# EUROPEAN PATENT SPECIFICATION

(11) **EP 3 747 472 B1**
(45) Date of publication and mention of the grant of the patent: **03.04.2024**
(21) Application number: 20171647.9
(22) Date of filing: 15.09.2016
(51) Int. Cl.: A61K 31/4985, A61K 31/519, A61K 39/395, C07K 16/28, C07K 14/70, A61P 35/02, A61K 45/06, A61K 31/454, A61P 35/00

(54) **THERAPEUTIC COMBINATIONS OF A CD19 INHIBITOR AND A BTK INHIBITOR**
THERAPEUTISCHE KOMBINATIONEN AUS EINEM CD19-INHIBITOR UND EINEM BTK-INHIBITOR
COMBINAISONS THÉRAPEUTIQUES D'UN INHIBITEUR CD19 ET D'UN INHIBITEUR BTK

(30) Priority: 15.09.2015 US 201562218958 P; 19.10.2015 US 201562243646 P; 11.01.2016 US 201662277474 P
(43) Date of publication of application: 09.12.2020
(62) Divisional of application: 16770565.6
(73) Proprietor: Acerta Pharma B.V., 5349 AB Oss (NL)
(72) Inventor: ROTHBAUM, Wayne, Delray Beach, Florida 33483 (US); LANNUTTI, Brian, Solana Beach, California 92075 (US)
(74) Representative: D Young & Co LLP

(56) References cited:
- WO-A1-2013/010868
- WO-A1-2014/153270
- WO-A2-2011/153514
- WO-A2-2013/059738
- WO-A2-2015/110923
- M RUELLA: "COMBINATION OF IBRUTINIB AND ANTI-CD19 CHIMERIC ANTIGEN RECEPTOR T CELLS FOR THE TREATMENT OF RELAPSING/REFRACTORY MANTLE CELL LYMPHOMA (MCL)", 20TH CONGRESS OF THE EUROPEAN HEMATOLOGY ASSOCIATION, JUN 11-14, 2015, VIENNA, AUSTRIA, 13 June 2015 (2015-06-13), page 1, XP055281361,
- WU JINGJING ET AL: "Blinatumomab: a bispecific T cell engager (BiTE) antibody against CD19/CD3 for refractory acute lymphoid leukemia.", JOURNAL OF HEMATOLOGY & ONCOLOGY, vol. 8, 104, 4 September 2015 (2015-09-04) , page 7PP, XP002764048, ISSN: 1756-8722, DOI: 10.1186/s13045-015-0195-4

## Description

### FIELD OF THE INVENTION

Therapeutic combinations of a Bruton's tyrosine kinase (BTK) inhibitor and a CD19 inhibitor and uses of the therapeutic combinations are disclosed herein. In particular, a combination of a BTK inhibitor and a CD19 inhibitor, such as a CD19-targeted antibody or a T cell or NK cell expressing a CD19-targeted chimeric antigen receptor, and compositions and uses thereof are disclosed.

### BACKGROUND OF THE INVENTION

Bruton's Tyrosine Kinase (BTK) is a Tec family non-receptor protein kinase expressed in B cells and myeloid cells. The function of BTK in signaling pathways activated by the engagement of the B cell receptor (BCR) and FCER1 on mast cells is well established. Functional mutations in BTK in humans result in a primary immunodeficiency disease characterized by a defect in B cell development with a block between pro- and pre-B cell stages. The result is an almost complete absence of B lymphocytes, causing a pronounced reduction of serum immunoglobulin of all classes. These findings support a key role for BTK in the regulation of the production of auto-antibodies in autoimmune diseases.

Other diseases with an important role for dysfunctional B cells are B cell malignancies. The reported role for BTK in the regulation of proliferation and apoptosis of B cells indicates the potential for BTK inhibitors in the treatment of B cell lymphomas. BTK inhibitors have thus been developed as potential therapies, as described in D'Cruz and Uckun, OncoTargets and Therapy 2013, 6, 161-176.

The CD19 (cluster of differentiation 19) antigen is found on B cells throughout their lifespan. Nadler, et al., J. Immunol. 1983, 131, 244-50. CD19 is expressed on cancerous cells, including B cell acute lymphoblastic leukemia ("B-ALL") cancer cells. Nadler, et al., J. Clin. Invest. 1984, 74, 332-40. CD19 is also found on follicular dendritic cells. CD19 is the most widely expressed cell surface antigen in B-cell malignancies. CD19 is thus a target for immunotherapeutic approaches in cancer and inflammatory diseases using anti-CD19 monoclonal antibodies. Tedder, NatRev. Rheumatol. 2009, 5, 572-77. Similarly, CD19 has also been a target for chimeric antigen receptor (CAR) modified T cells and natural killer (NK) cells. Kochenderfer and Rosenberg, Nat. Rev. Clin. Oncol. 2013, 10, 267-76. These anti-CD19 therapies have shown efficacy in various diseases, including B cell malignancies such as acute lymphoblastic leukemia and chronic lymphocytic leukemia.

In many solid tumors, the supportive microenvironment (which may make up the majority of the tumor mass) is a dynamic force that enables tumor survival. The tumor microenvironment is generally defined as a complex mixture of "cells, soluble factors, signaling molecules, extracellular matrices, and mechanical cues that promote neoplastic transformation, support tumor growth and invasion, protect the tumor from host immunity, foster therapeutic resistance, and provide niches for dominant metastases to thrive," as described in Swartz, et al., Cancer Res., 2012, 72, 2473. Although tumors express antigens that should be recognized by T cells, tumor clearance by the immune system is rare because of immune suppression by the microenvironment. Addressing the tumor cells themselves with e.g. chemotherapy has also proven to be insufficient to overcome the protective effects of the microenvironment. New approaches are thus urgently needed for more effective treatment of solid tumors that take into account the role of the microenvironment.

The CD20 antigen, also called human B-lymphocyte-restricted differentiation antigen Bp35, or B1), is found on the surface of normal "pre-B" and mature B lymphocytes, including malignant B lymphocytes. Nadler, et al., J. Clin. Invest. 1981, 67, 134-40; Stashenko, et al., J. Immunol. 1980, 139, 3260-85. The CD20 antigen is a glycosylated integral membrane protein with a molecular weight of approximately 35 kD. Tedder, et al., Proc. Natl. Acad. Sci. USA, 1988, 85, 208-12. CD20 is also expressed on most B cell non-Hodgkin's lymphoma cells, but is not found on hematopoietic stem cells, pro-B cells, normal plasma cells, or other normal tissues. Anti-CD20 antibodies are currently used as therapies for many B cell hematological malignancies, including indolent non-Hodgkin's lymphoma (NHL), aggressive NHL, and chronic lymphocytic leukemia (CLL)/small lymphocytic leukemia (SLL). Lim, et. al., Haematologica 2010, 95, 135-43; Beers, et. al., Sem. Hematol. 2010, 47, 107-14; Klein, et al., mAbs 2013, 5, 22-33. However, there is an urgent need to provide for more efficiacious therapies in many B cell hematological malignancies.

M. Ruella (20th Congress of the European Hematology Association, 13 June 2015, Vienna, Austria, p.1-2) discloses the use of a combination of the BTK inhibitor ibrutinib and CTL019 in treatment of mantle cell lymphoma (MCL).
WO 2015/110923 A2 discloses the use of a BTK inhibitor or the combination of a BTK inhibitor and an anti-CD20 antibody in the treatment of chronic lymphocytic leukemia (CLL) and small lymphocytic leukemia (SLL).

The present invention provides the unexpected finding that the combination of a CD19 inhibitor selected from an anti-CD19 antibody or an anti-CD19 chimeric antigen receptor, or an antigen-binding fragment or conjugate thereof, and a BTK inhibitor of Formula (II) is synergistically effective in the treatment of any of several types of cancers such as leukemia, lymphoma, and solid tumor cancers. The present invention further provides the unexpected finding that the combination of an anti-CD20 antibody with a BTK inhibitor of Formula (II) and a CD 19 inhibitor selected from an anti-CD 19 antibody or an anti-CD19 chimeric antigen receptor, or an antigen-binding fragment or conjugate thereof, is synergistically effective in the treatment of any of several types of cancers such as leukemia, lymphoma, and solid tumor cancers.

### SUMMARY OF THE INVENTION

The invention provides a pharmaceutical combination for use in the treatment of cancer, comprising therapeutically effective amounts of (1) a CD19 inhibitor selected from an anti-CD19 antibody or an anti-CD19 chimeric antigen receptor, or an antigen-binding fragment or conjugate thereof, and (2) a Bruton's tyrosine kinase (BTK) inhibitor, wherein the BTK inhibitor is the compound of Formula (II): 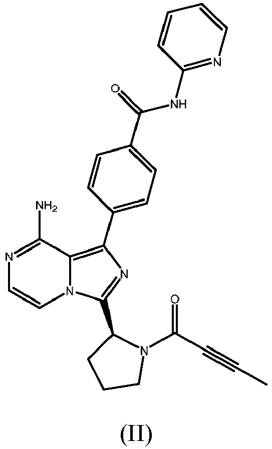 or a pharmaceutically acceptable salt thereof.

In an embodiment, the CD19 inhibitor is an anti-CD19 antibody. In an embodiment, the CD19 inhibitor is an anti-CD19 chimeric antigen receptor expressed in a T cell or a natural killer (NK) cell.

In an embodiment, the invention provides a pharmaceutical combination for use in treating cancer, comprising therapeutically effective amounts of (1) a CD19 inhibitor or an antigen-binding fragment or conjugate thereof, and (2) a BTK inhibitor of Formula (II) or a pharmaceutically acceptable salt thereof, wherein the CD19 inhibitor is selected from the group consisting of blinatumomab, coltuximab ravtansine, denintuzumab mafodotin, and an anti-CD19 chimeric antigen receptor, and antigen-binding fragments or conjugates thereof.

In an embodiment, the invention provides a pharmaceutical combination for use in treating cancer, comprising therapeutically effective amounts of (1) a CD19 inhibitor selected from an anti-CD19 antibody or an anti-CD19 chimeric antigen receptor, or an antigen-binding fragment or conjugate thereof, and (2) a BTK inhibitor of Formula (II) or a pharmaceutically acceptable salt thereof, further comprising a therapeutically effective amount of an anti-CD20 antibody selected from the group consisting of rituximab, obinutuzumab, ofatumumab, veltuzumab, tositumomab, ibritumomab, and fragments, derivatives, conjugates, variants, radioisotope-labeled complexes, biosimilars thereof, and combinations thereof.

In an embodiment, the invention provides a pharmaceutical combination for use in treating a cancer, comprising therapeutically effective amounts of (1) a CD19 inhibitor selected from an anti-CD19 antibody or an anti-CD19 chimeric antigen receptor, or an antigen-binding fragment or conjugate thereof, and (2) a Bruton's tyrosine kinase (BTK) inhibitor of Formula (II) or a pharmaceutically acceptable salt thereof, wherein the cancer is a B cell hematological malignancy, and wherein the B cell hematological malignancy is selected from the group consisting of chronic lymphocytic leukemia (CLL), small lymphocytic leukemia (SLL), non-Hodgkin's lymphoma (NHL), diffuse large B cell lymphoma (DLBCL), follicular lymphoma (FL), mantle cell lymphoma (MCL), Hodgkin's lymphoma, B cell acute lymphoblastic leukemia (B-ALL), Burkitt's lymphoma, Waldenström's macroglobulinemia (WM), Burkitt's lymphoma, multiple myeloma, and myelofibrosis. In an embodiment, the cancer is a solid tumor cancer, wherein the solid tumor cancer is selected from the group consisting of bladder cancer, non-small cell lung cancer, cervical cancer, anal cancer, pancreatic cancer, squamous cell carcinoma including head and neck cancer, renal cell carcinoma, melanoma, ovarian cancer, small cell lung cancer, glioblastoma, gastrointestinal stromal tumor, breast cancer, lung cancer, colorectal cancer, thyroid cancer, bone sarcoma, stomach cancer, oral cavity cancer, oropharyngeal cancer, gastric cancer, kidney cancer, liver cancer, prostate cancer, esophageal cancer, testicular cancer, gynecological cancer, colon cancer, and brain cancer. In an embodiment, the invention provides a pharmaceutical combination for use in treating cancer, comprising therapeutically effective amounts of (1) a CD19 inhibitor selected from an anti-CD19 antibody or an anti-CD19 chimeric antigen receptor, or an antigen-binding fragment or conjugate thereof, and (2) a Bruton's tyrosine kinase (BTK) inhibitor of Formula (II) or a pharmaceutically acceptable salt thereof, further comprising the step of administering a therapeutically effective amount of gemcitabine or albumin-bound paclitaxel. In an embodiment, the invention provides a pharmaceutical combination for use in treating a cancer in a human comprising (1) a therapeutically effective amount of a CD 19 inhibitor selected from an anti-CD19 antibody or an anti-CD19 chimeric antigen receptor, or an antigen-binding fragment or conjugater thereof, and (2) a therapeutically effective amount of a Bruton's tyrosine kinase (BTK) inhibitor of Formula (II) or a pharmaceutically acceptable salt thereof, wherein the therapeutically effective amount is effective to inhibit signaling between the tumor cells of the cancer and at least one tumor microenvironment selected from the group consisting of macrophages, monocytes, mast cells, helper T cells, cytotoxic T cells, regulatory T cells, natural killer cells, myeloid-derived suppressor cells, regulatory B cells, neutrophils, dendritic cells, and fibroblasts. In an embodiment, the cancer is a solid tumor cancer selected from the group consisting of bladder cancer, non-small cell lung cancer, cervical cancer, anal cancer, pancreatic cancer, squamous cell carcinoma including head and neck cancer, renal cell carcinoma, melanoma, ovarian cancer, small cell lung cancer, glioblastoma, gastrointestinal stromal tumor, breast cancer, lung cancer, colorectal cancer, thyroid cancer, bone sarcoma, stomach cancer, oral cavity cancer, oropharyngeal cancer, gastric cancer, kidney cancer, liver cancer, prostate cancer, esophageal cancer, testicular cancer, gynecological cancer, colon cancer, and brain cancer. In an embodiment, the CD19 inhibitor is selected from the group consisting of blinatumomab, coltuximab ravtansine, denintuzumab mafodotin, and an anti-CD19 chimeric antigen receptor, and antigen-binding fragments or conjugates thereof.

In some embodiments, the invention provides a composition comprising therapeutically effective amounts of (1) a CD19 inhibitor selected from an anti-CD19 antibody or an anti-CD19 chimeric antigen receptor, or an antigen-binding fragment or conjugate thereof; and (2) a BTK inhibitor of Formula (II) or a pharmaceutically acceptable salt thereof. This composition is typically a pharmaceutical composition. This composition is for use in the treatment of cancer.

In some embodiments, the invention provides a composition comprising therapeutically effective amounts of (1) a CD19 inhibitor selected from an anti-CD19 antibody or an anti-CD19 chimeric antigen receptor, or an antigen-binding fragment or conjugate thereof; (2) a BTK inhibitor of Formula (II) or a pharmaceutically acceptable salt thereof, for use in the treatment of cancer; and (3) a therapeutically effective amount of an anti-CD20 antibody selected from the group consisting of rituximab, obinutuzumab, ofatumumab, veltuzumab, tositumomab, ibritumomab, and fragments, derivatives, conjugates, variants, radioisotope-labeled complexes, and biosimilars thereof. This composition is typically a pharmaceutical composition. In an embodiment, this composition is for use in the treatment of cancer.

In some embodiments, the invention provides a pharmaceutical combination for use in treating leukemia, lymphoma or a solid tumor cancer comprising any of the foregoing compositions.

In some embodiments, the invention provides a pharmaceutical combination for use in treating leukemia, lymphoma or a solid tumor cancer comprising a therapeutically effective amount of a CD 19 inhibitor selected from an anti-CD19 antibody or an anti-CD19 chimeric antigen receptor, or an antigen-binding fragment or conjugate thereof and a BTK inhibitor of Formula (II).

In some embodiments, the invention provides a pharmaceutical combination for use in treating leukemia, lymphoma or a solid tumor cancer comprising a therapeutically effective amount of a CD 19 inhibitor selected from an anti-CD19 antibody or an anti-CD19 chimeric antigen receptor, or an antigen-binding fragment or conjugate thereof, a BTK inhibitor of Formula (II), and an anti-CD20 antibody.

In some embodiments, the invention provides a pharmaceutical combination for use in treating leukemia, lymphoma or a solid tumor cancer comprising a therapeutically effective amount of a CD 19 inhibitor selected from an anti-CD19 antibody or an anti-CD19 chimeric antigen receptor, or an antigen-binding fragment or conjugate thereof, a BTK inhibitor of Formula (II), and gemcitabine.

In some embodiments, the invention provides a pharmaceutical combination for use in treating leukemia, lymphoma or a solid tumor cancer comprising a therapeutically effective amount of a CD 19 inhibitor selected from an anti-CD19 antibody or an anti-CD19 chimeric antigen receptor, or an antigen-binding fragment or conjugate thereof, a BTK inhibitor of Formula (II), and albumin-bound paclitaxel.

In some embodiments, the invention provides a pharmaceutical combination for use in treating leukemia, lymphoma or a solid tumor cancer comprising a therapeutically effective amount of a CD 19 inhibitor selected from an anti-CD19 antibody or an anti-CD19 chimeric antigen receptor, or an antigen-binding fragment or conjugate thereof, a BTK inhibitor of Formula (II), and bendamustine.

In some embodiments, the invention provides a pharmaceutical combination for use in treating leukemia, lymphoma or a solid tumor cancer comprising a therapeutically effective amount of a CD 19 inhibitor selected from an anti-CD19 antibody or an anti-CD19 chimeric antigen receptor, or an antigen-binding fragment or conjugate thereof, a BTK inhibitor of Formula (II), and a combination of cyclophosphamide, doxorubicin, vincristine, and prednisone (CHOP).

In some embodiments, the invention provides a pharmaceutical combination for use in treating leukemia, lymphoma or a solid tumor cancer comprising a therapeutically effective amount of a CD 19 inhibitor selected from an anti-CD19 antibody or an anti-CD19 chimeric antigen receptor, or an antigen-binding fragment or conjugate thereof, a BTK inhibitor of Formula (II), and a combination of rituximab, cyclophosphamide, doxorubicin, vincristine, and prednisone (R-CHOP).

In some embodiments, the invention provides a pharmaceutical combination for use in treating leukemia, lymphoma or a solid tumor cancer comprising a therapeutically effective amount of a CD 19 inhibitor selected from an anti-CD19 antibody or an anti-CD19 chimeric antigen receptor, or an antigen-binding fragment or conjugate thereof, a BTK inhibitor of Formula (II), and a combination of fludarabine, cyclophosphamide, and rituximab (FCR).

In some embodiments, the invention provides a pharmaceutical combination for use in treating leukemia, lymphoma or a solid tumor cancer comprising a therapeutically effective amount of a CD 19 inhibitor selected from an anti-CD19 antibody or an anti-CD19 chimeric antigen receptor, or an antigen-binding fragment or conjugate thereof, a BTK inhibitor of Formula (II), and a combination of bendamustine and rituximab (BR).

### BRIEF DESCRIPTION OF THE DRAWINGS

The foregoing summary, as well as the following detailed description of the invention, will be better understood when read in conjunction with the appended drawings.
FIG. 1 illustrates *in vivo* potency of Formula (2) (labeled "BTK inhibitor") and Formula (10) (ibrutinib). Mice were gavaged at increasing drug concentration and sacrificed at one time point (3 hours post-dose). BCR is stimulated with IgM and the expression of activation markers CD69 and CD86 are monitored by flow cytometry to determine EC₅₀values. The results show that Formula (2) is more potent at inhibiting expression of activation makers than Formula (10) (ibrutinib).
FIG. 2 illustrates *in vitro* potency in whole blood of Formula (2), Formula (10) (ibrutinib) and Formula (17) (CC-292) in inhibition of signals through the B cell receptor.
FIG. 3 illustrates EGF receptor phosphorylation *in vitro* for Formula (2) and Formula (10) (ibrutinib).
FIG. 4 illustrates the effects of vehicle on flux at two timepoints in the ID8 syngeneic orthotropic ovarian cancer model.
FIG. 5 illustrates the effects of the BTK inhibitor of Formula (2) on flux at two timepoints, for comparison with FIG. 4, in the ID8 syngeneic orthotropic ovarian cancer model.
FIG. 6 illustrates tumor response to treatment with the BTK inhibitor of Formula (2) correlates with a significant reduction in immunosuppressive tumor associated lymphocytes in tumor-bearing mice, in comparison to a control (vehicle).
FIG. 7 illustrates the effects of treatment with the active pharmaceutical ingredient of Formula (2) on tumor volumes in the KPC pancreatic cancer model.
FIG. 8 illustrates the dosing schema used with the KrasLA2 non-small cell lung cancer (NSCLC) model.
FIG. 9 illustrates BTK inhibitory effects on MDSCs.

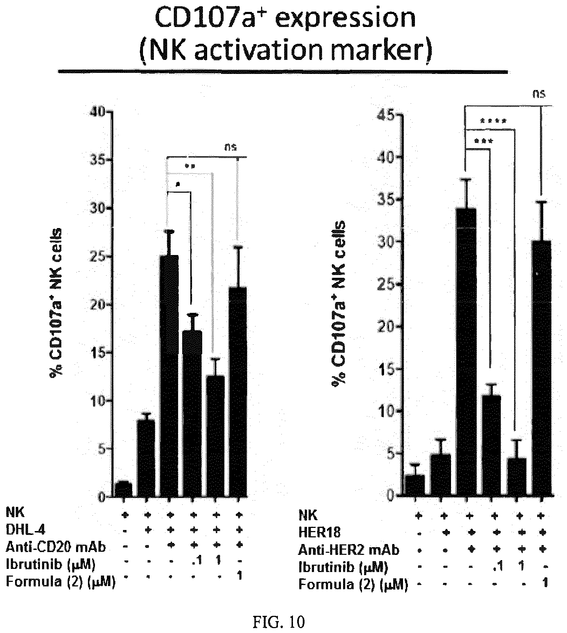

FIG. 10 shows *in vitro* analysis of antibody-dependent NK cell-mediated degranulation with BTK inhibitors. To evaluate NK cell function, purified NK cells were isolated from healthy peripheral blood mononuclear cells and cultured with 0.1 or 1 µM of Formula (10) (ibrutinib) or 1 µM of Formula (2) for 4 hours together with rituximab-coated (10 µg/mL) lymphoma cells, DHL4, or trastuzumab-coated (10 µg/mL) HER2⁺ breast cancer cells, HER18, and NK cells isolated and analyzed for degranulation by flow cytometry for CD107a⁺ mobilization. All *in vitro* experiments were performed in triplicate. Labels are defined as follows: *p = 0.01, **p = 0.002, ***p = 0.003, ****p = 0.0005.

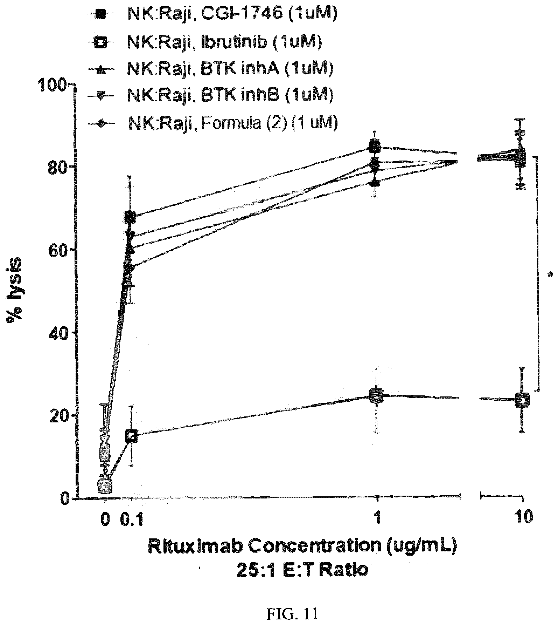

FIG. 11 shows that Formula (10) (ibrutinib) antagonizes antibody-dependent NK cell-mediated cytotoxicity using the Raji cell line. NK cell cytotoxicity as percent lysis of tumor cells was analyzed in chromium release assays with purified NK cells incubated with chromium-labeled Raji for 4 hours at variable rituximab concentrations at a constant effector:target ratio of 25:1 and Formula (10) (ibrutinib) (1 µM), Formula (2) (1 µM), or other interleukin-2 inducible tyrosine kinase (ITK) sparing BTK inhibitors CGI-1746, inhibA (1 µM) and BGB-3111 ("inhibB," 1 µM). All *in vitro* experiments were performed in triplicate. Labels are defined as follows: *p = 0.001.

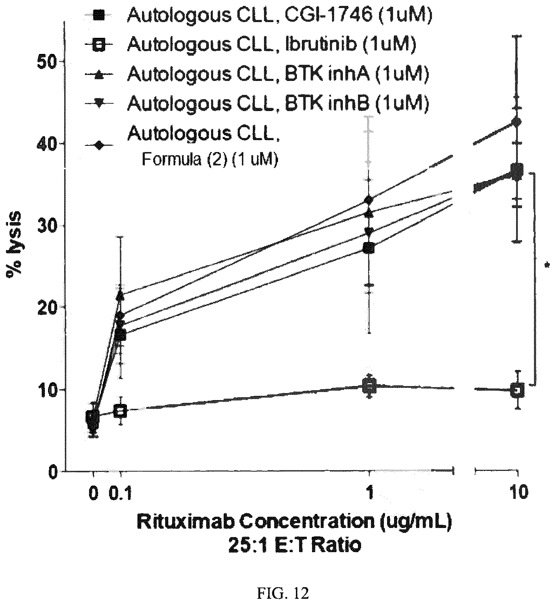

FIG. 12 shows that Formula (10) (ibrutinib) antagonizes antibody-dependent NK cell-mediated cytotoxicity in primary CLL cells. NK cell cytotoxicity as percent lysis of tumor cells was analyzed in chromium release assays with purified NK cells incubated with chromium-labeled Raji for 4 hours at variable rituximab concentrations at a constant effector:target ratio of 25:1 and Formula (10) (ibrutinib) (1 µM), Formula (2) (1 µM), or other ITK sparing BTK inhibitors CGI-1746, inhibA (1 µM) and BGB-3111 ("inhibB," 1 µM). All *in vitro* experiments were performed in triplicate. Labels are defined as follows: *p = 0.001.
FIG. 13 shows a summary of the results given in FIG. 12 at the highest concentration of rituximab ("Ab") (10 µg/mL).
FIG. 14 shows NK cell degranulation results for combinations of obinutuzumab with Formula (2) and Formula (10). The percentage of CD56⁺/CD107a⁺ NK cells observed in whole blood after pretreatment for 1 hour with the BTK inhibitors and stimulatation with MEC-1 cells opsonised with obinutuzumab at 1 µg/mL for 4 hours (n = 3) is shown.
FIG. 15 shows the effects of BTK inhibition on generalized NK cell mediated cytotoxicity.
FIG. 16 shows that Formula (2) has no adverse effect on T helper 17 (Th17) cells, which are a subset of T helper cells that produce interleukin 17 (IL-17), while Formula (10) (ibrutinib) strongly inhibits Th17 cells.
FIG. 17 shows that Formula (2) has no effect on regulatory T cell (Treg) development, while Formula (10) (ibrutinib) strongly increases Treg development.
FIG. 18 shows that Formula (2) has no effect on CD8⁺ T cell viability, development, while Formula (10) (ibrutinib) strongly affects CD8⁺ T cell viability at higher doses.
FIG. 19 illustrates the results of the cytotoxicity assay for CD8⁺ T cell function. Formula (10) (ibrutinib) affects CD8⁺ T cell function as measured by % cytotoxicity, while Formula (2) has no effect on CD8⁺ T cell function as measured by % cytotoxicity relative to vehicle.
FIG. 20 illustrates the results of IFN-γ level measurements for CD8⁺ T cell function. Formula (10) (ibrutinib) affects CD8⁺ T cell function as measured by IFN-γ level, while Formula (2) has no effect on CD8⁺ T cell function as measured by IFN-γ level relative to vehicle.

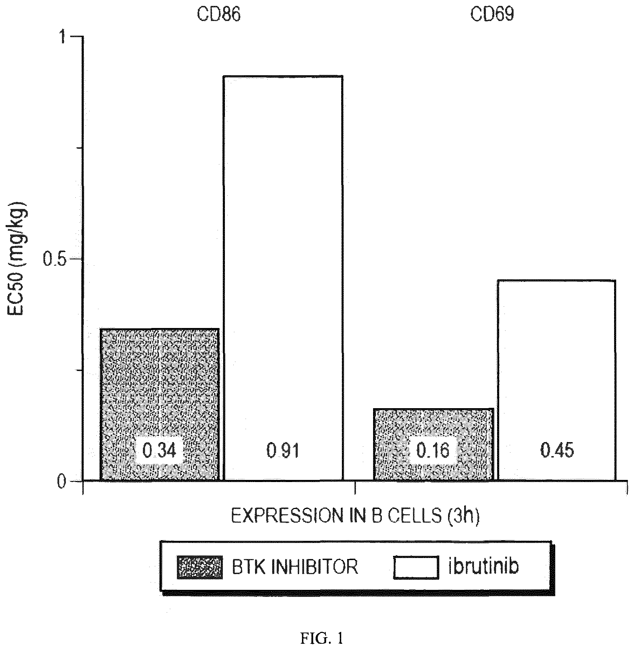

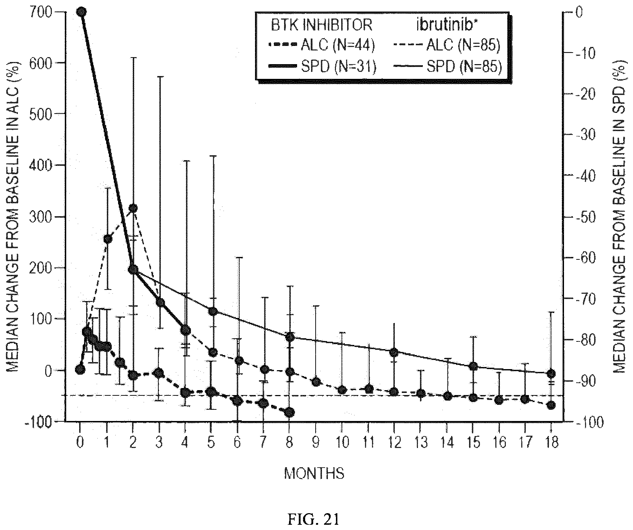

FIG. 21 illustrates the results of the clinical study of Formula (2) (labeled "BTK inhibitor") in CLL, which are shown in comparison to the results reported for Formula (10) (ibrutinib) in Figure 1A of Byrd, et al., N. Engl. J. Med. 2013, 369, 32-42. The results show that the BTK inhibitor of Formula (2) causes a much smaller relative increase and much faster decrease in absolute lymphocyte count (ALC) relative to the BTK inhibitor of Formula (10) (ibrutinib). The sum of the product of greatest diameters (SPD) also decreases more rapidly during treatment with the BTK inhibitor than with the BTK inhibitor of Formula (10) (ibrutinib).
FIG. 22 shows SPD of enlarged lymph nodes in CLL patients as a function of dose (cohort) of the BTK inhibitor of Formula (2).
FIG. 23 shows a comparison of progression-free survival (PFS) in CLL patients treated with the BTK inhibitor of Formula (10) (ibrutinib) or the BTK inhibitor of Formula (2). The ibrutinib data is taken from Byrd, etal., N. Engl. J. Med. 2013, 369, 32-42. CLL patients treated with Formula (2) for at least 8 days are included.
FIG. 24 shows a comparison of number of patients at risk in CLL patients treated with the BTK inhibitor of Formula (10) (ibrutinib) or the BTK inhibitor of Formula (2). CLL patients treated with Formula (2) for at least 8 days are included.
FIG. 25 shows a comparison of progression-free survival (PFS) in CLL patients exhibiting the 17p deletion and treated with the BTK inhibitor of Formula (10) (ibrutinib) or the BTK inhibitor of Formula (2). The ibrutinib data is taken from Byrd, et al., N. Engl. J. Med. 2013, 369, 32-42.
FIG. 26 shows a comparison of number of patients at risk in CLL patients exhibiting the 17p deletion and treated with the BTK inhibitor of Formula (10) (ibrutinib) or the BTK inhibitor of Formula (2). The ibrutinib data is taken from Byrd, et al., N. Engl. J. Med. 2013, 369, 32-42. CLL patients treated with Formula (2) for at least 8 days are included.
FIG. 27 shows improved BTK target occupancy of Formula (2) at lower dosage versus Formula (10) (ibrutinib) in relapsed/refractory CLL patients.
FIG. 28 shows the % change in myeloid-derived suppressor cell (MDSC) (monocytic) level over 28 days versus % ALC change at Cycle 1, day 28 (C1D28) with trendlines.
FIG. 29 shows the % change in MDSC (monocytic) level over 28 days versus % ALC change at Cycle 2, day 28 (C2D28) with trendlines.
FIG. 30 shows the % change in natural killer (NK) cell level over 28 days versus % ALC change at Cycle 1, day 28 (C2D28) with trendlines.
FIG. 31 shows the % change in NK cell level over 28 days versus % ALC change at Cycle 2, day 28 (C2D28) with trendlines.
FIG. 32 compares the % change in MDSC (monocytic) level and % change in NK cell level over 28 days versus % ALC change with the % change in level of CD4⁺ T cells, CD8⁺ T cells, CD4⁺/CD8⁺ T cell ratio, NK-T cells, PD-1⁺ CD4⁺ T cells, and PD-1⁺ CD8⁺ T cells, also versus % ALC change, at Cycle 1 day 28 (C1D28). Trendlines are shown for % change in MDSC (monocytic) level and % change in NK cell level.
FIG. 33 compares the % change in MDSC (monocytic) level and % change in NK cell level over 28 days versus % ALC change with the % change in level of CD4⁺ T cells, CD8⁺ T cells, CD4⁺/CD8⁺ T cell ratio, NK-T cells, PD-1⁺CD4⁺ T cells, and PD-1⁺ CD8⁺ T cells, also versus % ALC change, at Cycle 2 day 28 (C2D28). Trendlines are shown for % change in MDSC (monocytic) level and % change in NK cell level.

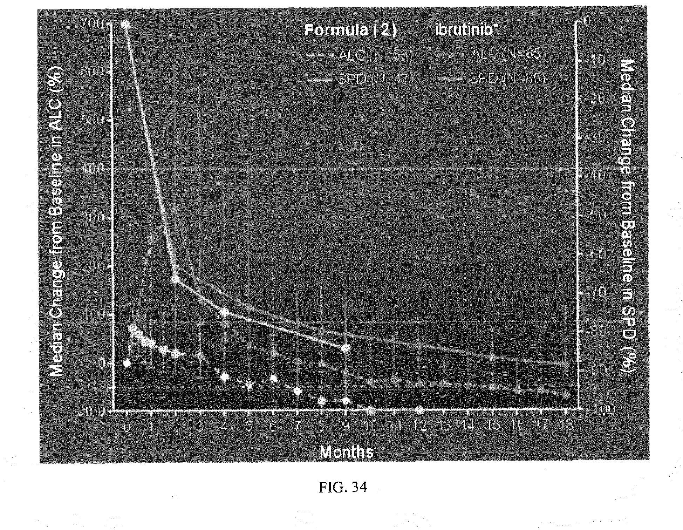

FIG. 34 shows updated the results of the clinical study of Formula (2) (labeled "BTK inhibitor") in CLL, which are shown in comparison to the results reported for ibrutinib in Figure 1A of Byrd, et al., N. Engl. J. Med. 2013, 369, 32-42. The results show that the BTK inhibitor of Formula (2) causes a much smaller relative increase and much faster decrease in absolute lymphocyte count (ALC) relative to the BTK inhibitor of Formula (10) (ibrutinib). The sum of the product of greatest diameters (SPD) also decreases more rapidly during treatment with the BTK inhibitor than with the BTK inhibitor of Formula (10) (ibrutinib).
FIG. 35 shows improved BTK target occupancy of Formula (2) at lower dosage versus ibrutinib in relapsed/refractory CLL patients, and includes BID dosing results.
FIG. 36 illustrates PFS for patients with 17p deletion.
FIG. 37 illustrates PFS across relapsed/refractory patients with 17p deletion and with 11q deletion and no 17p deletion.
FIG. 38 illustrates PFS for patients with 11q deletion and no 17p deletion.
FIG. 39 illustrates updated SPD results from the clinical study of Formula (2) in relapsed/refractory CLL patients.
FIG. 40 illustrates that treatment of CLL patients with Formula (2) resulted in increased apoptosis.
FIG. 41 illustrates a decrease in CXCL12 levels observed in patients treated with Formula (2).
FIG. 42 illustrates representative photomicrographs and comparison of maximal thrombus size in laser injured arterioles of VWF HA1 mutant mice infused with human platelets in the absence or presence of various BTK inhibitors. Representative photomicrographs are given as a comparison of maximal thrombus size in laser-injured arterioles (1 µM concentrations shown).
FIG. 43 illustrates a quantitative comparison obtained by *in vivo* analysis of early thrombus dynamics in a humanized mouse laser injury model using three BTK inhibitors at a concentration 1 µM.
FIG. 44 illustrates the results of GPVI platelet aggregation studies of Formula (2) (IC50 = 1.15 µM) and Formula (10) (ibrutinib, IC50 = 0.13 µM).
FIG. 45 illustrates the results of GPVI platelet aggregation studies of Formula (2) and Formula (10) (ibrutinib).

### BRIEF DESCRIPTION OF THE SEQUENCE LISTING

SEQ ID NO:1 is the amino acid sequence of the anti-CD19 monoclonal antibody blinatumomab.
SEQ ID NO:2 is the variable heavy chain CDR1 amino acid sequence of the anti-CD19 monoclonal antibody blinatumomab (corresponding to SEQ ID NO:52 in U.S. Patent No. 7,635,472).
SEQ ID NO:3 is the variable heavy chain CDR2 amino acid sequence of the anti-CD19 monoclonal antibody blinatumomab (corresponding to SEQ ID NO:53 in U.S. Patent No. 7,635,472).
SEQ ID NO:4 is the variable heavy chain CDR3 amino acid sequence of the anti-CD19 monoclonal antibody blinatumomab (corresponding to SEQ ID NO:54 in U.S. Patent No. 7,635,472).
SEQ ID NO:5 is the variable light chain CDR1 amino acid sequence of the anti-CD19 monoclonal antibody blinatumomab (corresponding to SEQ ID NO:55 in U.S. Patent No. 7,635,472).
SEQ ID NO:6 is the variable light chain CDR2 amino acid sequence of the anti-CD19 monoclonal antibody blinatumomab (corresponding to SEQ ID NO:56 in U.S. Patent No. 7,635,472).
SEQ ID NO:7 is the variable light chain CDR3 amino acid sequence of the anti-CD19 monoclonal antibody blinatumomab (corresponding to SEQ ID NO:57 in U.S. Patent No. 7,635,472).
SEQ ID NO:8 is the heavy chain amino acid sequence of the anti-CD19 monoclonal antibody coltuximab (corresponding to SEQ ID NO:8 in U.S. Patent Application Publication No. US 2014/0199300 A1).
SEQ ID NO:9 is the light chain amino acid sequence of the anti-CD19 monoclonal antibody coltuximab (corresponding to SEQ ID NO:7 in U.S. Patent Application Publication No. US 2014/0199300 A1).
SEQ ID NO:10 is the variable heavy chain CDR1 amino acid sequence of the anti-CD19 monoclonal antibody coltuximab (corresponding to SEQ ID NO:4 in U.S. Patent Application Publication No. US 2014/0199300 A1).
SEQ ID NO: 11 is the variable heavy chain CDR2 amino acid sequence of the anti-CD19 monoclonal antibody coltuximab (corresponding to SEQ ID NO:5 in U.S. Patent Application Publication No. US 2014/0199300 A1).
SEQ ID NO:12 is the variable heavy chain CDR3 amino acid sequence of the anti-CD19 monoclonal antibody coltuximab (corresponding to SEQ ID NO:6 in U.S. Patent Application Publication No. US 2014/0199300 A1).
SEQ ID NO:13 is the variable light chain CDR1 amino acid sequence of the anti-CD19 monoclonal antibody coltuximab (corresponding to SEQ ID NO:1 in U.S. Patent Application Publication No. US 2014/0199300 A1).
SEQ ID NO:14 is the variable light chain CDR2 amino acid sequence of the anti-CD19 monoclonal antibody coltuximab (corresponding to SEQ ID NO:2 in U.S. Patent Application Publication No. US 2014/0199300 A1).
SEQ ID NO:15 is the variable light chain CDR3 amino acid sequence of the anti-CD19 monoclonal antibody coltuximab (corresponding to SEQ ID NO:3 in U.S. Patent Application Publication No. US 2014/0199300 A1).
SEQ ID NO:16 is the heavy chain amino acid sequence of the anti-CD19 monoclonal antibody denintuzumab.
SEQ ID NO:17 is the light chain amino acid sequence of the anti-CD19 monoclonal antibody denintuzumab.
SEQ ID NO:18 is the variable heavy chain CDR1 amino acid sequence of the anti-CD19 monoclonal antibody denintuzumab.
SEQ ID NO:19 is the variable heavy chain CDR2 amino acid sequence of the anti-CD19 monoclonal antibody denintuzumab.
SEQ ID NO:20 is the variable heavy chain CDR3 amino acid sequence of the anti-CD19 monoclonal antibody denintuzumab.
SEQ ID NO:21 is the variable light chain CDR1 amino acid sequence of the anti-CD19 monoclonal antibody denintuzumab.
SEQ ID NO:22 is the variable light chain CDR2 amino acid sequence of the anti-CD19 monoclonal antibody denintuzumab.
SEQ ID NO:23 is the variable light chain CDR3 amino acid sequence of the anti-CD19 monoclonal antibody denintuzumab.
SEQ ID NO:24 is the amino acid sequence of the anti-CD19 FMC63 scFv variable heavy chain (V_{H}) domain.
SEQ ID NO:25 is the amino acid sequence of the anti-CD19 FMC63 scFv variable heavy chain (V_{L}) domain.
SEQ ID NO:26 is the amino acid sequence of the anti-CD19 SJ25C1 scFv variable heavy chain (V_{H}) domain.
SEQ ID NO:27 is the amino acid sequence of the anti-CD19 SJ25C1 scFv variable heavy chain (V_{L}) domain.
SEQ ID NO:28 is the amino acid sequence of a humanized anti-CD19 scFv domain.
SEQ ID NO:29 is the amino acid sequence of a humanized anti-CD19 scFv domain.
SEQ ID NO:30 is the amino acid sequence of a humanized anti-CD19 scFv domain.
SEQ ID NO:31 is the amino acid sequence of a humanized anti-CD19 scFv domain.
SEQ ID NO:32 is the amino acid sequence of a humanized anti-CD19 scFv domain.
SEQ ID NO:33 is the amino acid sequence of a humanized anti-CD19 scFv domain.
SEQ ID NO:34 is the amino acid sequence of a humanized anti-CD19 scFv domain.
SEQ ID NO:35 is the amino acid sequence of a humanized anti-CD19 scFv domain.
SEQ ID NO:36 is the amino acid sequence of a humanized anti-CD19 scFv domain.
SEQ ID NO:37 is the amino acid sequence of a humanized anti-CD19 scFv domain.
SEQ ID NO:38 is the amino acid sequence of a humanized anti-CD19 scFv domain.
SEQ ID NO:39 is the amino acid sequence of a humanized anti-CD19 scFv domain.
SEQ ID NO:40 is the amino acid sequence of a CD3ζ signaling domain.
SEQ ID NO:41 is the amino acid sequence of a CD28 signaling domain.
SEQ ID NO:42 is the amino acid sequence of a 4-1BB signaling domain.
SEQ ID NO:43 is the amino acid sequence of a CD8α transmembrane domain.
SEQ ID NO:44 is the amino acid sequence of a CD8α hinge domain.
SEQ ID NO:45 is the amino acid sequence of a murine anti-CD19 chimeric antigen receptor with the format anti-CD19(scFv):CD8α(transmembrane/hinge):4-1BB(signaling):CD3ζ(signaling).
SEQ ID NO:46 is the amino acid sequence of an anti-CD19 chimeric antigen receptor with the format anti-CD19(scFv):IgG1 Fc(transmembrane):CD3ζ(signaling). The chimeric antigen receptor comprises amino acids 23-634 of SEQ ID NO:46.
SEQ ID NO:47 is the amino acid sequence of an anti-CD19 chimeric antigen receptor with the format anti-CD19(scFv):IgG4(hinge):CD28(transmembrane):4-1BB(signaling): CD3ζ(signaling):T2A: EGFRt.
SEQ ID NO:48 is the amino acid sequence of a humanized anti-CD19 chimeric antigen receptor with the format anti-CD19(scFv):CD8α(transmembrane/hinge):4-1BB(signaling):CD3ζ(signalmg).
SEQ ID NO:49 is the amino acid sequence of a humanized anti-CD19 chimeric antigen receptor with the format anti-CD19(scFv):CD8α(transmembrane/hinge):4-1BB(signaling):CD3ζ(signaling).
SEQ ID NO:50 is the amino acid sequence of a humanized anti-CD19 chimeric antigen receptor with the format anti-CD19(scFv):CD8α(transmembrane/hinge):4-1BB(signaling):CD3ζ(signaling).
SEQ ID NO:51 is the amino acid sequence of a humanized anti-CD19 chimeric antigen receptor with the format anti-CD19(scFv):CD8α(transmembrane/hinge):4-1BB(signaling): CD3ζ(signaling).
SEQ ID NO:52 is the amino acid sequence of a humanized anti-CD19 chimeric antigen receptor with the format anti-CD19(scFv):CD8α(transmembrane/hinge):4-1BB(signaling): CD3ζ(signaling).
SEQ ID NO:53 is the amino acid sequence of a humanized anti-CD19 chimeric antigen receptor with the format anti-CD19(scFv):CD8α(transmembrane/hinge):4-1BB(signaling): CD3ζ(signaling).
SEQ ID NO:54 is the amino acid sequence of a humanized anti-CD19 chimeric antigen receptor with the format anti-CD19(scFv):CD8α(transmembrane/hinge):4-1BB(signaling):CD3ζ(signaling).
SEQ ID NO:55 is the amino acid sequence of a humanized anti-CD19 chimeric antigen receptor with the format anti-CD19(scFv):CD8α(transmembrane/hinge):4-1BB(signaling):CD3ζ(signaling).
SEQ ID NO:56 is the amino acid sequence of a humanized anti-CD19 chimeric antigen receptor with the format anti-CD19(scFv):CD8α(transmembrane/hinge):4-1BB(signaling):CD3ζ(signaling).
SEQ ID NO:57 is the amino acid sequence of a humanized anti-CD19 chimeric antigen receptor with the format anti-CD19(scFv):CD8α(transmembrane/hinge):4-1BB(signaling):CD3ζ(signaling).
SEQ ID NO:58 is the amino acid sequence of a humanized anti-CD19 chimeric antigen receptor with the format anti-CD19(scFv):CD8α(transmembrane/hinge):4-1BB(signaling): CD3ζ(signaling).
SEQ ID NO:59 is the amino acid sequence of a humanized anti-CD19 chimeric antigen receptor with the format anti-CD19(scFv):CD8α(transmembrane/hinge):4-1BB(signaling): CD3ζ(signaling).
SEQ ID NO:60 is the heavy chain amino acid sequence of the anti-CD20 monoclonal antibody rituximab.
SEQ ID NO:61 is the light chain amino acid sequence of the anti-CD20 monoclonal antibody rituximab.
SEQ ID NO:62 is the heavy chain amino acid sequence of the anti-CD20 monoclonal antibody obinutuzumab.
SEQ ID NO:63 is the light chain amino acid sequence of the anti-CD20 monoclonal antibody obinutuzumab.
SEQ ID NO:64 is the variable heavy chain amino acid sequence of the anti-CD20 monoclonal antibody ofatumumab.
SEQ ID NO:65 is the variable light chain amino acid sequence of the anti-CD20 monoclonal antibody ofatumumab.
SEQ ID NO:66 is the Fab fragment heavy chain amino acid sequence of the anti-CD20 monoclonal antibody ofatumumab.
SEQ ID NO:67 is the Fab fragment light chain amino acid sequence of the anti-CD20 monoclonal antibody ofatumumab.
SEQ ID NO:68 is the heavy chain amino acid sequence of the anti-CD20 monoclonal antibody veltuzumab.
SEQ ID NO:69 is the light chain amino acid sequence of the anti-CD20 monoclonal antibody veltuzumab.
SEQ ID NO:70 is the heavy chain amino acid sequence of the anti-CD20 monoclonal antibody tositumomab.
SEQ ID NO:71 is the light chain amino acid sequence of the anti-CD20 monoclonal antibody tositumomab.
SEQ ID NO:72 is the heavy chain amino acid sequence of the anti-CD20 monoclonal antibody ibritumomab.
SEQ ID NO:73 is the light chain amino acid sequence of the anti-CD20 monoclonal antibody ibritumomab.
SEQ ID NO:74 is the heavy chain amino acid sequence of the PD-1 inhibitor nivolumab.
SEQ ID NO:75 is the light chain amino acid sequence of the PD-1 inhibitor nivolumab.
SEQ ID NO:76 is the heavy chain variable region (V_{H}) amino acid sequence of the PD-1 inhibitor nivolumab.
SEQ ID NO:77 is the light chain variable region (V_{L}) amino acid sequence of the PD-1 inhibitor nivolumab.
SEQ ID NO:78 is the heavy chain CDR1 amino acid sequence of the PD-1 inhibitor nivolumab.
SEQ ID NO:79 is the heavy chain CDR2 amino acid sequence of the PD-1 inhibitor nivolumab.
SEQ ID NO:80 is the heavy chain CDR3 amino acid sequence of the PD-1 inhibitor nivolumab.
SEQ ID NO:81 is the light chain CDR1 amino acid sequence of the PD-1 inhibitor nivolumab.
SEQ ID NO:82 is the light chain CDR2 amino acid sequence of the PD-1 inhibitor nivolumab.
SEQ ID NO:83 is the light chain CDR3 amino acid sequence of the PD-1 inhibitor nivolumab.
SEQ ID NO:84 is the heavy chain amino acid sequence of the PD-1 inhibitor pembrolizumab.
SEQ ID NO:85 is the light chain amino acid sequence of the PD-1 inhibitor pembrolizumab.
SEQ ID NO: 86 is the heavy chain variable region (V_{H}) amino acid sequence of the PD-1 inhibitor pembrolizumab.
SEQ ID NO:87 is the light chain variable region (V_{L}) amino acid sequence of the PD-1 inhibitor pembrolizumab.
SEQ ID NO:88 is the heavy chain CDR1 amino acid sequence of the PD-1 inhibitor pembrolizumab.
SEQ ID NO: 89 is the heavy chain CDR2 amino acid sequence of the PD-1 inhibitor pembrolizumab.
SEQ ID NO:90 is the heavy chain CDR3amino acid sequence of the PD-1 inhibitor pembrolizumab.
SEQ ID NO:91 is the light chain CDR1 amino acid sequence of the PD-1 inhibitor pembrolizumab.
SEQ ID NO:92 is the light chain CDR2 amino acid sequence of the PD-1 inhibitor pembrolizumab.
SEQ ID NO:93 is the light chain CDR3 amino acid sequence of the PD-1 inhibitor pembrolizumab.
SEQ ID NO:94 is the heavy chain amino acid sequence of the PD-1 inhibitor pidilizumab.
SEQ ID NO:95 is the light chain amino acid sequence of the PD-1 inhibitor pidilizumab.
SEQ ID NO:96 is the heavy chain variable region (V_{H}) amino acid sequence of the PD-1 inhibitor pidilizumab.
SEQ ID NO:97 is the light chain variable region (V_{L}) amino acid sequence of the PD-1 inhibitor pidilizumab.
SEQ ID NO:98 is the heavy chain amino acid sequence of the PD-L1 inhibitor durvalumab.
SEQ ID NO:99 is the light chain amino acid sequence of the PD-L1 inhibitor durvalumab.
SEQ ID NO: 100 is the heavy chain variable region (V_{H}) amino acid sequence of the PD-L1 inhibitor durvalumab.
SEQ ID NO: 101 is the light chain variable region (V_{L}) amino acid sequence of the PD-L1 inhibitor durvalumab.
SEQ ID NO: 102 is the heavy chain CDR1 amino acid sequence of the PD-L1 inhibitor durvalumab.
SEQ ID NO: 103 is the heavy chain CDR2 amino acid sequence of the PD-L1 inhibitor durvalumab.
SEQ ID NO: 104 is the heavy chain CDR3 amino acid sequence of the PD-L1 inhibitor durvalumab.
SEQ ID NO:105 is the light chain CDR1 amino acid sequence of the PD-L1 inhibitor durvalumab.
SEQ ID NO: 106 is the light chain CDR2 amino acid sequence of the PD-L1 inhibitor durvalumab.
SEQ ID NO:107 is the light chain CDR3 amino acid sequence of the PD-L1 inhibitor durvalumab.
SEQ ID NO:108 is the heavy chain amino acid sequence of the PD-L1 inhibitor atezolizumab.
SEQ ID NO: 109 is the light chain amino acid sequence of the PD-L1 inhibitor atezolizumab.
SEQ ID NO:110 is the heavy chain variable region (V_{H}) amino acid sequence of the PD-L1 inhibitor atezolizumab.
SEQ ID NO: 111 is the light chain variable region (V_{L}) amino acid sequence of the PD-L1 inhibitor atezolizumab.
SEQ ID NO: 112 is the heavy chain CDR1 amino acid sequence of the PD-L1 inhibitor atezolizumab.
SEQ ID NO: 113 is the heavy chain CDR2 amino acid sequence of the PD-L1 inhibitor atezolizumab.
SEQ ID NO: 114 is the heavy chain CDR3 amino acid sequence of the PD-L1 inhibitor atezolizumab.
SEQ ID NO:115 is the light chain CDR1 amino acid sequence of the PD-L1 inhibitor atezolizumab.
SEQ ID NO: 116 is the light chain CDR2 amino acid sequence of the PD-L1 inhibitor atezolizumab.
SEQ ID NO:117 is the light chain CDR3 amino acid sequence of the PD-L1 inhibitor atezolizumab.
SEQ ID NO:118 is the heavy chain amino acid sequence of the PD-L1 inhibitor avelumab.
SEQ ID NO: 119 is the light chain amino acid sequence of the PD-L1 inhibitor avelumab.
SEQ ID NO:120 is the heavy chain variable region (V_{H}) amino acid sequence of the PD-L1 inhibitor avelumab.
SEQ ID NO: 121 is the heavy chain variable region (V_{H}) amino acid sequence of the PD-L1 inhibitor avelumab.
SEQ ID NO: 122 is the heavy chain CDR1 amino acid sequence of the PD-L1 inhibitor avelumab.
SEQ ID NO: 123 is the heavy chain CDR2 amino acid sequence of the PD-L1 inhibitor avelumab.
SEQ ID NO: 124 is the heavy chain CDR3 amino acid sequence of the PD-L1 inhibitor avelumab.
SEQ ID NO: 125 is the light chain CDR1 amino acid sequence of the PD-L1 inhibitor avelumab.
SEQ ID NO: 126 is the light chain CDR2 amino acid sequence of the PD-L1 inhibitor avelumab.
SEQ ID NO: 127 is the light chain CDR3 amino acid sequence of the PD-L1 inhibitor avelumab.

### DETAILED DESCRIPTION OF THE INVENTION

Unless defined otherwise, all technical and scientific terms used herein have the same meaning as is commonly understood by one of skill in the art to which this invention belongs.

### Definitions

The terms "co-administration," "co-administering," "administered in combination with," "administering in combination with," "simultaneous," and "concurrent," as used herein, encompass administration of two or more active pharmaceutical ingredients (in a preferred embodiment of the present invention, for example, at least one CD19 inhibitor and at least one BTK inhibitor) to a subject so that both active pharmaceutical ingredients and/or their metabolites are present in the subject at the same time. Co-administration includes simultaneous administration in separate compositions, administration at different times in separate compositions, or administration in a composition in which two or more active pharmaceutical ingredients are present. Simultaneous administration in separate compositions and administration in a composition in which both agents are present are preferred.

The term *"in vivo"* refers to an event that takes place in a subject's body.

The term *"in vitro"* refers to an event that takes places outside of a subject's body. *In vitro* assays encompass cell-based assays in which cells alive or dead are employed and may also encompass a cell-free assay in which no intact cells are employed.

The term "effective amount" or "therapeutically effective amount" refers to that amount of a compound or combination of compounds as described herein that is sufficient to effect the intended application including disease treatment. A therapeutically effective amount may vary depending upon the intended application (*in vitro* or *in vivo*), or the subject and disease condition being treated (*e.g.,* the weight, age and gender of the subject), the severity of the disease condition, the manner of administration, which can readily be determined by one of ordinary skill in the art. The term also applies to a dose that will induce a particular response in target cells (*e.g.,* the reduction of platelet adhesion and/or cell migration). The specific dose will vary depending on the particular compounds chosen, the dosing regimen to be followed, whether the compound is administered in combination with other compounds, timing of administration, the tissue to which it is administered, and the physical delivery system in which the compound is carried.

A "therapeutic effect" as that term is used herein, encompasses a therapeutic benefit and/or a prophylactic benefit. A prophylactic effect includes delaying or eliminating the appearance of a disease or condition, delaying or eliminating the onset of symptoms of a disease or condition, slowing, halting, or reversing the progression of a disease or condition, or any combination thereof.

The terms "QD," "qd," or "q.d." mean *quaque die,* once a day, or once daily. The terms "BID," "bid," or "b.i.d." mean *bis in die,* twice a day, or twice daily. The terms "TID," "tid," or "t.i.d." mean *ter in die,* three times a day, or three times daily. The terms "QID," "qid," or "q.i.d." mean *quater in die,* four times a day, or four times daily.

The term "pharmaceutically acceptable salt" refers to salts derived from a variety of organic and inorganic counter ions known in the art. Pharmaceutically acceptable acid addition salts can be formed with inorganic acids and organic acids. Preferred inorganic acids from which salts can be derived include, for example, hydrochloric acid, hydrobromic acid, sulfuric acid, nitric acid and phosphoric acid. Preferred organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid and salicylic acid. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese and aluminum. Organic bases from which salts can be derived include, for example, primary, secondary, and tertiary amines, substituted amines including naturally occurring substituted amines, cyclic amines and basic ion exchange resins. Specific examples include isopropylamine, trimethylamine, diethylamine, triethylamine, tripropylamine, and ethanolamine. In some embodiments, the pharmaceutically acceptable base addition salt is chosen from ammonium, potassium, sodium, calcium, and magnesium salts. The term "cocrystal" refers to a molecular complex derived from a number of cocrystal formers known in the art. Unlike a salt, a cocrystal typically does not involve hydrogen transfer between the cocrystal and the drug, and instead involves intermolecular interactions, such as hydrogen bonding, aromatic ring stacking, or dispersive forces, between the cocrystal former and the drug in the crystal structure.

"Pharmaceutically acceptable carrier" or "pharmaceutically acceptable excipient" is intended to include any and all solvents, dispersion media, coatings, antibacterial and antifungal agents, isotonic and absorption delaying agents, and inert ingredients. The use of such pharmaceutically acceptable carriers or pharmaceutically acceptable excipients for active pharmaceutical ingredients is well known in the art. Except insofar as any conventional pharmaceutically acceptable carrier or pharmaceutically acceptable excipient is incompatible with the active pharmaceutical ingredient, its use in the therapeutic compositions of the invention is contemplated. Additional active pharmaceutical ingredients, such as other drugs, can also be incorporated into the described compositions and methods.

"Prodrug" is intended to describe a compound that may be converted under physiological conditions or by solvolysis to a biologically active compound described herein. Thus, the term "prodrug" refers to a precursor of a biologically active compound that is pharmaceutically acceptable. A prodrug may be inactive when administered to a subject, but is converted *in vivo* to an active compound, for example, by hydrolysis. The prodrug compound often offers the advantages of solubility, tissue compatibility or delayed release in a mammalian organism (see, *e.g.,* Bundgaard, H., Design of Prodrugs (1985) (Elsevier, Amsterdam). The term "prodrug" is also intended to include any covalently bonded carriers, which release the active compound *in vivo* when administered to a subject. Prodrugs of an active compound, as described herein, may be prepared by modifying functional groups present in the active compound in such a way that the modifications are cleaved, either in routine manipulation or *in vivo,* to yield the active parent compound. Prodrugs include, for example, compounds wherein a hydroxy, amino or mercapto group is bonded to any group that, when the prodrug of the active compound is administered to a mammalian subject, cleaves to form a free hydroxy, free amino or free mercapto group, respectively. Examples of prodrugs include acetates, formates and benzoate derivatives of an alcohol, various ester derivatives of a carboxylic acid, or acetamide, formamide and benzamide derivatives of an amine functional group in the active compound.

Unless otherwise stated, the chemical structures depicted herein are intended to include compounds which differ only in the presence of one or more isotopically enriched atoms. For example, compounds where one or more hydrogen atoms is replaced by deuterium or tritium, or wherein one or more carbon atoms is replaced by ¹³C- or ¹⁴C-enriched carbons, are within the scope of this invention.

When ranges are used herein to describe, for example, physical or chemical properties such as molecular weight or chemical formulae, all combinations and subcombinations of ranges and specific embodiments therein are intended to be included. Use of the term "about" when referring to a number or a numerical range means that the number or numerical range referred to is an approximation within experimental variability (or within statistical experimental error), and thus the number or numerical range may vary. The variation is typically from 0% to 15%, preferably from 0% to 10%, more preferably from 0% to 5% of the stated number or numerical range. The term "comprising" (and related terms such as "comprise" or "comprises" or "having" or "including") includes those embodiments such as, for example, an embodiment of any composition of matter, method or process that "consist of" or "consist essentially of" the described features.

"Isomers" are different compounds that have the same molecular formula. "Stereoisomers" are isomers that differ only in the way the atoms are arranged in space - *i.e.,* having a different stereochemical configuration. "Enantiomers" are a pair of stereoisomers that are non-superimposable mirror images of each other. A 1:1 mixture of a pair of enantiomers is a "racemic" mixture. The term "(±)" is used to designate a racemic mixture where appropriate. "Diastereoisomers" are stereoisomers that have at least two asymmetric atoms, but which are not mirror-images of each other. The absolute stereochemistry is specified according to the Cahn-Ingold-Prelog R-S system. When a compound is a pure enantiomer the stereochemistry at each chiral carbon can be specified by either (*R*) or (*S*). Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers and can thus give rise to enantiomers, diastereomers, and other stereoisomeric forms that can be defined, in terms of absolute stereochemistry, as (*R*) or (*S*). The present chemical entities, pharmaceutical compositions and methods are meant to include all such possible isomers, including racemic mixtures, optically pure forms and intermediate mixtures. Optically active (*R*)- and (*S*)-isomers can be prepared using chiral synthons or chiral reagents, or resolved using conventional techniques. When the compounds described herein contain olefinic double bonds or other centers of geometric asymmetry, and unless specified otherwise, it is intended that the compounds include both *E* and *Z* geometric isomers.

"Moiety" refers to a specific segment or functional group of a molecule. Chemical moieties are often recognized chemical entities embedded in or appended to a molecule.

"Solvate" refers to a compound in physical association with one or more molecules of a pharmaceutically acceptable solvent.

The compounds disclosed herein for use in the invention also include crystalline and amorphous forms of those compounds, including, for example, polymorphs, pseudopolymorphs, solvates, hydrates, unsolvated polymorphs (including anhydrates), conformational polymorphs, and amorphous forms of the compounds, as well as mixtures thereof. "Crystalline form" and "polymorph" are intended to include all crystalline and amorphous forms of the compound, including, for example, polymorphs, pseudopolymorphs, solvates, hydrates, unsolvated polymorphs (including anhydrates), conformational polymorphs, and amorphous forms, as well as mixtures thereof, unless a particular crystalline or amorphous form is referred to.

The combinations for use in the invention also include antibodies. The terms "antibody" and its plural form "antibodies" refer to whole immunoglobulins and any antigen-binding fragment ("antigen-binding portion") or single chains thereof. An "antibody" further refers to a glycoprotein comprising at least two heavy (H) chains and two light (L) chains interconnected by disulfide bonds, or an antigen-binding portion thereof. Each heavy chain is comprised of a heavy chain variable region (abbreviated herein as V_{H}) and a heavy chain constant region. The heavy chain constant region is comprised of three domains, CH1, CH2 and CH3. Each light chain is comprised of a light chain variable region (abbreviated herein as V_{L}) and a light chain constant region. The light chain constant region is comprised of one domain, C_{L}. The V_{H} and V_{L} regions of an antibody may be further subdivided into regions of hypervariability, which are referred to as complementarity determining regions (CDR) or hypervariable regions (HVR), and which can be interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen epitope or epitopes. The constant regions of the antibodies may mediate the binding of the immunoglobulin to host tissues or factors, including various cells of the immune system (e.g., effector cells) and the first component (Clq) of the classical complement system.

The terms "monoclonal antibody," "mAb," "monoclonal antibody composition," or their plural forms refer to a preparation of antibody molecules of single molecular composition. A monoclonal antibody composition displays a single binding specificity and affinity for a particular epitope. Monoclonal antibodies specific to, *e.g.,* CD19, CD20, PD-1, PD-L1, or PD-L2 can be made using knowledge and skill in the art of injecting test subjects with CD19, CD20, PD-1, PD-L1, or PD-L2 antigen and then isolating hybridomas expressing antibodies having the desired sequence or functional characteristics. DNA encoding the monoclonal antibodies is readily isolated and sequenced using conventional procedures (e.g., by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. Recombinant production of antibodies will be described in more detail below.

The terms "antigen-binding portion" or "antigen-binding fragment" of an antibody (or simply "antibody portion"), as used herein, refers to one or more fragments of an antibody that retain the ability to specifically bind to an antigen (*e.g.,* CD19, CD20, PD-1, PD-L1, or PD-L2). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the V_{L}, V_{H}, C_{L} and CH1 domains; (ii) a F(ab')2 fragment, a bivalent fragment comprising two Fab fragments linked by a disulfide bridge at the hinge region; (iii) a Fd fragment consisting of the V_{H} and CH1 domains; (iv) a Fv fragment consisting of the V_{L} and V_{H} domains of a single arm of an antibody, (v) a domain antibody (dAb) fragment (Ward et al., Nature, 1989, 341, 544-546), which may consist of a V_{H} or a V_{L} domain; and (vi) an isolated complementarity determining region (CDR). Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules known as single chain Fv (scFv); see, *e.g.,* Bird et al., Science 1988, 242, 423-426; and Huston et al., Proc. Natl. Acad. Sci. USA 1988, 85, 5879-5883). Such scFv antibodies are also intended to be encompassed within the terms "antigen-binding portion" or "antigen-binding fragment" of an antibody. These antibody fragments are obtained using conventional techniques known to those with skill in the art, and the fragments are screened for utility in the same manner as are intact antibodies.

The term "human antibody," as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies of the invention may include amino acid residues not encoded by human germline immunoglobulin sequences (e.g., mutations introduced by random or site-specific mutagenesis *in vitro* or by somatic mutation *in vivo).* The term "human antibody", as used herein, is not intended to include antibodies in which CDR sequences derived from the germline of another mammalian species, such as a mouse, have been grafted onto human framework sequences.

The term "human monoclonal antibody" refers to antibodies displaying a single binding specificity which have variable regions in which both the framework and CDR regions are derived from human germline immunoglobulin sequences. In one embodiment, the human monoclonal antibodies are produced by a hybridoma which includes a B cell obtained from a transgenic nonhuman animal, e.g., a transgenic mouse, having a genome comprising a human heavy chain transgene and a light chain transgene fused to an immortalized cell.

The term "recombinant human antibody", as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom (described further below), (b) antibodies isolated from a host cell transformed to express the human antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

As used herein, "isotype" refers to the antibody class (e.g., IgM or IgG1) that is encoded by the heavy chain constant region genes.

The phrases "an antibody recognizing an antigen" and "an antibody specific for an antigen" are used interchangeably herein with the term "an antibody which binds specifically to an antigen."

The term "human antibody derivatives" refers to any modified form of the human antibody, e.g., a conjugate of the antibody and another active pharmaceutical ingredient or antibody. The terms "conjugate," "antibody-drug conjugate", "ADC," or "immunoconjugate" refers to an antibody, or a fragment thereof, conjugated to a therapeutic moiety, such as a bacterial toxin, a cytotoxic drug or a radionuclide-containing toxin. Toxic moieties can be conjugated to antibodies of the invention using methods available in the art.

The terms "humanized antibody," "humanized antibodies," and "humanized" are intended to refer to antibodies in which CDR sequences derived from the germline of another mammalian species, such as a mouse, have been grafted onto human framework sequences. Additional framework region modifications may be made within the human framework sequences. Humanized forms of non-human (for example, murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a 15 hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 1986, 321, 522-525; Riechmann et al., Nature 1988, 332, 323-329; and Presta, Curr. Op. Struct. Biol. 1992, 2, 593-596.

The term "chimeric antibody" is intended to refer to antibodies in which the variable region sequences are derived from one species and the constant region sequences are derived from another species, such as an antibody in which the variable region sequences are derived from a mouse antibody and the constant region sequences are derived from a human antibody.

A "diabody" is a small antibody fragment with two antigen-binding sites. The fragments comprises a heavy chain variable domain (V_{H}) connected to a light chain variable domain (V_{L}) in the same polypeptide chain (V_{H}-V_{L} or V_{L}-V_{H}). By using a linker that is too short to allow pairing between the two domains on the same chain, the domains are forced to pair with the complementary domains of another chain and create two antigen-binding sites. Diabodies are described more fully in, *e.g.,* European Patent No. EP 404,097, International Patent Publication No. WO 93/11161; and Bolliger et al., Proc. Natl. Acad. Sci. USA 1993, 90, 6444-6448.

The term "glycosylation" refers to a modified derivative of an antibody. An aglycoslated antibody lacks glycosylation. Glycosylation can be altered to, for example, increase the affinity of the antibody for antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Aglycosylation may increase the affinity of the antibody for antigen, as described in U.S. Patent Nos. 5,714,350 and 6,350,861. Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the invention to thereby produce an antibody with altered glycosylation. For example, the cell lines Ms704, Ms705, and Ms709 lack the fucosyltransferase gene, FUT8 (alpha (1,6) fucosyltransferase), such that antibodies expressed in the Ms704, Ms705, and Ms709 cell lines lack fucose on their carbohydrates. The Ms704, Ms705, and Ms709 FUT8-/- cell lines were created by the targeted disruption of the FUT8 gene in CHO/DG44 cells using two replacement vectors (see e.g. U.S. Patent Publication No. 2004/0110704 or Yamane-Ohnuki, et al. Biotechnol. Bioeng., 2004, 87, 614-622). As another example, European Patent No. EP 1,176,195 describes a cell line with a functionally disrupted FUT8 gene, which encodes a fucosyl transferase, such that antibodies expressed in such a cell line exhibit hypofucosylation by reducing or eliminating the alpha 1,6 bond-related enzyme, and also describes cell lines which have a low enzyme activity for adding fucose to the N-acetylglucosamine that binds to the Fc region of the antibody or does not have the enzyme activity, for example the rat myeloma cell line YB2/0 (ATCC CRL 1662). International Patent Publication WO 03/035835 describes a variant CHO cell line, Lec 13 cells, with reduced ability to attach fucose to Asn(297)-linked carbohydrates, also resulting in hypofucosylation of antibodies expressed in that host cell (see also Shields, et al., J. Biol. Chem. 2002, 277, 26733-26740. International Patent Publication WO 99/54342 describes cell lines engineered to express glycoprotein-modifying glycosyl transferases (e.g., beta(1,4)-N-acetylglucosaminyltransferase III (GnTIII)) such that antibodies expressed in the engineered cell lines exhibit increased bisecting GlcNac structures which results in increased ADCC activity of the antibodies (see also Umana, etal., Nat. Biotech. 1999, 17, 176-180). Alternatively, the fucose residues of the antibody may be cleaved off using a fucosidase enzyme. For example, the fucosidase alpha-L-fucosidase removes fucosyl residues from antibodies as described in Tarentino, et al., Biochem. 1975, 14, 5516-5523.

"Pegylation" refers to a modified antibody, or a fragment thereof, that typically is reacted with polyethylene glycol (PEG), such as a reactive ester or aldehyde derivative of PEG, under conditions in which one or more PEG groups become attached to the antibody or antibody fragment. Pegylation may, for example, increase the biological (e.g., serum) half life of the antibody. Preferably, the pegylation is carried out via an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C₁-C₁₀) alkoxy- or aryloxy-polyethylene glycol or polyethylene glycol-maleimide. The antibody to be pegylated may be an aglycosylated antibody. Methods for pegylation are known in the art and can be applied to the antibodies of the invention, as described for example in European Patent Nos. EP 0154316 and EP 0401384.

The term "conservative amino acid substitutions" in means amino acid sequence modifications which do not abrogate the binding of the antibody to the antigen. Conservative amino acid substitutions include the substitution of an amino acid in one class by an amino acid of the same class, where a class is defined by common physicochemical amino acid side chain properties and high substitution frequencies in homologous proteins found in nature, as determined, for example, by a standard Dayhoff frequency exchange matrix or BLOSUM matrix. Six general classes of amino acid side chains have been categorized and include: Class I (Cys); Class II (Ser, Thr, Pro, Ala, Gly); Class III (Asn, Asp, Gln, Glu); Class IV (His, Arg, Lys); Class V (Ile, Leu, Val, Met); and Class VI (Phe, Tyr, Trp). For example, substitution of an Asp for another class III residue such as Asn, Gln, or Glu, is a conservative substitution. Thus, a predicted nonessential amino acid residue in an OX40 agonist antibody is preferably replaced with another amino acid residue from the same class. Methods of identifying amino acid conservative substitutions which do not eliminate antigen binding are well-known in the art (see, *e.g.,* Brummell, et al., Biochemistry 1993, 32, 1180-1187; Kobayashi, et al., Protein Eng. 1999, 12, 879-884 (1999); and Burks, et al., Proc. Natl. Acad. Sci. USA 1997, 94, 412-417.

The terms "sequence identity," "percent identity," and "sequence percent identity" in the context of two or more nucleic acids or polypeptides, refer to two or more sequences or subsequences that are the same or have a specified percentage of nucleotides or amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. Suitable programs to determine percent sequence identity include for example the BLAST suite of programs available from the U.S. Government's National Center for Biotechnology Information BLAST web site. Comparisons between two sequences can be carried using either the BLASTN or BLASTP algorithm. BLASTN is used to compare nucleic acid sequences, while BLASTP is used to compare amino acid sequences. ALIGN, ALIGN-2 (Genentech, South San Francisco, California) or MegAlign, available from DNASTAR, are additional publicly available software programs that can be used to align sequences. One skilled in the art can determine appropriate parameters for maximal alignment by particular alignment software. In certain embodiments, the default parameters of the alignment software are used.

Certain embodiments of the present invention comprise a variant of an antibody, e.g., a CD19 inhibitor that is an antibody, an anti-CD20 antibody, an anti-PD-1 antibody, an anti-PD-L1 antibody, and/or an anti-PD-L2 antibody. As used herein, the term "variant" encompasses antibodies which comprise an amino acid sequence which differs from the amino acid sequence of a reference antibody by way of one or more substitutions, deletions and/or additions at certain positions within or adjacent to the amino acid sequence of the reference antibody. The variant may comprise one or more conservative substitutions in its amino acid sequence as compared to the amino acid sequence of a reference antibody. Conservative substitutions may involve, *e.g.,* the substitution of similarly charged or uncharged amino acids. The variant retains the ability to specifically bind to the antigen of the reference antibody.

The term "radioisotope-labeled complex" refers to both non-covalent and covalent attachment of a radioactive isotope, such as ⁹⁰Y, ¹¹¹In, or ¹³¹I, to an antibody, including conjugates.

The term "biosimilar" means a biological product that is highly similar to a U.S. licensed reference biological product notwithstanding minor differences in clinically inactive components, and for which there are no clinically meaningful differences between the biological product and the reference product in terms of the safety, purity, and potency of the product. Furthermore, a similar biological or "biosimilar" medicine is a biological medicine that is similar to another biological medicine that has already been authorized for use by the European Medicines Agency. The term "biosimilar" is also used synonymously by other national and regional regulatory agencies. Biological products or biological medicines are medicines that are made by or derived from a biological source, such as a bacterium or yeast. They can consist of relatively small molecules such as human insulin or erythropoietin, or complex molecules such as monoclonal antibodies. For example, if the reference anti-CD20 monoclonal antibody is rituximab, an anti-CD20 biosimilar monoclonal antibody approved by drug regulatory authorities with reference to rituximab is a "biosimilar to" rituximab or is a "biosimilar thereof' of rituximab. In Europe, a similar biological or "biosimilar" medicine is a biological medicine that is similar to another biological medicine that has already been authorized for use by the European Medicines Agency (EMA). The relevant legal basis for similar biological applications in Europe is Article 6 of Regulation (EC) No 726/2004 and Article 10(4) of Directive 2001/83/EC, as amended and therefore in Europe, the biosimilar may be authorised, approved for authorisation or subject of an application for authorisation under Article 6 of Regulation (EC) No 726/2004 and Article 10(4) of Directive 2001/83/EC. The already authorized original biological medicinal product may be referred to as a "reference medicinal product" in Europe. Some of the requirements for a product to be considered a biosimilar are outlined in the CHMP Guideline on Similar Biological Medicinal Products. In addition, product specific guidelines, including guidelines relating to monoclonal antibody biosimilars, are provided on a product-by-product basis by the EMA and published on its website. A biosimilar as described herein may be similar to the reference medicinal product by way of quality characteristics, biological activity, mechanism of action, safety profiles and/or efficacy. In addition, the biosimilar may be used or be intended for use to treat the same conditions as the reference medicinal product. Thus, a biosimilar as described herein may be deemed to have similar or highly similar quality characteristics to a reference medicinal product. Alternatively, or in addition, a biosimilar as described herein may be deemed to have similar or highly similar biological activity to a reference medicinal product. Alternatively, or in addition, a biosimilar as described herein may be deemed to have a similar or highly similar safety profile to a reference medicinal product. Alternatively, or in addition, a biosimilar as described herein may be deemed to have similar or highly similar efficacy to a reference medicinal product. As described herein, a biosimilar in Europe is compared to a reference medicinal product which has been authorised by the EMA. However, in some instances, the biosimilar may be compared to a biological medicinal product which has been authorised outside the European Economic Area (a non-EEA authorised "comparator") in certain studies. Such studies include for example certain clinical and in vivo non-clinical studies. As used herein, the term "biosimilar" also relates to a biological medicinal product which has been or may be compared to a non-EEA authorised comparator. Certain biosimilars are proteins such as antibodies, antibody fragments (for example, antigen binding portions) and fusion proteins. A protein biosimilar may have an amino acid sequence that has minor modifications in the amino acid structure (including for example deletions, additions, and/or substitutions of amino acids) which do not significantly affect the function of the polypeptide. The biosimilar may comprise an amino acid sequence having a sequence identity of 97% or greater to the amino acid sequence of its reference medicinal product, e.g., 97%, 98%, 99% or 100%. The biosimilar may comprise one or more post-translational modifications, for example, glycosylation, oxidation, deamidation, and/or truncation which is/are different to the post-translational modifications of the reference medicinal product, provided that the differences do not result in a change in safety and/or efficacy of the medicinal product. The biosimilar may have an identical or different glycosylation pattern to the reference medicinal product. Particularly, although not exclusively, the biosimilar may have a different glycosylation pattern if the differences address or are intended to address safety concerns associated with the reference medicinal product. Additionally, the biosimilar may deviate from the reference medicinal product in for example its strength, pharmaceutical form, formulation, excipients and/or presentation, providing safety and efficacy of the medicinal product is not compromised. The biosimilar may comprise differences in for example pharmacokinetic (PK) and/or pharmacodynamic (PD) profiles as compared to the reference medicinal product but is still deemed sufficiently similar to the reference medicinal product as to be authorised or considered suitable for authorisation. In certain circumstances, the biosimilar exhibits different binding characteristics as compared to the reference medicinal product, wherein the different binding characteristics are considered by a Regulatory Authority such as the EMA not to be a barrier for authorisation as a similar biological product. The term "biosimilar" is also used synonymously by other national and regional regulatory agencies.

The term "binding molecule" as used herein includes molecules that contain at least one antigen binding site that specifically binds to CD19, CD20, PD-1, PD-L1, or PD-L2. By "specifically binds" it is meant that the binding molecules exhibit essentially background binding to, *e.g.,* non-CD19 molecules. An isolated binding molecule that specifically binds to, *e.g.,* CD19 may, however, have cross-reactivity to CD19 molecules from other species. A CD19 binding molecule may also be referred to herein as a "CD19 inhibitor." A PD-1 or PD-L1 binding molecule may also be referred to herein as a "PD-1 inhibitor" or a "PD-L1 inhibitor."

The term "hematological malignancy" refers to mammalian cancers and tumors of the hematopoietic and lymphoid tissues, including tissues of the blood, bone marrow, lymph nodes, and lymphatic system. Hematological malignancies are also referred to as "liquid tumors." Hematological malignancies include ALL, CLL, SLL, acute myelogenous leukemia (AML), chronic myelogenous leukemia (CML), acute monocytic leukemia (AMoL), Hodgkin's lymphoma, and non-Hodgkin's lymphomas. The term "B cell hematological malignancy" refers to hematological malignancies that affect B cells.

The term "solid tumor" refers to an abnormal mass of tissue that usually does not contain cysts or liquid areas. Solid tumors may be benign or malignant. The term "solid tumor cancer" refers to malignant, neoplastic, or cancerous solid tumors. Solid tumor cancers include sarcomas, carcinomas, and lymphomas, such as cancers of the lung, breast, prostate, colon, rectum, and bladder. The tissue structure of solid tumors includes interdependent tissue compartments including the parenchyma (cancer cells) and the supporting stromal cells in which the cancer cells are dispersed and which may provide a supporting microenvironment.

The term "microenvironment," as used herein, may refer to the tumor microenvironment as a whole or to an individual subset of cells within the microenvironment.

The term "autologous" refers to any material derived from the same individual to whom it is later to be re-introduced into the individual.

The term "allogeneic" refers to any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically.

The term "chimeric antigen receptor" refers to a recombinant polypeptide construct comprising at least an extracellular antigen binding domain, a transmembrane domain and a cytoplasmic signaling domain (also referred to as "an intracellular signaling domain") comprising a functional signaling domain derived from a stimulatory molecule as defined below. In an embodiment, the stimulatory molecule is the zeta (ζ) chain associated with the T cell receptor complex. In an embodiment, the cytoplasmic signaling domain further comprises one or more functional signaling domains derived from at least one costimulatory molecule. In one aspect, the costimulatory molecule is chosen from 4-1BB (CD137), CD27 and/or CD28. In an embodiment, the CAR comprises a chimeric fusion protein comprising an extracellular antigen recognition domain, a transmembrane domain and an intracellular signaling domain comprising a functional signaling domain derived from a stimulatory molecule. In an embodiment, the CAR comprises a chimeric fusion protein comprising an extracellular antigen recognition domain, a transmembrane domain and an intracellular signaling domain comprising a functional signaling domain derived from a co-stimulatory molecule and a functional signaling domain derived from a stimulatory molecule. In an embodiment, the CAR comprises a chimeric fusion protein comprising an extracellular antigen recognition domain, a transmembrane domain and an intracellular signaling domain comprising two functional signaling domains derived from one or more co-stimulatory molecule(s) and a functional signaling domain derived from a stimulatory molecule. In an embodiment, the CAR comprises a chimeric fusion protein comprising an extracellular antigen recognition domain, a transmembrane domain and an intracellular signaling domain comprising at least two functional signaling domains derived from one or more costimulatory molecule(s) and a functional signaling domain derived from a stimulatory molecule. In one aspect the CAR comprises an optional leader sequence at the amino-terminus (N-terminus) of the CAR fusion protein. In an embodiment, the CAR further comprises a leader sequence at the N-terminus of the extracellular antigen recognition domain, wherein the leader sequence is optionally cleaved from the antigen recognition domain (*e.g.,* a scFv) during cellular processing and localization of the CAR to the cellular membrane. The term "signaling domain" refers to the functional portion of a protein which acts by transmitting information within the cell to regulate cellular activity via defined signaling pathways by generating second messengers or functioning as effectors by responding to such messengers.

For the avoidance of doubt, it is intended herein that particular features (for example integers, characteristics, values, uses, diseases, formulae, compounds or groups) described in conjunction with a particular aspect, embodiment or example of the invention are to be understood as applicable to any other aspect, embodiment or example described herein unless incompatible therewith. Thus such features may be used where appropriate in conjunction with any of the definition, claims or embodiments defined herein. All of the features disclosed in this specification (including any accompanying claims, abstract and drawings), and/or all of the steps of any method or process so disclosed, may be combined in any combination, except combinations where at least some of the features and/or steps are mutually exclusive. The invention is not restricted to any details of any disclosed embodiments.

### Co-administration of Compounds

An aspect of the invention is a composition, such as a pharmaceutical composition for use in the treatment of cancer, comprising a combination of a BTK inhibitor of Formula (II) and a CD19 inhbitor selected from an anti-CD19 antibody or an anti-CD19 chimeric antigen receptor, or an antigen-binding fragment or conjugate thereof.

Another aspect is a kit containing a BTK inhibitor of Formula (II) and a CD19 inhibitor selected from an anti-CD19 antibody or an anti-CD19 chimeric antigen receptor, or an antigen-binding fragment or conjugate thereof, wherein each of the inhibitors is formulated into a separate pharmaceutical composition, and wherein said separate pharmaceutical compositions are formulated for co-administration for use in the treatment of cancer.

Another aspect of the invention is a pharmaceutical composition for use in, treating cancer in a subject, such as leukemia, lymphoma or a solid tumor cancer, comprising a therapeutically effective amount of a combination of a BTK inhibitor of Formula (II) and a CD19 inhibitor selected from an anti-CD19 antibody or an anti-CD19 chimeric antigen receptor, or an antigen-binding fragment or conjugate thereof. In an embodiment, the foregoing use exhibits synergistic effects that may result in greater efficacy, less side effects, the use of less active pharmaceutical ingredient to achieve a given clinical result, or other synergistic effects. The pharmaceutical composition comprising the combination, and the kit, are both for use in treating such disease or condition.

In a preferred embodiment, the solid tumor cancer is selected from the group consisting of breast, lung, colorectal, thyroid, bone sarcoma, and stomach cancers.

In a preferred embodiment, the leukemia is selected from the group consisting of acute myelogenous leukemia (AML), chronic myelogenous leukemia (CML), acute lymphoblastic leukemia (ALL), B cell chronic lymphocytic leukemia (B-CLL), and chronic lymphoid leukemia (CLL).

In a preferred embodiment, the lymphoma is selected from the group consisting of Burkitt's lymphoma, mantle cell lymphoma, follicular lymphoma, indolent B-cell non-Hodgkin's lymphoma, histiocytic lymphoma, activated B-cell like diffuse large B cell lymphoma (DLBCL-ABC), germinal center B-cell like diffuse large B cell lymphoma (DLBCL-GCB), and diffuse large B cell lymphoma (DLBCL).

In an embodiment, the BTK inhibitor of Formula (II) is in the form of a pharmaceutically acceptable salt.

In a preferred embodiment, the CD19 inhibitor is in the form of a monoclonal antibody, an antigen-binding fragment, an antibody-drug conjugate, or a biosimilar thereof. In a preferred embodiment, the CD19 inhibitor is a NK cell or T cell expressing an anti-CD19 chimeric antigen receptor.

In an embodiment, the CD19 inhibitor selected from an anti-CD19 antibody or an anti-CD19 chimeric antigen receptor, or an antigen-binding fragment or conjugate thereof is administered to the subject before administration of the BTK inhibitor of Formula (II).

In an embodiment, the CD19 inhibitor selected from an anti-CD19 antibody or an anti-CD19 chimeric antigen receptor, or an antigen-binding fragment or conjugate thereof is administered concurrently with the administration of the BTK inhibitor of Formula (II).

In an embodiment, the CD19 inhibitor selected from an anti-CD19 antibody or an anti-CD19 chimeric antigen receptor, or an antigen-binding fragment or conjugate thereof is administered to the subject after administration of the BTK inhibitor of Formula (II).

In a preferred embodiment, the subject is a mammal, such as a human. In an embodiment, the subject is a human. In an embodiment, the subject is a companion animal. In an embodiment, the subject is a canine, feline, or equine.

### BTK Inhibitors

The BTK inhibitor is a BTK inhibitor of Formula (II) as described in more detail in the following paragraphs.

The BTK inhibitor is a compound of Formula (2): 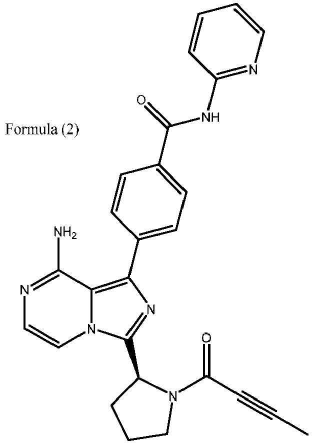 or a pharmaceutically acceptable salt thereof. The preparation of this compound is described in International Patent Application Publication No. WO 2013/010868 and U.S. Patent Application Publication No. US 2014/0155385 A1.

The BTK inhibitor is (*S*)-4-(8-amino-3-(1-(but-2-ynoyl)pyrrolidin-2-yl)imidazo[1,5-*a*]pyrazin-1-yl)-*N*-(pyridin-2-yl)benzamide or pharmaceutically acceptable salt therof.

Not part of the present invention, but when used as a comparator in the Examples below, the BTK inhibitor is ibrutinib or a pharmaceutically acceptable salt, solvate, hydrate, cocrystal, or prodrug thereof. In this context, the BTK inhibitor is (*R*)-1-(3-(4-amino-3-(4-phenoxyphenyl)-1*H*-pyrazolo[3,4-*d*]pyrimidin-1-yl)piperidin-1-yl)prop-2-en-1-one. In this context, the BTK inhibitor is 1-[(3*R*)-3-[4-amino-3-(4-phenoxyphenyl)-1*H*-pyrazolo[3,4-*d*]pyrimidin-1-yl]piperidin-1-yl]prop-2-en-1-one. In this context, the BTK inhibitor has the structure of Formula (10): 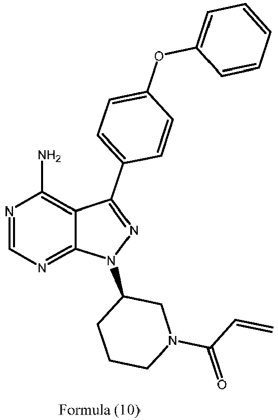 or an enantiomer thereof, or a pharmaceutically acceptable salt, solvate, hydrate, cocrystal, or prodrug thereof.

Not part of the present invention, but used as a comparator in the Examples which follow, the BTK inhibitor is CC-292 (also known as AVL-292), or a pharmaceutically acceptable salt, solvate, hydrate, cocrystal, or prodrug thereof, preferably a hydrochloride salt or a besylate salt thereof. In this context, the BTK inhibitor is a compound of Formula (17): 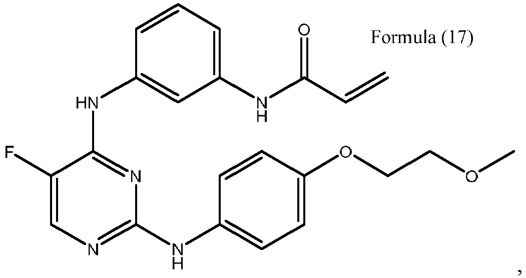 which is *N*-(3-((5-fluoro-2-((4-(2-methoxyethoxy)phenyl)amino)pyrimidin-4-yl)amino)phenyl)acrylamide, or a pharmaceutically acceptable salt, solvate, hydrate, cocrystal, or prodrug thereof, or in an exemplary embodiment is a hydrochloride salt or a besylate salt thereof. The preparation of this compound is described in U.S. Patent Application Publication No. 2010/0029610 A1 at Example 20. The preparation of the besylate salt of this compound is described in U.S. Patent Application Publication No. 2012/0077832 A1. In an embodiment, the BTK inhibitor is a compound selected from the structures disclosed in U.S. Patent Application Publication No. 2010/0029610 A1 or No. 2012/0077832 A1.

### CD19 Inhibitors, Anti-CD19 Antibodies, and Anti-CD19 Cell Therapies

The CD19 inhibitor is selected from an anti-CD19 antibody or an anti-CD19 chimeric antigen receptor, or an antigen-binding fragment or conjugate thereof as described in more detail in the following paragraphs. In preferred embodiments, the compositions described herein provide a combination of a CD 19 inhibitor selected from an anti-CD19 antibody or an anti-CD19 chimeric antigen receptor, or an antigen-binding fragment or conjugate thereof with a BTK inhibitor of Formula (II), and use of the combination in the treatment of cancer.

In a preferred embodiment, the CD19 inhibitor is an anti-CD19 antibody, such as an anti-CD19 monoclonal antibody (*i.e.,* an antibody derived from a single cell line). In a preferred embodiment, the CD19 inhibitor is an antibody that binds specifically to CD19 antigen. In an embodiment, the CD19 inhibitor is a CD19 receptor antagonist. In an embodiment, the CD19 inhibitor is a CD19 receptor blocker. In some embodiments, the CD19 inhibitor is an anti-CD19 antibody, such as an anti-CD19 monoclonal antibody, conjugated to a small molecule that is cytotoxic. In some embodiments, the CD19 inhibitor is an anti-CD19 antibody, such as an anti-CD19 monoclonal antibody, conjugated to a radioisotope. In some embodiments, the CD19 inhibitor is an anti-CD19 antibody that effects its function through antibody-dependent cellular toxicity (ADCC), for example through NK cell cytotoxicity. In some embodiments, the CD19 inhibitor is an anti-CD19 antibody that effects its function through antibody-dependent cell phagocytosis (ADCP). In some embodiments, the CD19 inhibitor is an anti-CD19 antibody that effects its function through complement-dependent cytotoxicity (CDC).

In some embodiments, the compositions and methods described include a CD19 inhibitor that binds human CD19 with a K_{D} of about 100 pM or lower, binds human CD19 with a K_{D} of about 90 pM or lower, binds human CD19 with a K_{D} of about 80 pM or lower, binds human CD19 with a K_{D} of about 70 pM or lower, binds human CD19 with a K_{D} of about 60 pM or lower, binds human CD-19 with a K_{D} of about 50 pM or lower, binds human CD19 with a K_{D} of about 40 pM or lower, or binds human CD19 with a K_{D} of about 30 pM or lower.

In some embodiments, the compositions and methods described include a CD19 inhibitor that binds to human CD19 with a k_{assoc} of about 7.5 × 10⁵ 1/M·s or faster, binds to human CD19 with a k_{assoc} of about 7.5 × 10⁵ 1IM.s or faster, binds to human CD19 with a k_{assoc} of about 8 × 10⁵ 1/M·s or faster, binds to human CD19 with a k_{assoc} of about 8.5 × 10⁵ 1/M-s or faster, binds to human CD19 with a k_{assoc} of about 9 × 10⁵ 1/M·s or faster, binds to human CD19 with a k_{assoc} of about 9.5 × 10⁵ 1/M·s or faster, or binds to human CD19 with a k_{assoc} of about 1 × 10⁶ 1/M·s or faster.

In some embodiments, the compositions and methods described include a CD19 inhibitor that binds to human CD19 with a k_{dissoc} of about 2 × 10⁻⁵ 1/s or slower, binds to human CD19 with a k_{dissoc} of about 2.1 × 10⁻⁵ 1/s or slower , binds to human CD19 with a k_{dissoc} of about 2.2 × 10⁻⁵ 1/s or slower, binds to human CD19 with a k_{dissoc} of about 2.3 × 10⁻⁵ 1/s or slower, binds to human CD19 with a k_{dissoc} of about 2.4 × 10⁻⁵ 1/s or slower, binds to human CD19 with a k_{dissoc} of about 2.5 × 10⁻⁵ 1/s or slower, binds to human CD19 with a k_{dissoc} of about 2.6 × 10⁻⁵ l/s or slower or binds to human CD19 with a k_{dissoc} of about 2.7 × 10⁻⁵ 1/s or slower, binds to human CD19 with a k_{dissoc} of about 2.8 × 10⁻⁵ 1/s or slower, binds to human CD19 with a k_{dissoc} of about 2.9 × 10⁻⁵ 1/s or slower, or binds to human CD19 with a k_{dissoc} of about 3 × 10⁻⁵ 1/s or slower.

In some embodiments, the compositions and methods described include a CD19 inhibitor that binds to human CD19 with an IC₅₀ of about 10 nM or lower, binds to human CD19 with an IC₅₀ of about 9 nM or lower, binds to human CD19 with an IC₅₀ of about 8 nM or lower, binds to human CD19 with an IC₅₀ of about 7 nM or lower, binds to human CD19 with an IC₅₀ of about 6 nM or lower, binds to human CD19 with an IC₅₀ of about 5 nM or lower, binds to human CD19 with an IC₅₀ of about 4 nM or lower, binds to human CD19 with an IC₅₀ of about 3 nM or lower, binds to human CD19 with an IC₅₀ of about 2 nM or lower, or binds to human CD19 with an IC₅₀ of about 1 nM or lower.

In an embodiment, the CD19 inhibitor is blinatumomab, also known as AMG103, BITE MT-103, MEDI-538, and MT103, or an antigen-binding fragment, conjugate, variant, or biosimilar thereof. Blinatumomab is an immunoglobulin scFv-scFv construct. In an embodiment, the CD19 inhibitor has the format scFv-scFv. In an embodiment, the CD19 inhibitor has the format scFv(V_{L}[kappa]-V_{H}) - (scFv-V_{H}-V_{L}[kappa]), where V_{H} represents an immunoglobulin heavy chain, V_{L} represents an immunoglobulin heavy chain, and kappa represents a human light chain encoded by the immunoglobulin kappa locus. In an embodiment, the CD19 inhibitor is a bispecific T cell engager (BITE) antibody construct. Garber, Nature Rev. Drug Disc. 2014,13, 799-801. In an embodiment, blinatumomab has disulfide bridges located at 23-92, 148-222, 277-351, and 415-479; and N-glycosylation sites located at 307 (but a Pro located at 308). Blinatumomab has been described for the treatment of B cell diseases such as relapsed B cell non-Hodgkin lymphoma and chronic lymphocytic leukemia. Baeuerle, et al., Cancer Res. 2009, 69, 4941-4944. In an embodiment, the CD19 inhibitor comprises a bispecific single chain antibody construct comprising binding domains specific for human CD3 and human CD19, wherein the corresponding variable heavy chain regions (V_{H}) and the corresponding variable light chain regions (V_{L}) regions are arranged, from N-terminus to C-terminus, in the order, V_{H}(CD19)-V_{L}(CD19)-V_{H}(CD3)-V_{L}(CD3), or V_{H}(CD3)-V_{L}(CD3)-V_{H}(CD19)-V_{L}(CD19).

In an embodiment, the CD19 inhibitor is an anti-CD19 biosimilar monoclonal antibody approved by drug regulatory authorities with reference to blinatumomab. In an embodiment, the CD19 inhibitor has a sequence identity of greater than 90% to SEQ ID NO: 1. In an embodiment, the CD19 inhibitor has a sequence identity of greater than 95% to SEQ ID NO: 1. In an embodiment, the CD19 inhibitor has a sequence identity of greater than 96% to SEQ ID NO:1. In an embodiment, the CD19 inhibitor has a sequence identity of greater than 97% to SEQ ID NO: 1. In an embodiment, the CD19 inhibitor has a sequence identity of greater than 98% to SEQ ID NO: 1. In an embodiment, the CD19 inhibitor has a sequence identity of greater than 99% to SEQ ID NO: 1.

In an embodiment, the CD19 inhibitor is an anti-CD19 monoclonal antibody comprising a V_{H} region comprising at least one CDR1 region comprising the amino acid sequence of SEQ ID NO:2; at least one CDR2 region comprising the amino acid sequence of SEQ ID NO:3; and at least one CDR3 region comprising the amino acid sequence of SEQ ID NO:4; and a V_{L} region comprising at least one CDR1 region comprising the amino acid sequence of SEQ ID NO:5; at least one CDR2 region comprising the amino acid sequence of SEQ ID NO:6; and at least one CDR3 region comprising the amino acid sequence of SEQ ID NO:7.

In an embodiment, the CD19 inhibitor is an anti-CD19 antibody construct described in U.S. Patent No. 7,635,472.

In an embodiment, the CD19 inhibitor is coltuximab, or an antigen-binding fragment, conjugate, variant, or biosimilar thereof. In an embodiment, the CD19 inhibitor is a coltuximab conjugate. In an embodiment, the CD19 inhibitor is a coltuximab maytansinoid conjugate. In an embodiment, the CD19 inhibitor is coltuximab ravtansine. The preparation and properties of coltuximab ravtansine are described in, *e.g.,* Hong, et al., Mol. Pharm. 2015, 12, 1703-16. Coltuximab ravtansine (also known as SAR3419 or huB4-DM4) is an antibody-drug conjugate (ADC) targeting CD19 that is a conjugate of a derivative of the potent microtubule-acting cytotoxic agent, maytansine, to a version of the anti-CD19 antibody, anti-B4, which was humanized as an IgG1 by variable domain resurfacing. In an embodiment, the CD19 inhibitor comprises a monoclonal antibody having a heavy chain sequence of SEQ ID NO:8, a light chain sequence of SEQ ID NO:9, intra-heavy chain disulfide bridges at the following positions: 22-96, 147-203, 264-324, 370-428, 22"-96", 147"-203", 264"-324", and 370"-428", intra-light chain disulfide bridges at the following positions: 23'-87', 131'-191', 23‴-87‴, and 131‴-191‴, inter-heavy-light chain disulfide bridges at the following positions: 223-211', 223"-211‴, inter-heavy-heavy chain bridges at the following positions: 229-229", 232-232", and N-glycosylation sites at 300 and 300', wherein the monoclonal antibody is conjugated to N2'-Deacetyl-N2'-(4-mercapto-4-methyl-1-oxopentyl)-maytansine (ravtansine; DM 4) by N-succinimidyl-4-(2-pyridyldithio)butyrate (SPDB) linkers. In an embodiment, the CD19 inhibitor is a compound of Formula (44): 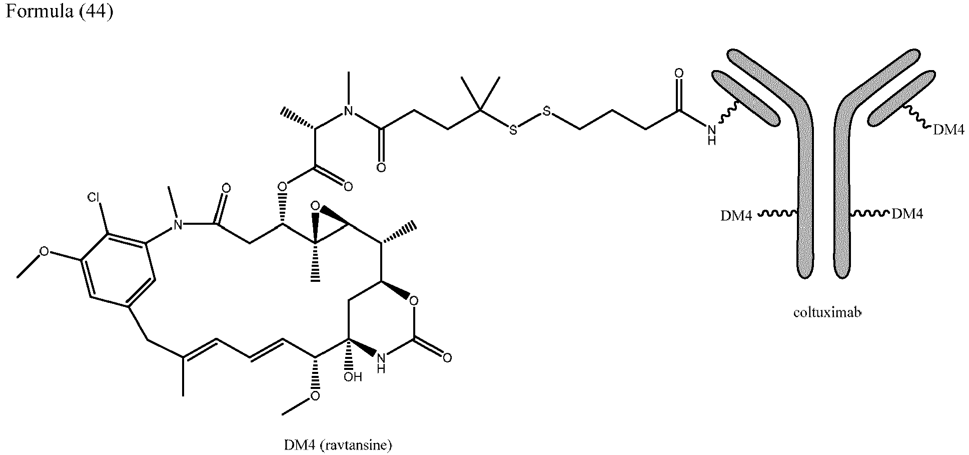 wherein the wavy line ( 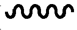 ) represents a SPDB linker, or antigen-binding fragments, conjugates, variants, or biosimilars thereof. In an embodiment, the CD 19 inhibitor of Formula (44) has a ravtansine (DM4) substitution, reported as a ravtansine to antibody ratio, of between 3 to 1 and 5 to 1. In an embodiment, the CD19 inhibitor of Formula (44) has a ravtansine (DM4) substitution, reported as a ravtansine:antibody ratio, of between 3.5 to 1 and 4.5 to 1. In an embodiment, the CD19 inhibitor of Formula (44) has an average ravtansine (DM4) substitution, reported as a ravtansine:antibody ratio, selected from the group consisting of 3 to 1, 3.2 to 1, 3.4 to 1, 3.6 to 1, 3.8 to 1, 4.0 to 1, 4.2 to 1, 4.4 to 1, 4.6 to 1, 4.8 to 1, and 5.0 to 1.

In an embodiment, the CD19 inhibitor includes a cleavable linker selected from the group consisting of: hydrazone and hydrazide moieties; disulfide containing linkers such as N-succinimidyl-4-(2-pyridyldithio)pentanoate (SPP) and N-succinimidyl-4-(2-pyridyldithio)butyrate (SPDB); 4-(4'-acetylphenoxy)butanoic acid (AcBut) linker; dipeptides valine-citrulline (Val-Cit) and phenylalanine-lysine (Phe-Lys) and non-cleavable linkers which include: amide moieties; SMCC (succinimidyl-4-[*N-*maleimidomethyl]cyclohexane-1-carboxylate) and maleimidocaproyl (mc) moieties.

In an embodiment, the CD19 inhibitor is an anti-CD19 biosimilar monoclonal antibody or conjugate thereof approved by drug regulatory authorities with reference to coltuximab or coltuximab ravtansine. In an embodiment, the CD19 inhibitor has a sequence identity of greater than 90% to SEQ ID NO:8. In an embodiment, the CD19 inhibitor has a sequence identity of greater than 90% to SEQ ID NO:9. In an embodiment, the CD19 inhibitor has a sequence identity of greater than 95% to SEQ ID NO:8. In an embodiment, the CD19 inhibitor has a sequence identity of greater than 95% to SEQ ID NO:9. In an embodiment, the CD19 inhibitor has a sequence identity of greater than 96% to SEQ ID NO:8. In an embodiment, the CD19 inhibitor has a sequence identity of greater than 96% to SEQ ID NO:9. In an embodiment, the CD19 inhibitor has a sequence identity of greater than 97% to SEQ ID NO:8. In an embodiment, the CD19 inhibitor has a sequence identity of greater than 97% to SEQ ID NO:9. In an embodiment, the CD19 inhibitor has a sequence identity of greater than 98% to SEQ ID NO:8. In an embodiment, the CD19 inhibitor has a sequence identity of greater than 98% to SEQ ID NO:9. In an embodiment, the CD19 inhibitor has a sequence identity of greater than 99% to SEQ ID NO: 8. In an embodiment, the CD19 inhibitor has a sequence identity of greater than 99% to SEQ ID NO:9.

In an embodiment, the CD19 inhibitor is an anti-CD19 monoclonal antibody comprising a V_{H} region comprising at least one CDR1 region comprising the amino acid sequence of SEQ ID NO:10; at least one CDR2 region comprising the amino acid sequence of SEQ ID NO: 11; and at least one CDR3 region comprising the amino acid sequence of SEQ ID NO:12; and wherein said V_{L} region comprises at least one CDR1 region comprising the amino acid sequence of SEQ ID NO:13; at least one CDR2 region comprising the amino acid sequence of SEQ ID NO:14; and at least one CDR3 region comprising the amino acid sequence of SEQ ID NO:15.

In an embodiment, the CD19 inhibitor is an anti-CD19 antibody described in U.S. Patent Application Publication Nos. US 2004/0235840 A1 and US 2014/0199300 A1 and U.S. Patent Nos. 7,276,497, 7,473,796, 7,851,432, 8,435,528, and 8,841,425.

In an embodiment, the CD19 inhibitor is denintuzumab, also known as huBU12, or an antigen-binding fragment, conjugate, variant, or biosimilar thereof. In an embodiment, the CD19 inhibitor is a denintuzumab conjugate. In an embodiment, the CD19 inhibitor is denintuzumab mafodotin, also known as SGN-19A, SGN-CD19A, and hBU12-491, or antigen-binding fragments, conjugates, variants, or biosimilars thereof. In an embodiment, the CD19 inhibitor comprises a monoclonal antibody having a heavy chain sequence of SEQ ID NO:17, a light chain sequence of SEQ ID NO: 18, disulfide bridges at the following positions: 22-97, 22"-97", 23'-87', 23‴-87‴, 133'-193', 133"'-193"', 147-203, 147"-203", 223-213', 223"-213"', 229-229", 232-232", 264-324, 264"-324", 370-428, and 370"-428" (where an average of two disulfide bridges are not present, giving an average of 4 cysteine linked to mafodotin), and N-glycosylation sites at Asn-300 and Asn-300", wherein the monoclonal antibody is conjugated to mafodotin via cysteine residues as shown in Formula (45):

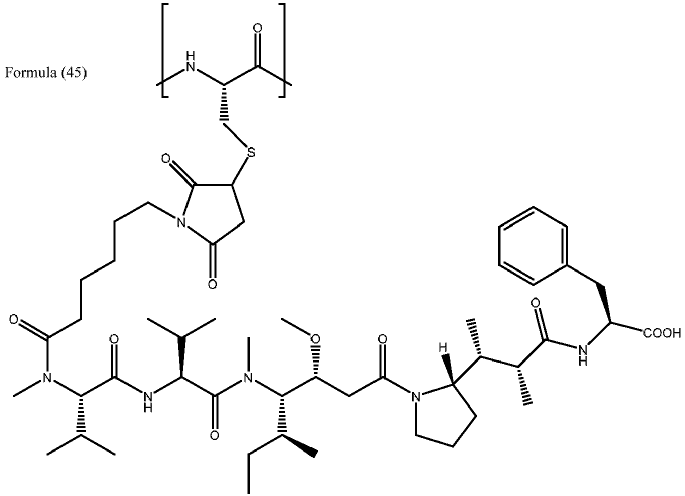

In an embodiment, the CD19 inhibitor is an immunoglobulin G1, anti-(human CD19 antigen) (human-Mus musculus monoclonal hBU12 heavy chain), disulfide with human-Mus musculus monoclonal hBU12 κ-chain, dimer, tetrakis(thioether) with N-[6-(3-mercapto-2,5-dioxo-1-pyrrolidinyl)-1-oxohexyl]-N-methyl-L-valyl-L-valyl-(3R,4S,5S)-3-methoxy-5-methyl-4-(methylamino)heptanoyl- (αR,βR,2S)-β-methoxy-α-methyl-2-pyrrolidinepropanoyl-L-phenylalanine. In an embodiment, the CD19 inhibitor is an immunoglobulin G1-kappa auristatin F conjugate, anti-[Homo sapiens CD19 (B lymphocyte surface antigen B4, Leu-12)], humanized monoclonal antibody; gamma1 heavy chain (1-450) [humanized VH (Homo sapiens IGHV4-31*02 (84.80%) -(IGHD)-IGHJ4*01) [10.7.12] (1-120) -Homo sapiens IGHG1*01 (CH1 (121-218), hinge (219-233), CH2 (234- 343), CH3 (344-448), CHS (449-450)) (121-450)], (223-213')-disulfide with kappa light chain (1'-213') [humanized V-KAPPA (Homo sapiens IGKV3-11*01 (85.30%) -IGKJ2*02) [5.3.9] (1'-106') - Homo sapiens IGKC*01 (107'-213')]; dimer (229-229":232-232")- bisdisulfide; conjugated, on an average of 4 cysteinyl, to monomethylauristatin F (MMAF), via a noncleavable maleimidocaproyl (mc) linker.

In an embodiment, the CD19 inhibitor is an anti-CD19 biosimilar monoclonal antibody or conjugate thereof approved by drug regulatory authorities with reference to denintuzumab or denintuzumab mafodotin. In an embodiment, the CD19 inhibitor has a sequence identity of greater than 90% to SEQ ID NO: 16. In an embodiment, the CD19 inhibitor has a sequence identity of greater than 90% to SEQ ID NO: 17. In an embodiment, the CD19 inhibitor has a sequence identity of greater than 95% to SEQ ID NO:16. In an embodiment, the CD19 inhibitor has a sequence identity of greater than 95% to SEQ ID NO: 17. In an embodiment, the CD19 inhibitor has a sequence identity of greater than 96% to SEQ ID NO: 16. In an embodiment, the CD19 inhibitor has a sequence identity of greater than 96% to SEQ ID NO: 17. In an embodiment, the CD19 inhibitor has a sequence identity of greater than 97% to SEQ ID NO:16. In an embodiment, the CD19 inhibitor has a sequence identity of greater than 97% to SEQ ID NO: 17. In an embodiment, the CD19 inhibitor has a sequence identity of greater than 98% to SEQ ID NO: 16. In an embodiment, the CD19 inhibitor has a sequence identity of greater than 98% to SEQ ID NO:17. In an embodiment, the CD19 inhibitor has a sequence identity of greater than 99% to SEQ ID NO: 16. In an embodiment, the CD19 inhibitor has a sequence identity of greater than 99% to SEQ ID NO: 17.

In an embodiment, the CD19 inhibitor comprises an anti-CD19 monoclonal antibody comprising a V_{H} region comprising at least one CDR1 region comprising the amino acid sequence of SEQ ID NO: 18; at least one CDR2 region comprising the amino acid sequence of SEQ ID NO: 19; and at least one CDR3 region comprising the amino acid sequence of SEQ ID NO:20; and wherein said V_{L} region comprises at least one CDR1 region comprising the amino acid sequence of SEQ ID NO:21; at least one CDR2 region comprising the amino acid sequence of SEQ ID NO:22; and at least one CDR3 region comprising the amino acid sequence of SEQ ID NO:23.

In an embodiment, the CD 19 inhibitor comprises an anti-CD 19 antibody construct described in U.S. Patent Application Publication No. US 2009/0136526 A1 and U.S. Patent No. 7,968,687.

In an embodiment, the CD19 inhibitor comprises a single-chain variable fragment (scFv) antibody construct, or an antigen-binding fragment, conjugate, variant, or biosimilar thereof. In an embodiment, the CD19 inhibitor comprises an anti-CD19 scFv antibody comprising a variable heavy (V_{H}) chain selected from the group consisting of SEQ ID NO:24 and SEQ ID NO:26. In an embodiment, the CD19 inhibitor comprises an anti-CD19 scFv antibody comprising a variable light (V_{L}) chain selected from the group consisting of SEQ ID NO:25 and SEQ ID NO:27. In an embodiment, the CD19 inhibitor comprises an anti-CD19 scFv antibody, the preparation of which is described, e.g., in U.S. Patent Application Publication No. 2014/0271635 A1. In an embodiment, the CD19 inhibitor comprises an anti-CD19 scFv antibody selected from the group consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, and SEQ ID NO:39. In an embodiment, the CD19 inhibitor comprises an anti-CD19 scFv antibody having a sequence identity of greater than 99% to an scFv antibody selected from the group consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, and SEQ ID NO:39. In an embodiment, the CD19 inhibitor comprises an anti-CD19 scFv antibody having a sequence identity of greater than 98% to an scFv antibody selected from the group consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, and SEQ ID NO:39.

In an embodiment, the CD19 inhibitor is selected from the group consisting of MDX-1342 (Bristol-Myers Squibb), inebilizumab (also known as MEDI-551, AstraZeneca), MOR-208 (Morphosys), DT2219ARL (Scott and White Hospital and Clinic), AFM11 (Affimed), GBR401 (Glenmark), CD19xCD3 dual affinity retargeting antibody (DART, Macrogeneics), and Combotox (Montefiore Medical Center). Additional examples of CD19 inhibitors comprising anti-CD19 antibodies are disclosed in U.S. Patent Nos. 7,919,576; 8,097,703; 8,242,252; 7,968,687; 9,073,993; 8,679,492; 8,840,888; 7,812,116; 8,524,867; 8,679,492; 8,323,653; 8,444,973; 7,575,923; 8,147,831; 8,883,992; 8,337,840; 8,097,703; 7,902,338; 8,486,395; 8,624,001; U.S. Patent Application Publication Nos. US 2009/0142349, US 2012/0294853, US 2009/0136526, US 2011/0312088, US 2009/0068235, US 2010/0272723, US 2012/0082664, US 2014/0086906, US 2008/0260731, US 2014/0112916, US 2010/0104509, US 2014/0286934, US 2010/0215651, US 2014/0271635, US 2015/0086575, US 2012/0121504, US 2012/0141505, US 2011/0165185, US 2012/0328618, US 2004/0136908, US 2005/0048071, US 2015/0071928, US 2013/0115657, US 2007/0166306, US 2014/0056896, US 2006/0233791, US 2010/0158901, US 2006/0148008, US 2011/0104150, US 2013/0084294, US 2006/0263357, US 2006/0280738, US 2009/0220501, US 2009/0246195, and US 2014/0199307; and PCT Patent Application Publication Nos. WO 2009/052431, WO 2003/055907, WO 2011/147834, WO 2010/095031, WO 2008/109493, WO 2010/132532, WO 2002/077029, WO 2002/080987, WO 2003/048209, WO 2007/082715, WO 2011/051307, and WO 2012/146394.

Non-limiting embodiments of anti-CD19 antibody amino acid sequences are summarized in Table 1.

**TABLE 1. Anti-CD19 antibody sequences.**

| **Identifier** | **Sequence (One-Letter Amino Acid Symbols)** | |
|---|---|---|
| SEQ ID NO:1 blinatumomab | DIQLTQSPAS LAVSLGQRAT ISCKASQSVD YDGDSYLNWY QQIPGQPPKL LIYDASNLVS 60 GIPPRFSGSG SGTDFTLNIH PVEKVDAATY HCQQSTEDPW TFGGGTKLEI KGGGGSGGGG 120 SGGGGSQVQL QQSGAELVRP GSSVKISCKA SGYAFSSYWM NWVKQRPGQG LEWIGQIWPG 180 DGDTNYNGKF KGKATLTADE SSSTAYMQLS SLASEDSAVY FCARRETTTV GRYYYAMDYW 240 GQGTTVTVSS GGGGSDIKLQ QSGAELARPG ASVKMSCKTS GYTFTRYTMH WVKQRPGQGL 300 EWIGYINPSR GYTNYNQKFK DKATLTTDKS SSTAYMQLSS LTSEDSAVYY CARYYDDHYC 360 LDYWGQGTTL TVSSVEGGSG GSGGSGGSGG VDDIQLTQSP AIMSASPGEK VTMTCRASSS 420 VSYMNWYQQK SGTSPKRWIY DTSKVASGVP YRFSGSGSGT SYSLTISSME AEDAATYYCQ 480 QWSSNPLTFG AGTKLELKHH HHHH 504 | |
| SEQ ID NO:2 blinatumomab variable heavy chain CDR1 | GYTFTRYTMH | 10 |
| SEQ ID NO:3 blinatumomab variable heavy chain CDR2 | YINPSRGYTN YNQKVK | 16 |
| SEQ ID NO:4 blinatumomab variable heavy chain CDR3 | YYDDHYCLDY | 10 |
| SEQ ID NO:5 blinatumomab variable light chain CDR1 | RASSSVSYMN | 10 |
| SEQ ID NO:6 blinatumomab variable light chain CDR2 | DTSKVAS | 7 |
| SEQ ID NO:7 blinatumomab variable light chain CDR3 | QQWSSNPLT | 9 |
| SEQ ID NO:8 coltuximab heavy chain | QVQLVQPGAE VVKPGASVKL SCKTSGYTFT SNWMHWVKQA PGQGLEWIGE IDPSDSYTNY 60 NQNFQGKAKL TVDKSTSTAY MEVSSLRSDD TAVYYCARGS NPYYYAMDYW GQGTSVTVSS 120 ASTKGPSVFP LAPSSKSTSG GTAALGCLVK DYFPEPVTVS WNSGALTSGV HTFPAVLQSS 180 GLYSLSSVVT VPSSSLGTQT YICNVNHKPS NTKVDKKVEP KSCDKTHTCP PCPAPELLGG 240 PSVFLFPPKP KDTLMISRTP EVTCVVVDVS HEDPEVKFNW YVDGVEVHNA KTKPREEQYN 300 STYRVVSVLT VLHQDWLNGK EYKCKVSNKA LPAPIEKTIS KAKGQPREPQ VYTLPPSRDE 360 LTKNQVSLTC LVKGFYPSDI AVEWESNGQP ENNYKTTPPV LDSDGSFFLY SKLTVDKSRW 420 QQGNVFSCSV MHEALHNHYT QKSLSLSPGK 450 | |
| SEQ ID NO:9 coltuximab light chain | EIVLTQSPAI MSASPGERVT MTCSASSGVN YMHWYQQKPG TSPRRWIYDT SKLASGVPAR 60 FSGSGSGTDY SLTISSMEPE DAATYYCHQR GSYTFGGGTK LEIKRTVAAP SVFIFPPSDE 120 QLKSGTASVV CLLNNFYPRE AKVQWKVDNA LQSGNSQESV TEQDSKDSTY SLSSTLTLSK 180 ADYEKHKVYA CEVTHQGLSS PVTKSFNRGE C 211 | |
| SEQ ID NO:10 coltuximab variable heavy chain CDR1 | SNWMH | 5 |
| SEQ ID NO:11 coltuximab variable heavy chain CDR2 | EIDPSDSYTN | 10 |
| SEQ ID NO:12 coltuximab variable heavy chain CDR3 | GSNPYYYAMD Y | 11 |
| SEQ ID NO:13 coltuximab variable light chain CDR1 | SASSGVNYMH | 10 |
| SEQ ID NO:14 coltuximab variable light chain CDR2 | DTSKLAS | 7 |
| SEQ ID NO:15 coltuximab variable light chain CDR3 | HQRGSYT | 7 |
| SEQ ID NO:16 denintuzumab heavy chain | QVQLQESGPG LVKPSQTLSL TCTVSGGSIS TSGMGVGWIR QHPGKGLEWI GHIWWDDDKR 60 YNPALKSRVT ISVDTSKNQF SLKLSSVTAA DTAVYYCARM ELWSYYFDYW GQGTLVTVSS 120 ASTKGPSVFP LAPSSKSTSG GTAALGCLVK DYFPEPVTVS WNSGALTSGV HTFPAVLQSS 180 GLYSLSSVVT VPSSSLGTQT YICNVNHKPS NTKVDKKVEP KSCDKTHTCP PCPAPELLGG 240 PSVFLFPPKP KDTLMISRTP EVTCVVVDVS HEDPEVKFNW YVDGVEVHNA KTKPREEQYN 300 STYRVVSVLT VLHQDWLNGK EYKCKVSNKA LPAPIEKTIS KAKGQPREPQ VYTLPPSRDE 360 LTKNQVSLTC LVKGFYPSDI AVEWESNGQP ENNYKTTPPV LDSDGSFFLY SKLTVDKSRW 420 QQGNVFSCSV MHEALHNHYT QKSLSLSPGK 450 | |
| SEQ ID NO:17 denintuzumab light chain | EIVLTQSPAT LSLSPGERAT LSCSASSSVS YMHWYQQKPG QAPRLLIYDT SKLASGIPAR 60 FSGSGSGTDF TLTISSLEPE DVAVYYCFQG SVYPFTFGQG TKLEIKRTVA APSVFIFPPS 120 DEQLKSGTAS VVCLLNNFYP REAKVQWKVD NALQSGNSQE SVTEQDSKDS TYSLSSTLTL 180 SKADYEKHKV YACEVTHQGL SSPVTKSFNR GEC 213 | |
| SEQ ID NO:18 denintuzumab variable heavy chain CDR1 | TSGMGVG | 7 |
| SEQ ID NO:19 denintuzumab variable heavy chain CDR2 | HIWWDDDKRY NPALKS | 16 |
| SEQ ID NO:20 denintuzumab variable heavy chain CDR3 | MELWSYYFDY | 10 |
| SEQ ID NO:21 denintuzumab variable light chain CDR1 | SASSSVSYMH | 10 |
| SEQ ID NO:22 denintuzumab variable light chain CDR2 | DTSKLAS | 7 |
| SEQ ID NO:23 denintuzumab variable light chain CDR3 | FQGSVYPFT | 9 |
| SEQ ID NO:24 FMC63 scFv variable heavy chain (V_{H}) domain | DIQMTQTTSS LSASLGDRVT ISCRASQDIS KYLNWYQQKP DGTVKLLIYH TSRLHSGVPS 60 RFSGSGSGTD YSLTISNLEQ EDIATYFCQQ GNTLPYTFGG GTKLEIT 107 | |
| SEQ ID NO:25 FMC63 scFv variable light chain (V_{L}) domain | EVKLQESGPG LVAPSQSLSV TCTVSGVSLP DYGVSWIRQP PRKGLEWLGV IWGSETTYYN 60 SALKSRLTII KDNSKSQVFL KMNSLQTDDT AIYYCAKHYY YGGSYAMDYW GQGTSVTVSS 120 | |
| SEQ ID NO:26 SJ25C1 scFv variable heavy chain (V_{H}) domain | LESGAELVRP GSSVKISCKA SGYAFSSYWM NWVKQRPGQG LEWIGQIYPG DGDTNYNGKF 60 KGQATLTADK SSSTAYMQLS GLTSEDSAVY SCARKTISSV VDFYFDNWGQ GTTVT 115 | |
| SEQ ID NO:27 SJ25C1 scFv variable light chain (V_{L}) domain | ELVLTQSPKF MSTSVGDRVS VTCKASQNVG TNVAWYQQKP GQSPKPLIYS ATYRNSGVPD 60 RFTGSGSGTD FTLTITNVQS KDLADYFYFC QYNRYPYTSG GGTKLEIKRR S 111 | |
| SEQ ID NO:28 humanized scFv domain | EIVMTQSPAT LSLSPGERAT LSCRASQDIS KYLNWYQQKP GQAPRLLIYH TSRLHSGIPA 60 RFSGSGSGTD YTLTISSLQP EDFAVYFCQQ GNTLPYTFGQ GTKLEIKGGG GSGGGGSGGG 120 GSQVQLQESG PGLVKPSETL SLTCTVSGVS LPDYGVSWIR QPPGKGLEWI GVIWGSETTY 180 | |
| | YSSSLKSRVT ISKDNSKNQV SLKLSSVTAA DTAVYYCAKH YYYGGSYAMD YWGQGTLVTV 240 SS 242 | |
| SEQ ID NO:29 humanized scFv domain | EIVMTQSPAT LSLSPGERAT LSCRASQDIS KYLNWYQQKP GQAPRLLIYH TSRLHSGIPA 60 RFSGSGSGTD YTLTISSLQP EDFAVYFCQQ GNTLPYTFGQ GTKLEIKGGG GSGGGGSGGG 120 GSQVQLQESG PGLVKPSETL SLTCTVSGVS LPDYGVSWIR QPPGKGLEWI GVIWGSETTY 180 YQSSLKSRVT ISKDNSKNQV SLKLSSVTAA DTAVYYCAKH YYYGGSYAMD YWGQGTLVTV 240 SS 242 | |
| SEQ ID NO:30 humanized scFv domain | QVQLQESGPG LVKPSETLSL TCTVSGVSLP DYGVSWIRQP PGKGLEWIGV IWGSETTYYS 60 SSLKSRVTIS KDNSKNQVSL KLSSVTAADT AVYYCAKHYY YGGSYAMDYW GQGTLVTVSS 120 GGGGSGGGGS GGGGSEIVMT QSPATLSLSP GERATLSCRA SQDISKYLNW YQQKPGQAPR 180 LLIYHTSRLH SGIPARFSGS GSGTDYTLTI SSLQPEDFAV YFCQQGNTLP YTFGQGTKLE 240 IK 242 | |
| SEQ ID NO:31 humanized scFv domain | QVQLQESGPG LVKPSETLSL TCTVSGVSLP DYGVSWIRQP PGKGLEWIGV IWGSETTYYQ 60 SSLKSRVTIS KDNSKNQVSL KLSSVTAADT AVYYCAKHYY YGGSYAMDYW GQGTLVTVSS 120 GGGGSGGGGS GGGGSEIVMT QSPATLSLSP GERATLSCRA SQDISKYLNW YQQKPGQAPR 180 LLIYHTSRLH SGIPARFSGS GSGTDYTLTI SSLQPEDFAV YFCQQGNTLP YTFGQGTKLE 240 IK 242 | |
| SEQ ID NO:32 humanized scFv domain | EIVMTQSPAT LSLSPGERAT LSCRASQDIS KYLNWYQQKP GQAPRLLIYH TSRLHSGIPA 60 RFSGSGSGTD YTLTISSLQP EDFAVYFCQQ GNTLPYTFGQ GTKLEIKGGG GSGGGGSGGG 120 GSGGGGSQVQ LQESGPGLVK PSETLSLTCT VSGVSLPDYG VSWIRQPPGK GLEWIGVIWG 180 SETTYYSSSL KSRVTISKDN SKNQVSLKLS SVTAADTAVY YCAKHYYYGG SYAMDYWGQG 240 TLVTVSS 247 | |
| SEQ ID NO:33 humanized scFv domain | EIVMTQSPAT LSLSPGERAT LSCRASQDIS KYLNWYQQKP GQAPRLLIYH TSRLHSGIPA 60 RFSGSGSGTD YTLTISSLQP EDFAVYFCQQ GNTLPYTFGQ GTKLEIKGGG GSGGGGSGGG 120 GSGGGGSQVQ LQESGPGLVK PSETLSLTCT VSGVSLPDYG VSWIRQPPGK GLEWIGVIWG 180 SETTYYQSSL KSRVTISKDN SKNQVSLKLS SVTAADTAVY YCAKHYYYGG SYAMDYWGQG 240 TLVTVSS 247 | |
| SEQ ID NO:34 humanized scFv domain | QVQLQESGPG LVKPSETLSL TCTVSGVSLP DYGVSWIRQP PGKGLEWIGV IWGSETTYYS 60 SSLKSRVTIS KDNSKNQVSL KLSSVTAADT AVYYCAKHYY YGGSYAMDYW GQGTLVTVSS 120 GGGGSGGGGS GGGGSGGGGS EIVMTQSPAT LSLSPGERAT LSCRASQDIS KYLNWYQQKP 180 GQAPRLLIYH TSRLHSGIPA RFSGSGSGTD YTLTISSLQP EDFAVYFCQQ GNTLPYTFGQ 240 GTKLEIK 247 | |
| SEQ ID NO:35 humanized scFv domain | QVQLQESGPG LVKPSETLSL TCTVSGVSLP DYGVSWIRQP PGKGLEWIGV IWGSETTYYQ 60 SSLKSRVTIS KDNSKNQVSL KLSSVTAADT AVYYCAKHYY YGGSYAMDYW GQGTLVTVSS 120 GGGGSGGGGS GGGGSGGGGS EIVMTQSPAT LSLSPGERAT LSCRASQDIS KYLNWYQQKP 180 GQAPRLLIYH TSRLHSGIPA RFSGSGSGTD YTLTISSLQP EDFAVYFCQQ GNTLPYTFGQ 240 GTKLEIK 247 | |
| SEQ ID NO:36 humanized scFv domain | EIVMTQSPAT LSLSPGERAT LSCRASQDIS KYLNWYQQKP GQAPRLLIYH TSRLHSGIPA 60 RFSGSGSGTD YTLTISSLQP EDFAVYFCQQ GNTLPYTFGQ GTKLEIKGGG GSGGGGSGGG 120 GSGGGGSQVQ LQESGPGLVK PSETLSLTCT VSGVSLPDYG VSWIRQPPGK GLEWIGVIWG 180 SETTYYNSSL KSRVTISKDN SKNQVSLKLS SVTAADTAVY YCAKHYYYGG SYAMDYWGQG 240 TLVTVSS 247 | |
| SEQ ID NO:37 humanized scFv domain | QVQLQESGPG LVKPSETLSL TCTVSGVSLP DYGVSWIRQP PGKGLEWIGV IWGSETTYYN 60 SSLKSRVTIS KDNSKNQVSL KLSSVTAADT AVYYCAKHYY YGGSYAMDYW GQGTLVTVSS 120 GGGGSGGGGS GGGGSGGGGS EIVMTQSPAT LSLSPGERAT LSCRASQDIS KYLNWYQQKP 180 GQAPRLLIYH TSRLHSGIPA RFSGSGSGTD YTLTISSLQP EDFAVYFCQQ GNTLPYTFGQ 240 GTKLEIK 247 | |
| SEQ ID NO:38 humanized scFv domain | EIVMTQSPAT LSLSPGERAT LSCRASQDIS KYLNWYQQKP GQAPRLLIYH TSRLHSGIPA 60 RFSGSGSGTD YTLTISSLQP EDFAVYFCQQ GNTLPYTFGQ GTKLEIKGGG GSGGGGSGGG 120 GSQVQLQESG PGLVKPSETL SLTCTVSGVS LPDYGVSWIR QPPGKGLEWI GVIWGSETTY 180 YNSSLKSRVT ISKDNSKNQV SLKLSSVTAA DTAVYYCAKH YYYGGSYAMD YWGQGTLVTV 240 SS 242 | |
| SEQ ID NO:39 humanized scFv domain | QVQLQESGPG LVKPSETLSL TCTVSGVSLP DYGVSWIRQP PGKGLEWIGV IWGSETTYYN 60 SSLKSRVTIS KDNSKNQVSL KLSSVTAADT AVYYCAKHYY YGGSYAMDYW GQGTLVTVSS 120 GGGGSGGGGS GGGGSEIVMT QSPATLSLSP GERATLSCRA SQDISKYLNW YQQKPGQAPR 180 LLIYHTSRLH SGIPARFSGS GSGTDYTLTI SSLQPEDFAV YFCQQGNTLP YTFGQGTKLE 240 IK 242 | |

In an embodiment, the CD19 inhibitor is an anti-CD19 chimeric antigen receptor. In an embodiment, the CD19 inhibitor is a cell modified to express an anti-CD19 chimeric antigen receptor. In an embodiment, the CD19 inhibitor is a T cell modified to express an anti-CD19 chimeric antigen receptor (CAR-T). In an embodiment, the CD19 inhibitor is a CD8⁺ T cell modified to express an anti-CD19 chimeric antigen receptor (CAR-T). In an embodiment, the CD19 inhibitor is a natural killer (NK) cell modified to express an anti-CD19 chimeric antigen receptor (CAR-NK). In an embodiment, the chimeric antigen receptor includes an intracellular signaling domain, a transmembrane domain, and an anti-CD19 extracellular domain. In an embodiment, the chimeric antigen receptor includes an intracellular signaling domain, a transmembrane domain, and an anti-CD19 extracellular domain wherein the heavy variable chain is selected from the group consisting of SEQ ID NO:24 and SEQ ID NO:26 and the light variable chain is selected from the group consisting of SEQ ID NO:25 and SEQ ID NO:27. In an embodiment, the chimeric antigen receptor includes an intracellular signaling domain, a transmembrane domain, and an anti-CD19 extracellular domain selected from the group consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, and SEQ ID NO:39. In an embodiment, the chimeric antigen receptor includes an intracellular signaling domain, a transmembrane domain, and an anti-CD19 extracellular domain, wherein the anti-CD19 extracellular domain has a sequence identity of greater than 99% to a sequence selected from the group consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, and SEQ ID NO:39. In an embodiment, the chimeric antigen receptor includes an intracellular signaling domain, a transmembrane domain, and an anti-CD19 extracellular domain, wherein the anti-CD19 extracellular domain has a sequence identity of greater than 98% to a sequence selected from the group consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, and SEQ ID NO:39. In an embodiment, the chimeric antigen receptor includes an intracellular signaling domain, a transmembrane domain, and an anti-CD19 extracellular domain, wherein the anti-CD19 extracellular domain has a sequence identity of greater than 97% to a sequence selected from the group consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, and SEQ ID NO:39. In an embodiment, the chimeric antigen receptor includes an intracellular signaling domain, a transmembrane domain, and an anti-CD19 extracellular domain, wherein the anti-CD19 extracellular domain has a sequence identity of greater than 96% to a sequence selected from the group consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, and SEQ ID NO:39. In an embodiment, the chimeric antigen receptor includes an intracellular signaling domain, a transmembrane domain, and an anti-CD19 extracellular domain, wherein the anti-CD19 extracellular domain has a sequence identity of greater than 95% to a sequence selected from the group consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, and SEQ ID NO:39. In an embodiment, the chimeric antigen receptor includes an intracellular signaling domain, a transmembrane domain, and an anti-CD19 extracellular domain wherein the anti-CD19 extracellular domain is a Fab.

In an embodiment, anti-CD19 chimeric antigen receptors may be prepared by synthesis of polynucleotides translated from any of the sequences provided herein, followed by insertion of the polynucleotide into a viral vector to transduce cells that produce virus for transfection of isolated human T cells or NK cells, which may be expanded and subsequently adoptively transferred back into the human to treat a disease. In an embodiment, a lentiviral vector is used to transduce cells that produce virus for transfection of isolated human T cells or NK cells, which may be subsequently adoptively transferred back into the human to treat a disease. Suitable lentiviral transduction methods for T cells and NK cells are described in U.S. Patent No. 6,627,442. Lentiviral transduction is generally described in Dull, et al., J. Virology 1998, 72, 8463-71, and Zufferey, et al., Nature Biotechnol. 1997, 15, 871-75. In an embodiment, a gammaretroviral vector is used to transduce cells that produce virus for transfection of isolated human T cells or NK cells, which may be subsequently adoptively transferred back into the human to treat a disease. In an embodiment, ribonucleic acid (RNA) electroporation is used to transduce isolated human T cells or NK cells, which may be subsequently adoptively transferred back into the human to treat a disease. Suitable RNA electroporation methods are described in U.S. Patent Application Publication No. US 2014/0227237 A1. RNA electroporation methods suitable for use with CARs are generally described in Zhao, et al., Cancer Res. 2010, 70, 9053-61. In an embodiment, transposon based methods such as the Sleeping Beauty (SB) system are used to transduce isolated human T cells or NK cells, which may be subsequently adoptively transferred back into the human to treat a disease. Suitable SB transposon methods are described in International Patent Application Publication No. WO 2014/186469 A2. SB transposon systems suitable for use with CARs are generally described in Hackett, et al., Mol. Therapy 2010, 18, 674-83.

In an embodiment, the adoptively transferred cells are autologous. In an embodiment, the adoptively transferred cells are allogeneic. The preparation of allogeneic cells is described in US 2014/0349402 A1.

In an embodiment, the transfected T cells or NK cells are expanded prior to adoptive transfer to a human. Suitable expansion methods for T cells and NK cells are described in U.S. Patent Nos. 5,858,358; 7,977,095; 7,435,596; and 8,026,097; and U.S. Patent Application Publication No. US 2015/0225697 A1. In an embodiment, the invention provides a method of treating a hyperproliferative disease in a patient, comprising co-administering to a mammal in need thereof therapeutically effective amounts of (1) a plurality of T cells or NK cells modified to express an anti-CD19 chimeric antigen receptor (CAR), wherein the plurality of T cells or NK cells is first obtained from the patient by apheresis, the plurality of T cells or NK cells is modified to express the CAR by viral transfection, the plurality of T cells or NK cells is expanded and activated by a cell expansion method, and the plurality of T cells or NK cells is adoptively transferred (e.g., by injection or transplantation) into the patient, and (2) a Bruton's tyrosine kinase (BTK) inhibitor of Formula (II) or a pharmaceutically acceptable salt thereof.

The preparation of anti-CD19 chimeric antigen receptors suitable for use with the presently discloses methods and compositions is described in U.S. Patent Nos. 7,446,179 and 7,446,190, and U.S. Patent Application Publication Nos. US 2004/0126363 A1, US 2015/0038684 A1, US 2013/0287748 A1, and US 2013/0288368 A1.

In an embodiment, the anti-CD19 chimeric antigen receptor includes one or more signaling domains selected from the group consisting of a CD3ζ signaling domain, a CD28 signaling domain, a 4-1BB (CD137) signaling domain, a CD27 signaling domain, an ICOS signaling domain, an OX40 signaling domain, an FcyRIII signaling domain, and an FcεRI signaling domain. In an embodiment, the anti-CD19 chimeric antigen receptor includes one or more signaling domains selected from the group consisting of SEQ ID NO:40, SEQ ID NO:41, and SEQ ID NO:42. In an embodiment, the anti-CD19 chimeric antigen receptor includes one or more signaling domains with a sequence identity of greater than 99% to a sequence selected from the group consisting of SEQ ID NO:40, SEQ ID NO:41, and SEQ ID NO:42. In an embodiment, the anti-CD19 chimeric antigen receptor includes one or more signaling domains with a sequence identity of greater than 98% to a sequence selected from the group consisting of SEQ ID NO:40, SEQ ID NO:41, and SEQ ID NO:42. In an embodiment, the anti-CD19 chimeric antigen receptor includes one or more signaling domains with a sequence identity of greater than 97% to a sequence selected from the group consisting of SEQ ID NO:40, SEQ ID NO:41, and SEQ ID NO:42. In an embodiment, the anti-CD19 chimeric antigen receptor includes one or more signaling domains with a sequence identity of greater than 96% to a sequence selected from the group consisting of SEQ ID NO:40, SEQ ID NO:41, and SEQ ID NO:42. In an embodiment, the anti-CD19 chimeric antigen receptor includes one or more signaling domains with a sequence identity of greater than 95% to a sequence selected from the group consisting of SEQ ID NO:40, SEQ ID NO:41, and SEQ ID NO:42. Several non-limiting signaling domains for use with chimeric antigen receptors are given in Table 2. Signaling domains suitable for use with CD19 chimeric antigen receptors are described in U.S. Patent Nos. 6,410,319; 7,446,190; 7,070,995 and 7,265,209; and U.S. Patent Application Publication Nos. US 2014/0219975 A1, US 2014/0322275 A1, US 2015/0031624 A1, and US 2014/0301993.

In an embodiment, the anti-CD19 chimeric antigen receptor includes a transmembrane domain. In an embodiment, the anti-CD19 chimeric antigen receptor includes a hinge domain. Suitable, non-limiting transmembrane and hinge domains for use with anti-CD19 chimeric antigen receptors are given in SEQ ID NO:43 and SEQ ID NO:44 in Table 2. Other suitable transmembrane and hinge domains for use with CD19 chimeric antigen receptors are described below in the examples of chimeric antigen receptors, and also in U.S. Patent Nos. 7,446,179 and 7,446,190, and U.S. Patent Application Publication Nos. US 2004/0126363 A1, US 2015/0038684 A1, US 2013/0287748 A1, and US 2013/0288368 A1.

**TABLE 2. Signaling, transmembrane, and hinge domains for use with anti-CD19 CARs.**

| **Identifier** | **Sequence (One-Letter Amino Acid Symbols)** | |
|---|---|---|
| SEQ ID NO:40 CD3ζ signaling domain | RVKFSRSADA PAYQQGQNQL YNELNLGRRE EYDVLDKRRG RDPEMGGKPR RKNPQEGLYN 60 ELQKDKMAEA YSEIGMKGER RRGKGHDGLY QGLSTATKDT YDALHMQALP PR 112 | |
| SEQ ID NO:41 CD28 signaling domain | RSKRSRLLHS DYMNMTPRRP GPTRKHYQPY APPRDFAAYR S | 41 |
| SEQ ID NO:42 4-1BB (CD137) signaling domain | KRGRKKLLYI FKQPFMRPVQ TTQEEDGCSC RFPEEEEGGC EL | 42 |
| SEQ ID NO:43 CD8α transmembrane domain | IYIWAPLAGT CGVLLLSLVI TLYC | 24 |
| SEQ ID NO:44 CD8α hinge domain | TTTPAPRPPT PAPTIASQPL SLRPEACRPA AGGAVHTRGL DFACD | 45 |

In a preferred embodiment, the CD19 inhibitor is an anti-CD19 chimeric antigen receptor selected from the group consisting of an anti-CD19:transmembrane: CD3ζ chimeric antigen receptor, an anti-CD19:transmembrane:4-1BB:CD3ζ chimeric antigen receptor, an anti-CD19:transmembrane:CD28:CD3ζ chimeric antigen receptor, an anti-CD19:transmembrane:4-1BB:CD28:CD3ζ chimeric antigen receptor, an anti-CD19:transmembrane:CD28:4-1BB:CD3ζ chimeric antigen receptor, an anti-CD19:transmembrane:CD27 chimeric antigen receptor, an anti-CD19:transmembrane:CD27:CD3ζ chimeric antigen receptor, an anti-CD19:transmembrane:OX40 chimeric antigen receptor, and an anti-CD19:transmembrane: OX40: CD3ζ chimeric antigen receptor. In an embodiment, the anti-CD19 chimeric antigen receptor further comprises a "suicide switch" domain that allows for cells transduced with the receptor to be controlled through, *e.g*., administration of a compound to a subject to activate the switch. In an embodiment, the suicide switch is a caspase 9 suicide switch.

In an embodiment, the CD 19 inhibitor is an anti-CD19 chimeric antigen receptor selected from the group consisting of SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, and SEQ ID NO:59. Other suitable anti-CD19 CARs are described in U.S. Patent Nos. 7,446,179 and 7,446,190, and U.S. Patent Application Publication Nos. US 2004/0126363 A1, US 2015/0038684 A1, US 2013/0287748 A1, and US 2013/0288368 A1. Suitable anti-CD19 chimeric antigen receptor amino acid sequences are given in Table 3.

**TABLE 3. Anti-CD19 chimeric antigen receptor sequences.**

| **Identifier** | **Sequence (One-Letter Amino Acid Symbols)** | |
|---|---|---|
| SEQ ID NO:45 murine anti-CD19 chimeric antigen receptor (anti-CD19:CD8α:4-1BB:CD3ζ) | MALPVTALLL PLALLLHAAR PDIQMTQTTS SLSASLGDRV TISCRASQDI SKYLNWYQQK 60 PDGTVKLLIY HTSRLHSGVP SRFSGSGSGT DYSLTISNLE QEDIATYFCQ QGNTLPYTFG 120 GGTKLEITGG GGSGGGGSGG GGSEVKLQES GPGLVAPSQS LSVTCTVSGV SLPDYGVSWI 180 RQPPRKGLEW LGVIWGSETT YYNSALKSRL TIIKDNSKSQ VFLKMNSLQT DDTAIYYCAK 240 HYYYGGSYAM DYWGQGTSVT VSSTTTPAPR PPTPAPTIAS QPLSLRPEAC RPAAGGAVHT 300 RGLDFACDIY IWAPLAGTCG VLLLSLVITL YCKRGRKKLL YIFKQPFMRP VQTTQEEDGC 360 SCRFPEEEEG GCELRVKFSR SADAPAYQQG QNQLYNELNL GRREEYDVLD KRRGRDPEMG 420 GKPRRKNPQE GLYNELQKDK MAEAYSEIGM KGERRRGKGH DGLYQGLSTA TKDTYDALHM 480 QALPPR 486 | |
| SEQ ID NO:46 anti-CD19 chimeric antigen receptor (anti-CD19: IgG1Fc:CD3ζ) | MLLLVTSLLL CELPHPAFLL IPDIQMTQTT SSLSASLGDR VTISCRASQD ISKYLNWYQQ 60 KPDGTVKLLI YHTSRLHSGV PSRFSGSGSG TDYSLTISNL EQEDIATYFC QQGNTLPYTF 120 GGGTKLEITG STSGSGKPGS GEGSTKGEVK LQESGPGLVA PSQSLSVTCT VSGVSLPDYG 180 VSWIRQPPRK GLEWLGVIWG SETTYYNSAL KSRLTIIKDN SKSQVFLKMN SLQTDDTAIY 240 YCAKHYYYGG SYAMDYWGQG TSVTVSSVEP KSSDKTHTCP PCPAPELLGG PSVFLFPPKP 300 KDTLMISRTP EVTCVVVDVS HEDPEVKFNW YVDGVEVHNA KTKPREEQYN STYRVVSVLT 360 VLHQDWLNGK EYKCKVSNKA LPAPIEKTIS KAKGQPREPQ VYTLPPSRDE LTKNQVSLTC 420 LVKGFYPSDI AVEWESNGQP ENNYKTTPPV LDSDGSFFLY SKLTVDKSRW QQGNVFSCSV 480 MHEALHNHYT QKSLSLSPGK MALIVLGGVA GLLLFIGLGI FFRVKFSRSA DAPAYQQGQN 540 QLYNELNLGR REEYDVLDKR RGRDPEMGGK PRRKNPQEGL YNELQKDKMA EAYSEIGMKG 600 ERRRGKGHDG LYQGLSTATK DTYDALHMQA LPPR 634 | |
| SEQ ID NO:47 anti-CD19 chimeric antigen receptor (anti-CD19ScFv:IgG4hinge: CD28tm:41BB:CD3Zeta: T2A:EGFRt) | MLLLVTSLLL CELPHPAFLL IPDIQMTQTT SSLSASLGDR VTISCRASQD ISKYLNWYQQ 60 KPDGTVKLLI YHTSRLHSGV PSRFSGSGSG TDYSLTISNL EQEDIATYFC QQGNTLPYTF 120 GGGTKLEITG STSGSGKPGS GEGSTKGEVK LQESGPGLVA PSQSLSVTCT VSGVSLPDYG 180 VSWIRQPPRK GLEWLGVIWG SETTYYNSAL KSRLTIIKDN SKSQVFLKMN SLQTDDTAIY 240 YCAKHYYYGG SYAMDYWGQG TSVTVSSESK YGPPCPPCPM FWVLVVVGGV LACYSLLVTV 300 AFIIFWVKRG RKKLLYIFKQ PFMRPVQTTQ EEDGCSCRFP EEEEGGCELR VKFSRSADAP 360 AYQQGQNQLY NELNLGRREE YDVLDKRRGR DPEMGGKPRR KNPQEGLYNE LQKDKMAEAY 420 SEIGMKGERR RGKGHDGLYQ GLSTATKDTY DALHMQALPP RLEGGGEGRG SLLTCGDVEE 480 NPGPRMLLLV TSLLLCELPH PAFLLIPRKV CNGIGIGEFK DSLSINATNI KHFKNCTSIS 540 GDLHILPVAF RGDSFTHTPP LDPQELDILK TVKEITGFLL IQAWPENRTD LHAFENLEII 600 RGRTKQHGQF SLAVVSLNIT SLGLRSLKEI SDGDVIISGN KNLCYANTIN WKKLFGTSGQ 660 KTKIISNRGE NSCKATGQVC HALCSPEGCW GPEPRDCVSC RNVSRGRECV DKCNLLEGEP 720 REFVENSECI QCHPECLPQA MNITCTGRGP DNCIQCAHYI DGPHCVKTCP AGVMGENNTL 780 VWKYADAGHV CHLCHPNCTY GCTGPGLEGC PTNGPKIPSI ATGMVGALLL LLVVALGIGL 840 FM 842 | |
| SEQ ID NO:48 humanized anti-CD19 chimeric antigen receptor (anti-CD19:CD8α:4-1BB:CD3ζ) | MALPVTALLL PLALLLHAAR PEIVMTQSPA TLSLSPGERA TLSCRASQDI SKYLNWYQQK 60 PGQAPRLLIY HTSRLHSGIP ARFSGSGSGT DYTLTISSLQ PEDFAVYFCQ QGNTLPYTFG 120 QGTKLEIKGG GGSGGGGSGG GGSQVQLQES GPGLVKPSET LSLTCTVSGV SLPDYGVSWI 180 RQPPGKGLEW IGVIWGSETT YYSSSLKSRV TISKDNSKNQ VSLKLSSVTA ADTAVYYCAK 240 HYYYGGSYAM DYWGQGTLVT VSSTTTPAPR PPTPAPTIAS QPLSLRPEAC RPAAGGAVHT 300 RGLDFACDIY IWAPLAGTCG VLLLSLVITL YCKRGRKKLL YIFKQPFMRP VQTTQEEDGC 360 SCRFPEEEEG GCELRVKFSR SADAPAYKQG QNQLYNELNL GRREEYDVLD KRRGRDPEMG 420 GKPRRKNPQE GLYNELQKDK MAEAYSEIGM KGERRRGKGH DGLYQGLSTA TKDTYDALHM 480 QALPPR 486 | |
| SEQ ID NO:49 humanized anti-CD19 chimeric antigen receptor (anti-CD19:CD8α:4-1BB:CD3ζ) | MALPVTALLL PLALLLHAAR PEIVMTQSPA TLSLSPGERA TLSCRASQDI SKYLNWYQQK 60 PGQAPRLLIY HTSRLHSGIP ARFSGSGSGT DYTLTISSLQ PEDFAVYFCQ QGNTLPYTFG 120 QGTKLEIKGG GGSGGGGSGG GGSQVQLQES GPGLVKPSET LSLTCTVSGV SLPDYGVSWI 180 RQPPGKGLEW IGVIWGSETT YYQSSLKSRV TISKDNSKNQ VSLKLSSVTA ADTAVYYCAK 240 HYYYGGSYAM DYWGQGTLVT VSSTTTPAPR PPTPAPTIAS QPLSLRPEAC RPAAGGAVHT 300 RGLDFACDIY IWAPLAGTCG VLLLSLVITL YCKRGRKKLL YIFKQPFMRP VQTTQEEDGC 360 SCRFPEEEEG GCELRVKFSR SADAPAYKQG QNQLYNELNL GRREEYDVLD KRRGRDPEMG 420 GKPRRKNPQE GLYNELQKDK MAEAYSEIGM KGERRRGKGH DGLYQGLSTA TKDTYDALHM 480 QALPPR 486 | |
| SEQ ID NO:50 humanized anti-CD19 chimeric antigen receptor (anti-CD19:CD8α:4-1BB:CD3ζ) | MALPVTALLL PLALLLHAAR PQVQLQESGP GLVKPSETLS LTCTVSGVSL PDYGVSWIRQ 60 PPGKGLEWIG VIWGSETTYY SSSLKSRVTI SKDNSKNQVS LKLSSVTAAD TAVYYCAKHY 120 YYGGSYAMDY WGQGTLVTVS SGGGGSGGGG SGGGGSEIVM TQSPATLSLS PGERATLSCR 180 ASQDISKYLN WYQQKPGQAP RLLIYHTSRL HSGIPARFSG SGSGTDYTLT ISSLQPEDFA 240 VYFCQQGNTL PYTFGQGTKL EIKTTTPAPR PPTPAPTIAS QPLSLRPEAC RPAAGGAVHT 300 RGLDFACDIY IWAPLAGTCG VLLLSLVITL YCKRGRKKLL YIFKQPFMRP VQTTQEEDGC 360 SCRFPEEEEG GCELRVKFSR SADAPAYKQG QNQLYNELNL GRREEYDVLD KRRGRDPEMG 420 GKPRRKNPQE GLYNELQKDK MAEAYSEIGM KGERRRGKGH DGLYQGLSTA TKDTYDALHM 480 QALPPR 486 | |
| SEQ ID NO:51 humanized anti-CD19 | MALPVTALLL PLALLLHAAR PQVQLQESGP GLVKPSETLS LTCTVSGVSL PDYGVSWIRQ 60 PPGKGLEWIG VIWGSETTYY QSSLKSRVTI SKDNSKNQVS LKLSSVTAAD TAVYYCAKHY 120 | |
| chimeric antigen receptor (anti-CD19:CD8α:4-1BB:CD3ζ) | YYGGSYAMDY WGQGTLVTVS SGGGGSGGGG SGGGGSEIVM TQSPATLSLS PGERATLSCR 180 ASQDISKYLN WYQQKPGQAP RLLIYHTSRL HSGIPARFSG SGSGTDYTLT ISSLQPEDFA 240 VYFCQQGNTL PYTFGQGTKL EIKTTTPAPR PPTPAPTIAS QPLSLRPEAC RPAAGGAVHT 300 RGLDFACDIY IWAPLAGTCG VLLLSLVITL YCKRGRKKLL YIFKQPFMRP VQTTQEEDGC 360 SCRFPEEEEG GCELRVKFSR SADAPAYKQG QNQLYNELNL GRREEYDVLD KRRGRDPEMG 420 GKPRRKNPQE GLYNELQKDK MAEAYSEIGM KGERRRGKGH DGLYQGLSTA TKDTYDALHM 480 QALPPR 486 | |
| SEQ ID NO:52 humanized anti-CD19 chimeric antigen receptor (anti-CD19:CD8α:4-1BB:CD3ζ) | MALPVTALLL PLALLLHAAR PEIVMTQSPA TLSLSPGERA TLSCRASQDI SKYLNWYQQK 60 PGQAPRLLIY HTSRLHSGIP ARFSGSGSGT DYTLTISSLQ PEDFAVYFCQ QGNTLPYTFG 120 QGTKLEIKGG GGSGGGGSGG GGSGGGGSQV QLQESGPGLV KPSETLSLTC TVSGVSLPDY 180 GVSWIRQPPG KGLEWIGVIW GSETTYYSSS LKSRVTISKD NSKNQVSLKL SSVTAADTAV 240 YYCAKHYYYG GSYAMDYWGQ GTLVTVSSTT TPAPRPPTPA PTIASQPLSL RPEACRPAAG 300 GAVHTRGLDF ACDIYIWAPL AGTCGVLLLS LVITLYCKRG RKKLLYIFKQ PFMRPVQTTQ 360 EEDGCSCRFP EEEEGGCELR VKFSRSADAP AYKQGQNQLY NELNLGRREE YDVLDKRRGR 420 DPEMGGKPRR KNPQEGLYNE LQKDKMAEAY SEIGMKGERR RGKGHDGLYQ GLSTATKDTY 480 DALHMQALPP R 491 | |
| SEQ ID NO:53 humanized anti-CD19 chimeric antigen receptor (anti-CD19:CD8α:4-1BB:CD3ζ) | MALPVTALLL PLALLLHAAR PEIVMTQSPA TLSLSPGERA TLSCRASQDI SKYLNWYQQK 60 PGQAPRLLIY HTSRLHSGIP ARFSGSGSGT DYTLTISSLQ PEDFAVYFCQ QGNTLPYTFG 120 QGTKLEIKGG GGSGGGGSGG GGSGGGGSQV QLQESGPGLV KPSETLSLTC TVSGVSLPDY 180 GVSWIRQPPG KGLEWIGVIW GSETTYYQSS LKSRVTISKD NSKNQVSLKL SSVTAADTAV 240 YYCAKHYYYG GSYAMDYWGQ GTLVTVSSTT TPAPRPPTPA PTIASQPLSL RPEACRPAAG 300 GAVHTRGLDF ACDIYIWAPL AGTCGVLLLS LVITLYCKRG RKKLLYIFKQ PFMRPVQTTQ 360 EEDGCSCRFP EEEEGGCELR VKFSRSADAP AYKQGQNQLY NELNLGRREE YDVLDKRRGR 420 DPEMGGKPRR KNPQEGLYNE LQKDKMAEAY SEIGMKGERR RGKGHDGLYQ GLSTATKDTY 480 DALHMQALPP R 491 | |
| SEQ ID NO:54 humanized anti-CD19 chimeric antigen receptor (anti-CD19:CD8α:4-1BB:CD3ζ) | MALPVTALLL PLALLLHAAR PQVQLQESGP GLVKPSETLS LTCTVSGVSL PDYGVSWIRQ 60 PPGKGLEWIG VIWGSETTYY SSSLKSRVTI SKDNSKNQVS LKLSSVTAAD TAVYYCAKHY 120 YYGGSYAMDY WGQGTLVTVS SGGGGSGGGG SGGGGSGGGG SEIVMTQSPA TLSLSPGERA 180 TLSCRASQDI SKYLNWYQQK PGQAPRLLIY HTSRLHSGIP ARFSGSGSGT DYTLTISSLQ 240 PEDFAVYFCQ QGNTLPYTFG QGTKLEIKTT TPAPRPPTPA PTIASQPLSL RPEACRPAAG 300 GAVHTRGLDF ACDIYIWAPL AGTCGVLLLS LVITLYCKRG RKKLLYIFKQ PFMRPVQTTQ 360 EEDGCSCRFP EEEEGGCELR VKFSRSADAP AYKQGQNQLY NELNLGRREE YDVLDKRRGR 420 DPEMGGKPRR KNPQEGLYNE LQKDKMAEAY SEIGMKGERR RGKGHDGLYQ GLSTATKDTY 480 DALHMQALPP R 491 | |
| SEQ ID NO:55 humanized anti-CD19 chimeric antigen receptor (anti-CD19:CD8α:4-1BB:CD3ζ) | MALPVTALLL PLALLLHAAR PQVQLQESGP GLVKPSETLS LTCTVSGVSL PDYGVSWIRQ 60 PPGKGLEWIG VIWGSETTYY QSSLKSRVTI SKDNSKNQVS LKLSSVTAAD TAVYYCAKHY 120 YYGGSYAMDY WGQGTLVTVS SGGGGSGGGG SGGGGSGGGG SEIVMTQSPA TLSLSPGERA 180 TLSCRASQDI SKYLNWYQQK PGQAPRLLIY HTSRLHSGIP ARFSGSGSGT DYTLTISSLQ 240 PEDFAVYFCQ QGNTLPYTFG QGTKLEIKTT TPAPRPPTPA PTIASQPLSL RPEACRPAAG 300 GAVHTRGLDF ACDIYIWAPL AGTCGVLLLS LVITLYCKRG RKKLLYIFKQ PFMRPVQTTQ 360 EEDGCSCRFP EEEEGGCELR VKFSRSADAP AYKQGQNQLY NELNLGRREE YDVLDKRRGR 420 DPEMGGKPRR KNPQEGLYNE LQKDKMAEAY SEIGMKGERR RGKGHDGLYQ GLSTATKDTY 480 DALHMQALP 489 | |
| SEQ ID NO:56 humanized anti-CD19 chimeric antigen receptor (anti-CD19:CD8α:4-1BB:CD3ζ) | MALPVTALLL PLALLLHAAR PEIVMTQSPA TLSLSPGERA TLSCRASQDI SKYLNWYQQK 60 PGQAPRLLIY HTSRLHSGIP ARFSGSGSGT DYTLTISSLQ PEDFAVYFCQ QGNTLPYTFG 120 QGTKLEIKGG GGSGGGGSGG GGSGGGGSQV QLQESGPGLV KPSETLSLTC TVSGVSLPDY 180 GVSWIRQPPG KGLEWIGVIW GSETTYYNSS LKSRVTISKD NSKNQVSLKL SSVTAADTAV 240 YYCAKHYYYG GSYAMDYWGQ GTLVTVSSTT TPAPRPPTPA PTIASQPLSL RPEACRPAAG 300 GAVHTRGLDF ACDIYIWAPL AGTCGVLLLS LVITLYCKRG RKKLLYIFKQ PFMRPVQTTQ 360 EEDGCSCRFP EEEEGGCELR VKFSRSADAP AYKQGQNQLY NELNLGRREE YDVLDKRRGR 420 DPEMGGKPRR KNPQEGLYNE LQKDKMAEAY SEIGMKGERR RGKGHDGLYQ GLSTATKDTY 480 DALHMQALPP R 491 | |
| SEQ ID NO:57 humanized anti-CD19 chimeric antigen receptor (anti-CD19:CD8α:4-1BB:CD3ζ) | MALPVTALLL PLALLLHAAR PEIVMTQSPA TLSLSPGERA TLSCRASQDI SKYLNWYQQK 60 PGQAPRLLIY HTSRLHSGIP ARFSGSGSGT DYTLTISSLQ PEDFAVYFCQ QGNTLPYTFG 120 QGTKLEIKGG GGSGGGGSGG GGSGGGGSQV QLQESGPGLV KPSETLSLTC TVSGVSLPDY 180 GVSWIRQPPG KGLEWIGVIW GSETTYYNSS LKSRVTISKD NSKNQVSLKL SSVTAADTAV 240 YYCAKHYYYG GSYAMDYWGQ GTLVTVSSTT TPAPRPPTPA PTIASQPLSL RPEACRPAAG 300 GAVHTRGLDF ACDIYIWAPL AGTCGVLLLS LVITLYCKRG RKKLLYIFKQ PFMRPVQTTQ 360 EEDGCSCRFP EEEEGGCELR VKFSRSADAP AYKQGQNQLY NELNLGRREE YDVLDKRRGR 420 DPEMGGKPRR KNPQEGLYNE LQKDKMAEAY SEIGMKGERR RGKGHDGLYQ GLSTATKDTY 480 DALHMQALPP R 491 | |
| SEQ ID NO:58 humanized anti-CD19 chimeric antigen receptor (anti-CD19:CD8α:4- | MALPVTALLL PLALLLHAAR PQVQLQESGP GLVKPSETLS LTCTVSGVSL PDYGVSWIRQ 60 PPGKGLEWIG VIWGSETTYY NSSLKSRVTI SKDNSKNQVS LKLSSVTAAD TAVYYCAKHY 120 YYGGSYAMDY WGQGTLVTVS SGGGGSGGGG SGGGGSGGGG SEIVMTQSPA TLSLSPGERA 180 TLSCRASQDI SKYLNWYQQK PGQAPRLLIY HTSRLHSGIP ARFSGSGSGT DYTLTISSLQ 240 PEDFAVYFCQ QGNTLPYTFG QGTKLEIKTT TPAPRPPTPA PTIASQPLSL RPEACRPAAG 300 | |
| 1BB:CD3ζ) | GAVHTRGLDF ACDIYIWAPL AGTCGVLLLS LVITLYCKRG RKKLLYIFKQ PFMRPVQTTQ 360 EEDGCSCRFP EEEEGGCELR VKFSRSADAP AYKQGQNQLY NELNLGRREE YDVLDKRRGR 420 DPEMGGKPRR KNPQEGLYNE LQKDKMAEAY SEIGMKGERR RGKGHDGLYQ GLSTATKDTY 480 DALHMQALPP R 491 | |
| SEQ ID NO:59 humanized anti-CD19 chimeric antigen receptor (anti-CD19:CD8α:4-1BB:CD3ζ) | MALPVTALLL PLALLLHAAR PEIVMTQSPA TLSLSPGERA TLSCRASQDI SKYLNWYQQK 60 PGQAPRLLIY HTSRLHSGIP ARFSGSGSGT DYTLTISSLQ PEDFAVYFCQ QGNTLPYTFG 120 QGTKLEIKGG GGSGGGGSGG GGSQVQLQES GPGLVKPSET LSLTCTVSGV SLPDYGVSWI 180 RQPPGKGLEW IGVIWGSETT YYNSSLKSRV TISKDNSKNQ VSLKLSSVTA ADTAVYYCAK 240 HYYYGGSYAM DYWGQGTLVT VSSTTTPAPR PPTPAPTIAS QPLSLRPEAC RPAAGGAVHT 300 RGLDFACDIY IWAPLAGTCG VLLLSLVITL YCKRGRKKLL YIFKQPFMRP VQTTQEEDGC 360 SCRFPEEEEG GCELRVKFSR SADAPAYKQG QNQLYNELNL GRREEYDVLD KRRGRDPEMG 420 GKPRRKNPQE GLYNELQKDK MAEAYSEIGM KGERRRGKGH DGLYQGLSTA TKDTYDALHM 480 QALPPR 486 | |

In an embodiment, the CD19 inhibitor selected from an anti-CD19 antibody or an anti-CD19 chimeric antigen receptor, or an antigen-binding fragment or conjugate thereof is co-administered with a BTK inhibitor of Formula (II) and another agent to manage toxicities related to the infusion of chimeric antigen receptor modified T cells or NK cells, including cytokine release syndrome and tumor lysis syndrome. In an embodiment, the CD19 inhibitor is co-administered with a BTK inhibitor and a TNFα inhibitor. In an embodiment, the CD19 inhibitor is co-administered with a BTK inhibitor of Formula (II) and etanercept. In an embodiment, the CD19 inhibitor is co-administered with a BTK inhibitor of Formula (II) and an antibody against the IL-6 receptor. In an embodiment, the CD19 inhibitor is co-administered with a BTK inhibitor of Formula (II) and tocilizumab. Suitable compositions comprising tocilizumab, etanercept, and chimeric antigen receptor modified T cells, as well as other agents, and methods of preparation and therapeutic use of the same, are described in U.S. Patent Application Publication No. US 20150202286 A1 and International Patent Publication No. WO 2014/011984 A1.

### Pharmaceutical Compositions

In an embodiment, the invention provides a pharmaceutical composition for use in the treatment of cancer.

In some embodiments, the invention provides pharmaceutical compositions for treating solid tumor cancers, lymphomas and leukemia.

In preferred embodiments, the invention provides a composition comprising therapeutically effective amounts of (1) a CD19 inhibitor selected from an anti-CD19 antibody, or an anti-CD19 chimeric antigen receptor or an antigen-binding fragmentor conjugate thereof; and (2) a BTK inhibitor of Formula (II) or a pharmaceutically acceptable salt for use in the treatment of cancer. This composition is typically a pharmaceutical composition.

In preferred embodiments, the invention provides a composition comprising therapeutically effective amounts of (1) a CD19 inhibitor selected from an anti-CD19 antibody, or an anti-CD19 chimeric antigen receptor or an antigen-binding fragment or conjugate thereof; (2) a BTK inhibitor of Formula (II) or a pharmaceutically acceptable salt thereof; and (3) an anti-CD20 antibody selected from the group consisting of rituximab, obinutuzumab, ofatumumab, veltuzumab, tositumomab, ibritumomab, and fragments, derivatives, conjugates, variants, radioisotope-labeled complexes, and biosimilars thereof, for use in the treatment of cancer. This composition is typically a pharmaceutical composition.

In some embodiments, the invention provides a composition for use in the treatment of cancer comprising therapeutically effective amounts of (1) a CD19 inhibitor selected from an anti-CD19 antibody or an anti-CD19 chimeric antigen receptor, or an antigen-binding fragmentor conjugate thereof; (2) a BTK inhibitor of Formula (II) or a pharmaceutically acceptable salt thereof; and (3) a compound selected from the group consisting of gemcitabine, albumin-bound paclitaxel, bendamustine, fludarabine, cyclophosphamide, chlorambucil, an anticoagulant or antiplatelet active pharmaceutical ingredient, or combinations thereof. This composition is typically a pharmaceutical composition.

In some embodiments, the invention provides a composition comprising therapeutically effective amounts of (1) a CD19 inhibitor selected from an anti-CD19 antibody or an anti-CD19 chimeric antigen receptor, or an antigen-binding fragment or conjugate thereof; (2) a BTK inhibitor of Formula (II) or a pharmaceutically acceptable salt thereof, for use in the treatment of cancer; (3) an anti-CD20 antibody selected from the group consisting of rituximab, obinutuzumab, ofatumumab, veltuzumab, tositumomab, ibritumomab, and fragments, derivatives, conjugates, variants, radioisotope-labeled complexes, biosimilars thereof, and combinations thereof; and (4) a compound selected from the group consisting of gemcitabine, albumin-bound paclitaxel, bendamustine, fludarabine, cyclophosphamide, chlorambucil, an anticoagulant or antiplatelet active pharmaceutical ingredient, and combinations thereof. This composition is typically a pharmaceutical composition.

In some embodiments, the invention provides a composition comprising therapeutically effective amounts of (1) a CD19 inhibitor selected from an anti-CD19 antibody or an anti-CD19 chimeric antigen receptor, or an antigen-binding fragment or conjugate thereof; and (2) a BTK inhibitor having the structure: 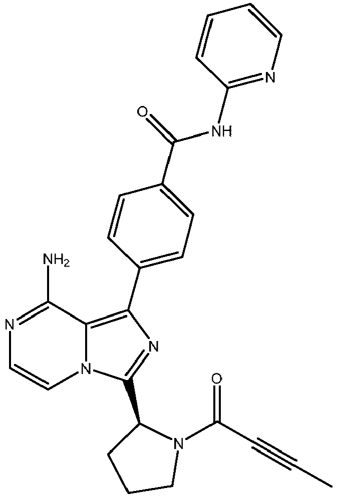 or a pharmaceutically acceptable salt thereof, for use in the treatment of cancer. This composition is typically a pharmaceutical composition.

In some embodiments, the invention provides a composition comprising therapeutically effective amounts of (1) a CD19 inhibitor selected from the group consisting of blinatumomab, coltuximab ravtansine, denintuzumab mafodotin, and an anti-CD19 chimeric antigen receptor, and antigen-binding fragments or conjugates thereof; and (2) a BTK inhibitor of Formula (II) or a pharmaceutically acceptable salt thereof. This composition is typically a pharmaceutical composition.

In some embodiments, the invention provides a composition comprising therapeutically effective amounts of (1) a CD19 inhibitor selected from the group consisting of blinatumomab, coltuximab ravtansine, denintuzumab mafodotin, and an anti-CD19 chimeric antigen receptor, and antigen-binding fragmentsor conjugates thereof; and (2) a BTK inhibitor of Formula (II) or a pharmaceutically acceptable salt thereof for use in the treatment of cancer. This composition is typically a pharmaceutical composition.

In some embodiments, the invention provides a composition comprising therapeutically effective amounts of (1) a CD19 inhibitor selected from the group consisting of blinatumomab, coltuximab ravtansine, denintuzumab mafodotin, and an anti-CD19 chimeric antigen receptor, and antigen-binding fragments or conjugates thereof; (2) a BTK inhibitor of Formula (II) or a pharmaceutically acceptable salt thereof, for use in the treatment of cancer; and (3) an anti-CD20 antibody selected from the group consisting of rituximab, obinutuzumab, ofatumumab, veltuzumab, tositumomab, ibritumomab, and fragments, derivatives, conjugates, variants, radioisotope-labeled complexes, and biosimilars thereof. This composition is typically a pharmaceutical composition.

In some embodiments, the invention provides a composition comprising therapeutically effective amounts of (1) a CD19 inhibitor selected from the group consisting of blinatumomab, coltuximab ravtansine, denintuzumab mafodotin, and an anti-CD19 chimeric antigen receptor, and antigen-binding fragments or conjugates thereof; (2) a BTK inhibitor of Formula (II) or a pharmaceutically acceptable salt thereof, for use in the treatment of cancer; and (3) an anti-CD20 antibody selected from the group consisting of rituximab, obinutuzumab, ofatumumab, veltuzumab, tositumomab, ibritumomab, and fragments, derivatives, conjugates, variants, radioisotope-labeled complexes, and biosimilars thereof. This composition is typically a pharmaceutical composition.

The pharmaceutical compositions are typically formulated to provide a therapeutically effective amount of a combination as described herein, *i.e.,* a combination of a CD19 inhibitor selected from an anti-CD19 antibody or an anti-CD19 chimeric antigen receptor, or an antigen-binding fragment or conjugate thereof, and a BTK inhibitor of Formula (II) as the active ingredients. Where desired, the pharmaceutical compositions contain a pharmaceutically acceptable salt and/or coordination complex of one or more of the active ingredients. Typically, the pharmaceutical compositions also comprise one or more pharmaceutically acceptable excipients, carriers, including inert solid diluents and fillers, diluents, including sterile aqueous solution and various organic solvents, permeation enhancers, solubilizers and adjuvants.

The pharmaceutical compositions described above are preferably for use in the treatment of the diseases and conditions described below. In a preferred embodiment, the pharmaceutical compositions are for use in the treatment of cancer. In preferred embodiments, the pharmaceutical compositions are for use in treating solid tumor cancers, lymphomas, and leukemias.

The pharmaceutical compositions of the present invention, including any of the foregoing embodiments of compositions, are for use in the treatment of cancer. In one embodiment, the pharmaceutical compositions of the present invention are for use in the treatment of a cancer selected from the group consisting of bladder cancer, squamous cell carcinoma including head and neck cancer, pancreatic ductal adenocarcinoma (PDA), pancreatic cancer, colon carcinoma, mammary carcinoma, breast cancer, fibrosarcoma, mesothelioma, renal cell carcinoma, lung carcinoma, thyoma, prostate cancer, colorectal cancer, ovarian cancer, acute myeloid leukemia, thymus cancer, brain cancer, squamous cell cancer, skin cancer, eye cancer, retinoblastoma, melanoma, intraocular melanoma, oral cavity and oropharyngeal cancers, gastric cancer, stomach cancer, cervical cancer, renal cancer, kidney cancer, liver cancer, ovarian cancer, esophageal cancer, testicular cancer, gynecological cancer, thyroid cancer, acquired immune deficiency syndrome (AIDS)-related cancers (e.g., lymphoma and Kaposi's sarcoma), viral-induced cancer, glioblastoma, esophogeal tumors, hematological neoplasms, non-small-cell lung cancer, chronic myelocytic leukemia, diffuse large B-cell lymphoma, esophagus tumor, follicle center lymphoma, head and neck tumor, hepatitis C virus infection, hepatocellular carcinoma, Hodgkin's disease, metastatic colon cancer, multiple myeloma, non-Hodgkin's lymphoma, indolent non-Hodgkin's lymphoma, ovary tumor, pancreas tumor, renal cell carcinoma, small-cell lung cancer, stage IV melanoma, chronic lymphocytic leukemia, B-cell acute lymphoblastic leukemia (ALL), mature B-cell ALL, follicular lymphoma, mantle cell lymphoma, and Burkitt's lymphoma.

In an embodiment, the molar ratio of the CD19 inhibitor selected from an anti-CD19 antibody or an anti-CD19 chimeric antigen receptor, or an antigen-binding fragment or conjugate thereof to the BTK inhibitor of Formula (II) in the pharmaceutical compositions is in the range from about 10:1 to about 1: 10, preferably from about 2.5:1 to about 1:2.5, and more preferably about about 1:1. In an embodiment, the weight ratio of the CD19 inhibitor selected from an anti-CD19 antibody or an anti-CD19 chimeric antigen receptor, or an antigen-binding fragment or conjugate thereof to the BTK inhibitor of Formula (II) in the pharmaceutical compositions is selected from the group consisting of about 20:1, about 19:1, about 18:1, about 17: 1, about 16:1, about 15:1, about 14: 1, about 13: 1, about 12:1, about 11: 1, about 10:1, about 9:1, about 8:1, about 7:1, about 6:1, about 5:1, about 4:1, about 3:1, about 2:1, about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, and about 1:20.

In some embodiments, the concentration of any one of the CD19 inhibitor selected from an anti-CD19 antibody or an anti-CD19 chimeric antigen receptor, or an antigen-binding fragment or conjugate thereof and BTK inhibitor of Formula (II) provided in the pharmaceutical compositions for use in the invention is independently less than, for example, 100%, 90%, 80%, 70%, 60%, 50%, 40%, 30%, 20%, 19%, 18%, 17%, 16%, 15%, 14%, 13%, 12%, 11%, 10%, 9%, 8%, 7%, 6%, 5%, 4%, 3%, 2%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002% or 0.0001% w/w, w/v or v/v of the pharmaceutical composition.

In some embodiments, the concentration of any one of the CD19 inhibitor selected from an anti-CD19 antibody or an anti-CD19 chimeric antigen receptor, or an antigen-binding fragment or conjugate thereof and BTK inhibitor of Formula (II) provided in the pharmaceutical compositions for use in the invention is independently greater than 90%, 80%, 70%, 60%, 50%, 40%, 30%, 20%, 19.75%, 19.50%, 19.25% 19%, 18.75%, 18.50%, 18.25% 18%, 17.75%, 17.50%, 17.25% 17%, 16.75%, 16.50%, 16.25% 16%, 15.75%, 15.50%, 15.25% 15%, 14.75%, 14.50%, 14.25% 14%, 13.75%, 13.50%, 13.25% 13%, 12.75%, 12.50%, 12.25% 12%, 11.75%, 11.50%, 11.25% 11%, 10.75%, 10.50%, 10.25% 10%, 9.75%, 9.50%, 9.25% 9%, 8.75%, 8.50%, 8.25% 8%, 7.75%, 7.50%, 7.25% 7%, 6.75%, 6.50%, 6.25% 6%, 5.75%, 5.50%, 5.25% 5%, 4.75%, 4.50%, 4.25%, 4%, 3.75%, 3.50%, 3.25%, 3%, 2.75%, 2.50%, 2.25%, 2%, 1.75%, 1.50%, 125%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002% or 0.0001% w/w, w/v, or v/v of the pharmaceutical composition.

In some embodiments, the concentration of any one of the CD19 inhibitor selected from an anti-CD19 antibody or an anti-CD19 chimeric antigen receptor, or an antigen-binding fragment or conjugate thereof and BTK inhibitor of Formula (II) provided in the pharmaceutical compositions is independently in the range from about 0.0001% to about 50%, about 0.001% to about 40%, about 0.01% to about 30%, about 0.02% to about 29%, about 0.03% to about 28%, about 0.04% to about 27%, about 0.05% to about 26%, about 0.06% to about 25%, about 0.07% to about 24%, about 0.08% to about 23%, about 0.09% to about 22%, about 0.1% to about 21%, about 0.2% to about 20%, about 0.3% to about 19%, about 0.4% to about 18%, about 0.5% to about 17%, about 0.6% to about 16%, about 0.7% to about 15%, about 0.8% to about 14%, about 0.9% to about 12% or about 1% to about 10% w/w, w/v or v/v of the pharmaceutical composition.

In some embodiments, the concentration of any one of the CD19 inhibitor selected from an anti-CD19 antibody or an anti-CD19 chimeric antigen receptor, or an antigen-binding fragment or conjugate thereof and BTK inhibitor of Formula (II) provided in the pharmaceutical compositions is independently in the range from about 0.001% to about 10%, about 0.01% to about 5%, about 0.02% to about 4.5%, about 0.03% to about 4%, about 0.04% to about 3.5%, about 0.05% to about 3%, about 0.06% to about 2.5%, about 0.07% to about 2%, about 0.08% to about 1.5%, about 0.09% to about 1%, about 0.1% to about 0.9% w/w, w/v or v/v of the pharmaceutical composition.

In some embodiments, the amount of any one of the CD19 inhibitor selected from an anti-CD19 antibody or an anti-CD19 chimeric antigen receptor, or an antigen-binding fragment or conjugate thereof and BTK inhibitor of Formula (II) provided in the pharmaceutical compositions is independently equal to or less than 10 g, 9.5 g, 9.0 g, 8.5 g, 8.0 g, 7.5 g, 7.0 g, 6.5 g, 6.0 g, 5.5 g, 5.0 g, 4.5 g, 4.0 g, 3.5 g, 3.0 g, 2.5 g, 2.0 g, 1.5 g, 1.0 g, 0.95 g, 0.9 g, 0.85 g, 0.8 g, 0.75 g, 0.7 g, 0.65 g, 0.6 g, 0.55 g, 0.5 g, 0.45 g, 0.4 g, 0.35 g, 0.3 g, 0.25 g, 0.2 g, 0.15 g, 0.1 g, 0.09 g, 0.08 g, 0.07 g, 0.06 g, 0.05 g, 0.04 g, 0.03 g, 0.02 g, 0.01 g, 0.009 g, 0.008 g, 0.007 g, 0.006 g, 0.005 g, 0.004 g, 0.003 g, 0.002 g, 0.001 g, 0.0009 g, 0.0008 g, 0.0007 g, 0.0006 g, 0.0005 g, 0.0004 g, 0.0003 g, 0.0002 g, or 0.0001 g.

In some embodiments, the amount of any one of the CD19 inhibitor selected from an anti-CD19 antibody or an anti-CD19 chimeric antigen receptor, or an antigen-binding fragment or conjugate thereof and BTK inhibitor of Formula (II) provided in the pharmaceutical compositions is independently more than 0.0001 g, 0.0002 g, 0.0003 g, 0.0004 g, 0.0005 g, 0.0006 g, 0.0007 g, 0.0008 g, 0.0009 g, 0.001 g, 0.0015 g, 0.002 g, 0.0025 g, 0.003 g, 0.0035 g, 0.004 g, 0.0045 g, 0.005 g, 0.0055 g, 0.006 g, 0.0065 g, 0.007 g, 0.0075 g, 0.008 g, 0.0085 g, 0.009 g, 0.0095 g, 0.01 g, 0.015 g, 0.02 g, 0.025 g, 0.03 g, 0.035 g, 0.04 g, 0.045 g, 0.05 g, 0.055 g, 0.06 g, 0.065 g, 0.07 g, 0.075 g, 0.08 g, 0.085 g, 0.09 g, 0.095 g, 0.1 g, 0.15 g, 0.2 g, 0.25 g, 0.3 g, 0.35 g, 0.4 g, 0.45 g, 0.5 g, 0.55 g, 0.6 g, 0.65 g, 0.7 g, 0.75 g, 0.8 g, 0.85 g, 0.9 g, 0.95 g, 1 g, 1.5 g, 2 g, 2.5, 3 g, 3.5, 4 g, 4.5 g, 5 g, 5.5 g, 6 g, 6.5 g, 7 g, 7.5 g, 8 g, 8.5 g, 9 g, 9.5 g, or 10 g.

Each of the CD19 inhibitor selected from an anti-CD19 antibody or an anti-CD19 chimeric antigen receptor, or an antigen-binding fragment or conjugate thereof and BTK inhibitor of Formula (II) is effective over a wide dosage range. For example, in the treatment of adult humans, dosages independently ranging from 0.01 to 1000 mg, from 0.5 to 100 mg, from 1 to 50 mg per day, and from 5 to 40 mg per day are examples of dosages that may be used. The exact dosage will depend upon the route of administration, the form in which the compound is administered, the gender and age of the subject to be treated, the body weight of the subject to be treated, and the preference and experience of the attending physician.

In a preferred embodiment, the pharmaceutical compositions of the present invention are for use in the treatment of cancer. In a preferred embodiment, the pharmaceutical compositions of the present invention are for use in the treatment of a cancer selected from the group consisting of bladder cancer, squamous cell carcinoma including head and neck cancer, pancreatic ductal adenocarcinoma (PDA), pancreatic cancer, colon carcinoma, mammary carcinoma, breast cancer, fibrosarcoma, mesothelioma, renal cell carcinoma, lung carcinoma, thyoma, prostate cancer, colorectal cancer, ovarian cancer, acute myeloid leukemia, thymus cancer, brain cancer, squamous cell cancer, skin cancer, eye cancer, retinoblastoma, melanoma, intraocular melanoma, oral cavity and oropharyngeal cancers, gastric cancer, stomach cancer, cervical cancer, renal cancer, kidney cancer, liver cancer, ovarian cancer, esophageal cancer, testicular cancer, gynecological cancer, thyroid cancer, acquired immune deficiency syndrome (AIDS)-related cancers (e.g., lymphoma and Kaposi's sarcoma), viral-induced cancer, glioblastoma, esophogeal tumors, hematological neoplasms, non-small-cell lung cancer, chronic myelocytic leukemia, diffuse large B-cell lymphoma, esophagus tumor, follicle center lymphoma, head and neck tumor, hepatitis C virus infection, hepatocellular carcinoma, Hodgkin's disease, metastatic colon cancer, multiple myeloma, non-Hodgkin's lymphoma, indolent non-Hodgkin's lymphoma, ovary tumor, pancreas tumor, renal cell carcinoma, small-cell lung cancer, stage IV melanoma, chronic lymphocytic leukemia, B-cell acute lymphoblastic leukemia (ALL), mature B-cell ALL, follicular lymphoma, mantle cell lymphoma, and Burkitt's lymphoma.

Described below are non-limiting pharmaceutical compositions and methods for preparing the same.

### Pharmaceutical Compositions for Oral Administration

In preferred embodiments, the invention provides a pharmaceutical composition for oral administration containing the combination of a CD19 inhibitor selected from an anti-CD19 antibody or an anti-CD19 chimeric antigen receptor, or an antigen-binding fragment or conjugate thereof and BTK inhibitor of Formula (II), and a pharmaceutical excipient suitable for oral administration for use in the treatment of cancer.

In preferred embodiments, the invention provides a solid pharmaceutical composition for oral administration containing: (i) an effective amount of each of a CD19 inhibitor selected from an anti-CD19 antibody or an anti-CD19 chimeric antigen receptor, or an antigen-binding fragment or conjugate thereof and BTK inhibitor of Formula (II) in combination and (ii) a pharmaceutical excipient suitable for oral administration for use in the treatment of cancer. In some embodiments, the composition further contains (iii) an effective amount of a fourth active pharmaceutical ingredient.

In preferred embodiments, the invention provides a solid pharmaceutical composition for oral administration containing: (i) an effective amount of a CD 19 inhibitor selected from an anti-CD19 antibody or an anti-CD19 chimeric antigen receptor, or an antigen-binding fragment or conjugate thereof in combination with a BTK inhibitor of Formula (II) and (ii) a pharmaceutical excipient suitable for oral administration for use in the treatment of cancer. In selected embodiments, the composition further contains (iii) an effective amount of a third active pharmaceutical ingredient.

In some embodiments, the pharmaceutical composition may be a liquid pharmaceutical composition suitable for oral consumption.

Pharmaceutical compositions for use in the invention suitable for oral administration can be presented as discrete dosage forms, such as capsules, sachets, tablets, liquids, or aerosol sprays each containing a predetermined amount of an active ingredient as a powder or in granules, a solution, or a suspension in an aqueous or non-aqueous liquid, an oil-in-water emulsion, a water-in-oil liquid emulsion, powders for reconstitution, powders for oral consumptions, bottles (including powders or liquids in a bottle), orally dissolving films, lozenges, pastes, tubes, gums, and packs. Such dosage forms can be prepared by any of the methods of pharmacy, but all methods include the step of bringing the active ingredient(s) into association with the carrier, which constitutes one or more necessary ingredients. In general, the compositions are prepared by uniformly and intimately admixing the active ingredient(s) with liquid carriers or finely divided solid carriers or both, and then, if necessary, shaping the product into the desired presentation. For example, a tablet can be prepared by compression or molding, optionally with one or more accessory ingredients. Compressed tablets can be prepared by compressing in a suitable machine the active ingredient in a free-flowing form such as powder or granules, optionally mixed with an excipient such as a binder, a lubricant, an inert diluent, and/or a surface active or dispersing agent. Molded tablets can be made by molding in a suitable machine a mixture of the powdered compound moistened with an inert liquid diluent.

The invention further encompasses the use of anhydrous pharmaceutical compositions and dosage forms since water can facilitate the degradation of some compounds. For example, water may be added *(e.g.,* 5%) in the pharmaceutical arts as a means of simulating long-term storage in order to determine characteristics such as shelf-life or the stability of formulations over time. Anhydrous pharmaceutical compositions and dosage forms for use inthe invention can be prepared using anhydrous or low moisture containing ingredients and low moisture or low humidity conditions. Pharmaceutical compositions and dosage forms for use in the invention which contain lactose can be made anhydrous if substantial contact with moisture and/or humidity during manufacturing, packaging, and/or storage is expected. An anhydrous pharmaceutical composition may be prepared and stored such that its anhydrous nature is maintained. Accordingly, anhydrous compositions may be packaged using materials known to prevent exposure to water such that they can be included in suitable formulary kits. Examples of suitable packaging include hermetically sealed foils, plastic or the like, unit dose containers, blister packs, and strip packs.

Each of the CD19 inhibitor selected from an anti-CD19 antibody or an anti-CD19 chimeric antigen receptor, or an antigen-binding fragment or conjugate thereof and BTK inhibitor of Formula (II) as active ingredients can be combined in an intimate admixture with a pharmaceutical carrier according to conventional pharmaceutical compounding techniques. The carrier can take a wide variety of forms depending on the form of preparation desired for administration. In preparing the compositions for an oral dosage form, any of the usual pharmaceutical media can be employed as carriers, such as, for example, water, glycols, oils, alcohols, flavoring agents, preservatives, and coloring agents in the case of oral liquid preparations (such as suspensions, solutions, and elixirs) or aerosols; or carriers such as starches, sugars, micro-crystalline cellulose, diluents, granulating agents, lubricants, binders, and disintegrating agents can be used in the case of oral solid preparations, in some embodiments without employing the use of lactose. For example, suitable carriers include powders, capsules, and tablets, with the solid oral preparations. If desired, tablets can be coated by standard aqueous or nonaqueous techniques.

Binders suitable for use in pharmaceutical compositions and dosage forms include corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, microcrystalline cellulose, and mixtures thereof.

Examples of suitable fillers for use in the pharmaceutical compositions and dosage forms disclosed herein include talc, calcium carbonate (e.g., granules or powder), microcrystalline cellulose, powdered cellulose, dextrates, kaolin, mannitol, silicic acid, sorbitol, starch, pre-gelatinized starch, and mixtures thereof.

Disintegrants may be used in the compositions for use in the invention to provide tablets that disintegrate when exposed to an aqueous environment. Too much of a disintegrant may produce tablets which disintegrate in the bottle. Too little may be insufficient for disintegration to occur, thus altering the rate and extent of release of the active ingredients from the dosage form. Thus, a sufficient amount of disintegrant that is neither too little nor too much to detrimentally alter the release of the active ingredient(s) may be used to form the dosage forms of the compounds disclosed herein. The amount of disintegrant used may vary based upon the type of formulation and mode of administration, and may be readily discernible to those of ordinary skill in the art. About 0.5 to about 15 weight percent of disintegrant, or about 1 to about 5 weight percent of disintegrant, may be used in the pharmaceutical composition. Disintegrants that can be used to form pharmaceutical compositions and dosage forms for use in the invention include agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums or mixtures thereof.

Lubricants which can be used to form pharmaceutical compositions and dosage forms for use in the invention include calcium stearate, magnesium stearate, sodium stearyl fumarate, mineral oil, light mineral oil, glycerin, sorbitol, mannitol, polyethylene glycol, other glycols, stearic acid, sodium lauryl sulfate, talc, hydrogenated vegetable oil *(e.g.,* peanut oil, cottonseed oil, sunflower oil, sesame oil, olive oil, corn oil, and soybean oil), zinc stearate, ethyl oleate, ethylaureate, agar, or mixtures thereof. Additional lubricants include, for example, a syloid silica gel, a coagulated aerosol of synthetic silica, silicified microcrystalline cellulose, or mixtures thereof. A lubricant can optionally be added in an amount of less than about 0.5% or less than about 1% (by weight) of the pharmaceutical composition.

When aqueous suspensions and/or elixirs are desired for oral administration, the active pharmacetical ingredient(s) may be combined with various sweetening or flavoring agents, coloring matter or dyes and, if so desired, emulsifying and/or suspending agents, together with such diluents as water, ethanol, propylene glycol, glycerin and various combinations thereof.

The tablets can be uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Surfactants which can be used to form pharmaceutical compositions and dosage forms for use in the invention include hydrophilic surfactants, lipophilic surfactants, and mixtures thereof. That is, a mixture of hydrophilic surfactants may be employed, a mixture of lipophilic surfactants may be employed, or a mixture of at least one hydrophilic surfactant and at least one lipophilic surfactant may be employed.

A suitable hydrophilic surfactant may generally have an HLB value of at least 10, while suitable lipophilic surfactants may generally have an HLB value of or less than about 10. An empirical parameter used to characterize the relative hydrophilicity and hydrophobicity of non-ionic amphiphilic compounds is the hydrophilic-lipophilic balance ("HLB" value). Surfactants with lower HLB values are more lipophilic or hydrophobic, and have greater solubility in oils, while surfactants with higher HLB values are more hydrophilic, and have greater solubility in aqueous solutions. Hydrophilic surfactants are generally considered to be those compounds having an HLB value greater than about 10, as well as anionic, cationic, or zwitterionic compounds for which the HLB scale is not generally applicable. Similarly, lipophilic (*i.e.,* hydrophobic) surfactants are compounds having an HLB value equal to or less than about 10. However, HLB value of a surfactant is merely a rough guide generally used to enable formulation of industrial, pharmaceutical and cosmetic emulsions.

Hydrophilic surfactants may be either ionic or non-ionic. Suitable ionic surfactants include alkylammonium salts; fusidic acid salts; fatty acid derivatives of amino acids, oligopeptides, and polypeptides; glyceride derivatives of amino acids, oligopeptides, and polypeptides; lecithins and hydrogenated lecithins; lysolecithins and hydrogenated lysolecithins; phospholipids and derivatives thereof; lysophospholipids and derivatives thereof; carnitine fatty acid ester salts; salts of alkylsulfates; fatty acid salts; sodium docusate; acylactylates; mono- and di-acetylated tartaric acid esters of mono- and di-glycerides; succinylated mono- and di-glycerides; citric acid esters of mono- and di-glycerides; and mixtures thereof.

Within the aforementioned group, ionic surfactants include, by way of example: lecithins, lysolecithin, phospholipids, lysophospholipids and derivatives thereof; carnitine fatty acid ester salts; salts of alkylsulfates; fatty acid salts; sodium docusate; acylactylates; mono- and di-acetylated tartaric acid esters of mono- and di-glycerides; succinylated mono- and di-glycerides; citric acid esters of mono- and di-glycerides; and mixtures thereof.

Ionic surfactants may be the ionized forms of lecithin, lysolecithin, phosphatidylcholine, phosphatidylethanolamine, phosphatidylglycerol, phosphatidic acid, phosphatidylserine, lysophosphatidylcholine, lysophosphatidylethanolamine, lysophosphatidylglycerol, lysophosphatidic acid, lysophosphatidylserine, PEG-phosphatidylethanolamine, PVP-phosphatidylethanolamine, lactylic esters of fatty acids, stearoyl-2-lactylate, stearoyl lactylate, succinylated monoglycerides, mono/diacetylated tartaric acid esters of mono/diglycerides, citric acid esters of mono/diglycerides, cholylsarcosine, caproate, caprylate, caprate, laurate, myristate, palmitate, oleate, ricinoleate, linoleate, linolenate, stearate, lauryl sulfate, teracecyl sulfate, docusate, lauroyl carnitines, palmitoyl carnitines, myristoyl carnitines, and salts and mixtures thereof.

Hydrophilic non-ionic surfactants may include alkylglucosides; alkylmaltosides; alkylthioglucosides; lauryl macrogolglycerides; polyoxyalkylene alkyl ethers such as polyethylene glycol alkyl ethers; polyoxyalkylene alkylphenols such as polyethylene glycol alkyl phenols; polyoxyalkylene alkyl phenol fatty acid esters such as polyethylene glycol fatty acids monoesters and polyethylene glycol fatty acids diesters; polyethylene glycol glycerol fatty acid esters; polyglycerol fatty acid esters; polyoxyalkylene sorbitan fatty acid esters such as polyethylene glycol sorbitan fatty acid esters; hydrophilic transesterification products of a polyol with at least one member of the group consisting of glycerides, vegetable oils, hydrogenated vegetable oils, fatty acids, and sterols; polyoxyethylene sterols, derivatives, and analogues thereof; polyoxyethylated vitamins and derivatives thereof; polyoxyethylene-polyoxypropylene block copolymers; and mixtures thereof; polyethylene glycol sorbitan fatty acid esters and hydrophilic transesterification products of a polyol with at least one member of the group consisting of triglycerides, vegetable oils, and hydrogenated vegetable oils. The polyol may be glycerol, ethylene glycol, polyethylene glycol, sorbitol, propylene glycol, pentaerythritol, or a saccharide.

Other hydrophilic-non-ionic surfactants include, without limitation, PEG-10 laurate, PEG-12 laurate, PEG-20 laurate, PEG-32 laurate, PEG-32 dilaurate, PEG-12 oleate, PEG-15 oleate, PEG-20 oleate, PEG-20 dioleate, PEG-32 oleate, PEG-200 oleate, PEG-400 oleate, PEG-15 stearate, PEG-32 distearate, PEG-40 stearate, PEG-100 stearate, PEG-20 dilaurate, PEG-25 glyceryl trioleate, PEG-32 dioleate, PEG-20 glyceryl laurate, PEG-30 glyceryl laurate, PEG-20 glyceryl stearate, PEG-20 glyceryl oleate, PEG-30 glyceryl oleate, PEG-30 glyceryl laurate, PEG-40 glyceryl laurate, PEG-40 palm kernel oil, PEG-50 hydrogenated castor oil, PEG-40 castor oil, PEG-35 castor oil, PEG-60 castor oil, PEG-40 hydrogenated castor oil, PEG-60 hydrogenated castor oil, PEG-60 corn oil, PEG-6 caprate/caprylate glycerides, PEG-8 caprate/caprylate glycerides, polyglyceryl-10 laurate, PEG-30 cholesterol, PEG-25 phyto sterol, PEG-30 soya sterol, PEG-20 trioleate, PEG-40 sorbitan oleate, PEG-80 sorbitan laurate, polysorbate 20, polysorbate 80, POE-9 lauryl ether, POE-23 lauryl ether, POE-10 oleyl ether, POE-20 oleyl ether, POE-20 stearyl ether, tocopheryl PEG-100 succinate, PEG-24 cholesterol, polyglyceryl-10oleate, Tween 40, Tween 60, sucrose monostearate, sucrose monolaurate, sucrose monopalmitate, PEG 10-100 nonyl phenol series, PEG 15-100 octyl phenol series, and poloxamers.

Suitable lipophilic surfactants include, by way of example only: fatty alcohols; glycerol fatty acid esters; acetylated glycerol fatty acid esters; lower alcohol fatty acids esters; propylene glycol fatty acid esters; sorbitan fatty acid esters; polyethylene glycol sorbitan fatty acid esters; sterols and sterol derivatives; polyoxyethylated sterols and sterol derivatives; polyethylene glycol alkyl ethers; sugar esters; sugar ethers; lactic acid derivatives of mono- and di-glycerides; hydrophobic transesterification products of a polyol with at least one member of the group consisting of glycerides, vegetable oils, hydrogenated vegetable oils, fatty acids and sterols; oilsoluble vitamins/vitamin derivatives; and mixtures thereof. Within this group, preferred lipophilic surfactants include glycerol fatty acid esters, propylene glycol fatty acid esters, and mixtures thereof, or are hydrophobic transesterification products of a polyol with at least one member of the group consisting of vegetable oils, hydrogenated vegetable oils, and triglycerides.

In an embodiment, the composition may include a solubilizer to ensure good solubilization and/or dissolution of the compound for use in the present invention and to minimize precipitation of the compound for use in the present invention. This can be especially important for compositions for non-oral use - *e.g*., compositions for injection. A solubilizer may also be added to increase the solubility of the hydrophilic drug and/or other components, such as surfactants, or to maintain the composition as a stable or homogeneous solution or dispersion.

Examples of suitable solubilizers include the following: alcohols and polyols, such as ethanol, isopropanol, butanol, benzyl alcohol, ethylene glycol, propylene glycol, butanediols and isomers thereof, glycerol, pentaerythritol, sorbitol, mannitol, transcutol, dimethyl isosorbide, polyethylene glycol, polypropylene glycol, polyvinylalcohol, hydroxypropyl methylcellulose and other cellulose derivatives, cyclodextrins and cyclodextrin derivatives; ethers of polyethylene glycols having an average molecular weight of about 200 to about 6000, such as tetrahydrofurfuryl alcohol PEG ether (glycofurol) or methoxy PEG; amides and other nitrogencontaining compounds such as 2-pyrrolidone, 2-piperidone, 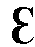 -caprolactam, N-alkylpyrrolidone, N-hydroxyalkylpyrrolidone, N-alkylpiperidone, N-alkylcaprolactam, dimethylacetamide and polyvinylpyrrolidone; esters such as ethyl propionate, tributylcitrate, acetyl triethylcitrate, acetyl tributyl citrate, triethylcitrate, ethyl oleate, ethyl caprylate, ethyl butyrate, triacetin, propylene glycol monoacetate, propylene glycol diacetate, epsilon. -caprolactone and isomers thereof, δ-valerolactone and isomers thereof, β-butyrolactone and isomers thereof; and other solubilizers known in the art, such as dimethyl acetamide, dimethyl isosorbide, N-methyl pyrrolidones, monooctanoin, diethylene glycol monoethyl ether, and water.

Mixtures of solubilizers may also be used. Examples include triacetin, triethylcitrate, ethyl oleate, ethyl caprylate, dimethylacetamide, N-methylpyrrolidone, N-hydroxyethylpyrrolidone, polyvinylpyrrolidone, hydroxypropyl methylcellulose, hydroxypropyl cyclodextrins, ethanol, polyethylene glycol 200-100, glycofurol, transcutol, propylene glycol, and dimethyl isosorbide. Particularly preferred solubilizers include sorbitol, glycerol, triacetin, ethyl alcohol, PEG-400, glycofurol and propylene glycol.

The amount of solubilizer that can be included is not particularly limited. The amount of a given solubilizer may be limited to a bioacceptable amount, which may be readily determined by one of skill in the art. In some circumstances, it may be advantageous to include amounts of solubilizers far in excess of bioacceptable amounts, for example to maximize the concentration of the drug, with excess solubilizer removed prior to providing the composition to a patient using conventional techniques, such as distillation or evaporation. Thus, if present, the solubilizer can be in a weight ratio of 10%, 25%, 50%, 100%, or up to about 200% by weight, based on the combined weight of the drug, and other excipients. If desired, very small amounts of solubilizer may also be used, such as 5%, 2%, 1% or even less. Typically, the solubilizer may be present in an amount of about 1% to about 100%, more typically about 5% to about 25% by weight.

The composition can further include one or more pharmaceutically acceptable additives and excipients. Such additives and excipients include, without limitation, detackifiers, antifoaming agents, buffering agents, polymers, antioxidants, preservatives, chelating agents, viscomodulators, tonicifiers, flavorants, colorants, odorants, opacifiers, suspending agents, binders, fillers, plasticizers, lubricants, and mixtures thereof.

In addition, an acid or a base may be incorporated into the composition to facilitate processing, to enhance stability, or for other reasons. Examples of pharmaceutically acceptable bases include amino acids, amino acid esters, ammonium hydroxide, potassium hydroxide, sodium hydroxide, sodium hydrogen carbonate, aluminum hydroxide, calcium carbonate, magnesium hydroxide, magnesium aluminum silicate, synthetic aluminum silicate, synthetic hydrocalcite, magnesium aluminum hydroxide, diisopropylethylamine, ethanolamine, ethylenediamine, triethanolamine, triethylamine, triisopropanolamine, trimethylamine, and tris(hydroxymethyl)aminomethane (TRIS). Also suitable are bases that are salts of a pharmaceutically acceptable acid, such as acetic acid, acrylic acid, adipic acid, alginic acid, alkanesulfonic acid, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, oxalic acid, para-bromophenylsulfonic acid, propionic acid, p-toluenesulfonic acid, salicylic acid, stearic acid, succinic acid, tannic acid, tartaric acid, thioglycolic acid, toluenesulfonic acid, and uric acid. Salts of polyprotic acids, such as sodium phosphate, disodium hydrogen phosphate, and sodium dihydrogen phosphate can also be used. When the base is a salt, the cation can be any convenient and pharmaceutically acceptable cation, such as ammonium, alkali metals and alkaline earth metals. Example may include sodium, potassium, lithium, magnesium, calcium and ammonium.

Suitable acids are pharmaceutically acceptable organic or inorganic acids. Examples of suitable inorganic acids include hydrochloric acid, hydrobromic acid, hydriodic acid, sulfuric acid, nitric acid, boric acid, and phosphoric acid. Examples of suitable organic acids include acetic acid, acrylic acid, adipic acid, alginic acid, alkanesulfonic acids, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, methanesulfonic acid, oxalic acid, para-bromophenylsulfonic acid, propionic acid, p-toluenesulfonic acid, salicylic acid, stearic acid, succinic acid, tannic acid, tartaric acid, thioglycolic acid, toluenesulfonic acid and uric acid.

### Pharmaceutical Compositions for Injection

In preferred embodiments, the invention provides a pharmaceutical composition for injection , for use in the treatment of cancer containing the combination of the CD19 inhibitor selected from an anti-CD19 antibody or an anti-CD19 chimeric antigen receptor, or an antigen-binding fragment or conjugate thereof and BTK inhibitor of Formula (II), and a pharmaceutical excipient suitable for injection. Components and amounts of agents in the compositions are as described herein.

The forms in which the compositions for use in the present invention may be incorporated for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles.

Aqueous solutions in saline are also conventionally used for injection. Ethanol, glycerol, propylene glycol and liquid polyethylene glycol (and suitable mixtures thereof), cyclodextrin derivatives, and vegetable oils may also be employed. The proper fluidity can be maintained, for example, by the use of a coating, such as lecithin, for the maintenance of the required particle size in the case of dispersion and by the use of surfactants. The prevention of the action of microorganisms can be brought about by various antibacterial and antifungal agents, for example, parabens, chlorobutanol, phenol, sorbic acid and thimerosal.

Sterile injectable solutions are prepared by incorporating the combination of the CD19 inhibitor selected from an anti-CD19 antibody or an anti-CD19 chimeric antigen receptor, or an antigen-binding fragment or conjugate thereof and BTK inhibitor of Formula (II) in the required amounts in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, certain desirable methods of preparation are vacuum-drying and freeze-drying techniques which yield a powder of the active ingredient plus any additional desired ingredient from a previously sterile-filtered solution thereof.

### Pharmaceutical Compositions for Topical Delivery

In preferred embodiments, the invention provides a pharmaceutical composition for transdermal delivery for use in the treatment of cancer containing the combination of the CD19 inhibitor selected from an anti-CD19 antibody or an anti-CD19 chimeric antigen receptor, or an antigen-binding fragment or conjugate thereof and BTK inhibitor for Formula (II) and a pharmaceutical excipient suitable for transdermal delivery.

Compositions for use in the present invention can be formulated into preparations in solid, semi-solid, or liquid forms suitable for local or topical administration, such as gels, water soluble jellies, creams, lotions, suspensions, foams, powders, slurries, ointments, solutions, oils, pastes, suppositories, sprays, emulsions, saline solutions, dimethylsulfoxide (DMSO)-based solutions. In general, carriers with higher densities are capable of providing an area with a prolonged exposure to the active ingredients. In contrast, a solution formulation may provide more immediate exposure of the active ingredient to the chosen area.

The pharmaceutical compositions also may comprise suitable solid or gel phase carriers or excipients, which are compounds that allow increased penetration of, or assist in the delivery of, therapeutic molecules across the stratum corneum permeability barrier of the skin. There are many of these penetration-enhancing molecules known to those trained in the art of topical formulation. Examples of such carriers and excipients include humectants (*e.g*., urea), glycols (*e.g.,* propylene glycol), alcohols (*e.g.,* ethanol), fatty acids (*e.g.,* oleic acid), surfactants (*e.g.,* isopropyl myristate and sodium lauryl sulfate), pyrrolidones, glycerol monolaurate, sulfoxides, terpenes (*e.g*., menthol), amines, amides, alkanes, alkanols, water, calcium carbonate, calcium phosphate, various sugars, starches, cellulose derivatives, gelatin, and polymers such as polyethylene glycols.

Another exemplary formulation for use in the present invention employs transdermal delivery devices ("patches"). Such transdermal patches may be used to provide continuous or discontinuous infusion of the combination of the CD19 inhibitor selected from an anti-CD19 antibody or an anti-CD19 chimeric antigen receptor, or an antigen-binding fragment or conjugate thereof and BTK inhibitor of Formula (II) in controlled amounts, either with or without another active pharmaceutical ingredient.

The construction and use of transdermal patches for the delivery of pharmaceutical agents is well known in the art. See, *e.g.,* U.S. Patent Nos. 5,023,252; 4,992,445 and 5,001,139. Such patches may be constructed for continuous, pulsatile, or on demand delivery of pharmaceutical agents.

### Pharmaceutical Compositions for Inhalation

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described supra. Preferably the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices that deliver the formulation in an appropriate manner. Dry powder inhalers may also be used to provide inhaled delivery of the compositions.

### Other Pharmaceutical Compositions

Pharmaceutical compositions may also be prepared from compositions described herein and one or more pharmaceutically acceptable excipients suitable for sublingual, buccal, rectal, intraosseous, intraocular, intranasal, epidural, or intraspinal administration. Preparations for such pharmaceutical compositions are well-known in the art. See, *e.g*., Anderson, Philip O.; Knoben, James E.; Troutman, William G, eds., Handbook of Clinical Drug Data, Tenth Edition, McGraw-Hill, 2002; and Pratt and Taylor, eds., Principles of Drug Action, Third Edition, Churchill Livingston, N.Y., 1990.

Administration of the combination of the CD19 inhibitor selected from an anti-CD19 antibody or an anti-CD19 chimeric antigen receptor, or an antigen-binding fragment or conjugate thereof and BTK inhibitor of Formula (II) or pharmaceutical compositions of these compounds can be effected by any method that enables delivery of the compounds to the site of action. These methods include oral routes, intraduodenal routes, parenteral injection (including intravenous, intraarterial, subcutaneous, intramuscular, intravascular, intraperitoneal or infusion), topical (*e.g.,* transdermal application), rectal administration, via local delivery by catheter or stent or through inhalation. The combination of compounds can also be administered intraadiposally or intrathecally.

The compositions for use in the invention may also be delivered via an impregnated or coated device such as a stent, for example, or an artery-inserted cylindrical polymer. Such a method of administration may, for example, aid in the prevention or amelioration of restenosis following procedures such as balloon angioplasty. Without being bound by theory, compounds for use in the invention may slow or inhibit the migration and proliferation of smooth muscle cells in the arterial wall which contribute to restenosis. A compound for use in the invention may be administered, for example, by local delivery from the struts of a stent, from a stent graft, from grafts, or from the cover or sheath of a stent. In some embodiments, a compound for use in the invention is admixed with a matrix. Such a matrix may be a polymeric matrix, and may serve to bond the compound to the stent. Polymeric matrices suitable for such use, include, for example, lactone-based polyesters or copolyesters such as polylactide, polycaprolactonglycolide, polyorthoesters, polyanhydrides, polyaminoacids, polysaccharides, polyphosphazenes, poly(ether-ester) copolymers (e.g. PEO-PLLA); polydimethylsiloxane, polyethylenevinylacetate), acrylate-based polymers or copolymers (*e.g*., polyhydroxyethyl methylmethacrylate, polyvinyl pyrrolidinone), fluorinated polymers such as polytetrafluoroethylene and cellulose esters. Suitable matrices may be nondegrading or may degrade with time, releasing the compound or compounds. The combination of the CD19 inhibitor selected from an anti-CD19 antibody or an anti-CD19 chimeric antigen receptor, or an antigen-binding fragment or conjugate thereof and BTK inhibitor of Formula (II) may be applied to the surface of the stent by various methods such as dip/spin coating, spray coating, dipcoating, and/or brush-coating. The compounds may be applied in a solvent and the solvent may be allowed to evaporate, thus forming a layer of compound onto the stent. Alternatively, the compound may be located in the body of the stent or graft, for example in microchannels or micropores. When implanted, the compound diffuses out of the body of the stent to contact the arterial wall. Such stents may be prepared by dipping a stent manufactured to contain such micropores or microchannels into a solution of the compound for use in the invention in a suitable solvent, followed by evaporation of the solvent. Excess drug on the surface of the stent may be removed via an additional brief solvent wash. In yet other embodiments, compounds for use in the invention may be covalently linked to a stent or graft. A covalent linker may be used which degrades *in vivo,* leading to the release of the compound for use in the invention. Any bio-labile linkage may be used for such a purpose, such as ester, amide or anhydride linkages.

Exemplary parenteral administration forms include solutions or suspensions of active compound in sterile aqueous solutions, for example, aqueous propylene glycol or dextrose solutions. Such dosage forms can be suitably buffered, if desired.

The invention also provides kits as defined above for use in the treatment of cancer. The kits include each of the CD 19 inhibitor selected from an anti-CD19 antibody or an anti-CD19 chimeric antigen receptor, or an antigen-binding fragment or conjugate thereof and BTK inhibitor of Formula (II), either alone or in combination in suitable packaging, and written material that can include instructions for use, discussion of clinical studies and listing of side effects. Such kits may also include information, such as scientific literature references, package insert materials, clinical trial results, and/or summaries of these, which indicate or establish the activities and/or advantages of the composition, and/or which describe dosing, administration, side effects, drug interactions, or other information useful to the health care provider. Such information may be based on the results of various studies, for example, studies using experimental animals involving in vivo models and studies based on human clinical trials. The kit may further contain another active pharmaceutical ingredient. In selected embodiments, the CD19 inhibitor selected from an anti-CD19 antibody or an anti-CD19 chimeric antigen receptor, or an antigen-binding fragment or conjugate thereof and BTK inhibitor of Formula (II) and another active pharmaceutical ingredient are provided as separate compositions in separate containers within the kit. In selected embodiments, the CD19 inhibitor selected from an anti-CD19 antibody or an anti-CD19 chimeric antigen receptor, or an antigen-binding fragment or conjugate thereof and BTK inhibitor of Formula (II) and the agent are provided as a single composition within a container in the kit. Suitable packaging and additional articles for use (e.g., measuring cup for liquid preparations, and foil wrapping to minimize exposure to air) are known in the art and may be included in the kit. Kits described herein can be provided, marketed and/or promoted to health providers, including physicians, nurses, pharmacists, and formulary officials. Kits may also, in selected embodiments, be marketed directly to the consumer.

In some embodiments, the invention provides a kit for use in the treatment of cancer comprising (1) a composition comprising a therapeutically effective amount of a CD19 inhibitor selected from an anti-CD19 antibody or an anti-CD19 chimeric antigen receptor, or an antigen-binding fragment or conjugate thereof; and (2) a composition comprising a therapeutically effective amount of a BTK inhibitor of Formula (II) or a pharmaceutically acceptable salt thereof. These compositions are typically pharmaceutical compositions. The kit is for co-administration of the CD 19 inhibitor selected from an anti-CD19 antibody or an anti-CD19 chimeric antigen receptor, or an antigen-binding fragment or conjugate thereof and the BTK inhibitor of Formula (II), either simultaneously or separately.

In some embodiments, the invention provides a kit for use in the treatment of cancer comprising (1) a composition comprising a therapeutically effective amount of CD19 inhibitor selected from an anti-CD19 antibody or an anti-CD19 chimeric antigen receptor, or an antigen-binding fragment or conjugate thereof; (2) a composition comprising a therapeutically effective amount of a BTK inhibitor of Formula (II) or a pharmaceutically acceptable salt thereof; and/or (3) a composition comprising a therapeutically effective amount of an anti-CD20 antibody selected from the group consisting of rituximab, obinutuzumab, ofatumumab, veltuzumab, tositumomab, ibritumomab, and fragments, derivatives, conjugates, variants, radioisotope-labeled complexes, and biosimilars thereof. These compositions are typically pharmaceutical compositions. The kit is for co-administration of the CD19 inhibitor selected from an anti-CD19 antibody or an anti-CD19 chimeric antigen receptor, or an antigen-binding fragment or conjugate thereof, the BTK inhibitor of Formula (II), and/or the anti-CD20 antibody, either simultaneously or separately.

In some embodiments, the invention provides a kit for use in the treatment of cancer comprising (1) a composition comprising a therapeutically effective amount of a CD19 inhibitor selected from an anti-CD19 antibody or an anti-CD19 chimeric antigen receptor, or an antigen-binding fragment or conjugate thereof; (2) a composition comprising a therapeutically effective amount of a BTK inhibitor of Formula (II) or a pharmaceutically acceptable salt thereof; and/or (3) a composition comprising a therapeutically effective amount of gemcitabine, albumin-bound paclitaxel, bendamustine, fludarabine, cyclophosphamide, chlorambucil, an anticoagulant or antiplatelet active pharmaceutical ingredient, or combinations thereof. These compositions are typically pharmaceutical compositions. The kit is for co-administration of the CD19 inhibitor selected from an anti-CD19 antibody or an anti-CD19 chimeric antigen receptor, or an antigen-binding fragment or conjugate thereof, BTK inhibitor of Formula (II), gemcitabine, albumin-bound paclitaxel, bendamustine, fludarabine, cyclophosphamide, chlorambucil, and/or the anticoagulant or the antiplatelet active pharmaceutical ingredient, either simultaneously or separately.

In some embodiments, the invention provides a kit for use in the treatment of cancer comprising (1) a composition comprising a therapeutically effective amount of a CD19 inhibitor selected from an anti-CD19 antibody or an anti-CD19 chimeric antigen receptor, or an antigen-binding fragment or conjugate thereof; (2) a composition comprising a therapeutically effective amount of a BTK inhibitor of Formula (II) or a pharmaceutically acceptable salt thereof; (3) a composition comprising a therapeutically effective amount of an anti-CD20 antibody selected from the group consisting of rituximab, obinutuzumab, ofatumumab, veltuzumab, tositumomab, ibritumomab, and fragments, derivatives, conjugates, variants, radioisotope-labeled complexes, biosimilars thereof, and combinations thereof; and/or (4) a composition comprising a therapeutically effective amount of gemcitabine, albumin-bound paclitaxel, bendamustine, fludarabine, cyclophosphamide, chlorambucil, an anticoagulant or antiplatelet active pharmaceutical ingredient, or combinations thereof. These compositions are typically pharmaceutical compositions. The kit is for co-administration of the CD19 inhibitor selected from an anti-CD19 antibody or an anti-CD19 chimeric antigen receptor, or an antigen-binding fragment or conjugate thereof, BTK inhibitor of Formula (II), anti-CD20 antibody, gemcitabine, albumin-bound paclitaxel, bendamustine, fludarabine, cyclophosphamide, chlorambucil, and/or the anticoagulant or the antiplatelet active pharmaceutical ingredient, either simultaneously or separately.

The kits described above are preferably for use in the treatment of cancer. In preferred embodiments, the kits are for use in treating solid tumor cancers, lymphomas and leukemias.

The kits of the present invention are for use in the treatment of cancer. In a preferred embodiment, the kits of the present invention are for use in the treatment of a cancer selected from the group consisting of bladder cancer, squamous cell carcinoma including head and neck cancer, pancreatic ductal adenocarcinoma (PDA), pancreatic cancer, colon carcinoma, mammary carcinoma, breast cancer, fibrosarcoma, mesothelioma, renal cell carcinoma, lung carcinoma, thyoma, prostate cancer, colorectal cancer, ovarian cancer, acute myeloid leukemia, thymus cancer, brain cancer, squamous cell cancer, skin cancer, eye cancer, retinoblastoma, melanoma, intraocular melanoma, oral cavity and oropharyngeal cancers, gastric cancer, stomach cancer, cervical cancer, renal cancer, kidney cancer, liver cancer, ovarian cancer, esophageal cancer, testicular cancer, gynecological cancer, thyroid cancer, acquired immune deficiency syndrome (AIDS)-related cancers (e.g., lymphoma and Kaposi's sarcoma), viral-induced cancer, glioblastoma, esophogeal tumors, hematological neoplasms, non-small-cell lung cancer, chronic myelocytic leukemia, diffuse large B-cell lymphoma, esophagus tumor, follicle center lymphoma, head and neck tumor, hepatitis C virus infection, hepatocellular carcinoma, Hodgkin's disease, metastatic colon cancer, multiple myeloma, non-Hodgkin's lymphoma, indolent non-Hodgkin's lymphoma, ovary tumor, pancreas tumor, renal cell carcinoma, small-cell lung cancer, stage IV melanoma, chronic lymphocytic leukemia, B-cell acute lymphoblastic leukemia (ALL), mature B-cell ALL, follicular lymphoma, mantle cell lymphoma, and Burkitt's lymphoma.

### Dosages and Dosing Regimens

The amounts of BTK inhibitor of Formula (II) and CD19 inhibitor selected from an anti-CD19 antibody or an anti-CD19 chimeric antigen receptor, or an antigen-binding fragment or conjugate thereof administered will be dependent on the human or mammal being treated, the severity of the disorder or condition, the rate of administration, the disposition of the compounds and the discretion of the prescribing physician. However, an effective dosage of each is in the range of about 0.001 to about 100 mg per kg body weight per day, such as about 1 to about 35 mg/kg/day, in single or divided doses. For a 70 kg human, this would amount to about 0.05 to 7 g/day, such as about 0.05 to about 2.5 g/day. In some instances, dosage levels below the lower limit of the aforesaid range may be more than adequate, while in other cases still larger doses may be employed without causing any harmful side effect - *e.g.,* by dividing such larger doses into several small doses for administration throughout the day. The dosage of BTK inhibitor of Formula (II) and CD19 inhibitor selected from an anti-CD19 antibody or an anti-CD19 chimeric antigen receptor, or an antigen-binding fragment or conjugate thereof may be provided in units of mg/kg of body mass or in mg/m² of body surface area. In an embodiment, the ratio of the dose of the CD19 inhibitor selected from an anti-CD19 antibody or an anti-CD19 chimeric antigen receptor, or an antigen-binding fragment or conjugate thereof to the dose of the BTK inhibitor of Formula (II) in mg/kg or in mg/m² is in the range from 10:1 to 1:10, preferably from 2.5:1 to 1:2.5, and more preferably about 1:1. In an embodiment, the ratio of the CD19 inhibitor selected from an anti-CD19 antibody or an anti-CD19 chimeric antigen receptor, or an antigen-binding fragment or conjugate thereof to the BTK inhibitor of Formula (II) in mg/kg or in mg/m² is selected from the group consisting of about 20:1, about 19:1, about 18:1, about 17:1, about 16:1, about 15:1, about 14:1, about 13:1, about 12:1, about 11:1, about 10:1, about 9:1, about 8:1, about 7:1, about 6:1, about 5:1, about 4:1, about 3:1, about 2:1, about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, and about 1:20.

In some embodiments, the combination of the CD19 inhibitor selected from an anti-CD19 antibody or an anti-CD19 chimeric antigen receptor, or an antigen-binding fragment or conjugate thereof and BTK inhibitor of Formula (II) is administered in a single dose. Such administration may be by injection, *e.g*., intravenous injection, in order to introduce the CD19 inhibitor selected from an anti-CD19 antibody or an anti-CD19 chimeric antigen receptor, or an antigen-binding fragment or conjugate thereof and BTK inhibitor of Formula (II) quickly. However, other routes, including the preferred oral route, may be used as appropriate. A single dose of the combination of the CD19 inhibitor selected from an anti-CD19 antibody or an anti-CD19 chimeric antigen receptor, or an antigen-binding fragment or conjugate thereof and BTK inhibitor of Formula (II) may also be used for treatment of an acute condition.

In some embodiments, the combination of the CD19 inhibitor selected from an anti-CD19 antibody or an anti-CD19 chimeric antigen receptor, or an antigen-binding fragment or conjugate thereof and BTK inhibitor of Formula (II) is administered in multiple doses. Dosing may be once, twice, three times, four times, five times, six times, or more than six times per day. Dosing may be once a month, once every two weeks, once a week, or once every other day. In other embodiments, the combination of the CD19 inhibitor selected from an anti-CD19 antibody or an anti-CD19 chimeric antigen receptor, or an antigen-binding fragment or conjugate thereof and BTK inhibitor of Formula (II) is administered about once per day to about 6 times per day. In some embodiments, the combination of the CD19 inhibitor selected from an anti-CD19 antibody or an anti-CD19 chimeric antigen receptor, or an antigen-binding fragment or conjugate thereof and BTK inhibitor of Formula (II) is administered once daily, while in other embodiments, the combination of the CD19 inhibitor selected from an anti-CD19 antibody or an anti-CD19 chimeric antigen receptor, or an antigen-binding fragment or conjugate thereof, and BTK inhibitor of Formula (II) is administered twice daily, and in other embodiments the combination of the CD 19 inhibitor selected from an anti-CD19 antibody or an anti-CD19 chimeric antigen receptor, or an antigen-binding fragment or conjugate thereof and BTK inhibitor of Formula (II) is administered three times daily. In some embodiments, a BTK inhibitor of Formula (II) disclosed herein is administered in combination with a CD19 inhibitor selected from an anti-CD19 antibody or an anti-CD19 chimeric antigen receptor, or an antigen-binding fragment or conjugate thereof once daily, while in other embodiments a BTK inhibitor of Formula (II) disclosed herein is administered in combination with a CD 19 inhibitor selected from an anti-CD19 antibody or an anti-CD19 chimeric antigen receptor, or an antigen-binding fragment or conjugate thereof twice daily, and in other embodiments a BTK inhibitor of Formula (II) disclosed herein is administered in combination with a CD19 inhibitor selected from an anti-CD19 antibody or an anti-CD19 chimeric antigen receptor, or an antigen-binding fragment or conjugate thereof three times daily. In some embodiments, a CD19 inhibitor selected from an anti-CD19 antibody or an anti-CD19 chimeric antigen receptor, or an antigen-binding fragment or conjugate thereof disclosed herein, wherein the CD19 inhibitor is a T cell or NK cell expressing an anti-CD19 chimeric antigen receptor, is administered as a single infusion, and a BTK inhibitor of Formula (II) disclosed herein is administered once or twice daily by oral administration for a period of at least 1 week.

Administration of the active pharmaceutical ingredients of the invention may continue as long as necessary. In selected embodiments, the combination of the CD19 inhibitor selected from an anti-CD19 antibody or an anti-CD19 chimeric antigen receptor, or an antigen-binding fragment or conjugate thereof and BTK inhibitor of Formula (II) is administered for more than 1, 2, 3, 4, 5, 6, 7, 14, or 28 days. In some embodiments, the combination of the CD19 inhibitor selected from an anti-CD19 antibody or an anti-CD19 chimeric antigen receptor, or an antigen-binding fragment or conjugate thereof and BTK inhibitor of Formula (II) is administered for less than 28, 14, 7, 6, 5, 4, 3, 2, or 1 day. In selected embodiments, the combination of the CD19 inhibitor selected from an anti-CD19 antibody or an anti-CD19 chimeric antigen receptor, or an antigen-binding fragment or conjugate thereof and BTK inhibitor of Formula (II) is administered chronically on an ongoing basis - *e.g.,* for the treatment of chronic effects. In another embodiment the administration of the combination of the CD19 inhibitor selected from an anti-CD19 antibody or an anti-CD19 chimeric antigen receptor, or an antigen-binding fragment or conjugate thereof and BTK inhibitor of Formula (II) continues for less than about 7 days. In yet another embodiment the administration continues for more than about 6, 10, 14, 28 days, two months, six months, or one year. In some cases, continuous dosing is achieved and maintained as long as necessary.

In some embodiments, an effective dosage of a BTK inhibitor of Formula (II) disclosed herein is in the range of about 1 mg to about 500 mg, about 10 mg to about 300 mg, about 20 mg to about 250 mg, about 25 mg to about 200 mg, about 10 mg to about 200 mg, about 20 mg to about 150 mg, about 30 mg to about 120 mg, about 10 mg to about 90 mg, about 20 mg to about 80 mg, about 30 mg to about 70 mg, about 40 mg to about 60 mg, about 45 mg to about 55 mg, about 48 mg to about 52 mg, about 50 mg to about 150 mg, about 60 mg to about 140 mg, about 70 mg to about 130 mg, about 80 mg to about 120 mg, about 90 mg to about 110 mg, about 95 mg to about 105 mg, about 150 mg to about 250 mg, about 160 mg to about 240 mg, about 170 mg to about 230 mg, about 180 mg to about 220 mg, about 190 mg to about 210 mg, about 195 mg to about 205 mg, or about 198 to about 202 mg. In some embodiments, an effective dosage of a BTK inhibitor of Formula (II) disclosed herein is about 25 mg, about 50 mg, about 75 mg, about 100 mg, about 125 mg, about 150 mg, about 175 mg, about 200 mg, about 225 mg, or about 250 mg.

In some embodiments, an effective dosage of a BTK inhibitor of Formula (II) disclosed herein is in the range of about 0.01 mg/kg to about 4.3 mg/kg, about 0.15 mg/kg to about 3.6 mg/kg, about 0.3 mg/kg to about 3.2 mg/kg, about 0.35 mg/kg to about 2.85 mg/kg, about 0.15 mg/kg to about 2.85 mg/kg, about 0.3 mg to about 2.15 mg/kg, about 0.45 mg/kg to about 1.7 mg/kg, about 0.15 mg/kg to about 1.3 mg/kg, about 0.3 mg/kg to about 1.15 mg/kg, about 0.45 mg/kg to about 1 mg/kg, about 0.55 mg/kg to about 0.85 mg/kg, about 0.65 mg/kg to about 0.8 mg/kg, about 0.7 mg/kg to about 0.75 mg/kg, about 0.7 mg/kg to about 2.15 mg/kg, about 0.85 mg/kg to about 2 mg/kg, about 1 mg/kg to about 1.85 mg/kg, about 1.15 mg/kg to about 1.7 mg/kg, about 1.3 mg/kg mg to about 1.6 mg/kg, about 1.35 mg/kg to about 1.5 mg/kg, about 2.15 mg/kg to about 3.6 mg/kg, about 2.3 mg/kg to about 3.4 mg/kg, about 2.4 mg/kg to about 3.3 mg/kg, about 2.6 mg/kg to about 3.15 mg/kg, about 2.7 mg/kg to about 3 mg/kg, about 2.8 mg/kg to about 3 mg/kg, or about 2.85 mg/kg to about 2.95 mg/kg. In some embodiments, an effective dosage of a BTK inhibitor of Formula (II) disclosed herein is about 0.35 mg/kg, about 0.7 mg/kg, about 1 mg/kg, about 1.4 mg/kg, about 1.8 mg/kg, about 2.1 mg/kg, about 2.5 mg/kg, about 2.85 mg/kg, about 3.2 mg/kg, or about 3.6 mg/kg.

In some embodiments, an effective dosage of a CD19 inhibitor selected from an anti-CD19 antibody or an anti-CD19 chimeric antigen receptor, or an antigen-binding fragment or conjugate thereof disclosed herein is in the range of about 1 mg to about 500 mg, about 10 mg to about 300 mg, about 20 mg to about 250 mg, about 25 mg to about 200 mg, about 1 mg to about 50 mg, about 5 mg to about 45 mg, about 10 mg to about 40 mg, about 15 mg to about 35 mg, about 20 mg to about 30 mg, about 23 mg to about 28 mg, about 50 mg to about 150 mg, about 60 mg to about 140 mg, about 70 mg to about 130 mg, about 80 mg to about 120 mg, about 90 mg to about 110 mg, or about 95 mg to about 105 mg, about 98 mg to about 102 mg, about 150 mg to about 250 mg, about 160 mg to about 240 mg, about 170 mg to about 230 mg, about 180 mg to about 220 mg, about 190 mg to about 210 mg, about 195 mg to about 205 mg, or about 198 to about 207 mg. In some embodiments, an effective dosage of a CD19 inhibitor disclosed herein is about 25 mg, about 50 mg, about 75 mg, about 100 mg, about 125 mg, about 150 mg, about 175 mg, about 200 mg, about 225 mg, or about 250 mg. In some embodiments, an effective dosage of a CD 19 inhibitor selected from an anti-CD19 antibody or an anti-CD19 chimeric antigen receptor, or an antigen-binding fragment or conjugate thereof disclosed herein, wherein the CD 19 inhibitor is a T cell or an NK cell expressing an anti-CD19 chimeric antigen receptor, is at least 1 × 10⁵ cells, 1 × 10⁶ cells, 1 × 10⁷ cells, 1 × 10⁸ cells, 1 × 10⁹ cells, or 1 × 10¹⁰ cells.

In some embodiments, an effective dosage of a CD19 inhibitor selected from an anti-CD19 antibody or an anti-CD19 chimeric antigen receptor, or an antigen-binding fragment or conjugate thereof disclosed herein is in the range of about 0.01 mg/kg to about 4.3 mg/kg, about 0.15 mg/kg to about 3.6 mg/kg, about 0.3 mg/kg to about 3.2 mg/kg, about 0.35 mg/kg to about 2.85 mg/kg, about 0.01 mg/kg to about 0.7 mg/kg, about 0.07 mg/kg to about 0.65 mg/kg, about 0.15 mg/kg to about 0.6 mg/kg, about 0.2 mg/kg to about 0.5 mg/kg, about 0.3 mg/kg to about 0.45 mg/kg, about 0.3 mg/kg to about 0.4 mg/kg, about 0.7 mg/kg to about 2.15 mg/kg, about 0.85 mg/kg to about 2 mg/kg, about 1 mg/kg to about 1.85 mg/kg, about 1.15 mg/kg to about 1.7 mg/kg, about 1.3 mg/kg to about 1.6 mg/kg, about 1.35 mg/kg to about 1.5 mg/kg, about 1.4 mg/kg to about 1.45 mg/kg, about 2.15 mg/kg to about 3.6 mg/kg, about 2.3 mg/kg to about 3.4 mg/kg, about 2.4 mg/kg to about 3.3 mg/kg, about 2.6 mg/kg to about 3.15 mg/kg, about 2.7 mg/kg to about 3 mg/kg, about 2.8 mg/kg to about 3 mg/kg, or about 2.85 mg/kg to about 2.95 mg/kg. In some embodiments, an effective dosage of a CD19 inhibitor selected from an anti-CD19 antibody or an anti-CD19 chimeric antigen receptor, or an antigen-binding fragment or conjugate thereof disclosed herein is about 0.4 mg/kg, about 0.7 mg/kg, about 1 mg/kg, about 1.4 mg/kg, about 1.8 mg/kg, about 2.1 mg/kg, about 2.5 mg/kg, about 2.85 mg/kg, about 3.2 mg/kg, or about 3.6 mg/kg.

In some embodiments, a combination of a BTK inhibitor of Formula (II) and a CD19 inhibitor selected from an anti-CD19 antibody or an anti-CD19 chimeric antigen receptor, or an antigen-binding fragment or conjugate thereof is administered at a dosage of 10 to 200 mg BID, including 10, 20, 30, 40, 50, 60, 70, 80, 90, 100, or 150 mg BID, for the BTK inhibitor of Formula (II), and 1 to 500 mg BID, including 1, 5, 10, 15, 25, 50, 75, 100, 150, 200, 300, 400, or 500 mg BID for the CD 19 inhibitor selected from an anti-CD19 antibody or an anti-CD19 chimeric antigen receptor, or an antigen-binding fragment or conjugate thereof.

In some instances, dosage levels below the lower limit of the aforesaid ranges may be more than adequate, while in other cases still larger doses may be employed without causing any harmful side effect - *e.g.,* by dividing such larger doses into several small doses for administration throughout the day.

An effective amount of the combination of the CD19 inhibitor selected from an anti-CD19 antibody or an anti-CD19 chimeric antigen receptor, or an antigen-binding fragment or conjugate thereof and BTK inhibitor of Formula (II) may be administered in either single or multiple doses by any of the accepted modes of administration of agents having similar utilities, including rectal, buccal, intranasal and transdermal routes, by intra-arterial injection, intravenously, intraperitoneally, parenterally, intramuscularly, subcutaneously, orally, topically, or as an inhalant.

### Use in Treating Solid Tumor Cancers Hematological Malignancies, and Other Diseases

The compositions and combinations of inhibitors described above can be used in treating BTK-mediated disorders and diseases. In a preferred embodiment, they are for use in treating cancer.

In some embodiments, the invention provides a pharmaceutical combination for use in treating cancer in a mammal that comprises a therapeutically effective amount of a CD19 inhibitor selected from an anti-CD19 antibody or an anti-CD19 chimeric antigen receptor, or an antigen-binding fragmentor conjugate thereof and a BTK inhibitor of Formula (II) or a pharmaceutically acceptable salt thereof.

In some embodiments, the cancer is a solid tumor cancer selected from the group consisting of bladder cancer, squamous cell carcinoma, head and neck cancer, pancreatic ductal adenocarcinoma (PDA), pancreatic cancer, colon carcinoma, mammary carcinoma, breast cancer, fibrosarcoma, mesothelioma, renal cell carcinoma, lung carcinoma, thyoma, prostate cancer, colorectal cancer, ovarian cancer, acute myeloid leukemia, thymus cancer, brain cancer, squamous cell cancer, skin cancer, eye cancer, retinoblastoma, melanoma, intraocular melanoma, oral cavity cancer, oropharyngeal cancer, gastric cancer, stomach cancer, cervical cancer, renal cancer, kidney cancer, liver cancer, ovarian cancer, prostate cancer, colorectal cancer, esophageal cancer, testicular cancer, gynecological cancer, thyroid cancer, acquired immune deficiency syndrome (AIDS)-related cancers (e.g., lymphoma and Kaposi's sarcoma), viral-induced cancers such as cervical carcinoma (human papillomavirus), B-cell lymphoproliferative disease, nasopharyngeal carcinoma (Epstein-Barr virus), Kaposi's sarcoma and primary effusion lymphomas (Kaposi's sarcoma herpesvirus), hepatocellular carcinoma (hepatitis B and hepatitis C viruses), and T-cell leukemias (Human T-cell leukemia virus-1), glioblastoma, esophogeal tumors, head and neck tumor, metastatic colon cancer, head and neck squamous cell carcinoma, ovary tumor, pancreas tumor, renal cell carcinoma, hematological neoplasms, small-cell lung cancer, non-small-cell lung cancer, stage IV melanoma, and glioma.

In some embodiments, the cancer is a B cell hematological malignancy selected from the group consisting of chronic lymphocytic leukemia (CLL), small lymphocytic leukemia (SLL), non-Hodgkin's lymphoma (NHL), diffuse large B cell lymphoma (DLBCL), follicular lymphoma (FL), mantle cell lymphoma (MCL), Hodgkin's lymphoma, B cell acute lymphoblastic leukemia (B-ALL), Burkitt's lymphoma, Waldenström's macroglobulinemia (WM), Burkitt's lymphoma, multiple myeloma, myelodysplatic syndromes, or myelofibrosis. In an embodiment, the invention relates to a method of treating a cancer in a mammal, wherein the cancer is chronic myelocytic leukemia, acute myeloid leukemia, DLBCL (including activated B-cell (ABC) and germinal center B-cell (GCB) subtypes), follicle center lymphoma, Hodgkin's disease, multiple myeloma, indolent non-Hodgkin's lymphoma, and mature B-cell ALL.

In some embodiments, the cancer is a subtype of CLL. A number of subtypes of CLL have been characterized. CLL is often classified for immunoglobulin heavy-chain variableregion (IgV_{H}) mutational status in leukemic cells. R. N. Damle, et al., Blood 1999, 94, 1840-47; T. J. Hamblin, et al., Blood 1999, 94, 1848-54. Patients with IgV_{H} mutations generally survive longer than patients without IgV_{H} mutations. ZAP70 expression (positive or negative) is also used to characterize CLL. L. Z. Rassenti, et al., N. Engl. J. Med. 2004, 351, 893-901. The methylation of ZAP-70 at CpG3 is also used to characterize CLL, for example by pyrosequencing. R. Claus, et al., J. Clin. Oncol. 2012, 30, 2483-91; J. A. Woyach, et al., Blood 2014, 123, 1810-17. CLL is also classfied by stage of disease under the Binet or Rai criteria. J. L. Binet, et al., Cancer 1977, 40, 855-64; K. R. Rai, T. Han, Hematol. Oncol. Clin. North Am. 1990, 4, 447-56. Other common mutations, such as 11q deletion, 13q deletion, and 17p deletion can be assessed using well-known techniques such as fluorescence *in situ* hybridization (FISH). In an embodiment, the invention relates to a method of treating a CLL in a human, wherein the CLL is selected from the group consisting of IgV_{H} mutation negative CLL, ZAP-70 positive CLL, ZAP-70 methylated at CpG3 CLL, CD38 positive CLL, chronic lymphocytic leukemia characterized by a 17p13.1 (17p) deletion, and CLL characterized by a 11q22.3 (11q) deletion.

In some embodiments, the cancer is a CLL wherein the CLL has undergone a Richter's transformation. Methods of assessing Richter's transformation, which is also known as Richter's syndrome, are described in P. Jain and S. O'Brien, Oncology, 2012, 26, 1146-52. Richter's transformation is a subtype of CLL that is observed in 5-10% of patients. It involves the development of aggressive lymphoma from CLL and has a generally poor prognosis.

In some embodiments, the cancer is a CLL or SLL in a patient, wherein the patient is sensitive to lymphocytosis. In an embodiment, the invention relates to a method of treating CLL or SLL in a patient, wherein the patient exhibits lymphocytosis caused by a disorder selected from the group consisting of a viral infection, a bacterial infection, a protozoal infection, or a post-splenectomy state. In an embodiment, the viral infection in any of the foregoing embodiments is selected from the group consisting of infectious mononucleosis, hepatitis, and cytomegalovirus. In an embodiment, the bacterial infection in any of the foregoing embodiments is selected from the group consisting of pertussis, tuberculosis, and brucellosis.

In some embodiments, the cancer is selected from the group consisting of myeloproliferative disorders (MPDs), myeloproliferative neoplasms, polycythemia vera (PV), essential thrombocythemia (ET), primary myelofibrosis (PMF), myelodysplastic syndrome, chronic myelogenous leukemia (BCR-ABL1-positive), chronic neutrophilic leukemia, chronic eosinophilic leukemia, or mastocytosis.

Efficacy of the methods, compounds, and combinations of compounds described herein in treating, preventing and/or managing the indicated diseases or disorders can be tested using various animal models known in the art. Models for determining efficacy of treatments for pancreatic cancer are described in Herreros-Villanueva, et al., World J. Gastroenterol. 2012, 18, 1286-1294. Models for determining efficacy of treatments for breast cancer are described, e.g., in Fantozzi, Breast Cancer Res. 2006, 8, 212. Models for determining efficacy of treatments for ovarian cancer are described, *e.g.,* in Mullany, et al., Endocrinology 2012, 153, 1585-92; and Fong, et al., J. Ovarian Res. 2009, 2, 12. Models for determining efficacy of treatments for melanoma are described, *e.g.,* in Damsky, et al., Pigment Cell & Melanoma Res. 2010, 23, 853-859. Models for determining efficacy of treatments for lung cancer are described, *e.g*., in Meuwissen, et al., Genes & Development, 2005, 19, 643-664. Models for determining efficacy of treatments for lung cancer are described, *e.g.,* in Kim, Clin. Exp. Otorhinolaryngol. 2009, 2, 55-60; and Sano, Head Neck Oncol. 2009, 1, 32. Models for determining efficacy of treatments for colorectal cancer, including the CT26 model, are described in Castle, et al., BMC Genomics, 2013, 15, 190; Endo, et al., Cancer Gene Therapy, 2002, 9, 142-148; Roth et al., Adv. Immunol. 1994, 57, 281-351; Fearon, et al., Cancer Res. 1988, 48, 2975-2980. Models for determining the efficacy of treatments for DLBCL included the PiBCL1 murine model and BALB/c (haplotype H-2^{d}) mice. Illidge, et al., Cancer Biother. & Radiopharm. 2000, 15, 571-80. Models for determining the efficacy of treatments for NHL include the 38C13 murine model with C3H/HeN (haplotype 2-H^{k}) mice or alternatively the 38C13 Her2/neu model. Timmerman, et al., Blood, 2001, 97, 1370-77; Penichet, et al., Cancer Immunolog. Immunother. 2000, 49, 649-662. Models for determining the efficacy of treatments for CLL include the BCL1 model using BALB/c (haplotype H-2^{d}) mice. Dutt, et al., Blood, 2011, 117, 3230-29.

In selected embodiments, the invention provides a pharmaceutical combination for use in treating a solid tumor cancer with a composition including a combination of a CD19 inhibitor selected from an anti-CD19 antibody or an anti-CD19 chimeric antigen receptor, or an antigen-binding fragment or conjugate thereof and a BTK inhibitor of Formula (II), wherein the dose is effective to inhibit signaling between the solid tumor cells and at least one microenvironment selected from the group consisting of macrophages, monocytes, mast cells, helper T cells, cytotoxic T cells, regulatory T cells, natural killer cells, myeloid-derived suppressor cells, regulatory B cells, neutrophils, dendritic cells, and fibroblasts. In an embodiment, the invention provides a pharmaceutical combination for use in treating pancreatic cancer, breast cancer, ovarian cancer, melanoma, lung cancer, head and neck cancer, and colorectal cancer using a combination of a BTK inhibitor of Formula (II), a CD19 inhibitor selected from an anti-CD19 antibody or an anti-CD19 chimeric antigen receptor, or an antigen-binding fragment or conjugate thereof, and gemcitabine, or a pharmaceutically-acceptable salt, cocrystal, hydrate, solvate, or prodrug thereof.

In some embodiments, the invention provides pharmaceutical compositions of a combination of a CD 19 inhibitor selected from an anti-CD19 antibody or an anti-CD19 chimeric antigen receptor, or an antigen-binding fragment or conjugate thereof and a BTK inhibitor of Formula (II) for use in the treatment of cancer as described herein. In some embodiments, the invention provides pharmaceutical compositions of a combination of a CD19 inhibitor selected from an anti-CD19 antibody or an anti-CD19 chimeric antigen receptor, or an antigen-binding fragment or conjugate thereof and a BTK inhibitor of Formula (II) for use in the treatment of disorders such as myeloproliferative disorders (MPDs), myeloproliferative neoplasms, polycythemia vera (PV), essential thrombocythemia (ET), primary myelofibrosis (PMF), myelodysplastic syndrome, chronic myelogenous leukemia (BCR-ABL1-positive), chronic neutrophilic leukemia, chronic eosinophilic leukemia, or mastocytosis . The invention further provides a composition as described herein for the prevention of blastocyte implantation in a mammal.

### Combinations of BTK Inhibitors and CD19 Inhibitors with Anti-CD20 Antibodies

The combinations of the BTK inhibitors of Formula (II) with CD19 inhibitors selected from an anti-CD19 antibody or an anti-CD19 chimeric antigen receptor, or an antigen-binding fragment or conjugate thereof may also be safely co-administered with immunotherapeutic antibodies such as the anti-CD20 antibodies rituximab, obinutuzumab, ofatumumab, veltuzumab, tositumomab, and ibritumomab, and or antigen-binding fragments, derivatives, conjugates, variants, and radioisotope-labeled complexes thereof, which may be given alone or with conventional chemotherapeutic active pharmaceutical ingredients such as those described herein. In an embodiment, the foregoing combinations exhibit synergistic effects that may result in greater efficacy, less side effects, the use of less active pharmaceutical ingredient to achieve a given clinical result, or other synergistic effects.

In an embodiment, the invention provides a pharmaceutical combination for use in treating a hematological malignancy or a solid tumor cancer in a human comprising a BTK inhibitor of Formula (2) or a pharmaceutically acceptable salt thereof, and a CD19 inhibitor selected from an anti-CD19 antibody or an anti-CD19 chimeric antigen receptor, or an antigen-binding fragment or conjugate, thereof, and further comprising an anti-CD20 antibody, wherein the anti-CD20 antibody is a monoclonal antibody or an antigen-binding fragment, derivative, conjugate, variant, or radioisotope-labeled complex thereof. In an embodiment, the invention provides a pharmaceutical combination for use in treating a hematological malignancy or a solid tumor cancer in a human comprising a BTK inhibitor of Formula (2) or a pharmaceutically acceptable salt thereof, a CD19 inhibitor selected from an anti-CD19 antibody or an anti-CD19 chimeric antigen receptor, or an antigen-binding fragment or conjugate thereof, and further comprising an anti-CD20 antibody, wherein the anti-CD20 antibody is an anti-CD20 monoclonal antibody or an antigen-binding fragment, derivative, conjugate, variant, or radioisotope-labeled complex thereof, and wherein the anti-CD20 antibody specifically binds to human CD20 with a K_{D} selected from the group consisting of 1×10⁻⁷ M or less, 5×10⁻⁸ M or less, 1×10⁻⁸ M or less, and 5×10⁻⁹ M or less.

In an embodiment, the invention provides a pharmaceutical combination for use in treating a hematological malignancy or a solid tumor cancer in a human comprising a BTK inhibitor of Formula (2) or a pharmaceutically acceptable salt thereof, and a CD19 inhibitor selected from an anti-CD19 antibody or an anti-CD19 chimeric antigen receptor, or an antigen-binding fragment or conjugate thereof, and further comprising an Type I anti-CD20 antibody, or an antigen-binding fragment, derivative, conjugate, variant, or radioisotope-labeled complex thereof. In an embodiment, the invention provides a pharmaceutical combination for use in treating a hematological malignancy or a solid tumor cancer in a human comprising a BTK inhibitor of Formula (2) or a pharmaceutically acceptable salt thereof, and a CD19 inhibitor selected from an anti-CD19 antibody or an anti-CD19 chimeric antigen receptor, or an antigen-binding fragment or conjugate thereof, and further comprising an Type II anti-CD20 antibody, or an antigen-binding fragment, derivative, conjugate, variant, or radioisotope-labeled complex thereof.

In selected embodiments, the combinations of the BTK inhibitor of Formula (II) with CD19 inhibitors selected from an anti-CD19 antibody or an anti-CD19 chimeric antigen receptor, or an antigen-binding fragment or conjugate thereof and the anti-CD20 monoclonal antibody are administered sequentially. In selected embodiments, the combinations of the BTK inhibitor of Formula (II) and CD19 inhibitors selected from an anti-CD19 antibody or an anti-CD19 chimeric antigen receptor, or an antigen-binding fragment or conjugate thereof and the anti-CD20 monoclonal antibody are administered concomitantly. In selected embodiments, the combinations of the BTK inhibitor of Formula (II) with CD19 inhibitors selected from an anti-CD19 antibody or an anti-CD19 chimeric antigen receptor, or an antigen-binding fragment or conjugate thereof are administered before the anti-CD20 monoclonal antibody. In selected embodiments, the combinations of the BTK inhibitor of Formula (II) with CD19 inhibitors selected from an anti-CD19 antibody or an anti-CD19 chimeric antigen receptor, or an antigen-binding fragment or conjugate thereof are administered after the anti-CD20 monoclonal antibody. In selected embodiments, the combinations of the BTK inhibitor of Formula (II) with CD19 inhibitors selected from an anti-CD19 antibody or an anti-CD19 chimeric antigen receptor, or an antigen-binding fragment or conjugate thereof and the anti-CD20 monoclonal antibody are administered over the same time period, and the BTK inhibitor administration continues after the anti-CD20 monoclonal antibody administration is completed.

In an embodiment, the anti-CD20 monoclonal antibody is rituximab, or an antigen-binding fragment, derivative, conjugate, variant, or radioisotope-labeled complex thereof. Rituximab is a chimeric murine-human monoclonal antibody directed against CD20, and its structure comprises an IgG1 kappa immunoglobulin containing murine light- and heavy-chain variable region sequences and human constant region sequences. Rituximab is composed of two heavy chains of 451 amino acids and two light chains of 213 amino acids. The amino acid sequence for the heavy chains of rituximab is set forth in SEQ ID NO:60. The amino acid sequence for the light chains of rituximab is set forth in SEQ ID NO:61. Rituximab is commercially available, and its properties and use in cancer and other diseases is described in more detail in Rastetter, et al., Ann. Rev. Med. 2004, 55, 477-503, and in Plosker and Figgett, Drugs, 2003, 63, 803-43. In an embodiment, the anti-CD20 monoclonal antibody is an anti-CD20 biosimilar monoclonal antibody approved by drug regulatory authorities with reference to rituximab. In an embodiment, the anti-CD20 monoclonal antibody has a heavy chain sequence identity of greater than 90% to SEQ ID NO:60. In an embodiment, the anti-CD20 monoclonal antibody has a light chain sequence identity of greater than 90% to SEQ ID NO:61. In an embodiment, the anti-CD20 monoclonal antibody has a heavy chain sequence identity of greater than 95% to SEQ ID NO:60. In an embodiment, the anti-CD20 monoclonal antibody has a light chain sequence identity of greater than 95% to SEQ ID NO:61. In an embodiment, the anti-CD20 monoclonal antibody has a heavy chain sequence identity of greater than 98% to SEQ ID NO:60. In an embodiment, the anti-CD20 monoclonal antibody has a light chain sequence identity of greater than 98% to SEQ ID NO:61. In an embodiment, the anti-CD20 monoclonal antibody has a heavy chain sequence identity of greater than 99% to SEQ ID NO:60. In an embodiment, the anti-CD20 monoclonal antibody has a light chain sequence identity of greater than 99% to SEQ ID NO:61.

In an embodiment, the anti-CD20 monoclonal antibody is obinutuzumab, or an antigen-binding fragment, derivative, conjugate, variant, or radioisotope-labeled complex thereof. Obinutuzumab is also known as afutuzumab or GA-101. Obinutuzumab is a humanized monoclonal antibody directed against CD20. The amino acid sequence for the heavy chains of obinutuzumab is set forth in SEQ ID NO:62. The amino acid sequence for the light chains of obinutuzumab is set forth in SEQ ID NO:63. Obinutuzumab is commercially available, and its properties and use in cancer and other diseases is described in more detail in Robak, Curr. Opin. Investig. Drugs 2009, 10, 588-96. In an embodiment, the anti-CD20 monoclonal antibody is an anti-CD20 biosimilar monoclonal antibody approved by drug regulatory authorities with reference to obinutuzumab. In an embodiment, the anti-CD20 monoclonal antibody has a heavy chain sequence identity of greater than 90% to SEQ ID NO:62. In an embodiment, the anti-CD20 monoclonal antibody has a light chain sequence identity of greater than 90% to SEQ ID NO:63. In an embodiment, the anti-CD20 monoclonal antibody has a heavy chain sequence identity of greater than 95% to SEQ ID NO:62. In an embodiment, the anti-CD20 monoclonal antibody has a light chain sequence identity of greater than 95% to SEQ ID NO:63. In an embodiment, the anti-CD20 monoclonal antibody has a heavy chain sequence identity of greater than 98% to SEQ ID NO:62. In an embodiment, the anti-CD20 monoclonal antibody has a light chain sequence identity of greater than 98% to SEQ ID NO:63. In an embodiment, the anti-CD20 monoclonal antibody has a heavy chain sequence identity of greater than 99% to SEQ ID NO:62. In an embodiment, the anti-CD20 monoclonal antibody has a light chain sequence identity of greater than 99% to SEQ ID NO:63. In an embodiment, the anti-CD20 monoclonal antibody obinutuzumab is an immunoglobulin G1, anti-(human B-lymphocyte antigen CD20 (membrane-spanning 4-domains subfamily A member 1, B-lymphocyte surface antigen B1, Leu-16 or Bp35)), humanized mouse monoclonal obinutuzumab des-CH31 07 - K -y 1 heavy chain (222-219')-disulfide with humanized mouse monoclonal obinutuzumab κ light chain dimer (228-228":231- 231")-bisdisulfide antibody.

In an embodiment, the anti-CD20 monoclonal antibody is ofatumumab, or an antigen-binding fragment, derivative, conjugate, variant, or radioisotope-labeled complex thereof. Ofatumumab is described in Cheson, J. Clin. Oncol. 2010, 28, 3525-30. The crystal structure of the Fab fragment of ofatumumab has been reported in Protein Data Bank reference 3GIZ and in Du, et al., Mol. Immunol. 2009, 46, 2419-2423. Ofatumumab is commercially available, and its preparation, properties, and use in cancer and other diseases are described in more detail in U.S. Patent No. 8,529,202 B2. In an embodiment, the anti-CD20 monoclonal antibody is an anti-CD20 biosimilar monoclonal antibody approved by drug regulatory authorities with reference to ofatumumab. In an embodiment, the anti-CD20 monoclonal antibody has a variable heavy chain sequence identity of greater than 90% to SEQ ID NO:64. In an embodiment, the anti-CD20 monoclonal antibody has a variable light chain sequence identity of greater than 90% to SEQ ID NO:65. In an embodiment, the anti-CD20 monoclonal antibody has a variable heavy chain sequence identity of greater than 95% to SEQ ID NO:64. In an embodiment, the anti-CD20 monoclonal antibody has a variable light chain sequence identity of greater than 95% to SEQ ID NO:65. In an embodiment, the anti-CD20 monoclonal antibody has a variable heavy chain sequence identity of greater than 98% to SEQ ID NO:64. In an embodiment, the anti-CD20 monoclonal antibody has a variable light chain sequence identity of greater than 98% to SEQ ID NO:65. In an embodiment, the anti-CD20 monoclonal antibody has a variable heavy chain sequence identity of greater than 99% to SEQ ID NO:64. In an embodiment, the anti-CD20 monoclonal antibody has a variable light chain sequence identity of greater than 99% to SEQ ID NO:65. In an embodiment, the anti-CD20 monoclonal antibody has a Fab fragment heavy chain sequence identity of greater than 90% to SEQ ID NO:66. In an embodiment, the anti-CD20 monoclonal antibody has a Fab fragment light chain sequence identity of greater than 90% to SEQ ID NO:67. In an embodiment, the anti-CD20 monoclonal antibody has a Fab fragment heavy chain sequence identity of greater than 95% to SEQ ID NO:66. In an embodiment, the anti-CD20 monoclonal antibody has a Fab fragment light chain sequence identity of greater than 95% to SEQ ID NO:67. In an embodiment, the anti-CD20 monoclonal antibody has a Fab fragment heavy chain sequence identity of greater than 98% to SEQ ID NO:66. In an embodiment, the anti-CD20 monoclonal antibody has a Fab fragment light chain sequence identity of greater than 98% to SEQ ID NO:67. In an embodiment, the anti-CD20 monoclonal antibody has a Fab fragment heavy chain sequence identity of greater than 99% to SEQ ID NO:66. In an embodiment, the anti-CD20 monoclonal antibody has a Fab fragment light chain sequence identity of greater than 99% to SEQ ID NO:67. In an embodiment, the anti-CD20 monoclonal antibody ofatumumab is an immunoglobulin G1, anti-(human B-lymphocyte antigen CD20 (membrane-spanning 4-domains subfamily A member 1, B-lymphocyte surface antigen B1, Leu-16 or Bp35)); human monoclonal ofatumumab-CD20 γ1 heavy chain (225-214')-disulfide with human monoclonal ofatumumab-CD20 κ light chain, dimer (231-231":234-234")-bisdisulfide antibody.

In an embodiment, the anti-CD20 monoclonal antibody is veltuzumab, or an antigen-binding fragment, derivative, conjugate, variant, or radioisotope-labeled complex thereof. Veltuzumab is also known as hA20. Veltuzumab is described in Goldenberg, et al., Leuk. Lymphoma 2010, 51, 747-55. In an embodiment, the anti-CD20 monoclonal antibody is an anti-CD20 biosimilar monoclonal antibody approved by drug regulatory authorities with reference to veltuzumab. In an embodiment, the anti-CD20 monoclonal antibody has a heavy chain sequence identity of greater than 90% to SEQ ID NO:68. In an embodiment, the anti-CD20 monoclonal antibody has a light chain sequence identity of greater than 90% to SEQ ID NO:69. In an embodiment, the anti-CD20 monoclonal antibody has a heavy chain sequence identity of greater than 95% to SEQ ID NO:68. In an embodiment, the anti-CD20 monoclonal antibody has a light chain sequence identity of greater than 95% to SEQ ID NO:69. In an embodiment, the anti-CD20 monoclonal antibody has a heavy chain sequence identity of greater than 98% to SEQ ID NO:68. In an embodiment, the anti-CD20 monoclonal antibody has a light chain sequence identity of greater than 98% to SEQ ID NO:69. In an embodiment, the anti-CD20 monoclonal antibody has a heavy chain sequence identity of greater than 99% to SEQ ID NO:68. In an embodiment, the anti-CD20 monoclonal antibody has a light chain sequence identity of greater than 99% to SEQ ID NO:69. In an embodiment, the anti-CD20 monoclonal antibody ofatumumab is an immunoglobulin G1, anti-(human B-lymphocyte antigen CD20 (membrane-spanning 4-domains subfamily A member 1, Leu-16, Bp35)); [218- arginine,360-glutamic acid,362-methionine]humanized mouse monoclonal hA20 γ1 heavy chain (224-213')-disulfide with humanized mouse monoclonal hA20 κ light chain (230-230":233-233")-bisdisulfide dimer

In an embodiment, the anti-CD20 monoclonal antibody is tositumomab, or an antigen-binding fragment, derivative, conjugate, variant, or radioisotope-labeled complex thereof. In an embodiment, the anti-CD20 monoclonal antibody is ¹³¹I-labeled tositumomab. In an embodiment, the anti-CD20 monoclonal antibody is an anti-CD20 biosimilar monoclonal antibody approved by drug regulatory authorities with reference to tositumomab. In an embodiment, the anti-CD20 monoclonal antibody has a heavy chain sequence identity of greater than 90% to SEQ ID NO:70. In an embodiment, the anti-CD20 monoclonal antibody has a light chain sequence identity of greater than 90% to SEQ ID NO: 71. In an embodiment, the anti-CD20 monoclonal antibody has a heavy chain sequence identity of greater than 95% to SEQ ID NO:70. In an embodiment, the anti-CD20 monoclonal antibody has a light chain sequence identity of greater than 95% to SEQ ID NO:71. In an embodiment, the anti-CD20 monoclonal antibody has a heavy chain sequence identity of greater than 98% to SEQ ID NO:70. In an embodiment, the anti-CD20 monoclonal antibody has a light chain sequence identity of greater than 98% to SEQ ID NO:71. In an embodiment, the anti-CD20 monoclonal antibody has a heavy chain sequence identity of greater than 99% to SEQ ID NO:70. In an embodiment, the anti-CD20 monoclonal antibody has a light chain sequence identity of greater than 99% to SEQ ID NO:71.

In an embodiment, the anti-CD20 monoclonal antibody is ibritumomab, or an antigen-binding fragment, derivative, conjugate, variant, or radioisotope-labeled complex thereof. The active form of ibritumomab used in therapy is ibritumomab tiuxetan. When used with ibritumomab, the chelator tiuxetan (diethylene triamine pentaacetic acid) is complexed with a radioactive isotope such as ⁹⁰Y or ¹¹¹In. In an embodiment, the anti-CD20 monoclonal antibody is ibritumomab tiuxetan, or radioisotope-labeled complex thereof. In an embodiment, the anti-CD20 monoclonal antibody is an anti-CD20 biosimilar monoclonal antibody approved by drug regulatory authorities with reference to tositumomab. In an embodiment, the anti-CD20 monoclonal antibody has a heavy chain sequence identity of greater than 90% to SEQ ID NO:72. In an embodiment, the anti-CD20 monoclonal antibody has a light chain sequence identity of greater than 90% to SEQ ID NO:73. In an embodiment, the anti-CD20 monoclonal antibody has a heavy chain sequence identity of greater than 95% to SEQ ID NO:72. In an embodiment, the anti-CD20 monoclonal antibody has a light chain sequence identity of greater than 95% to SEQ ID NO:73. In an embodiment, the anti-CD20 monoclonal antibody has a heavy chain sequence identity of greater than 98% to SEQ ID NO:72. In an embodiment, the anti-CD20 monoclonal antibody has a light chain sequence identity of greater than 98% to SEQ ID NO:73. In an embodiment, the anti-CD20 monoclonal antibody has a heavy chain sequence identity of greater than 99% to SEQ ID NO:72. In an embodiment, the anti-CD20 monoclonal antibody has a light chain sequence identity of greater than 99% to SEQ ID NO:73.

In an embodiment, an anti-CD20 antibody selected from the group consisting of obinutuzumab, ofatumumab, veltuzumab, tositumomab, and ibritumomab, and or antigen-binding fragments, derivatives, conjugates, variants, and radioisotope-labeled complexes thereof, is administered to a subject by infusing a dose selected from the group consisting of about 10 mg, about 20 mg, about 25 mg, about 50 mg, about 75 mg, 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, about 1800 mg, about 1900 mg, and about 2000 mg. In an embodiment, the anti-CD20 antibody is admininstered weekly. In an embodiment, the anti-CD20 antibody is admininstered every two weeks. In an embodiment, the anti-CD20 antibody is admininstered every three weeks. In an embodiment, the anti-CD20 antibody is admininstered monthly. In an embodiment, the anti-CD20 antibody is administered at a lower initial dose, which is escalated when administered at subsequent intervals admininstered monthly. For example, the first infusion can deliver 300 mg of anti-CD20 antibody, and subsequent weekly doses could deliver 2,000 mg of anti-CD20 antibody for eight weeks, followed by monthly doses of 2,000 mg of anti-CD20 antibody. During any of the foregoing embodiments, the combinations of the BTK inhibitors of Formula (II) with CD 19 inhibitors selected from an anti-CD19 antibody or an anti-CD19 chimeric antigen receptor, or an antigen-binding fragment or conjugate thereof may be administered daily, twice daily, or at different intervals as described above, at the dosages described above.

In an embodiment, the invention provides a kit comprising a first composition comprising combinations of the BTK inhibitor of Formula (II) with a CD 19 inhibitor selected from an anti-CD19 antibody or an anti-CD19 chimeric antigen receptor, or an antigen-binding fragment or conjugate thereof and an anti-CD20 antibody selected from the group consisting of rituximab, obinutuzumab, ofatumumab, veltuzumab, tositumomab, and ibritumomab, or an antigen-binding fragment, derivative, conjugate, variant, or radioisotope-labeled complex thereof, for use in the treatment of CLL or SLL, hematological malignancies, B cell malignancies or, or any of the other diseases described herein. The compositions are typically pharmaceutical compositions. The kit is for use in co-administration of the anti-CD20 antibody and the BTK inhibitor of Formula (II), either simultaneously or separately, in the treatment of CLL or SLL, hematological malignancies, B cell malignancies, or any of the other diseases described herein.

The anti-CD20 antibody sequences referenced in the foregoing are summarized in Table 4.

**TABLE 4. Anti-CD20 antibody sequences.**

| **Identifier** | **Sequence (One-Letter Amino Acid Symbols)** |
|---|---|
| SEQ ID NO:60 rituximab heavy chain | QVQLQQPGAE LVKPGASVKM SCKASGYTFT SYNMHWVKQT PGRGLEWIGA IYPGNGDTSY 60 NQKFKGKATL TADKSSSTAY MQLSSLTSED SAVYYCARST YYGGDWYFNV WGAGTTVTVS 120 AASTKGPSVF PLAPSSKSTS GGTAALGCLV KDYFPEPVTV SWNSGALTSG VHTFPAVLQS 180 SGLYSLSSVV TVPSSSLGTQ TYICNVNHKP SNTKVDKKVE PKSCDKTHTC PPCPAPELLG 240 GPSVFLFPPK PKDTLMISRT PEVTCVVVDV SHEDPEVKFN WYVDGVEVHN AKTKPREEQY 300 NSTYRVVSVL TVLHQDWLNG KEYKCKVSNK ALPAPIEKTI SKAKGQPREP QVYTLPPSRD 360 ELTKNQVSLT CLVKGFYPSD IAVEWESNGQ PENNYKTTPP VLDSDGSFFL YSKLTVDKSR 420 WQQGNVFSCS VMHEALHNHY TQKSLSLSPG K 451 |
| SEQ ID NO:61 rituximab light chain | QIVLSQSPAI LSASPGEKVT MTCRASSSVS YIHWFQQKPG SSPKPWIYAT SNLASGVPVR 60 FSGSGSGTSY SLTISRVEAE DAATYYCQQW TSNPPTFGGG TKLEIKRTVA APSVFIFPPS 120 DEQLKSGTAS VVCLLNNFYP REAKVQWKVD NALQSGNSQE SVTEQDSKDS TYSLSSTLTL 180 SKADYEKHKV YACEVTHQGL SSPVTKSFNR GEC 213 |
| SEQ ID NO:62 obinutuzumab heavy chain | QVQLVQSGAE VKKPGSSVKV SCKASGYAFS YSWINWVRQA PGQGLEWMGR IFPGDGDTDY 60 NGKFKGRVTI TADKSTSTAY MELSSLRSED TAVYYCARNV FDGYWLVYWG QGTLVTVSSA 120 STKGPSVFPL APSSKSTSGG TAALGCLVKD YFPEPVTVSW NSGALTSGVH TFPAVLQSSG 180 LYSLSSVVTV PSSSLGTQTY ICNVNHKPSN TKVDKKVEPK SCDKTHTCPP CPAPELLGGP 240 SVFLFPPKPK DTLMISRTPE VTCVVVDVSH EDPEVKFNWY VDGVEVHNAK TKPREEQYNS 300 TYRVVSVLTV LHQDWLNGKE YKCKVSNKAL PAPIEKTISK AKGQPREPQV YTLPPSRDEL 360 TKNQVSLTCL VKGFYPSDIA VEWESNGQPE NNYKTTPPVL DSDGSFFLYS KLTVDKSRWQ 420 QGNVFSCSVM HEALHNHYTQ KSLSLSPGK 449 |
| SEQ ID NO:63 obinutuzumab light chain | DIVMTQTPLS LPVTPGEPAS ISCRSSKSLL HSNGITYLYW YLQKPGQSPQ LLIYQMSNLV 60 SGVPDRFSGS GSGTDFTLKI SRVEAEDVGV YYCAQNLELP YTFGGGTKVE IKRTVAAPSV 120 FIFPPSDEQL KSGTASVVCL LNNFYPREAK VQWKVDNALQ SGNSQESVTE QDSKDSTYSL 180 SSTLTLSKAD YEKHKVYACE VTHQGLSSPV TKSFNRGEC 219 |
| SEQ ID NO:64 | EVQLVESGGG LVQPGRSLRL SCAASGFTFN DYAMHWVRQA PGKGLEWVST ISWNSGSIGY 60 |
| ofatumumab variable heavy chain | ADSVKGRFTI SRDNAKKSLY LQMNSLRAED TALYYCAKDI QYGNYYYGMD VWGQGTTVTV 120 SS 122 |
| SEQ ID NO:65 ofatumumab variable light chain | EIVLTQSPAT LSLSPGERAT LSCRASQSVS SYLAWYQQKP GQAPRLLIYD ASNRATGIPA 60 RFSGSGSGTD FTLTISSLEP EDFAVYYCQQ RSNWPITFGQ GTRLEIK 107 |
| SEQ ID NO:66 ofatumumab Fab fragment heavy chain | EVQLVESGGG LVQPGRSLRL SCAASGFTFN DYAMHWVRQA PGKGLEWVST ISWNSGSIGY 60 ADSVKGRFTI SRDNAKKSLY LQMNSLRAED TALYYCAKDI QYGNYYYGMD VWGQGTTVTV 120 SSASTKGPSV FPLAPGSSKS TSGTAALGCL VKDYFPEPVT VSWNSGALTS GVHTFPAVLQ 180 SSGLYSLSSV VTVPSSSLGT QTYICNVNHK PSNTKVDKKV EP 222 |
| SEQ ID NO:67 ofatumumab Fab fragment light chain | EIVLTQSPAT LSLSPGERAT LSCRASQSVS SYLAWYQQKP GQAPRLLIYD ASNRATGIPA 60 RFSGSGSGTD FTLTISSLEP EDFAVYYCQQ RSNWPITFGQ GTRLEIKRTV AAPSVFIFPP 120 SDEQLKSGTA SVVCLLNNFY PREAKVQWKV DNALQSGNSQ ESVTEQDSKD STYSLSSTLT 180 LSKADYEKHK VYACEVTHQG LSSPVTKSFN R 211 |
| SEQ ID NO:68 veltuzumab heavy chain | QVQLQQSGAE VKKPGSSVKV SCKASGYTFT SYNMHWVKQA PGQGLEWIGA IYPGMGDTSY 60 NQKFKGKATL TADESTNTAY MELSSLRSED TAFYYCARST YYGGDWYFDV WGQGTTVTVS 120 SASTKGPSVF PLAPSSKSTS GGTAALGCLV KDYFPEPVTV SWNSGALTSG VHTFPAVLQS 180 SGLYSLSSVV TVPSSSLGTQ TYICNVNHKP SNTKVDKRVE PKSCDKTHTC PPCPAPELLG 240 GPSVFLFPPK PKDTLMISRT PEVTCVVVDV SHEDPEVKFN WYVDGVEVHN AKTKPREEQY 300 NSTYRVVSVL TVLHQDWLNG KEYKCKVSNK ALPAPIEKTI SKAKGQPREP QVYTLPPSRE 360 EMTKNQVSLT CLVKGFYPSD IAVEWESNGQ PENNYKTTPP VLDSDGSFFL YSKLTVDKSR 420 WQQGNVFSCS VMHEALHNHY TQKSLSLSPG K 451 |
| SEQ ID NO:69 veltuzumab light chain | DIQLTQSPSS LSASVGDRVT MTCRASSSVS YIHWFQQKPG KAPKPWIYAT SNLASGVPVR 60 FSGSGSGTDY TFTISSLQPE DIATYYCQQW TSNPPTFGGG TKLEIKRTVA APSVFIFPPS 120 DEQLKSGTAS VVCLLNNFYP REAKVQWKVD NALQSGNSQE SVTEQDSKDS TYSLSSTLTL 180 SKADYEKHKV YACEVTHQGL SSPVTKSFNR GEC 213 |
| SEQ ID NO:70 tositumomab heavy chain | QAYLQQSGAE LVRPGASVKM SCKASGYTFT SYNMHWVKQT PRQGLEWIGA IYPGNGDTSY 60 NQKFKGKATL TVDKSSSTAY MQLSSLTSED SAVYFCARVV YYSNSYWYFD VWGTGTTVTV 120 SGPSVFPLAP SSKSTSGGTA ALGCLVKDYF PEPVTVSWNS GALTSGVHTF PAVLQSSGLY 180 SLSSVVTVPS SSLGTQTYIC NVNHKPSNTK VDKKAEPKSC DKTHTCPPCP APELLGGPSV 240 FLFPPKPKDT LMISRTPEVT CVVVDVSHED PEVKFNWYVD GVEVHNAKTK PREEQYNSTY 300 RVVSVLTVLH QDWLNGKEYK CKVSNKALPA PIEKTISKAK GQPREPQVYT LPPSRDELTK 360 NQVSLTCLVK GFYPSDIAVE WESNGQPENN YKTTPPVLDS DGSFFLYSKL TVDKSRWQQG 420 NVFSCSVMHE ALHNHYTQKS LSLSPGK 447 |
| SEQ ID NO:71 tositumomab light chain | QIVLSQSPAI LSASPGEKVT MTCRASSSVS YMHWYQQKPG SSPKPWIYAP SNLASGVPAR 60 FSGSGSGTSY SLTISRVEAE DAATYYCQQW SFNPPTFGAG TKLELKRTVA APSVFIFPPS 120 DEQLKSGTAS VVCLLNNFYP REAKVQWKVD NALQSGNSQE SVTEQDSKDS TYSLSSTLTL 180 SKADYEKHKV YACEVTHQGL SSPVTKSFNR 210 |
| SEQ ID NO:72 ibritumomab heavy chain | QAYLQQSGAE LVRPGASVKM SCKASGYTFT SYNMHWVKQT PRQGLEWIGA IYPGNGDTSY 60 NQKFKGKATL TVDKSSSTAY MQLSSLTSED SAVYFCARVV YYSNSYWYFD VWGTGTTVTV 120 SAPSVYPLAP VCGDTTGSSV TLGCLVKGYF PEPVTLTWNS GSLSSGVHTF PAVLQSDLYT 180 LSSSVTVTSS TWPSQSITCN VAHPASSTKV DKKIEPRGPT IKPCPPCKCP APNLLGGPSV 240 FIFPPKIKDV LMISLSPIVT CVVVDVSEDD PDVQISWFVN NVEVHTAQTQ THREDYNSTL 300 RVVSALPIQH QDWMSGKEFK CKVNNKDLPA PIERTISKPK GSVRAPQVYV LPPPEEEMTK 360 KQVTLTCMVT DFMPEDIYVE WTNNGKTELN YKNTEPVLDS DGSYFMYSKL RVEKKNWVER 420 NSYSCSVVHE GLHNHHTTKS FSR 443 |
| SEQ ID NO:73 ibritumomab light chain | QIVLSQSPAI LSASPGEKVT MTCRASSSVS YMHWYQQKPG SSPKPWIYAP SNLASGVPAR 60 FSGSGSGTSY SLTISRVEAE DAATYYCQQW SFNPPTFGAG TKLELKRADA APTVFIFPPS 120 DEQLKSGTAS VVCLLNNFYP REAKVQWKVD NALQSGNSQE SVTEQDSKDS TYSLSSTLTL 180 SKADYEKHKV YACEVTHQGL SSPVTKSFN 209 |

### Combinations of BTK Inhibitors and CD19 Inhibitors with Chemotherapeutic Active Pharmaceutical Ingredients

The combinations of the BTK inhibitors of Formula (II) with CD 19 inhibitors selected from an anti-CD19 antibody or an anti-CD19 chimeric antigen receptor, or an antigen-binding fragment or conjugate thereof may also be safely co-administered with chemotherapeutic active pharmaceutical ingredients such as gemcitabine, albumin-bound paclitaxel (nab-paclitaxel), and bendamustine or bendamustine hydrochloride. In a preferred embodiment, the invention provides a pharmaceutical combination for use in treating a hematological malignancy or a solid tumor cancer in a human comprising a BTK inhibitor of Formula (II) and a CD19 inhibitor selected from an anti-CD19 antibody or an anti-CD19 chimeric antigen receptor, or an antigen-binding fragment or conjugate thereof, and further comprising a therapeutically-effective amount of gemcitabine, or a pharmaceutically acceptable salt, prodrug, cocrystal, solvate or hydrate thereof. In an embodiment, the invention provides a pharmaceutical combination for use in treating a hematological malignancy or a solid tumor cancer in a human comprising a BTK inhibitor consisting of Formula (2) or a pharmaceutically acceptable salt thereof, and a CD 19 inhibitor selected from an anti-CD19 antibody or an anti-CD19 chimeric antigen receptor, or an antigen-binding fragment or conjugate thereof, and further comprising a therapeutically-effective amount of gemcitabine, or a pharmaceutically acceptable salt, prodrug, cocrystal, solvate or hydrate thereof. In an embodiment, the solid tumor cancer in any of the foregoing embodiments is pancreatic cancer.

In an embodiment, the invention provides a pharmaceutical combination for use in treating a hematological malignancy or a solid tumor cancer in a human comprising a BTK inhibitor of Formula (II) and a CD19 inhibitor selected from an anti-CD19 antibody or an anti-CD19 chimeric antigen receptor, or an antigen-binding fragment or conjugate thereof, and further comprising a therapeutically-effective amount of albumin-bound paclitaxel. In an embodiment, the solid tumor cancer in any of the foregoing embodiments is pancreatic cancer.

In an embodiment, the invention provides a pharmaceutical combination for use in treating a hematological malignancy or a solid tumor cancer in a human comprising a BTK inhibitor of Formula (II) and a CD19 inhibitor selected from an anti-CD19 antibody or an anti-CD19 chimeric antigen receptor, or an antigen-binding fragment or conjugate thereof, and further comprising a therapeutically-effective amount of bendamustine hydrochloride. In an embodiment, the invention provides a pharmaceutical combination for use in treating a hematological malignancy or a solid tumor cancer in a human comprising a BTK inhibitor of Formula (2) or a pharmaceutically-acceptable salt thereof, and a CD19 inhibitor selected from an anti-CD19 antibody or an anti-CD19 chimeric antigen receptor, or an antigen-binding fragment or conjugate thereof, , further comprising a therapeutically-effective amount of bendamustine hydrochloride, and further comprising a therapeutically-effective amount of rituximab (collectively referred to as "BR" or "BR chemotherapy"). BR chemotherapy has been shown to improve overall response rates in non-Hodgkin's lymphoma and mantle cell lymphomas and also demonstrated improved safety in comparison to alternative regimens, as described in Flinn, et al., Blood 2014, 123, 2944-52.

In an embodiment, the invention provides a pharmaceutical combination for use in treating a hematological malignancy or a solid tumor cancer in a human comprising BTK inhibitor of Formula (II) and a CD19 inhibitor selected from an anti-CD19 antibody or an anti-CD19 chimeric antigen receptor, or an antigen-binding fragment or conjugate thereof, and further comprising a therapeutically-effective amount of a combination of fludarabine, cyclophosphamide, and rituximab (which collectively may be referred to as "FCR" or "FCR chemotherapy"). FCR chemotherapy has been shown to improve survival in patients with cancer, as described in Hallek, et al., Lancet. 2010, 376, 1164-1174.

In an embodiment, the invention provides a pharmaceutical combination for use in treating a hematological malignancy or a solid tumor cancer in a human comprising a BTK inhibitor of Formula (II) and a CD19 inhibitor selected from an anti-CD19 antibody or an anti-CD19 chimeric antigen receptor, or an antigen-binding fragment or conjugate thereof, and further comprising a therapeutically-effective amount of a combination of rituximab, cyclophosphamide, doxorubicin hydrochloride (also referred to as hydroxydaunomycin), vincristine sulfate (also referred to as oncovin), and prednisone (which collectively may be referred to as "R-CHOP" or "R-CHOP chemotherapy"). R-CHOP chemotherapy has been shown to improve the 10-year progression-free and overall survival rates for patients with cancer, as described in Sehn, Blood, 2010, 116, 2000-2001.

In any of the foregoing embodiments, the chemotherapeutic active pharmaceutical ingredient or combinations thereof may be administered before, concurrently, or after administration of the CD 19 inhibitors selected from an anti-CD19 antibody or an anti-CD19 chimeric antigen receptor, or an antigen-binding fragment or conjugate thereof and the BTK inhibitors of Formula (II).

### Combinations of BTK Inhibitors and CD19 Inhibitors with PD-1, PD-L1, and/or PD-L2 Inhibitors

The combinations of the BTK inhibitors of Formula (II) with CD 19 inhibitors selected from an anti-CD19 antibody or an anti-CD19 chimeric antigen receptor, or an antigen-binding fragment or conjugate thereof may also be further combined with programmed death-1 (PD-1), programmed death ligand 1 (PD-L1), and/or programmed death ligand 2 (PD-L2) binding antibodies or inhibitors (*i.e.,* blockers). In a preferred embodiment, the PD-1 or PD-L1 inhibitor is selected from the group consisting of nivolumab, pembrolizumab, pidilizumab, durvalumab, atezolizumab, avelumab, and antigen-binding fragments, variants, conjugates, or biosimilars thereof. In an embodiment, the invention provides a pharmaceutical combination for use in treating a cancer in a human comprising a BTK inhibitor of Formula (II), or a pharmaceutically acceptable salt thereof, and a CD19 inhibitor, including an anti-CD19 antibody or an antibody fragment or conjugate, thereof, and further comprising an PD-1 or PD-L1 inhibitor, or an antigen-binding fragment, derivative, conjugate, variant, or biosimilar thereof.

Programmed death 1 (PD-1) is a 288-amino acid transmembrane immunocheckpoint receptor protein expressed by T cells, B cells, natural killer (NK) T cells, activated monocytes, and dendritic cells. PD-1, which is also known as CD279, is an immunoreceptor belonging to the CD28 family and in humans is encoded by the *Pdcd1* gene on chromosome 2. PD-1 consists of one immunoglobulin (Ig) superfamily domain, a transmembrane region, and an intracellular domain containing an immunoreceptor tyrosine-based inhibitory motif (ITIM) and an immunoreceptor tyrosine-based switch motif (ITSM). PD-1 and its ligands (PD-L1 and PD-L2) play a key role in immune tolerance, as described in Keir, et al., Annu. Rev. Immunol. 2008, 26, 677-704. PD-1 provides inhibitory signals that negatively regulate T cell immune responses. PD-L1 (also known as B7-H1 or CD274) and PD-L2 (also known as B7-DC or CD273) are expressed on tumor cells and stromal cells, which may be encountered by activated T cells expressing PD-1, leading to immunosuppression of the T cells. PD-L1 is a 290 amino acid transmembrane protein encoded by the *Cd274* gene on human chromosome 9. Blocking the interaction between PD-1 and its ligands PD-L1 and PD-L2 by use of a PD-1 inhibitor, a PD-L1 inhibitor, and/or a PD-L2 inhibitor can overcome immune resistance, as demonstrated in recent clinical studies, such as that described in Topalian, et al., N. Eng. J. Med. 2012, 366, 2443. PD-L1 is expressed on many tumor cell lines, while PD-L2 is expressed is expressed mostly on dendritic cells and a few tumor lines. In addition to T cells (which inducibly express PD-1 after activation), PD-1 is also expressed on B cells, natural killer cells, macrophages, activated monocytes, and dendritic cells.

In an embodiment, the PD-1 inhibitor may be any PD-1 inhibitor or PD-1 blocker known in the art. In particular, it is one of the PD-1 inhibitors or blockers described in more detail in the following paragraphs. The terms "inhibitor" and "blocker" are used interchangeably herein in reference to PD-1 inhibitors. For avoidance of doubt, references herein to a PD-1 inhibitor that is an antibody may refer to a compound or antigen-binding fragments, variants, conjugates, or biosimilars thereof. For avoidance of doubt, references herein to a PD-1 inhibitor may also refer to a compound or a pharmaceutically acceptable salt, ester, solvate, hydrate, cocrystal, or prodrug thereof.

In some embodiments, the compositions and methods described herein include a PD-1 inhibitor. In some embodiments, the PD-1 inhibitor is a small molecule. In a preferred embodiment, the PD-1 inhibitor is an antibody (*i.e.,* an anti-PD-1 antibody), a fragment thereof, including Fab fragments, or a single-chain variable fragment (scFv) thereof. In some embodiments the PD-1 inhibitor is a polyclonal antibody. In a preferred embodiment, the PD-1 inhibitor is a monoclonal antibody. In some embodiments, the PD-1 inhibitor competes for binding with PD-1, and/or binds to an epitope on PD-1. In an embodiment, the antibody competes for binding with PD-1, and/or binds to an epitope on PD-1. In some embodiments, the PD-1 inhibitor is included in a composition and is further combined with a BTK inhibitor of Formula (II) and CD19 inhibitor selected from an anti-CD19 antibody or an anti-CD19 chimeric antigen receptor, or an antigen-binding fragment or conjugate thereof. In some embodiments, an anti-PD-1 monoclonal antibody is included in a composition and is further combined with a BTK inhibitor of Formula (II) and a CD19 inhibitor selected from an anti-CD19 antibody or an anti-CD19 chimeric antigen receptor, or an antigen-binding fragment or conjugate thereof. In some embodiments, the PD-1 inhibitors provided herein are selective for PD-1, in that the compounds bind or interact with PD-1 at substantially lower concentrations than they bind or interact with other receptors.

In some embodiments, the compositions and methods described include a PD-1 inhibitor that binds human PD-1 with a K_{D} of about 100 pM or lower, binds human PD-1 with a K_{D} of about 90 pM or lower, binds human PD-1 with a K_{D} of about 80 pM or lower, binds human PD-1 with a K_{D} of about 70 pM or lower, binds human PD-1 with a K_{D} of about 60 pM or lower, binds human PD-1 with a K_{D} of about 50 pM or lower, binds human PD-1 with a K_{D} of about 40 pM or lower, or binds human PD-1 with a K_{D} of about 30 pM or lower.

In some embodiments, the compositions and methods described include a PD-1 inhibitor that binds to human PD-1 with a k_{assoc} of about 7.5 × 10⁵ l/M·s or faster, binds to human PD-1 with a k_{assoc} of about 7.5 × 10⁵ l/M·s or faster, binds to human PD-1 with a k_{assoc} of about 8 × 10⁵ l/M·s or faster, binds to human PD-1 with a k_{assoc} of about 8.5 × 10⁵ l/M·s or faster, binds to human PD-1 with a k_{assoc} of about 9 × 10⁵ l/M·s or faster, binds to human PD-1 with a k_{assoc} of about 9.5 × 10⁵ l/M·s or faster, or binds to human PD-1 with a k_{assoc} of about 1 × 10⁶ l/M·s or faster.

In some embodiments, the compositions and methods described include a PD-1 inhibitor that binds to human PD-1 with a k_{dissoc} of about 2 × 10⁻⁵ 1/s or slower, binds to human PD-1 with a k_{dissoc} of about 2.1 × 10⁻⁵ 1/s or slower , binds to human PD-1 with a k_{dissoc} of about 2.2 × 10⁻⁵ 1/s or slower, binds to human PD-1 with a k_{dissoc} of about 2.3 × 10⁻⁵ l/s or slower, binds to human PD-1 with a k_{dissoc} of about 2.4 × 10⁻⁵ l/s or slower, binds to human PD-1 with a k_{dissoc} of about 2.5 × 10⁻⁵ 1/s or slower, binds to human PD-1 with a k_{dissoc} of about 2.6 × 10⁻⁵ 1/s or slower or binds to human PD-1 with a k_{dissoc} of about 2.7 × 10⁻⁵ 1/s or slower, binds to human PD-1 with a k_{dissoc} of about 2.8 × 10⁻⁵ 1/s or slower, binds to human PD-1 with a k_{dissoc} of about 2.9 × 10⁻⁵ 1/s or slower, or binds to human PD-1 with a k_{dissoc} of about 3 × 10⁻⁵ 1/s or slower.

In some embodiments, the compositions and methods described include a PD-1 inhibitor that blocks or inhibits binding of human PD-Ll or human PD-L2 to human PD-1 with an IC₅₀ of about 10 nM or lower, blocks or inhibits binding of human PD-Ll or human PD-L2 to human PD-1 with an IC₅₀ of about 9 nM or lower, blocks or inhibits binding of human PD-Ll or human PD-L2 to human PD-1 with an IC₅₀ of about 8 nM or lower, blocks or inhibits binding of human PD-Ll or human PD-L2 to human PD-1 with an IC₅₀ of about 7 nM or lower, blocks or inhibits binding of human PD-Ll or human PD-L2 to human PD-1 with an IC₅₀ of about 6 nM or lower, blocks or inhibits binding of human PD-Ll or human PD-L2 to human PD-1 with an IC₅₀ of about 5 nM or lower, blocks or inhibits binding of human PD-Ll or human PD-L2 to human PD-1 with an IC₅₀ of about 4 nM or lower, blocks or inhibits binding of human PD-Ll or human PD-L2 to human PD-1 with an IC₅₀ of about 3 nM or lower, blocks or inhibits binding of human PD-Ll or human PD-L2 to human PD-1 with an IC₅₀ of about 2 nM or lower, or blocks or inhibits binding of human PD-Ll or human PD-L2 to human PD-1 with an IC₅₀ of about 1 nM or lower.

In an embodiment, an anti-PD-1 antibody comprises nivolumab (commercially available from Bristol-Myers Squibb Co.), or antigen-binding fragments, conjugates, or variants thereof. Nivolumab is referred to as 5C4 in International Patent Publication No. WO 2006/121168. Nivolumab is assigned Chemical Abstracts Service (CAS) registry number 946414-94-4 and is also known as BMS-936558, MDX-1106 or ONO-4538. Nivolumab is a fully human IgG4 antibody blocking the PD-1 receptor. The clinical safety and efficacy of nivolumab in various forms of cancer has been described in Wang et al., Cancer Immunol Res. 2014, 2, 846-56; Page et al., Ann. Rev. Med., 2014, 65, 185-202; and Weber, etal., J. Clin. Oncology, 2013, 31, 4311-4318. The nivolumab monoclonal antibody includes a heavy chain given by SEQ ID NO:74 and a light chain given by SEQ ID NO:75. Nivolumab has intra-heavy chain disulfide linkages at 22-96,140-196, 254-314, 360-418, 22"-96", 140"-196", 254"-314", and 360"-418"; intra-light chain disulfide linkages at 23'-88', 134'-194', 23‴-88‴, and 134‴-194‴; inter-heavy-light chain disulfide linkages at 127-214', 127"-214‴, inter-heavy-heavy chain disulfide linkages at 219-219" and 222-222"; and N-glycosylation sites (H CH₂ 84.4) at 290, 290". In an embodiment, the anti-PD-1 antibody is an immunoglobulin G4 kappa, anti-(human CD274) antibody. In an embodiment, an anti-PD-1 antibody comprises heavy and light chains having the sequences shown in SEQ ID NO:74 and SEQ ID NO:75, respectively, or antigen binding fragments, Fab fragments, single-chain variable fragments (scFv), variants, or conjugates thereof. In an embodiment, an anti-PD-1 antibody comprises heavy and light chains that are each at least 99% identical to the sequences shown in SEQ ID NO:74 and SEQ ID NO:75, respectively. In an embodiment, an anti-PD-1 antibody comprises heavy and light chains that are each at least 98% identical to the sequences shown in SEQ ID NO:74 and SEQ ID NO:75, respectively. In an embodiment, an anti-PD-1 antibody comprises heavy and light chains that are each at least 97% identical to the sequences shown in SEQ ID NO:74 and SEQ ID NO:75, respectively. In an embodiment, an anti-PD-1 antibody comprises heavy and light chains that are each at least 96% identical to the sequences shown in SEQ ID NO:74 and SEQ ID NO:75, respectively. In an embodiment, an anti-PD-1 antibody comprises heavy and light chains that are each at least 95% identical to the sequences shown in SEQ ID NO:74 and SEQ ID NO:75, respectively.

In an embodiment, the anti-PD-1 antibody comprises the heavy and light chain CDRs or variable regions (VRs) of nivolumab. In one embodiment, the anti-PD-1 antibody heavy chain variable region (V_{H}) comprises the sequence shown in SEQ ID NO:76, and the anti-PD-1 antibody light chain variable region (V_{L}) comprises the sequence shown in SEQ ID NO:77. In an embodiment, an anti-PD-1 antibody comprises V_{H} and V_{L} regions that are each at least 99% identical to the sequences shown in SEQ ID NO:76 and SEQ ID NO:77, respectively. In an embodiment, an anti-PD-1 antibody comprises V_{H} and V_{L} regions that are each at least 98% identical to the sequences shown in SEQ ID NO:76 and SEQ ID NO:77, respectively. In an embodiment, an anti-PD-1 antibody comprises V_{H} and V_{L} regions that are each at least 97% identical to the sequences shown in SEQ ID NO:76 and SEQ ID NO:77, respectively. In an embodiment, an anti-PD-1 antibody comprises V_{H} and V_{L} regions that are each at least 96% identical to the sequences shown in SEQ ID NO:76 and SEQ ID NO:77, respectively. In an embodiment, an anti-PD-1 antibody comprises V_{H} and V_{L} regions that are each at least 95% identical to the sequences shown in SEQ ID NO:76 and SEQ ID NO:77, respectively. In an alternative embodiment, the antibody comprises V_{H} and/or V_{L} regions having the amino acid sequences set forth in SEQ ID NO:76 and/or SEQ ID NO:77, respectively.

In an embodiment, the anti-PD-1 antibody comprises heavy chain CDR1, CDR2 and CDR3 domains having the sequences set forth in SEQ ID NO:78, SEQ ID NO:79, and SEQ ID NO:80, respectively, and light chain CDR1, CDR2 and CDR3 domains having the sequences set forth in SEQ ID NO:81, SEQ ID NO:82, and SEQ ID NO:83, respectively.

In an embodiment, an anti-PD-1 antibody comprises a heavy chain that comprises CDR1, CDR2 and CDR3 domains that are each at least 95% identical to the sequences shown in SEQ ID NO:78, SEQ ID NO:79, and SEQ ID NO:80, respectively. In an embodiment, an anti-PD-1 antibody comprises a heavy chain that comprises CDR1, CDR2 and CDR3 domains that are each at least 90% identical to the sequences shown in SEQ ID NO:78, SEQ ID NO:79, and SEQ ID NO:80, respectively. In an embodiment, an anti-PD-1 antibody comprises a heavy chain that comprises CDR1, CDR2 and CDR3 domains that are each at least 85% identical to the sequences shown in SEQ ID NO:78, SEQ ID NO:79, and SEQ ID NO:80, respectively. In an embodiment, an anti-PD-1 antibody comprises a heavy chain that comprises CDR1, CDR2 and CDR3 domains that are each at least 80% identical to the sequences shown in SEQ ID NO:78, SEQ ID NO:79, and SEQ ID NO:80, respectively. In another embodiment, the antibody competes for binding with, and/or binds to the same epitope on PD-1 as the aforementioned antibodies.

In an embodiment, an anti-PD-1 antibody comprises a light chain that comprises CDR1, CDR2 and CDR3 domains that are each at least 95% identical to the sequences shown in SEQ ID NO:81, SEQ ID NO:82, and SEQ ID NO:83, respectively. In an embodiment, an anti-PD-1 antibody comprises a light chain that comprises CDR1, CDR2 and CDR3 domains that are each at least 90% identical to the sequences shown in SEQ ID NO:81, SEQ ID NO:82, and SEQ ID NO:83, respectively. In an embodiment, an anti-PD-1 antibody comprises a light chain that comprises CDR1, CDR2 and CDR3 domains that are each at least 85% identical to the sequences shown in SEQ ID NO:81, SEQ ID NO:82, and SEQ ID NO:83, respectively. In an embodiment, an anti-PD-1 antibody comprises a light chain that comprises CDR1, CDR2 and CDR3 domains that are each at least 80% identical to the sequences shown in SEQ ID NO:81, SEQ ID NO:82, and SEQ ID NO:83, respectively. In another embodiment, the antibody competes for binding with, and/or binds to the same epitope on PD-1 as the aforementioned antibodies.

In an embodiment, the anti-PD-1 antibody is an antibody disclosed and/or prepared according to U.S. Patent No. 8,008,449 or U.S. Patent Application Publication Nos. 2009/0217401 A1 or 2013/0133091 A1. For example, in an embodiment, the monoclonal antibody includes 5C4 (referred to herein as nivolumab), 17D8, 2D3, 4H1, 4A11, 7D3, and 5F4, described in U.S. Patent No. 8,008,449. The PD-1 antibodies 17D8, 2D3, 4H1, 5C4, and 4A11, are all directed against human PD-1, bind specifically to PD-1 and do not bind to other members of the CD28 family. The sequences and CDR regions for these antibodies are provided in U.S. Patent No. 8,008,449, in particular in Figure 1 through Figure 12.

The anti-PD-1 antibody nivolumab may be prepared by the following procedure, as described in U.S. Patent No. 8,008,449. Immunization protocols utilized as antigen both (i) a recombinant fusion protein comprising the extracellular portion of PD-1 and (ii) membrane bound full-length PD-1. Both antigens were generated by recombinant transfection methods in a CHO cell line. Fully human monoclonal antibodies to PD-1 were prepared using the HCo7 strain of HuMab transgenic mice and the KM strain of transgenic transchromosomic mice, each of which express human antibody genes. In each of these mouse strains, the endogenous mouse kappa light chain gene has been homozygously disrupted as described in Chen et al. EMBO J. 1993, 12, 811-820 and the endogenous mouse heavy chain gene has been homozygously disrupted as described in Example 1 of International Patent Publication No. WO 01/09187. Each of these mouse strains carries a human kappa light chain transgene, KCo5, as described in Fishwild, et al. Nat. Biotechnology 1996, 14, 845-851. The HCo7 strain carries the HCo7 human heavy chain transgene as described in U.S. Patent Nos. 5,545,806; 5,625,825; and 5,545,807. The KM strain contains the SC20 transchromosome as described in International Patent Publication No. WO 02/43478. To generate fully human monoclonal antibodies to PD-1, HuMab mice and KM Mice^{™} were immunized with purified recombinant PD-1 fusion protein and PD-1-transfected CHO cells as antigen. General immunization schemes for HuMab mice are described in Lonberg, et al., Nature 1994, 368, 856-859; Fishwild, et al., Nat. Biotechnology 1996, 14, 845-851, and International Patent Publication No. WO 98/24884. The mice were 6-16 weeks of age upon the first infusion of antigen. A purified recombinant preparation (5-50 µg) of PD-1 fusion protein antigen and 5-10×10⁶ cells were used to immunize the HuMab mice and KM Mice^{™} intraperitonealy, subcutaneously (Sc) or via footpad injection. Transgenic mice were immunized twice with antigen in complete Freund's adjuvant or Ribi adjuvant IP, followed by 3-21 days IP (up to a total of 11 immunizations) with the antigen in incomplete Freund's or Ribi adjuvant. The immune response was monitored by retroorbital bleeds. The plasma was screened by ELISA (as described below), and mice with sufficient titers of anti-PD-1 human immunoglobulin were used for fusions. Mice were boosted intravenously with antigen 3 days before sacrifice and removal of the spleen. Typically, 10-35 fusions for each antigen were performed. Several dozen mice were immunized for each antigen. To select HuMab or KM Mice^{™} producing antibodies that bound PD-1, sera from immunized mice were tested by ELISA as described by Fishwild, et al., Nat. Biotechnology 1996, 14, 845-851. Briefly, microtiter plates were coated with purified recombinant PD-1 fusion protein from transfected CHO cells at 1-2 µg/ml in PBS, 100 µL/wells incubated at 4° C overnight then blocked with 200 µL/well of 5% fetal bovine serum in PBS/Tween (0.05%). Dilutions of sera from PD-1-immunized mice were added to each well and incubated for 1-2 hours at ambient temperature. The plates were washed with PBS/Tween and then incubated with a goat-anti-human IgG polyclonal antibody conjugated with horseradish peroxidase (HRP) for 1 hour at room temperature. After washing, the plates were developed with ABTS substrate (Sigma, A-1888, 0.22 mg/ml) and analyzed by spectrophotometer at OD 415-495. Mice that developed the highest titers of anti-PD-1 antibodies were used for fusions. Fusions were performed as described below and hybridoma supernatants were tested for anti-PD-1 activity by ELISA. The mouse splenocytes, isolated from the HuMab or KM mice, were fused to a mouse myeloma cell line either using PEG based upon standard protocols or electric field based electrofusion using a Cyto Pulse large chamber cell fusion electroporator (Cyto Pulse Sciences, Inc., Glen Burnie, Md.). The resulting hybridomas were then screened for the production of antigen-specific antibodies. Single cell suspensions of splenocytes from immunized mice were fused to one-fourth the number of SP2/0 nonsecreting mouse myeloma cells (ATCC, CRL 1581) with 50% PEG (Sigma). Cells were plated at approximately 1×105/well in flat bottom microtiter plate, followed by about two week incubation in selective medium containing 10% fetal bovine serum, 10% P388D1 (ATCC, CRL TIB-63) conditioned medium, 3-5% origen (IGEN) in DMEM (Mediatech, CRL 10013, with high glucose, L-glutamine and sodium pyruvate) plus 5 mM HEPES, 0.055 mM 2-mercaptoethanol, 50 mg/ml gentamycin and 1×HAT (Sigma, CRL P-7185). After 1-2 weeks, cells were cultured in medium in which the HAT was replaced with HT. Individual wells were then screened by ELISA (described above) for human anti-PD-1 monoclonal IgG antibodies. Once extensive hybridoma growth occurred, medium was monitored usually after 10-14 days. The antibody-secreting hybridomas were replated, screened again and, if still positive for human IgG, anti-PD-1 monoclonal antibodies were subcloned at least twice by limiting dilution. The stable subclones were then cultured in vitro to generate small amounts of antibody in tissue culture medium for further characterization. The antibody nivolumab may be produced in this manner, or by other known means given the disclosure of the amino acid sequences herein.

In another embodiment, the anti-PD-1 antibody comprises pembrolizumab, which is commercially available from Merck, or antigen-binding fragments, conjugates, or variants thereof. Pembrolizumab is assigned CAS registry number 1374853-91-4 and is also known as lambrolizumab, MK-3475, and SCH-900475. The structure, properties, uses, and preparation of pembrolizumab are described in International Patent Publication No. WO 2008/156712 A1, U.S. Patent No. 8,354,509 and U.S. Patent Application Publication Nos. US 2010/0266617 A1, US 2013/0108651 A1, and US 2013/0109843 A2. Pembrolizumab has an immunoglobulin G4, anti-(human protein PDCD1 (programmed cell death 1)) (human-Mus musculus monoclonal heavy chain), disulfide with human-Mus musculus monoclonal light chain, dimer structure. The structure of pembrolizumab may also be described as immunoglobulin G4, anti-(human programmed cell death 1); humanized mouse monoclonal [228-L-proline(H10-S>P)]γ4 heavy chain (134-218')-disulfide with humanized mouse monoclonal κ light chain dimer (226-226":229-229")-bisdisulfide. The clinical safety and efficacy of pembrolizumab in various forms of cancer is described in Fuerst, Oncology Times, 2014, 36, 35-36; Robert, et al., Lancet, 2014, 384, 1109-17; and Thomas et al., Exp. Opin. Biol. Ther., 2014, 14, 1061-1064. In an embodiment, the pembrolizumab monoclonal antibody includes a heavy chain given by SEQ ID NO:84 and a light chain given by SEQ ID NO:85, and includes the following disulfide bridges: 22-96, 22"-96", 23'-92', 23'"-92'", 134-218', 134"-218‴, 138'-198', 138‴-198‴, 147-203, 147"-203", 226-226", 229-229", 261-321, 261"-321", 367-425, and 367"-425", and the following glycosylation sites (N): Asn-297 and Asn-297". Pembrolizumab is an IgG4/kappa isotype with a stabilizing S228P mutation in the Fc region; insertion of this mutation in the IgG4 hinge region prevents the formation of half molecules typically observed for IgG4 antibodies. Pembrolizumab is heterogeneously glycosylated at Asn297 within the Fc domain of each heavy chain, yielding a molecular weight of approximately 149 kDa for the intact antibody. The dominant glycoform of pembrolizumab is the fucosylated agalacto diantennary glycan form (G0F).

In an embodiment, an anti-PD-1 antibody comprises heavy and light chains having the sequences shown in SEQ ID NO:84 and SEQ ID NO:85, respectively, or antigen binding fragments and variants thereof. In an embodiment, an anti-PD-1 antibody comprises heavy and light chains that are each at least 99% identical to the sequences shown in SEQ ID NO:84 and SEQ ID NO: 85, respectively, or antigen binding fragments and variants thereof. In an embodiment, an anti-PD-1 antibody comprises heavy and light chains that are each at least 98% identical to the sequences shown in SEQ ID NO:84 and SEQ ID NO:85, respectively, or antigen binding fragments and variants thereof. In an embodiment, an anti-PD-1 antibody comprises heavy and light chains that are each at least 97% identical to the sequences shown in SEQ ID NO:84 and SEQ ID NO:85, respectively, or antigen binding fragments and variants thereof. In an embodiment, an anti-PD-1 antibody comprises heavy and light chains that are each at least 96% identical to the sequences shown in SEQ ID NO:84 and SEQ ID NO:85, respectively, or antigen binding fragments and variants thereof. In an embodiment, an anti-PD-1 antibody comprises heavy and light chains that are each at least 95% identical to the sequences shown in SEQ ID NO:84 and SEQ ID NO:85, respectively, or antigen binding fragments and variants thereof.

In an embodiment, the anti-PD-1 antibody comprises the heavy and light chain CDRs or VRs of pembrolizumab. In one embodiment, the anti-PD-1 antibody V_{H} region comprises the sequence shown in SEQ ID NO:86, and the anti-PD-1 antibody V_{L} region comprises the sequence shown in SEQ ID NO:87. In an embodiment, an anti-PD-1 antibody comprises V_{H} and V_{L} regions that are each at least 99% identical to the sequences shown in SEQ ID NO:86 and SEQ ID NO:87, respectively. In an embodiment, an anti-PD-1 antibody comprises V_{H} and V_{L} regions that are each at least 98% identical to the sequences shown in SEQ ID NO:86 and SEQ ID NO: 87, respectively. In an embodiment, an anti-PD-1 antibody comprises V_{H} and V_{L} regions that are each at least 97% identical to the sequences shown in SEQ ID NO:86 and SEQ ID NO:87, respectively. In an embodiment, an anti-PD-1 antibody comprises V_{H} and V_{L} regions that are each at least 96% identical to the sequences shown in SEQ ID NO:86 and SEQ ID NO:87, respectively. In an embodiment, an anti-PD-1 antibody comprises V_{H} and V_{L} regions that are each at least 95% identical to the sequences shown in SEQ ID NO:86 and SEQ ID NO:87, respectively. In an alternative embodiment, the antibody comprises V_{H} and/or V_{L} regions having the amino acid sequences set forth in SEQ ID NO:86 and/or SEQ ID NO:87, respectively.

In an embodiment, the anti-PD-1 antibody comprises heavy chain CDR1, CDR2 and CDR3 domains having the sequences set forth in SEQ ID NO:88, SEQ ID NO:89, and SEQ ID NOVO, respectively, and light chain CDR1, CDR2 and CDR3 domains having the sequences set forth in SEQ ID NO:91, SEQ ID NO:92, and SEQ ID NO:93, respectively.

In an embodiment, an anti-PD-1 antibody comprises a heavy chain that comprises CDR1, CDR2 and CDR3 domains that are each at least 95% identical to the sequences shown in SEQ ID NO: 88, SEQ ID NO:89, and SEQ ID NOVO, respectively. In an embodiment, an anti-PD-1 antibody comprises a heavy chain that comprises CDR1, CDR2 and CDR3 domains that are each at least 90% identical to the sequences shown in SEQ ID NO:88, SEQ ID NO:89, and SEQ ID NOVO, respectively. In an embodiment, an anti-PD-1 antibody comprises a heavy chain that comprises CDR1, CDR2 and CDR3 domains that are each at least 85% identical to the sequences shown in SEQ ID NO:88, SEQ ID NO:89, and SEQ ID NO:90, respectively. In an embodiment, an anti-PD-1 antibody comprises a heavy chain that comprises CDR1, CDR2 and CDR3 domains that are each at least 80% identical to the sequences shown in SEQ ID NO:88, SEQ ID NO: 89, and SEQ ID NOVO, respectively. In another embodiment, the antibody competes for binding with, and/or binds to the same epitope on PD-1 as the aforementioned antibodies.

In an embodiment, an anti-PD-1 antibody comprises a light chain that comprises CDR1, CDR2 and CDR3 domains that are each at least 95% identical to the sequences shown in SEQ ID NO:91, SEQ ID NO:92, and SEQ ID NO:93, respectively. In an embodiment, an anti-PD-1 antibody comprises a light chain that comprises CDR1, CDR2 and CDR3 domains that are each at least 90% identical to the sequences shown in SEQ ID NO:91, SEQ ID NO:92, and SEQ ID NO:93, respectively. In an embodiment, an anti-PD-1 antibody comprises a light chain that comprises CDR1, CDR2 and CDR3 domains that are each at least 85% identical to the sequences shown in SEQ ID NO:91, SEQ ID NO:92, and SEQ ID NO:93, respectively. In an embodiment, an anti-PD-1 antibody comprises a light chain that comprises CDR1, CDR2 and CDR3 domains that are each at least 80% identical to the sequences shown in SEQ ID NO:91, SEQ ID NO:92, and SEQ ID NO:93, respectively. In another embodiment, the antibody competes for binding with, and/or binds to the same epitope on PD-1 as the aforementioned antibodies.

In an embodiment, the anti-PD-1 antibody is an antibody disclosed in U.S. Patent No. 8,354,509 or U.S. Patent Application Publication Nos. 2010/0266617 A1, 2013/0108651 A1, 2013/0109843 A2.

In an embodiment, the anti-PD-1 antibody is pidilizumab, which is also known as CT-011 (CureTech Ltd.), and which is disclosed in U.S. Patent No. 8,686,119 B2. The efficacy of pidilizumab in the treatment of cancers, such as hematological malignancies, is described in Berger, et al., Clin. Cancer Res. 2008, 14, 3044-51. The pidilizumab monoclonal antibody includes a heavy chain given by SEQ ID NO:94 and a light chain given by SEQ ID NO:95. Pidilizumab has intra-heavy chain disulfide linkages at 22-96, 144-200, 261-321, 367-425, 22"-96", 144"-200", 261"-321", and 367"-425"; intra-light chain disulfide linkages at 23'-87', 133'-193', 23"'-87'", and 133‴-193‴; inter-heavy-light chain disulfide linkages at 220-213' and 220"-213'", inter-heavy-heavy chain disulfide linkages at 226-226" 229-229"; and N-glycosylation sites (H CH₂ 84.4) at 297, 297".

In an embodiment, the anti-PD-1 antibody is an immunoglobulin G1 kappa, anti-(human CD274) humanized monoclonal antibody. In an embodiment, an anti-PD-1 antibody comprises heavy and light chains having the sequences shown in SEQ ID NO:94 and SEQ ID NO:95, respectively, or antigen binding fragments, variants, or conjugates thereof. In an embodiment, an anti-PD-1 antibody comprises heavy and light chains that are each at least 99% identical to the sequences shown in SEQ ID NO:94 and SEQ ID NO:95, respectively. In an embodiment, an anti-PD-1 antibody comprises heavy and light chains that are each at least 98% identical to the sequences shown in SEQ ID NO:94 and SEQ ID NO:95, respectively. In an embodiment, an anti-PD-1 antibody comprises heavy and light chains that are each at least 97% identical to the sequences shown in SEQ ID NO:94 and SEQ ID NO:95, respectively. In an embodiment, an anti-PD-1 antibody comprises heavy and light chains that are each at least 96% identical to the sequences shown in SEQ ID NO:94 and SEQ ID NO:95, respectively. In an embodiment, an anti-PD-1 antibody comprises heavy and light chains that are each at least 95% identical to the sequences shown in SEQ ID NO:94 and SEQ ID NO:95, respectively.

In an embodiment, an anti-PD-L1 antibody comprises V_{H} and V_{L} regions that are each at least 99% identical to the sequences shown in SEQ ID NO:96 and SEQ ID NO:97, respectively. In an embodiment, an anti-PD-L1 antibody comprises V_{H} and V_{L} regions that are each at least 98% identical to the sequences shown in SEQ ID NO:96 and SEQ ID NO:97, respectively. In an embodiment, an anti-PD-L1 antibody comprises V_{H} and V_{L} regions that are each at least 97% identical to the sequences shown in SEQ ID NO:96 and SEQ ID NO:97, respectively. In an embodiment, an anti-PD-L1 antibody comprises V_{H} and V_{L} regions that are each at least 96% identical to the sequences shown in SEQ ID NO:96 and SEQ ID NO:97, respectively. In an embodiment, an anti-PD-L1 antibody comprises V_{H} and V_{L} regions that are each at least 95% identical to the sequences shown in SEQ ID NO:96 and SEQ ID NO:97, respectively.

In an embodiment, anti-PD-1 antibodies and other PD-1 inhibitors include those described in U.S. Patent Nos. 8,287,856, 8,580,247, and 8,168,757 and U.S. Patent Application Publication Nos. 2009/0028857 A1, 2010/0285013 A1, 2013/0022600 A1, and 2011/0008369 A1. In another embodiment, antibodies that compete with any of these antibodies for binding to PD-1 are also included. In another embodiment, the anti-PD-1 antibody is an antibody disclosed in U.S. Patent No. 8,735,553 B1.

In an embodiment, the anti-PD-1 antibody is a commercially-available monoclonal antibody, such as anti-m-PD-1 clones J43 (Cat # BE0033-2) and RMP1-14 (Cat # BE0146) (Bio X Cell, Inc., West Lebanon, NH, USA). A number of commercially-available anti-PD-1 antibodies are known to one of ordinary skill in the art.

Monoclonal antibodies that inhibit or block PD-1 can be prepared by procedures known to those of ordinary knowledge and skill in the art, *e.g.,* by injecting test subjects with PD-1 antigen and then isolating hybridomas expressing antibodies having the desired sequence or functional characteristics. DNA encoding the monoclonal antibodies is readily isolated and sequenced using conventional procedures (*e.g.*, by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as *E*. *coli* cells, simian COS cells, Chinese hamster ovary (CHO) cells, myeloma cells, or other suitable cells that do not otherwise produce immunoglobulin protein, to obtain the synthesis of monoclonal antibodies in the recombinant host cells. The details of recombinant production of specific antibodies may be found in the references cited in the foregoing. Monoclonal antibodies that inhibit PD-1 can be prepared by standard molecular biology methods using the sequences provided herein by reverse translation and insertion into appropriate DNA or RNA vectors.

In an embodiment, the PD-1 inhibitor may be a small molecule or a peptide, or a peptide derivative, such as those described in U.S. Patent Nos. 8,907,053; 9,096,642; and 9,044,442 and U.S. Patent Application Publication No. 2015/0087581; 1,2,4 oxadiazole compounds and derivatives such as those described in U.S. Patent Application Publication No. 2015/0073024; cyclic peptidomimetic compounds and derivatives such as those described in U.S. Patent Application Publication No. 2015/0073042; cyclic compounds and derivatives such as those described in U.S. Patent Application Publication No. 2015/0125491; 1,3,4 oxadiazole and 1,3,4 thiadiazole compounds and derivatives such as those described in International Patent Application Publication No. WO 2015/033301; peptide-based compounds and derivatives such as those described in International Patent Application Publication Nos. WO 2015/036927 and WO 2015/04490, or a macrocyclic peptide-based compounds and derivatives such as those described in U.S. Patent Application Publication No. 2014/0294898.

The anti-PD-1 antibody sequences discussed and referenced in some of the foregoing embodiments are summarized in Table 5.

**TABLE 5. Anti-PD-1 antibody amino acid sequences.**

| **Identifier** | **Sequence (One-Letter Amino Acid Symbols)** | |
|---|---|---|
| SEQ ID NO:74 nivolumab heavy chain | QVQLVESGGG VVQPGRSLRL DCKASGITFS NSGMHWVRQA PGKGLEWVAV IWYDGSKRYY 60 ADSVKGRFTI SRDNSKNTLF LQMNSLRAED TAVYYCATND DYWGQGTLVT VSSASTKGPS 120 VFPLAPCSRS TSESTAALGC LVKDYFPEPV TVSWNSGALT SGVHTFPAVL QSSGLYSLSS 180 VVTVPSSSLG TKTYTCNVDH KPSNTKVDKR VESKYGPPCP PCPAPEFLGG PSVFLFPPKP 240 KDTLMISRTP EVTCVVVDVS QEDPEVQFNW YVDGVEVHNA KTKPREEQFN STYRVVSVLT 300 VLHQDWLNGK EYKCKVSNKG LPSSIEKTIS KAKGQPREPQ VYTLPPSQEE MTKNQVSLTC 360 LVKGFYPSDI AVEWESNGQP ENNYKTTPPV LDSDGSFFLY SRLTVDKSRW QEGNVFSCSV 420 MHEALHNHYT QKSLSLSLGK 440 | |
| SEQ ID NO:75 nivolumab light chain | EIVLTQSPAT LSLSPGERAT LSCRASQSVS SYLAWYQQKP GQAPRLLIYD ASNRATGIPA 60 RFSGSGSGTD FTLTISSLEP EDFAVYYCQQ SSNWPRTFGQ GTKVEIKRTV AAPSVFIFPP 120 SDEQLKSGTA SVVCLLNNFY PREAKVQWKV DNALQSGNSQ ESVTEQDSKD STYSLSSTLT 180 LSKADYEKHK VYACEVTHQG LSSPVTKSFN RGEC 214 | |
| SEQ ID NO:76 nivolumab variable heavy chain |  113 | |
| SEQ ID NO:77 nivolumab variable light chain |  107 | |
| SEQ ID NO:78 nivolumab heavy chain CDR1 | NSGMH | 5 |
| SEQ ID NO:79 nivolumab heavy chain CDR2 | VIWYDGSKRY YADSVKG | 17 |
| SEQ ID NO:80 nivolumab heavy chain CDR3 | NDDY | 4 |
| SEQ ID NO:81 nivolumab light chain CDR1 | RASQSVSSYL A | 11 |
| SEQ ID NO:82 nivolumab light chain CDR2 | DASNRAT | 7 |
| SEQ ID NO:83 nivolumab light chain CDR3 | QQSSNWPRT | 9 |
| SEQ ID NO:84 pembrolizumab heavy chain | QVQLVQSGVE VKKPGASVKV SCKASGYTFT NYYMYWVRQA PGQGLEWMGG INPSNGGTNF 60 NEKFKNRVTL TTDSSTTTAY MELKSLQFDD TAVYYCARRD YRFDMGFDYW GQGTTVTVSS 120 ASTKGPSVFP LAPCSRSTSE STAALGCLVK DYFPEPVTVS WNSGALTSGV HTFPAVLQSS 180 GLYSLSSVVT VPSSSLGTKT YTCNVDHKPS NTKVDKRVES KYGPPCPPCP APEFLGGPSV 240 FLFPPKPKDT LMISRTPEVT CVVVDVSQED PEVQFNWYVD GVEVHNAKTK PREEQFNSTY 300 RVVSVLTVLH QDWLNGKEYK CKVSNKGLPS SIEKTISKAK GQPREPQVYT LPPSQEEMTK 360 NQVSLTCLVK GFYPSDIAVE WESNGQPENN YKTTPPVLDS DGSFFLYSRL TVDKSRWQEG 420 NVFSCSVMHE ALHNHYTQKS LSLSLGK 447 | |
| SEQ ID NO:85 pembrolizumab light chain | EIVLTQSPAT LSLSPGERAT LSCRASKGVS TSGYSYLHWY QQKPGQAPRL LIYLASYLES 60 GVPARFSGSG SGTDFTLTIS SLEPEDFAVY YCQHSRDLPL TFGGGTKVEI KRTVAAPSVF 120 IFPPSDEQLK SGTASVVCLL NNFYPREAKV QWKVDNALQS GNSQESVTEQ DSKDSTYSLS 180 STLTLSKADY EKHKVYACEV THQGLSSPVT KSFNRGEC 218 | |
| SEQ ID NO:86 pembrolizumab variable heavy chain |  120 | |
| SEQ ID NO:87 pembrolizumab variable light chain |  111 | |
| SEQ ID NO:88 pembrolizumab heavy chain CDR1 | NYYMY | 5 |
| SEQ ID NO:89 pembrolizumab heavy chain CDR2 | GINPSNGGTN FNEKFK | 16 |
| SEQ ID NO:90 pembrolizumab heavy chain CDR3 | RDYRFDMGFD Y | 11 |
| SEQ ID NO:91 pembrolizumab light chain CDR1 | RASKGVSTSG YSYLH | 15 |
| SEQ ID NO:92 pembrolizumab light chain CDR2 | LASYLES | 7 |
| SEQ ID NO:93 pembrolizumab light chain CDR3 | QHSRDLPLT | 9 |
| SEQ ID NO:94 pidilizumab heavy chain | QVQLVQSGSE LKKPGASVKI SCKASGYTFT NYGMNWVRQA PGQGLQWMGW INTDSGESTY 60 AEEFKGRFVF SLDTSVNTAY LQITSLTAED TGMYFCVRVG YDALDYWGQG TLVTVSSAST 120 KGPSVFPLAP SSKSTSGGTA ALGCLVKDYF PEPVTVSWNS GALTSGVHTF PAVLQSSGLY 180 SLSSVVTVPS SSLGTQTYIC NVNHKPSNTK VDKRVEPKSC DKTHTCPPCP APELLGGPSV 240 FLFPPKPKDT LMISRTPEVT CVVVDVSHED PEVKFNWYVD GVEVHNAKTK PREEQYNSTY 300 RVVSVLTVLH QDWLNGKEYK CKVSNKALPA PIEKTISKAK GQPREPQVYT LPPSREEMTK 360 NQVSLTCLVK GFYPSDIAVE WESNGQPENN YKTTPPVLDS DGSFFLYSKL TVDKSRWQQG 420 NVFSCSVMHE ALHNHYTQKS LSLSPGK 447 | |
| SEQ ID NO:95 pidilizumab light chain | EIVLTQSPSS LSASVGDRVT ITCSARSSVS YMHWFQQKPG KAPKLWIYRT SNLASGVPSR 60 FSGSGSGTSY CLTINSLQPE DFATYYCQQR SSFPLTFGGG TKLEIKRTVA APSVFIFPPS 120 DEQLKSGTAS VVCLLNNFYP REAKVQWKVD NALQSGNSQE SVTEQDSKDS TYSLSSTLTL 180 SKADYEKHKV YACEVTHQGL SSPVTKSFNR GEC 213 | |
| SEQ ID NO:96 pidilizumab variable heavy chain | QVQLVQSGSE LKKPGASVKI SCKASGYTFT NYGMNWVRQA PGQGLQWMGW INTDSGESTY 60 AEEFKGRFVF SLDTSVNTAY LQITSLTAED TGMYFCVRVG YDALDYWGQG TLVTVSS 117 | |
| SEQ ID NO:97 pidilizumab variable light chain | EIVLTQSPSS LSASVGDRVT ITCSARSSVS YMHWFQQKPG KAPKLWIYRT SNLASGVPSR 60 FSGSGSGTSY CLTINSLQPE DFATYYCQQR SSFPLTFGGG TKLEIK 106 | |

The PD-L1 or PD-L2 inhibitor may be any PD-L1 or PD-L2 inhibitor or blocker known in the art. In particular, it is one of the PD-L1 or PD-L2 inhibitors or blockers described in more detail in the following paragraphs. The terms "inhibitor" and "blocker" are used interchangeably herein in reference to PD-L1 and PD-L2 inhibitors. For avoidance of doubt, references herein to a PD-L1 or PD-L2 inhibitor that is an antibody may refer to a compound or antigen-binding fragments, variants, conjugates, or biosimilars thereof. For avoidance of doubt, references herein to a PD-L1 or PD-L2 inhibitor may refer to a compound or a pharmaceutically acceptable salt, ester, solvate, hydrate, cocrystal, or prodrug thereof.

In some embodiments, the compositions and methods include a PD-L1 or PD-L2 inhibitor. In some embodiments, the PD-L1 and PD-L2 inhibitor is a small molecule. In some embodiments, the PD-L1 or PD-L2 inhibitor is an anti-PD-L1 or anti-PD-L2 antibody, a fragment thereof, including Fab fragments or single-chain variable fragments (scFv). In an aspect of the invention, the anti-PD-1 antibody or fragment thereof in any of the aforementioned embodiments is replaced by, or combined with, an anti-PD-Ll or anti-PD-L2 antibody or fragment thereof. In an embodiment, the antibody competes for binding with, and/or binds to an epitope on PD-L1 and/or PD-L2. In some embodiments, the PD-L1 or PD-L2 inhibitor is a monoclonal antibody. In some embodiments the PD-L1 or PD-L2 inhibitor is a polyclonal antibody. In some embodiments, a PD-L1 inhibitor is included in a composition and is further combined with a BTK inhibitor of Formula (II) and a CD19 inhibitor selected from an anti-CD19 antibody or an anti-CD19 chimeric antigen receptor, or an antigen-binding fragment or conjugate thereof. In some embodiments, an anti-PD-L1 monoclonal antibody is included in a composition and is further combined with a BTK inhibitor of Formula (II) and a CD19 inhibitor selected from an anti-CD19 antibody or an anti-CD19 chimeric antigen receptor, or an antigen-binding fragment or conjugate thereof. In some embodiments, a PD-L2 inhibitor is included in a composition and is further combined with a BTK inhibitor of Formula (II) and a CD19 inhibitor selected from an anti-CD19 antibody or an anti-CD19 chimeric antigen receptor, or an antigen-binding fragment or conjugate thereof. In some embodiments, an anti-PD-L2 monoclonal antibody is included in a composition and is further combined with a BTK inhibitor of Formula (II) and a CD 19 inhibitor selected from an anti-CD19 antibody or an anti-CD19 chimeric antigen receptor, or an antigen-binding fragment or conjugate thereof. In some embodiments, both a PD-1 inhibitor and a PD-L1 inhibitor are included in a composition and are further combined with a BTK inhibitor of Formula (II) and a CD19 inhibitor selected from an anti-CD19 antibody or an anti-CD19 chimeric antigen receptor, or an antigen-binding fragment or conjugate thereof. In some embodiments, both an anti-PD-1 monoclonal antibody and an anti-PD-L1 monoclonal antibody are included in a composition and are further combined with a BTK inhibitor of Formula (II) and a CD19 inhibitor selected from an anti-CD19 antibody or an anti-CD19 chimeric antigen receptor, or an antigen-binding fragment or conjugate thereof. In some embodiments, both a PD-1 inhibitor and a PD-L2 inhibitor are included in a composition and are further combined with a BTK inhibitor of Formula (II) and a CD19 inhibitor selected from an anti-CD19 antibody or an anti-CD19 chimeric antigen receptor, or an antigen-binding fragment or conjugate thereof. In some embodiments, both an anti-PD-1 monoclonal antibody and an anti-PD-L2 monoclonal antibody are included in a composition and are further combined with a BTK inhibitor of Formula (II) and a CD19 inhibitor selected from an anti-CD19 antibody or an anti-CD19 chimeric antigen receptor, or an antigen-binding fragment or conjugate thereof.

In preferred embodiments, the compositions described herein provide a combination of a PD-L1 and/or PD-L2 inhibitor with a BTK inhibitor of Formula (II) and a CD19 inhibitor selected from an anti-CD19 antibody or an anti-CD19 chimeric antigen receptor, or an antigen-binding fragment or conjugate thereof. In some embodiments, the PD-L1 inhibitors provided herein are selective for PD-L1, in that the compounds bind or interact with PD-L1 at substantially lower concentrations than they bind or interact with other receptors, including the PD-L2 receptor. In certain embodiments, the compounds bind to the PD-L2 receptor at a binding constant that is at least about a 2-fold higher concentration, about a 3-fold higher concentration, about a 5-fold higher concentration, about a 10-fold higher concentration, about a 20-fold higher concentration, about a 30-fold higher concentration, about a 50-fold higher concentration, about a 100-fold higher concentration, about a 200-fold higher concentration, about a 300-fold higher concentration, or about a 500-fold higher concentration than to the PD-L1 receptor.

Without being bound by any theory, it is believed that tumor cells express PD-L1, and that T cells express PD-1. However, PD-L1 expression by tumor cells is not required for efficacy of PD-1 or PD-L1 inhibitors or blockers. In an embodiment, the tumor cells express PD-L1. In another embodiment, the tumor cells do not express PD-L1. In some embodiments, the compositions described herein include a combination of a PD-1 and a PD-L1 antibody, such as those described herein, in combination with a BTK inhibitor of Formula (II). The administration of a combination of a PD-1 and a PD-L1 antibody and a BTK inhibitor of Formula (II) may be simultaneous or sequential.

In some embodiments, the compositions described include a PD-L1 and/or PD-L2 inhibitor that binds human PD-L1 and/or PD-L2 with a K_{D} of about 100 pM or lower, binds human PD-L1 and/or PD-L2 with a K_{D} of about 90 pM or lower, binds human PD-L1 and/or PD-L2 with a K_{D} of about 80 pM or lower, binds human PD-L1 and/or PD-L2 with a K_{D} of about 70 pM or lower, binds human PD-L1 and/or PD-L2 with a K_{D} of about 60 pM or lower, a K_{D} of about 50 pM or lower, binds human PD-L1 and/or PD-L2 with a K_{D} of about 40 pM or lower, or binds human PD-L1 and/or PD-L2 with a KD of about 30 pM or lower,

In some embodiments, the compositions described include a PD-L1 and/or PD-L2 inhibitor that binds to human PD-L1 and/or PD-L2 with a k_{assoc} of about 7.5 × 10⁵ 1/M·s or faster, binds to human PD-L1 and/or PD-L2 with a k_{assoc} of about 8 × 10⁵ 1/M·s or faster, binds to human PD-L1 and/ or PD-L2 with a k_{assoc} of about 8.5 × 10⁵ 1/M·s or faster, binds to human PD-L1 and/or PD-L2 with a k_{assoc} of about 9 × 10⁵ 1/M·s or faster, binds to human PD-L1 and/or PD-L2 with a k_{assoc} of about 9.5 × 10⁵ 1/M·s and/or faster, or binds to human PD-L1 and/or PD-L2 with a k_{assoc} of about 1 × 10⁶ 1/M·s or faster.

In some embodiments, the compositions for use described include a PD-L1 and/or PD-L2 inhibitor that binds to human PD-L1 or PD-L2 with a k_{dissoc} of about 2 × 10⁻⁵ 1/s or slower, binds to human PD-1 with a k_{dissoc} of about 2.1 × 10⁻⁵ 1/s or slower , binds to human PD-1 with a k_{dissoc} of about 2.2 × 10⁻⁵ 1/s or slower, binds to human PD-1 with a k_{dissoc} of about 2.3 × 10⁻⁵ 1/s or slower, binds to human PD-1 with a k_{dissoc} of about 2.4 × 10⁻⁵ 1/s or slower, binds to human PD-1 with a k_{dissoc} of about 2.5 × 10⁻⁵ 1/s or slower, binds to human PD-1 with a k_{dissoc} of about 2.6 × 10⁻⁵ 1/s or slower, binds to human PD-L1 or PD-L2 with a k_{dissoc} of about 2.7 × 10⁻⁵ 1/s or slower, or binds to human PD-L1 or PD-L2 with a k_{dissoc} of about 3 × 10⁻⁵ 1/s or slower.

In some embodiments, the compositions for use described include a PD-L1 and/or PD-L2 inhibitor that blocks or inhibits binding of human PD-Ll or human PD-L2 to human PD-1 with an IC₅₀ of about 10 nM or lower; blocks or inhibits binding of human PD-Ll or human PD-L2 to human PD-1 with an IC₅₀ of about 9 nM or lower; blocks or inhibits binding of human PD-Ll or human PD-L2 to human PD-1 with an IC₅₀ of about 8 nM or lower; blocks or inhibits binding of human PD-Ll or human PD-L2 to human PD-1 with an IC₅₀ of about 7 nM or lower; blocks or inhibits binding of human PD-Ll or human PD-L2 to human PD-1 with an IC₅₀ of about 6 nM or lower; blocks or inhibits binding of human PD-Ll or human PD-L2 to human PD-1 with an IC₅₀ of about 5 nM or lower; blocks or inhibits binding of human PD-Ll or human PD-L2 to human PD-1 with an IC₅₀ of about 4 nM or lower; blocks or inhibits binding of human PD-Ll or human PD-L2 to human PD-1 with an IC₅₀ of about 3 nM or lower; blocks or inhibits binding of human PD-Ll or human PD-L2 to human PD-1 with an IC₅₀ of about 2 nM or lower; or blocks human PD-1, or blocks binding of human PD-L1 or human PD-L2 to human PD-l with an IC₅₀ of about 1 nM or lower.

In an embodiment, the anti-PD-L1 antibody is durvalumab, also known as MEDI4736 (which is commercially available from Medimmune, LLC, Gaithersburg, Maryland, a subsidiary of AstraZeneca plc.), or antigen-binding fragments, conjugates, or variants thereof. In an embodiment, the anti-PD-L1 antibody is an antibody disclosed in U.S. Patent No. 8,779,108 or U.S. Patent Application Publication No. 2013/0034559. The clinical efficacy of durvalumab (MEDI4736, SEQ ID NO:98 and SEQ ID NO:99) has been described in: Page, et al., Ann. Rev. Med., 2014, 65, 185-202; Brahmer, et al., J. Clin. Oncol. 2014, 32, 5s (supplement, abstract 8021); and McDermott, et al., Cancer Treatment Rev., 2014, 40, 1056-64. The durvalumab (MEDI4736) monoclonal antibody includes a V_{H} region given by SEQ ID NO: 100 (corresponding to SEQ ID NO:72 in U.S. Patent No. 8,779,108) and a V_{L} region given by SEQ ID NO:101 (corresponding to SEQ ID NO:77 in U.S. Patent No. 8,779,108). The durvalumab monoclonal antibody includes disulfide linkages at 22-96, 22"-96", 23'-89', 23‴-89‴, 135'-195', 135‴-195‴, 148-204, 148"-204", 215'-224, 215"'-224", 230-230", 233-233", 265-325, 265"-325", 371-429, and 371"-429'; and N-glycosylation sites at Asn-301 and Asn-301".

In an embodiment, the anti-PD-L1 antibody is an immunoglobulin G1, anti-(human CD antigen CD274) (human monoclonal heavy chain), disulfide with human monoclonal κ-chain, dimer. In an embodiment, the anti-PD-L1 antibody comprises the heavy and light chains of durvalumab (MEDI4736). In an embodiment, an anti-PD-L1 antibody comprises heavy and light chains having the sequences shown in SEQ ID NO:98 and SEQ ID NO:99, respectively, or antigen binding fragments, variants, or conjugates thereof. In an embodiment, an anti-PD-L1 antibody comprises heavy and light chains that are each at least 99% identical to the sequences shown in SEQ ID NO:98 and SEQ ID NO:99, respectively. In an embodiment, an anti-PD-L1 antibody comprises heavy and light chains that are each at least 98% identical to the sequences shown in SEQ ID NO:98 and SEQ ID NO:99, respectively. In an embodiment, an anti-PD-L1 antibody comprises heavy and light chains that are each at least 97% identical to the sequences shown in SEQ ID NO:98 and SEQ ID NO:99, respectively. In an embodiment, an anti-PD-L1 antibody comprises heavy and light chains that are each at least 96% identical to the sequences shown in SEQ ID NO:98 and SEQ ID NO:99, respectively. In an embodiment, an anti-PD-L1 antibody comprises heavy and light chains that are each at least 95% identical to the sequences shown in SEQ ID NO:98 and SEQ ID NO:99, respectively.

In an embodiment, the anti-PD-L1 antibody comprises V_{H} and V_{L} regions having the sequences shown in SEQ ID NO: 100 (corresponding to SEQ ID NO:72 in U.S. Patent No. 8,779,108) and SEQ ID NO: 101 (corresponding to SEQ ID NO:77 in U.S. Patent No. 8,779,108), respectively, as described in U.S. Patent No. 8,779,108 or U.S. Patent Application Publication No. 2013/0034559 including antigen binding fragments, conjugates, and variants thereof. In an embodiment, an anti-PD-L1 antibody comprises V_{H} and V_{L} regions that are each at least 99% identical to the sequences shown in SEQ ID NO:100 and SEQ ID NO: 101, respectively. In an embodiment, an anti-PD-L1 antibody comprises V_{H} and V_{L} regions that are each at least 98% identical to the sequences shown in SEQ ID NO:100 and SEQ ID NO:101, respectively. In an embodiment, an anti-PD-L1 antibody comprises V_{H} and V_{L} regions that are each at least 97% identical to the sequences shown in SEQ ID NO:100 and SEQ ID NO:101, respectively. In an embodiment, an anti-PD-L1 antibody comprises V_{H} and V_{L} regions that are each at least 96% identical to the sequences shown in SEQ ID NO:100 and SEQ ID NO:101, respectively. In an embodiment, an anti-PD-L1 antibody comprises V_{H} and V_{L} regions that are each at least 95% identical to the sequences shown in SEQ ID NO:100 and SEQ ID NO:101, respectively. In an embodiment, an anti-PD-L1 antibody comprises V_{H} and V_{L} regions that are each at least 90% identical to the sequences shown in SEQ ID NO:100 and SEQ ID NO:101, respectively.

In an embodiment, the anti-PD-L1 antibody comprises heavy chain CDR1, CDR2 and CDR3 domains having the sequences set forth in SEQ ID NO: 102, SEQ ID NO:103, and SEQ ID NO:104, respectively, and light chain CDR1, CDR2 and CDR3 domains having the sequences set forth in SEQ ID NO: 105, SEQ ID NO:106, and SEQ ID NO:107, respectively.

In an embodiment, an anti-PD-L1 antibody comprises a heavy chain that comprises CDR1, CDR2 and CDR3 domains that are each at least 95% identical to the sequences shown in SEQ ID NO: 102, SEQ ID NO:103, and SEQ ID NO:104, respectively. In an embodiment, an anti-PD-L1 antibody comprises a heavy chain that comprises CDR1, CDR2 and CDR3 domains that are each at least 90% identical to the sequences shown in SEQ ID NO:102, SEQ ID NO:103, and SEQ ID NO:104, respectively. In an embodiment, an anti-PD-L1 antibody comprises a heavy chain that comprises CDR1, CDR2 and CDR3 domains that are each at least 85% identical to the sequences shown in SEQ ID NO: 102, SEQ ID NO:103, and SEQ ID NO:104, respectively. In an embodiment, an anti-PD-L1 antibody comprises a heavy chain that comprises CDR1, CDR2 and CDR3 domains that are each at least 80% identical to the sequences shown in SEQ ID NO:102, SEQ ID NO:103, and SEQ ID NO:104, respectively. In another embodiment, the antibody competes for binding with, and/or binds to the same epitope on PD-L1 as the aforementioned antibodies.

In an embodiment, an anti-PD-L1 antibody comprises a light chain that comprises CDR1, CDR2 and CDR3 domains that are each at least 95% identical to the sequences shown in SEQ ID NO: 105, SEQ ID NO: 106, and SEQ ID NO: 107, respectively. In an embodiment, an anti-PD-L1 antibody comprises a light chain that comprises CDR1, CDR2 and CDR3 domains that are each at least 90% identical to the sequences shown in SEQ ID NO: 105, SEQ ID NO: 106, and SEQ ID NO: 107, respectively. In an embodiment, an anti-PD-L1 antibody comprises a light chain that comprises CDR1, CDR2 and CDR3 domains that are each at least 85% identical to the sequences shown in SEQ ID NO:105, SEQ ID NO:106, and SEQ ID NO:107, respectively. In an embodiment, an anti-PD-L1 antibody comprises a light chain that comprises CDR1, CDR2 and CDR3 domains that are each at least 80% identical to the sequences shown in SEQ ID NO: 105, SEQ ID NO: 106, and SEQ ID NO: 107, respectively. In another embodiment, the antibody competes for binding with, and/or binds to the same epitope on PD-L1 as the aforementioned antibodies.

In an embodiment, anti-PD-L1 antibodies and other PD-L1 inhibitors include those described in U.S. Patent No. 8,779,108 and U.S. Patent Application Publication No. 2013/0034559A1. In another embodiment, antibodies that compete with any of these antibodies for binding to PD-L1 are also included.

In an embodiment, the anti-PD-L1 antibody is atezolizumab, also known as MPDL3280A or RG7446 (commercially available from Genentech, Inc., a subsidiary of Roche), or antigen-binding fragments, conjugates, or variants thereof. In an embodiment, the anti-PD-L1 antibody is an antibody disclosed in U.S. Patent No. 8,217,149. In an embodiment, the anti-PD-L1 antibody is an antibody disclosed in U.S. Patent Application Publication Nos. 2010/0203056 A1, 2013/0045200 A1, 2013/0045201 A1, 2013/0045202 A1, or 2014/0065135 A1. The atezolizumab monoclonal antibody includes a heavy chain given by SEQ ID NO: 108 and a light chain given by SEQ ID NO: 109. Atezolizumab has intra-heavy chain disulfide linkages (C23-C104) at 22-96, 145-201, 262-322, 368-426, 22"-96", 145"-201", 262"-322", and 368"-426"; intra-light chain disulfide linkages (C23-C104) at 23'-88', 134'-194', 23‴-88‴, and 134"'-194" ; intra-heavy-light chain disulfide linkages (h 5-CL 126) at 221-214' and 221"-214‴; intra-heavy-heavy chain disulfide linkages (h 11, h 14) at 227-227" and 230-230"; and N-glycosylation sites (H CH₂ N84.4>A) at 298 and 298'.

In an embodiment, the anti-PD-L1 antibody is an immunoglobulin G1 kappa, anti-(human PD-L1) humanized monoclonal antibody. In an embodiment, the anti-PD-L1 antibody comprises the heavy and light chains of atezolizumab (MPDL3280A). In an embodiment, an anti-PD-L1 antibody comprises heavy and light chains having the sequences shown in SEQ ID NO:108 and SEQ ID NO:109, respectively, or antigen binding fragments, variants, or conjugates thereof. In an embodiment, an anti-PD-L1 antibody comprises heavy and light chains that are each at least 99% identical to the sequences shown in SEQ ID NO: 108 and SEQ ID NO: 109, respectively. In an embodiment, an anti-PD-L1 antibody comprises heavy and light chains that are each at least 98% identical to the sequences shown in SEQ ID NO:108 and SEQ ID NO:109, respectively. In an embodiment, an anti-PD-L1 antibody comprises heavy and light chains that are each at least 97% identical to the sequences shown in SEQ ID NO:108 and SEQ ID NO:109, respectively. In an embodiment, an anti-PD-L1 antibody comprises heavy and light chains that are each at least 96% identical to the sequences shown in SEQ ID NO:108 and SEQ ID NO:109, respectively. In an embodiment, an anti-PD-L1 antibody comprises heavy and light chains that are each at least 95% identical to the sequences shown in SEQ ID NO:108 and SEQ ID NO:109, respectively.

In an embodiment, the anti-PD-L1 antibody comprises the heavy and light chain CDRs or VRs of atezolizumab (MPDL3280A). In an embodiment, the anti-PD-L1 antibody V_{H} region comprises the sequence shown in SEQ ID NO:110 (corresponding to SEQ ID NO:20 in U.S. Patent No. 8,217,149), and the anti-PD-L1 antibody V_{L} region comprises the sequence shown in SEQ ID NO: 111 (corresponding to SEQ ID NO:21 in U.S. Patent No. 8,217,149). In an embodiment, an anti-PD-L1 antibody comprises V_{H} and V_{L} regions that are each at least 99% identical to the sequences shown in SEQ ID NO:110 and SEQ ID NO: 111, respectively. In an embodiment, an anti-PD-L1 antibody comprises V_{H} and V_{L} regions that are each at least 98% identical to the sequences shown in SEQ ID NO:110 and SEQ ID NO: 111, respectively. In an embodiment, an anti-PD-L1 antibody comprises V_{H} and V_{L} regions that are each at least 97% identical to the sequences shown in SEQ ID NO:110 and SEQ ID NO: 111, respectively. In an embodiment, an anti-PD-L1 antibody comprises V_{H} and V_{L} regions that are each at least 96% identical to the sequences shown in SEQ ID NO:110 and SEQ ID NO:111, respectively. In an embodiment, an anti-PD-L1 antibody comprises V_{H} and V_{L} regions that are each at least 95% identical to the sequences shown in SEQ ID NO:110 and SEQ ID NO:111, respectively.

In an embodiment, the anti-PD-L1 antibody comprises a heavy chain variable region (V_{H}) polypeptide that comprises a CDR1, CDR2, and CDR3 sequence, wherein the CDR1 sequence is given by SEQ ID NO:112 (GFTFSX₁SWIH) (corresponding to SEQ ID NO:1 in U.S. Patent No. 8,217,149), the CDR2 sequence is SEQ ID NO:113 (AWIX₂PYGGSX₃YYADSVKG) (corresponding to SEQ ID NO:2 in U.S. Patent No. 8,217,149), and the CDR3 sequence is SEQ ID NO:114 (RHWPGGFDY) (corresponding to SEQ ID NO:3 in U.S. Patent No. 8,217,149), further wherein X₁ is D or G, X₂ is S or L, and X₃ is T or S, and the anti-PD-L1 antibody also comprises a light chain variable region (V_{L}) polypeptide that comprises a CDR1, CDR2, and CDR3 sequence wherein the CDR1 sequence is given by SEQ ID NO:115 (RASQX₄X₅X₆TX₇X₈A) (corresponding to SEQ ID NO:8 in U.S. Patent No. 8,217,149), the CDR2 sequence is given by SEQ ID NO:116 (SASX₉LX₁₀S) (corresponding to SEQ ID NO:9 in U.S. Patent No. 8,217,149), and the CDR3 sequence is SEQ ID NO:117 (QQX₁₁X₁₂X₁₃X₁₄PX₁₅T) (corresponding to SEQ ID NO:10 in U.S. Patent No. 8,217,149), further wherein further wherein: X₄ is D or V; X₅ is V or I; X₆ is S or N; X₇ is A or F; X₈ isV or L; X₉ is For T; X₁₀ is Y or A; X₁₁ is Y, G, F, or S; X₁₂ is L, Y, F or W; X₁₃ is Y, N, A, T, G, F or I; X₁₄ is H, V, P, T or I; and X₁₅ is A, W, R, P or T.

In an embodiment, the anti-PD-L1 antibody is avelumab, also known as MSB0010718C (commercially available from Merck KGaA/EMD Serono), or antigen-binding fragments, conjugates, or variants thereof. In an embodiment, the anti-PD-L1 antibody is an antibody disclosed in U.S. Patent Application Publication No. US 2014/0341917 A1. The avelumab monoclonal antibody includes a heavy chain given by SEQ ID NO:118 and a light chain given by SEQ ID NO:119. Avelumab has intra-heavy chain disulfide linkages (C23-C104) at 22-96, 147-203, 264-324, 370-428, 22"-96", 147"-203", 264"-324", and 370"-428"; intra-light chain disulfide linkages (C23-C104) at 22'-90', 138'-197', 22'"-90'", and 138‴-197‴; intra-heavy-light chain disulfide linkages (h 5-CL 126) at 223-215' and 223"-215‴; intra-heavy-heavy chain disulfide linkages (h 11, h 14) at 229-229" and 232-232"; N-glycosylation sites (H CH₂ N84.4) at 300, 300"; fucosylated complex bi-antennary CHO-type glycans; and H CHS K2 C-terminal lysine clipping at 450 and 450'.

In an embodiment, the anti-PD-L1 antibody is an immunoglobulin G1 lambda-1, anti-(human PD-L1) human monoclonal antibody. In an embodiment, the anti-PD-L1 antibody comprises the heavy and light chains of avelumab (MSB0010718C). In an embodiment, an anti-PD-L1 antibody comprises heavy and light chains having the sequences shown in SEQ ID NO:118 and SEQ ID NO:119, respectively, or antigen binding fragments, variants, or conjugates thereof. In an embodiment, an anti-PD-L1 antibody comprises heavy and light chains that are each at least 99% identical to the sequences shown in SEQ ID NO:118 and SEQ ID NO:119, respectively. In an embodiment, an anti-PD-L1 antibody comprises heavy and light chains that are each at least 98% identical to the sequences shown in SEQ ID NO:118 and SEQ ID NO:119, respectively. In an embodiment, an anti-PD-L1 antibody comprises heavy and light chains that are each at least 97% identical to the sequences shown in SEQ ID NO:118 and SEQ ID NO:119, respectively. In an embodiment, an anti-PD-L1 antibody comprises heavy and light chains that are each at least 96% identical to the sequences shown in SEQ ID NO:118 and SEQ ID NO:119, respectively. In an embodiment, an anti-PD-L1 antibody comprises heavy and light chains that are each at least 95% identical to the sequences shown in SEQ ID NO:118 and SEQ ID NO:119, respectively.

In an embodiment, the anti-PD-L1 antibody V_{H} region comprises the sequence given in SEQ ID NO: 120 (corresponding to SEQ ID NO:24 in U.S. Patent Application Publication No. US 2014/0341917 A1), and the anti-PD-L1 antibody V_{L} region comprises the sequence given in SEQ ID NO: 121 (corresponding to SEQ ID NO:25 in U.S. Patent Application Publication No. US 2014/0341917 A1). In an embodiment, an anti-PD-L1 antibody comprises V_{H} and V_{L} regions that are each at least 99% identical to the sequences shown in SEQ ID NO:120 and SEQ ID NO:121, respectively. In an embodiment, an anti-PD-L1 antibody comprises V_{H} and V_{L} regions that are each at least 98% identical to the sequences shown in SEQ ID NO:120 and SEQ ID NO:121, respectively. In an embodiment, an anti-PD-L1 antibody comprises V_{H} and V_{L} regions that are each at least 97% identical to the sequences shown in SEQ ID NO:120 and SEQ ID NO:121, respectively. In an embodiment, an anti-PD-L1 antibody comprises V_{H} and V_{L} regions that are each at least 96% identical to the sequences shown in SEQ ID NO:120 and SEQ ID NO:121, respectively. In an embodiment, an anti-PD-L1 antibody comprises V_{H} and V_{L} regions that are each at least 95% identical to the sequences shown in SEQ ID NO:120 and SEQ ID NO:121, respectively.

In an embodiment, the anti-PD-L1 antibody comprises a heavy chain variable region (V_{H}) polypeptide that comprises a CDR1, CDR2, and CDR3 sequence, wherein the CDR1 sequence is given by SEQ ID NO:122 (corresponding to SEQ ID NO:15 in U.S. Patent Application Publication No. US 2014/0341917 A1), the CDR2 sequence is given by SEQ ID NO:123 (corresponding to SEQ ID NO: 16 in U.S. Patent Application Publication No. US 2014/0341917 A1), and the CDR3 sequence is given by SEQ ID NO:124 (corresponding to SEQ ID NO:17 in U.S. Patent Application Publication No. US 2014/0341917 A1), and the anti-PD-L1 antibody also comprises a light chain variable region (V_{L}) polypeptide that comprises a CDR1, CDR2, and CDR3 sequence wherein the CDR1 sequence is given by SEQ ID NO:125 (corresponding to SEQ ID NO:18 in U.S. Patent Application Publication No. US 2014/0341917 A1), the CDR2 sequence is given by SEQ ID NO:126 (corresponding to SEQ ID NO:19 in U.S. Patent Application Publication No. US 2014/0341917 A1), and the CDR3 sequence is given by SEQ ID NO: 127 (corresponding to SEQ ID NO:20 in U.S. Patent Application Publication No. US 2014/0341917 A1).

In an embodiment, an anti-PD-L1 antibody comprises a heavy chain that comprises CDR1, CDR2 and CDR3 domains that are each at least 95% identical to the sequences shown in SEQ ID NO: 122, SEQ ID NO:123, and SEQ ID NO:124, respectively. In an embodiment, an anti-PD-L1 antibody comprises a heavy chain that comprises CDR1, CDR2 and CDR3 domains that are each at least 90% identical to the sequences shown in SEQ ID NO:122, SEQ ID NO: 123, and SEQ ID NO:124, respectively. In an embodiment, an anti-PD-L1 antibody comprises a heavy chain that comprises CDR1, CDR2 and CDR3 domains that are each at least 85% identical to the sequences shown in SEQ ID NO:122, SEQ ID NO:123, and SEQ ID NO:124, respectively. In an embodiment, an anti-PD-L1 antibody comprises a heavy chain that comprises CDR1, CDR2 and CDR3 domains that are each at least 80% identical to the sequences shown in SEQ ID NO:122, SEQ ID NO:123, and SEQ ID NO:124, respectively. In another embodiment, the antibody competes for binding with, and/or binds to the same epitope on PD-L1 as the aforementioned antibodies.

In an embodiment, an anti-PD-L1 antibody comprises a light chain that comprises CDR1, CDR2 and CDR3 domains that are each at least 95% identical to the sequences shown in SEQ ID NO: 125, SEQ ID NO:126, and SEQ ID NO:127, respectively. In an embodiment, an anti-PD-L1 antibody comprises a light chain that comprises CDR1, CDR2 and CDR3 domains that are each at least 90% identical to the sequences shown in SEQ ID NO:125, SEQ ID NO: 126, and SEQ ID NO:127, respectively. In an embodiment, an anti-PD-L1 antibody comprises a light chain that comprises CDR1, CDR2 and CDR3 domains that are each at least 85% identical to the sequences shown in SEQ ID NO:125, SEQ ID NO:126, and SEQ ID NO:127, respectively. In an embodiment, an anti-PD-L1 antibody comprises a light chain that comprises CDR1, CDR2 and CDR3 domains that are each at least 80% identical to the sequences shown in SEQ ID NO:125, SEQ ID NO: 126, and SEQ ID NO:127, respectively. In another embodiment, the antibody competes for binding with, and/or binds to the same epitope on PD-L1 as the aforementioned antibodies.

In an embodiment, anti-PD-L1 antibodies and other PD-L1 inhibitors include those described in U.S. Patent Application Publication No. 2014/0341917 A1. In another embodiment, antibodies that compete with any of these antibodies for binding to PD-L1 are also included.

In an embodiment, the anti-PD-L1 antibody is MDX-1105, also known as BMS-935559, which is disclosed in U.S. Patent No. US 7,943,743 B2. In an embodiment, the anti-PD-L1 antibody is selected from the anti-PD-L1 antibodies disclosed in U.S. Patent No. US 7,943,743 B2.

In an embodiment, the anti-PD-L1 antibody is a commercially-available monoclonal antibody, such as INVIVOMAB anti-m-PD-L1 clone 10F.9G2 (Catalog # BE0101, Bio X Cell, Inc., West Lebanon, NH, USA). In an embodiment, the anti-PD-L1 antibody is a commercially-available monoclonal antibody, such as AFFYMETRIX EBIOSCIENCE (MIH1). A number of commercially-available anti-PD-L1 antibodies are known to one of ordinary skill in the art.

In an embodiment, the anti-PD-L2 antibody is a commercially-available monoclonal antibody, such as BIOLEGEND 24F.10C12 Mouse IgG2a, κ isotype (catalog # 329602 Biolegend, Inc., San Diego, CA), SIGMA anti-PD-L2 antibody (catalog # SAB3500395, Sigma-Aldrich Co., St. Louis, MO), or other commercially-available anti-PD-L2 antibodies known to one of ordinary skill in the art.

Monoclonal antibodies that inhibit PD-L1 and/or PD-L2 can be prepared by procedures known to those of ordinary knowledge and skill in the art, e.g. by injecting test subjects with PD-L1 or PD-L2 antigen and then isolating hybridomas expressing antibodies having the desired sequence or functional characteristics. DNA encoding the monoclonal antibodies is readily isolated and sequenced using conventional procedures (e.g., by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as *E. coli* cells, simian COS cells, Chinese hamster ovary (CHO) cells, myeloma cells, or other suitable cells that do not otherwise produce immunoglobulin protein, to obtain the synthesis of monoclonal antibodies in the recombinant host cells. The details of recombinant production of specific antibodies may be found in the references cited in the foregoing. Monoclonal antibodies that inhibit PD-1 can be prepared by standard molecular biology methods using the sequences provided herein by reverse translation and insertion into appropriate DNA or RNA vectors.

The anti-PD-L1 antibody sequences referenced in some of the foregoing embodiments are summarized in Table 6.

**TABLE 6. Anti-PD-L1 antibody amino acid sequences.**

| **Identifier** | **Sequence (One-Letter Amino Acid Symbols)** | |
|---|---|---|
| SEQ ID NO:98 durvalumab (MEDI4736) heavy chain | EVQLVESGGG LVQPGGSLRL SCAASGFTFS RYWMSWVRQA PGKGLEWVAN IKQDGSEKYY 60 VDSVKGRFTI SRDNAKNSLY LQMNSLRAED TAVYYCAREG GWFGELAFDY WGQGTLVTVS 120 SASTKGPSVF PLAPSSKSTS GGTAALGCLV KDYFPEPVTV SWNSGALTSG VHTFPAVLQS 180 SGLYSLSSVV TVPSSSLGTQ TYICNVNHKP SNTKVDKRVE PKSCDKTHTC PPCPAPEFEG 240 GPSVFLFPPK PKDTLMISRT PEVTCVVVDV SHEDPEVKFN WYVDGVEVHN AKTKPREEQY 300 NSTYRVVSVL TVLHQDWLNG KEYKCKVSNK ALPASIEKTI SKAKGQPREP QVYTLPPSRE 360 EMTKNQVSLT CLVKGFYPSD IAVEWESNGQ PENNYKTTPP VLDSDGSFFL YSKLTVDKSR 420 WQQGNVFSCS VMHEALHNHY TQKSLSLSPG K 451 | |
| SEQ ID NO:99 durvalumab (MEDI4736) light chain | EVQLVESGGG LVQPGGSLRL SCAASGFTFS RYWMSWVRQA PGKGLEWVAN EIVLTQSPGT 60 LSLSPGERAT LSCRASQRVS SSYLAWYQQK PGQAPRLLIY DASSRATGIP DRFSGSGSGT 120 DFTLTISRLE PEDFAVYYCQ QYGSLPWTFG QGTKVEIKRT VAAPSVFIFP PSDEQLKSGT 180 ASVVCLLNNF YPREAKVQWK VDNALQSGNS QESVTEQDSK DSTYSLSSTL TLSKADYEKH 240 KVYACEVTHQ GLSSPVTKSF NRGEC 265 | |
| SEQ ID NO:100 durvalumab variable heavy chain | EVQLVESGGG LVQPGGSLRL SCAASGFTFS RYWMSWVRQA PGKGLEWVAN IKQDGSEKYY 60 VDSVKGRFTI SRDNAKNSLY LQMNSLRAED TAVYYCAREG GWFGELAFDY WGQGTLVTVS 120 S 121 | |
| SEQ ID NO:101 durvalumab variable light chain | EIVLTQSPGT LSLSPGERAT LSCRASQRVS SSYLAWYQQK PGQAPRLLIY DASSRATGIP 60 DRFSGSGSGT DFTLTISRLE PEDFAVYYCQ QYGSLPWTFG QGTKVEIK 108 | |
| SEQ ID NO:102 durvalumab heavy chain CDR1 | RYWMS | 5 |
| SEQ ID NO:103 durvalumab heavy chain CDR2 | NIKQDGSEKY YVDSVKG | 17 |
| SEQ ID NO:104 durvalumab heavy chain CDR3 | EGGWFGELAF DY | 12 |
| SEQ ID NO:105 durvalumab light chain CDR1 | RASQRVSSSY LA | 12 |
| SEQ ID NO:106 durvalumab light chain CDR2 | DASSRAT | 7 |
| SEQ ID NO:107 durvalumab light chain CDR3 | QQYGSLPWT | 9 |
| SEQ ID NO:108 atezolizumab (MPDL3280A) heavy chain | EVQLVESGGG LVQPGGSLRL SCAASGFTFS DSWIHWVRQA PGKGLEWVAW ISPYGGSTYY 60 ADSVKGRFTI SADTSKNTAY LQMNSLRAED TAVYYCARRH WPGGFDYWGQ GTLVTVSSAS 120 TKGPSVFPLA PSSKSTSGGT AALGCLVKDY FPEPVTVSWN SGALTSGVHT FPAVLQSSGL 180 YSLSSVVTVP SSSLGTQTYI CNVNHKPSNT KVDKKVEPKS CDKTHTCPPC PAPELLGGPS 240 VFLFPPKPKD TLMISRTPEV TCVVVDVSHE DPEVKFNWYV DGVEVHNAKT KPREEQYAST 300 YRVVSVLTVL HQDWLNGKEY KCKVSNKALP APIEKTISKA KGQPREPQVY TLPPSREEMT 360 KNQVSLTCLV KGFYPSDIAV EWESNGQPEN NYKTTPPVLD SDGSFFLYSK LTVDKSRWQQ 420 GNVFSCSVMH EALHNHYTQK SLSLSPGK 448 | |
| SEQ ID NO:109 atezolizumab (MPDL3280A) light chain | DIQMTQSPSS LSASVGDRVT ITCRASQDVS TAVAWYQQKP GKAPKLLIYS ASFLYSGVPS 60 RFSGSGSGTD FTLTISSLQP EDFATYYCQQ YLYHPATFGQ GTKVEIKRTV AAPSVFIFPP 120 SDEQLKSGTA SVVCLLNNFY PREAKVQWKV DNALQSGNSQ ESVTEQDSKD STYSLSSTLT 180 LSKADYEKHK VYACEVTHQG LSSPVTKSFN RGEC 214 | |
| SEQ ID NO:110 atezolizumab variable heavy chain | EVQLVESGGG LVQPGGSLRL SCAASGFTFS DSWIHWVRQA PGKGLEWVAW ISPYGGSTYY 60 ADSVKGRFTI SADTSKNTAY LQMNSLRAED TAVYYCARRH WPGGFDYWGQ GTLVTVSA 118 | |
| SEQ ID NO:111 atezolizumab variable light chain | DIQMTQSPSS LSASVGDRVT ITCRASQDVS TAVAWYQQKP GKAPKLLIYS ASFLYSGVPS 60 RFSGSGSGTD FTLTISSLQP EDFATYYCQQ YLYHPATFGQ GTKVEIKR 108 | |
| SEQ ID NO:112 atezolizumab heavy chain CDR1 | GFTFSXSWIH | 10 |
| SEQ ID NO:113 atezolizumab heavy chain CDR2 | AWIXPYGGSX YYADSVKG | 18 |
| SEQ ID NO:114 atezolizumab heavy chain CDR3 | RHWPGGFDY | 9 |
| SEQ ID NO:115 atezolizumab light chain CDR1 | RASQXXXTXX A | 11 |
| SEQ ID NO:116 atezolizumab light chain CDR2 | SASXLXS | 7 |
| SEQ ID NO:117 atezolizumab light chain CDR3 | QQXXXXPXT | 9 |
| SEQ ID NO:118 avelumab (MSB0010718C) heavy chain | EVQLLESGGG LVQPGGSLRL SCAASGFTFS SYIMMWVRQA PGKGLEWVSS IYPSGGITFY 60 ADTVKGRFTI SRDNSKNTLY LQMNSLRAED TAVYYCARIK LGTVTTVDYW GQGTLVTVSS 120 ASTKGPSVFP LAPSSKSTSG GTAALGCLVK DYFPEPVTVS WNSGALTSGV HTFPAVLQSS 180 GLYSLSSVVT VPSSSLGTQT YICNVNHKPS NTKVDKKVEP KSCDKTHTCP PCPAPELLGG 240 PSVFLFPPKP KDTLMISRTP EVTCVVVDVS HEDPEVKFNW YVDGVEVHNA KTKPREEQYN 300 | |
| | STYRVVSVLT VLHQDWLNGK EYKCKVSNKA LPAPIEKTIS KAKGQPREPQ VYTLPPSRDE 360 LTKNQVSLTC LVKGFYPSDI AVEWESNGQP ENNYKTTPPV LDSDGSFFLY SKLTVDKSRW 420 QQGNVFSCSV MHEALHNHYT QKSLSLSPGK 450 | |
| SEQ ID NO:119 avelumab (MSB0010718C) light chain | QSALTQPASV SGSPGQSITI SCTGTSSDVG GYNYVSWYQQ HPGKAPKLMI YDVSNRPSGV 60 SNRFSGSKSG NTASLTISGL QAEDEADYYC SSYTSSSTRV FGTGTKVTVL GQPKANPTVT 120 LFPPSSEELQ ANKATLVCLI SDFYPGAVTV AWKADGSPVK AGVETTKPSK QSNNKYAASS 180 YLSLTPEQWK SHRSYSCQVT HEGSTVEKTV APTECS 216 | |
| SEQ ID NO:120 avelumab variable heavy chain | EVQLLESGGG LVQPGGSLRL SCAASGFTFS SYIMMWVRQA PGKGLEWVSS IYPSGGITFY 60 ADTVKGRFTI SRDNSKNTLY LQMNSLRAED TAVYYCARIK LGTVTTVDYW GQGTLVTVSS 120 | |
| SEQ ID NO:121 avelumab variable light chain | QSALTQPASV SGSPGQSITI SCTGTSSDVG GYNYVSWYQQ HPGKAPKLMI YDVSNRPSGV 60 SNRFSGSKSG NTASLTISGL QAEDEADYYC SSYTSSSTRV FGTGTKVTVL 110 | |
| SEQ ID NO:122 avelumab heavy chain CDR1 | SYIMM | 5 |
| SEQ ID NO:123 avelumab heavy chain CDR2 | SIYPSGGITF YADTVKG | 17 |
| SEQ ID NO:124 avelumab heavy chain CDR3 | IKLGTVTTVD Y | 11 |
| SEQ ID NO:125 avelumab light chain CDR1 | TGTSSDVGGY NYVS | 14 |
| SEQ ID NO:126 avelumab light chain CDR2 | DVSNRPS | 7 |
| SEQ ID NO:127 avelumab light chain CDR3 | SSYTSSSTRV | 10 |

The preparation, properties, and uses of suitable PD-1 and PD-L1 inhibitors are described in, *e.g.,* U.S. Patent No. 8,008,449 or U.S. Patent Application Publication Nos. 2009/0217401 A1 or 2013/0133091 A1; U.S. Patent No. 8,354,509 and U.S. Patent Application Publication Nos. US 2010/0266617 A1, US 2013/0108651 A1, and US 2013/0109843 A2; U.S. Patent Nos. 8,287,856, 8,580,247, and 8,168,757 and U.S. Patent Application Publication Nos. US 2009/0028857 A1, US 2010/0285013 A1, US 2013/0022600 A1, and US 2011/0008369 A1; U.S. Patent No. 8,779,108 or U.S. Patent Application Publication No. US 2013/0034559 A1; U.S. Patent No. 8,217,149 and U.S. Patent Application Publication Nos. US 2010/0203056 A1, US 2013/0045200 A1, US 2013/0045201 A1, US 2013/0045202 A1, or US 2014/0065135 A1; and U.S. Patent Application Publication No. US 2014/0341917 A1.

While preferred embodiments of the invention are shown and described herein, such embodiments are provided by way of example only and are not intended to otherwise limit the scope of the invention as defined by the claims. Various alternatives to the described embodiments of the invention may be employed in practicing the invention.

### EXAMPLES

The embodiments encompassed herein are now described with reference to the following examples. These examples are provided for the purpose of illustration only and the disclosure encompassed herein should in no way be construed as being limited to these examples, but rather should be construed to encompass any and all variations which become evident as a result of the teachings provided herein.

### Example 1 - Preclinical Characteristics of BTK Inhibitors

The BTK inhibitor ibrutinib (Formula (10), (1-[(3*R*)-3-[4-amino-3-(4-phenoxyphenyl)-1*H*-pyrazolo[3,4-*d*]pyrimidin-1-yl]piperidin-1-yl]prop-2-en-1-one) is a first-generation BTK inhibitor. In clinical testing as a monotherapy in subjects with hematologic malignancies, ibrutinib was generally well tolerated at dose levels through 840 mg (the highest dose tested). Advani, et al., J. Clin. Oncol. 2013, 31, 88-94; Byrd, et al., N. Engl. J. Med. 2013, 369, 32-42; Wang, et al., N. Engl. J. Med. 2013, 369, 507-16. No maximum tolerated dose (MTD) was apparent within the tested dose range. Furthermore, subjects typically found the drug tolerable over periods extending to > 2 years. No subject had tumor lysis syndrome. No overt pattern of myelosuppression was associated with ibrutinib treatment. No drug-related reductions in circulating CD4⁺ T cells or serum immunoglobulins were noted. Adverse events with an apparent relationship to study drug included diarrhea and rash.

In subjects with heavily pretreated non-Hodgkin lymphoma (NHL), ibrutinib showed substantial antitumor activity, inducing durable regressions of lymphadenopathy and splenomegaly in most subjects. Improvements in disease-associated anemia and thrombocytopenia were observed. The pattern of changes in subjects with CLL was notable. Single-active pharmaceutical ingredient ibrutinib caused rapid and substantial reductions in lymph node size concomitant with a redistribution of malignant sites into the peripheral blood. An asymptomatic absolute lymphocyte count (ALC) increase was observed that was maximal during the first few months of treatment and generally decreased thereafter but could be persistent in some subjects or could be seen repeatedly in subjects who had interruption and resumption of drug therapy.

Collectively, these data with ibrutinib support the potential benefits of selective BTK inhibition in the treatment of subjects with relapsed lymphoid cancers. However, while highly potent in inhibiting BTK, ibrutinib has also shown *in vitro* activity against other kinases with a cysteine in the same position as Cys481 in BTK, to which the drug covalently binds. For example, ibrutinib inhibits epidermal growth factor receptor (EGFR), which may be the cause of ibrutinib-related diarrhea and rash. In addition, it is a substrate for both cytochrome P450 (CYP) enzymes 3A4/5 and 2D6, which increases the possibility of drug-drug interactions. These liabilities support the development of alternative BTK inhibitors for use in the therapy of lymphoid cancer.

The preclinical selectivity and potency characteristics of the second-generation BTK inhibitor of Formula (2) were compared to the first-generation BTK inhibitor of Formula (10) (ibrutinib). In Table 7, a kinome screen (performed by Life Technologies or based on literature data) is shown that compares these compounds.

**TABLE 7. Kinome Screen for BTK Inhibitors (IC₅₀, nM)**

| **3F-Cys Kinase** | **Formula (2)** | **Ibrutinib (Formula (10))** |
|---|---|---|
| Btk | 3.1 | 0.5 |
| Tec | 29 | 78 |
| Bmx | 39 | 0.80 |
| ITK | >1000 | 10.7 |
| Txk | 291 | 2.0 |
| EGFR | >1000 | 5.6 |
| ErbB2 | 912 | 9.4 |
| ErbB4 | 13.2 | 2.7 |
| Blk | >1000 | 0.5 |
| JAK-3 | >1000 | 16.1 |

The results shown in Table 7 are obtained from a 10 point biochemical assay generated from 10 point concentration curves. The BTK inhibitor of Formula (2) shows much greater selectivity for BTK compared to other kinases than ibrutinib.

A comparison of the *in vivo* potency results for the BTK inhibitors of Formula (2) and ibrutinib is shown in FIG. 1. CD86 and CD69 are cell surface proteins that are BCR activation markers. To obtain the *in vivo* potency results, mice were gavaged at increasing drug concentration and sacrificed at one time point (3 hours post-dose). BCR was stimulated with IgM and the expression of activation marker CD69 and CD86 are monitored by flow cytometry to determine EC₅₀ values.

*In vitro* and *in vivo* safety pharmacology studies with Formula (2) have demonstrated a favorable nonclinical safety profile. When screened at 10 µM in binding assays evaluating interactions with 80 known pharmacologic targets such as G-protein-coupled receptors, nuclear receptors, proteases, and ion channels, Formula (2) shows significant activity only against the A3 adenosine receptor; follow-up dose-response experiments indicated an IC₅₀ of 2.7 µM, suggesting a low clinical risk of off-target effects. Formula (2) at 10 µM showed no inhibition of *in vitro* EGFR phosphorylation in an A431 human epidermoid cancer cell line whereas ibrutinib had an IC₅₀ of 66 nM. The *in vitro* effect of Formula (2) on human ether-à-go-go-related gene (hERG) channel activity was investigated *in vitro* in human embryonic kidney cells stably transfected with hERG. Formula (2) inhibited hERG channel activity by 25% at 10 µM, suggesting a low clinical risk that Formula (2) would induce clinical QT prolongation as predicted by this assay. Formula (2) was well tolerated in standard *in vivo* Good Laboratory Practices (GLP) studies of pharmacologic safety. A functional observation battery in rats at doses through 300 mg/kg (the highest dose level) revealed no adverse effects on neurobehavioral effects or body temperature at any dose level. A study of respiratory function in rats also indicated no treatment-related adverse effects at doses through 300 mg/kg (the highest dose level). In a cardiovascular function study in awake telemeterized male beagle dogs, single doses of Formula (2) at dose levels through 30 mg/kg (the highest dose level) induced no meaningful changes in body temperature, cardiovascular, or electrocardiographic (ECG) (including QT interval) parameters. The results suggest that Formula (2) is unlikely to cause serious off-target effects or adverse effects on critical organ systems.

The drug-drug interaction potential of Formula (2) was also evaluated. *In vitro* experiments evaluating loss of parent drug as catalyzed by CYPs indicated that Formula (2) is metabolized by CYP3A4. *In vitro* metabolism studies using mouse, rat, dog, rabbit, monkey, and human hepatocytes incubated with ¹⁴C-labeled Formula (2) indicated two mono-oxidized metabolites and a glutathione conjugate. No unique human metabolite was identified. Preliminary evaluations of metabolism in the plasma, bile, and urine of rats, dogs, and monkeys indicated metabolic processes of oxidation, glutathione binding, and hydrolysis. It was shown that Formula (2) binds to glutathione but does not deplete glutathione *in vitro.* Nonclinical CYP interaction studies data indicate that Formula (2) is very unlikely to cause clinical drug-drug interactions through alteration of the metabolism of drugs that are substrates for CYP enzymes.

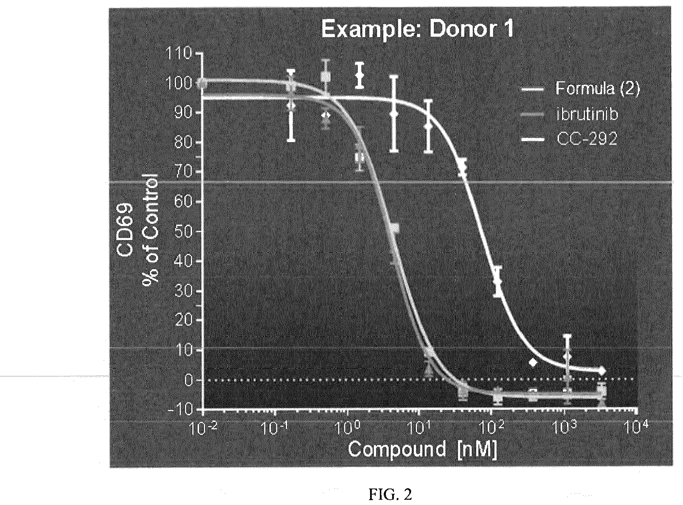

The *in vitro* potency in whole blood of Formula (2), Formula (10) (ibrutinib), and Formula (17) (CC-292) in inhibiting signals through the B cell receptor was also assessed. Blood from four healthy donors was incubated for 2 hours with the compounds shown over a concentration range, and then stimulated with anti-human IgD (10 µg/mL) for 18 hours. The mean fluorescent intensity (MFI) of CD69 (and CD86, data not shown) on gated CD19+ B cells was measured by flow cytometry. MFI values were normalized so that 100% represents CD69 level in stimulated cells without inhibitor, while 0% represents the unstimulated/no drug condition. The results are shown in FIG. 2. The EC₅₀ values obtained were 8.2 nM (95% confidence interval: 6.5 - 10.3), 6.1 nM (95% confidence interval: 5.2 - 7.2), and 121 nM (95% confidence interval: 94 - 155) for Formula (2), Formula (10) (ibrutinib), and Formula (17) (CC-292), respectively.

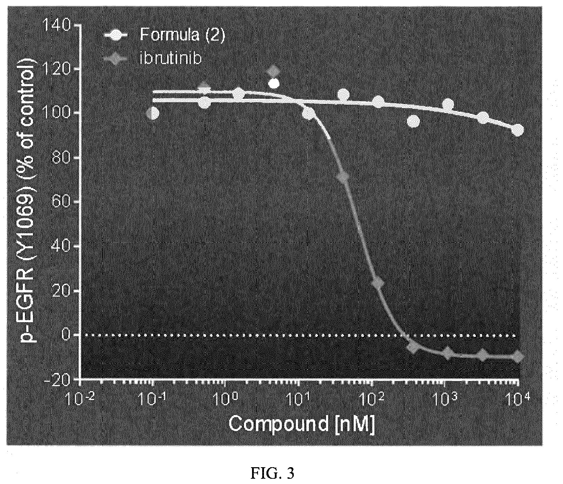

The EGF receptor phosphorylation *in vitro* was also determined for Formula (2) and Formula (10) (ibrutinib). Epidermoid carcinoma A431 cells were incubated for 2h with a dose titration of Formula (2) or Formula (10) (ibrutinib), before stimulation with EGF (100 ng/mL) for 5 min to induce EGFR phosphorylation (p-EGFR). Cells were fixed with 1.6% paraformaldehyde and permeabilized with 90% MeOH. Phosphoflow cytometry was performed with p-EGFR (Y1069). MFI values were normalized so that 100% represents the p-EGFR level in stimulated cells without inhibitor, while 0% represents the unstimulated/no drug condition. The results are shown in FIG. 3. EGF-induced p-EGFR inhibition was determined to be 7% at 10 µM for Formula (2), while ibrutinib has an EC₅₀ of 66 nM. The much more potent inhibition of EGF-induced p-EGFR by ibrutinib may be associated with increased side effects including diarrhea and rash.

### Example 2 - BTK Inhibitory Effects on Solid Tumor Microenvironment in an Orthotopic Pancreatic Cancer Model and Synergistic Combination of a BTK Inhibitor and Gemcitabine

A study was performed to observe potential reduction in tumor burden through modulation of tumor infiltrating MDSCs and TAMs using the BTK inhibitor of Formula (2) and/or gemcitabine ("Gem"). In this study, KPC derived mouse pancreatic cancer cells (KrasG12D;Trp53R172H;Pdx1-Cre) were injected into the pancreases. Animals were treated with (1) vehicle; (2) Formula (2), 15 mg/kg/BID given orally; (3) gemcitabine 15 mg/kg intravenous (IV) administered every 4 days for 3 injections; or (4) Formula (2), 15 mg/kg/BID given orally together with gemcitabine, 15 mg/kg IV administered every 4 days for 3 injections.

Single cell suspensions from tumor samples. Mouse tumor tissue was collected and stored in PBS/0.1% soybean trypsin inhibitor prior to enzymatic dissociation. Samples were finely minced with a scissors and mouse tissue was transferred into DMEM containing 1.0 mg/mL collagenase IV (Gibco), 0.1% soybean trypsin inhibitor, and 50 U/ml DNase (Roche) and incubated at 37 °C for 30 min. with constant stirring while human tissue was digested in 2.0 mg/ml collagenase IV, 1.0 mg/ml hyluronidase, 0.1% soybean trypsin inhibitor, and 50 U/ml DNase for 45 minutes. Suspensions were filtered through a 100 micron filter and washed with FACS buffer (PBS/0.5% BSA/2.0 mM EDTA) prior to staining. Two million total cells were stained with antibodies as indicated. Intracellular detection of FoxP3 was achieved following permeabilization with BD Perm Buffer III (BD Biosciences) and eBioscience Fix/Perm respectively. Following surface staining, samples were acquired on a BD Fortessa and analyzed using FlowJo (Treestar) software.

### Example 3 - BTK Inhibitory Effects on Solid Tumor Microenvironment in an Ovarian Cancer Model

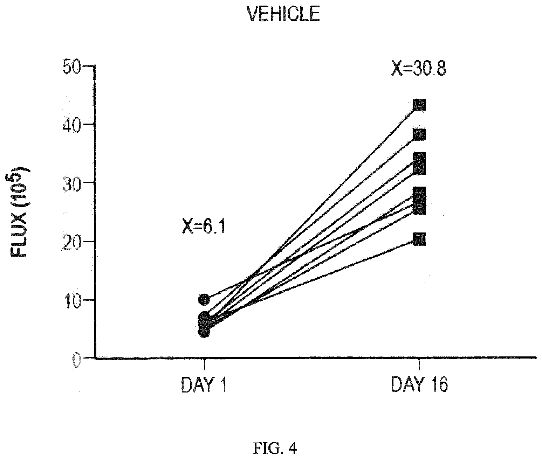

The ID8 syngeneic orthotropic ovarian cancer murine model was used to investigate the therapeutic efficacy of the BTK inhibitor of Formula (2) through treatment of the solid tumor microenvironment. Human ovarian cancer models, including the ID8 syngeneic orthotropic ovarian cancer model and other animal models, are described in Fong and Kakar, J. Ovarian Res. 2009, 2, 12; Greenaway, etal., Gynecol. Oncol. 2008, 108, 385-94; Urzua, et al., Tumour Biol. 2005, 26, 236-44; Janat-Amsbury, et al., Anticancer Res. 2006, 26, 3223-28; Janat-Amsbury, et al., Anticancer Res. 2006, 26, 2785-89. Animals were treated with vehicle or Formula (2), 15 mg/kg/BID given orally. The results of the study are shown in FIG. 4, FIG. 5, and FIG. 6.

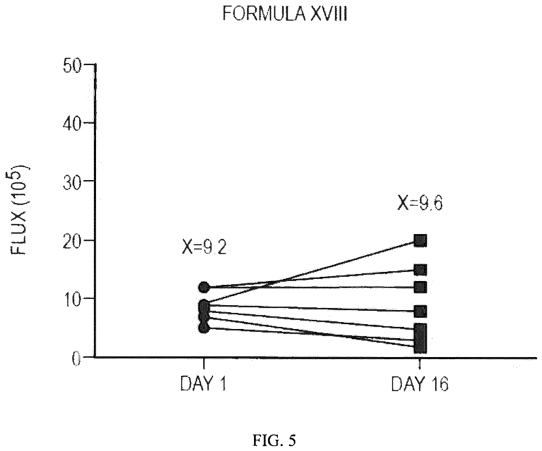

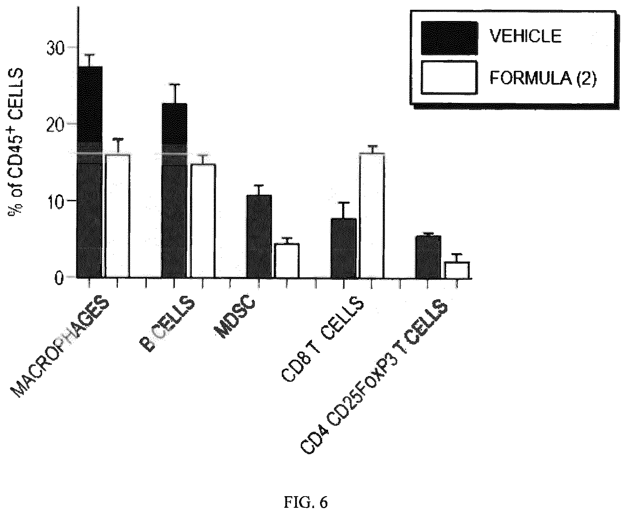

FIG. 4 and FIG. 5 demonstrate that the BTK inhibitor of Formula (2) impairs ID8 ovarian cancer growth in the ID8 syngeneic murine model. FIG. 6 shows that tumor response to treatment with the BTK inhibitor of Formula (2) correlates with a significant reduction in immunosuppressive tumor-associated lymphocytes in tumor-bearing mice.

### Example 4 - BTK Inhibitory Effects on Solid Tumor Microenvironment in a KPC Pancreatic Cancer Model

Given the potential for BTK inhibition to affect TAMs and MDSCs, single-active pharmaceutical ingredient Formula (2) was evaluated in mice with advanced pancreatic cancer arising as the result of genetic modifications of oncogenes KRAS and p53, and the pancreatic differentiation promoter PDX-1 (KPC mice). The KPC mouse model recapitulates many of the molecular, histopathologic, and clinical features of human disease (Westphalen and Olive, Cancer J. 2012, 18, 502-510). Combination therapy with gemcitabine was also evaluated in this model. Mice were enrolled after identification of spontaneously appearing tumors in the pancreas that were ≥ 100 mm³ (as assessed by high-resolution ultrasonography). Mice were treated with (1) vehicle (N=6); or (2) Formula (2), 15 mg/kg BID given orally (N=6).

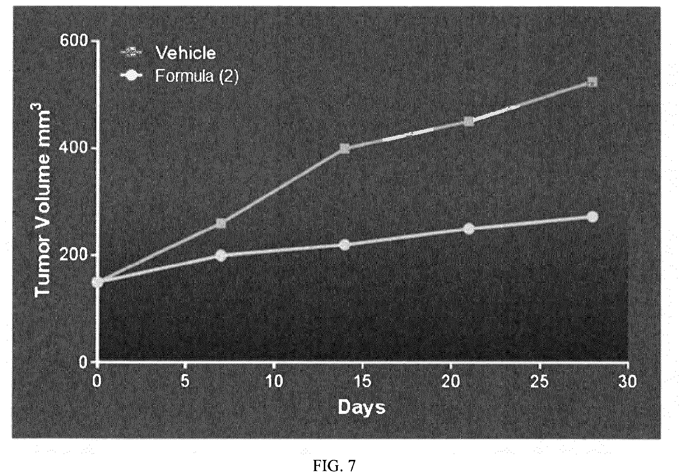

As shown in FIG. 7, treatment with single-active pharmaceutical ingredient Formula (2) substantially slowed pancreatic cancer growth and increased animal survival. With vehicle, tumor volumes predose averaged 152 mm³, and at day 28 averaged 525 mm³. In the cohort treated with Formula (2), tumor volumes predose averaged 165 mm³, and at day 28 averaged 272 mm³, indicating significant improvement. With vehicle, survival at day 14 was 5/6 animals, and at day 28 was 0/6 animals. With Formula (2), survival at day 14 was 6/6 animals, and at day 28 was 5/6 animals.

### Example 5 - BTK Inhibitory Effects on Solid Tumor Microenvironment in a Non-small Cell Lung Cancer (NSCLC) Model

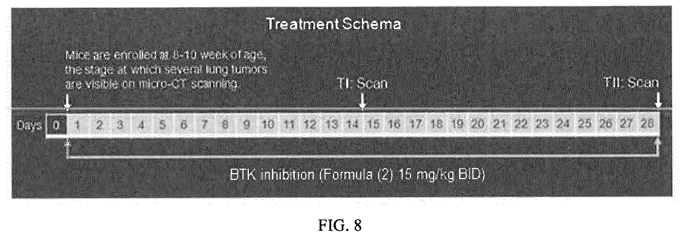

A genetic tumor model of NSCLC (KrasLA2) was studied as a model for lung cancer using the treatment schema shown in FIG. 8. The model is designed to have sporadic expression in single cells of G12D mutant Kras off its own promoter triggered by spontaneous intrachromosomal recombination. Johnson, et al. Nature 2001, 410, 1111-16. While the mutant Kras protein is expressed in a few cells in all tissues, tumor development is seen only in the lung at high penetrance.

### Example 6 - BTK Inhibitory Effects on MDSCs in the Solid Tumor Microenvironment

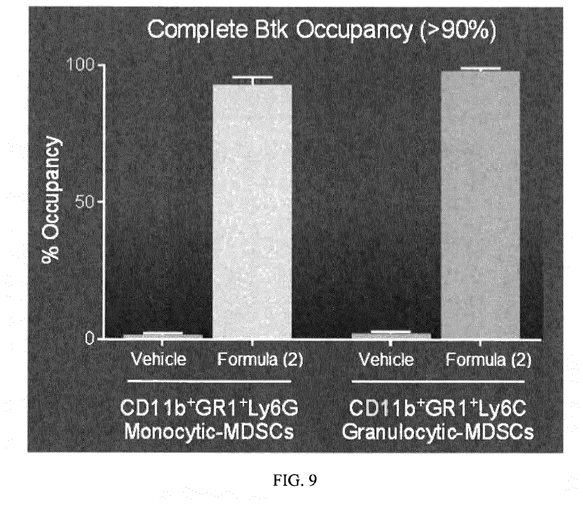

A molecular probe assay was used to calculate the percent irreversible occupancy of total BTK. MDSCs were purified from tumor bearing PDA mice (as described previously) dosed at 15 mg/kg BID of Formula (2). Complete BTK occupancy is observed for both the granulocytic and monocytic MDSC compartments on Day 8 at 4 hours post dose (N=5). The results are shown in FIG. 9.

### Example 7 - Effects of BTK Inhibitors on Antibody-Dependent NK Cell Mediated Cytotoxicity Using Rituximab

Rituximab-combination chemotherapy is today's standard of care in CD20⁺ B-cell malignancies. Previous studies investigated and determined that ibrutinib antagonizes rituximab antibody-dependent cell-mediated cytotoxicity (ADCC) mediated by NK cells. This may be due to ibrutinib's secondary irreversible binding to interleukin-2 inducible tyrosine kinase (ITK) which is required for FcR-stimulated NK cell function including calcium mobilization, granule release, and overall ADCC. Kohrt, et al., Blood 2014, 123, 1957-60.

In this example, the effects of Formula (2) and ibrutinib on NK cell function were evaluated in cell lines and primary NK cells from healthy volunteers and CLL patients. In summary, the results show that the activation of NK cells co-cultured with antibody-coated target cells was strongly inhibited by ibrutinib. The secretion of IFN-γ was reduced by 48% (p = 0.018) and 72% (p = 0.002) in cultures treated with ibrutinib at 0.1 and 1.0 µM respectively and NK cell degranulation was significantly (p = 0.002) reduced, compared with control cultures. Formula (2) treatment at 1 µM, a clinically relevant concentration, did not inhibit IFN-γ or NK cell degranulation. Rituximab-mediated ADCC was evaluated in NK cells from healthy volunteers as well as assays of NK cells from CLL patients targeting autologous CLL cells. In both cases, ADCC was not inhibited by Formula (2) treatment at 1 µM. In contrast, addition of ibrutinib to the ADCC assays strongly inhibited the rituximab-mediated cytotoxicity of target cells, and no increase over natural cytotoxicity was observed at any rituximab concentration. This result indicates that the combination of rituximab and Formula (2) provides an unexpected benefit in the treatment of B cell malignancies and other diseases where NK cell inhibition is undesirable.

BTK is a non-receptor enzyme in the Tec kinase family that is expressed among cells of hematopoietic origin, including B cells, myeloid cells, mast cells and platelets, where it regulates multiple cellular processes including proliferation, differentiation, apoptosis, and cell migration. Khan, Immunol Res. 2001, 23, 147-56; Mohamed, et al., Immunol Rev. 2009, 228, 58-73; Bradshaw, Cell Signal. 2010, 22, 1175-84. Functional null mutations of BTK in humans cause the inherited disease, X linked agammaglobulinemia, which is characterized by a lack of mature peripheral B cells. Vihinen, et al., Front Biosci. 2000, 5, D917-28. Conversely, BTK activation is implicated in the pathogenesis of several B-cell malignancies. Herman, et al., Blood 2011, 117, 6287-96; Kil, et al., Am. J. Blood Res. 2013, 3, 71-83; Tai, et al., Blood 2012, 120, 1877-87; Buggy, and Elias, Int. Rev. Immunol. 2012, 31, 119-32 (Erratum in: Int. Rev. Immunol. 2012, 31, 428). In addition, BTK-dependent activation of mast cells and other immunocytes in peritumoral inflammatory stroma has been shown to sustain the complex microenvironment needed for lymphoid and solid tumor maintenance. Soucek, et al., Neoplasia 2011, 13, 1093-100; Ponader, et al., Blood 2012, 119, 1182-89; de Rooij, et al., Blood 2012, 119, 2590-94. Taken together, these findings have suggested that inhibition of BTK may offer an attractive strategy for treating B cell neoplasms, other hematologic malignancies, and solid tumors.

Ibrutinib (PCI-32765, INMRUVICA), is a first-in-class therapeutic BTK inhibitor. This orally delivered, small-molecule drug is being developed by Pharmacyclics, Inc. for the therapy of B cell malignancies. As described above, in patients with heavily pretreated indolent non-Hodgkin lymphoma (iNHL), mantle cell lymphoma (MCL), and CLL, ibrutinib showed substantial antitumor activity, inducing durable regressions of lymphadenopathy and splenomegaly in the majority of patients. Advani, et al., J. Clin. Oncol. 2013, 31, 88-94; Byrd, etal., N. Engl. J. Med. 2013, 369, 32-42; Wang, et al., N. Engl. J. Med. 2013, 369, 507-16; O'Brien, et al., Blood 2012, 119, 1182-89. The pattern of changes in CLL was notable. Inhibition of BTK with ibrutinib caused rapid and substantial mobilization of malignant CLL cells from tissue sites into the peripheral blood, as described in Woyach, et al., Blood 2014, 123, 1810-17; this effect was consistent with decreased adherence of CLL to protective stromal cells. Ponader, et al., Blood 2012, 119, 1182-89; de Rooij, et al., Blood 2012, 119, 2590-94. Ibrutinib has been generally well tolerated. At dose levels associated with total BTK occupancy, no dose-limiting toxicities were identified and subjects found the drug tolerable over periods extending to >2.5 years.

Given the homology between BTK and ITK, it has been recently confirmed that ibrutinib irreversibly binds ITK. Dubovsky, et al., Blood 2013, 122, 2539-2549. ITK expression in Fc receptor (FcR)-stimulated NK cells leads to increased calcium mobilization, granule release, and cytotoxicity. Khurana, et al., J. Immunol. 2007, 178, 3575-3582. As rituximab is a backbone of lymphoma therapy, with mechanisms of action including ADCC, as well as direct induction of apoptosis and complement-dependent cytotoxicity, and FcR stimulation is requisite for ADCC, we investigated if ibrutinib or Formula (2) (the latter lacking ITK inhibition) influenced rituximab's anti-lymphoma activity *in vitro* by assessing NK cell IFN-γ secretion, degranulation by CD107a mobilization, and cytotoxicity by chromium release using CD20⁺ cell lines and autologous patient samples with chronic lymphocytic leukemia (CLL).

Formula (2) is a more selective inhibitor than ibrutinib, as shown previously. Formula (2) is not a potent inhibitor of ITK in contrast to ibrutinib, as shown herein (see, *e.g.,* Table 7). ITK is required for FcR-stimulated NK cell function including calcium mobilization, granule release, and overall ADCC. Anti-CD20 antibodies like rituximab are currently the standard of care for the treatment of many CD20⁺ B cell malignancies, often as part of combination regimens with older chemotherapeutic agents. The potential of ibrutinib or Formula (2) to antagonize ADCC was evaluated *in vitro.* Previous studies have determined that ibrutinib undesirably antagonizes rituximab ADCC effects mediated by NK cells. Kohrt, et al., Blood 2014, 123, 1957-60. We hypothesized that the BTK inhibitor of Formula (2), which does not have activity against ITK, may preserve NK cell function and therefore synergize rather than antagonize rituximab-mediated ADCC. Rituximab-dependent NK-cell mediated cytotoxicity was assessed using lymphoma cell lines as well as autologous CLL tumor cells.

Cell culture conditions were as follows. Cell lines Raji and DHL-4 were maintained in RPMI 1630 supplemented with fetal bovine serum, L-glutamine, 2-mercaptoethanol and penicillin-streptomycin at 37 °C in a humidified incubator. The HER18 cells were maintained in DEM supplemented with fetal bovine serum, penicillin-streptomycin and. Prior to assay, HER18 cells were harvested using trypsin-EDTA, washed with phosphate-buffered saline (PBS) containing 5% serum and viable cells were counted. For culture of primary target cells, peripheral blood from CLL patients was subject to density centrifugation to obtain peripheral blood mononuclear cells (PBMC). Cell preparations were washed and then subject to positive selection of CD5⁺CD19⁺ CLL cells using magnetic beads (MACS, Miltenyi Biotech). Cell preparations were used fresh after selection. NK cells from CLL patients and healthy volunteers were enriched from peripheral blood collected in sodium citrate anti-coagulant tubes and then subject to density centrifugation. Removal of non NK cells was performed using negative selection by MACS separation. Freshly isolated NK cells were washed three times, enumerated, and then used immediately for ADCC assays.

Cytokine secretion was determined as follows. Rituximab and trastuzumab-dependent NK-cell mediated degranulation and cytokine release were assessed using lymphoma and HER2+ breast cancer cell lines (DHL-4 and HER18, respectively). Target cells were cultured in flat-bottom plates containing 10 µg/mL of rituximab (DHL-4) or trastuzumab (HER18) and test articles (0.1 or 1 µM ibrutinib, 1 µM Formula (2), or DMSO vehicle control). NK cells from healthy donors were enriched as described above and then added to the target cells and incubated for 4 hours at 37 °C. Triplicate cultures were performed on NK cells from donors. After incubation, supernatants were harvested, centrifuged briefly, and then analyzed for interferon-γ using an enzyme-linked immunosorbent assay (ELISA) (R&D Systems, Minneapolis, MN, USA).

Lytic granule release was determined as follows. NK cells from healthy donors were enriched and cultured in the presence of target cells, monoclonal antibodies and test articles as described above. After 4 hours, the cultures were harvested and cells were pelleted, washed, and then stained for flow cytometry evaluation. Degranulation was evaluated via by flow cytometery by externalization of CD107a, a protein normally present on the inner leaflet of lytic granules, and gating on NK cells (CD3-CD16⁺ lymphocytes). The percentage of CD107a positive NK cells was quantified by comparison with a negative control (isotype control, unstained cells/FMO). Control cultures (NK cells cultured without target cells, or NK, target cell cocultures in the absence of appropriate monoclonal antibody) were also evaluated; all experiments were performed in triplicate.

ADCC assays were performed as follows. Briefly, target cells (Raji or primary CLL) were labeled by incubation at 37 °C with 100 pCi ⁵¹Cr for 4 hours prior to co-culture with NK cells. Cells were washed, enumerated, and then added in triplicate to prepared 96-well plates containing treated NK cells at an effector:target (E:T) ratio of 25:1. Rituximab (Genentech) was added to ADCC wells at concentrations of 0.1, 1.0 or 10 µg/mL and the assays were briefly mixed and then centrifuged to collect cells at the bottom of the wells. The effect of NK cell natural cytotoxicity was assessed in wells containing no rituximab. Cultures were incubated at 37 °C for 4 hours, and then centrifuged. Supernatants were harvested and ⁵¹Cr release was measured by liquid scintillation counting. All experiments were performed in triplicate.

Formula (2) had no inhibitory effect on rituximab-stimulated NK cell degranulation (< 2%) while ibrutinib reduced degranulation by ~50% (p = 0.24, FIG. 10). Formula (2) had no inhibitory effect while ibrutinib prevented trastuzumab-stimulated NK cell degranulation by ~84% at 1 µM (FIG. 10: **p = 0.002).

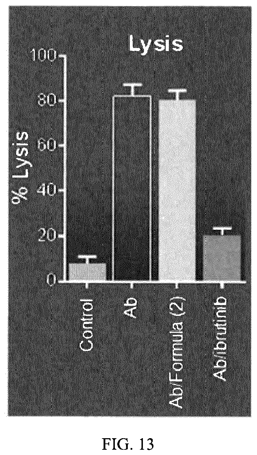

In Raji cell samples, *ex vivo* NK cell activity against autologous tumor cells was not inhibited by addition of Formula (2) at 1 µM, and increased cell lysis was observed with increasing concentrations of rituximab at a constant E:T ratio (FIG. 11). In primary CLL samples, *ex vivo* NK cell activity against autologous tumor cells was not inhibited by addition of Formula (2) at 1 µM, and increased cell lysis was observed with increasing concentrations of rituximab at a constant E:T ratio (FIG. 12). In contrast, addition of 1 µM ibrutinib completely inhibited ADCC, with less than 10% cell lysis at any rituximab concentration and no increase in cell lysis in the presence of rituximab, compared with cultures without rituximab. A graph highlighting the significant differences between Formula (2) and ibrutinib is shown in FIG. 13 (where "Ab" refers to rituximab). The difference between Formula (2) and ibrutinib was highly significant in this assay (p = 0.001).

In ADCC assays using healthy donor NK cells, antibody-dependent lysis of rituximab-coated Raji cells was not inhibited by addition of 1 µM of Formula (2) (FIG. 13). In these experiments, addition of rituximab stimulated a 5- to 8-fold increase in cell lysis at 0.1 and 1 µg/mL, compared with low (< 20%) natural cytotoxicity in the absence of rituximab. As previously reported, addition of 1 µM ibrutinib strongly inhibited the antibody-dependent lysis of target cells, with less than 20% cell lysis at all rituximab concentrations and no increase in ADCC with at higher rituximab concentrations.

Ibrutinib is clinically effective as monotherapy and in combination with rituximab, despite inhibition of ADCC *in vitro* and *in vivo* murine models due to ibrutinib's secondary irreversible binding to ITK. Preclinically, the efficacy of therapeutics which do not inhibit NK cell function, including Formula (2), is superior to ibrutinib. These results provide support for the unexpected property of Formula (2) as a synergistic and superior active pharmaceutical ingredient than ibrutinib to use in combinations with antibodies that have ADCC as a mechanism of action. The improved performance of Formula (2) in combination with anti-CD20 antibody therapies is expected to extend to its use in combination with CD19 and PD-1/PD-L1 inhibitors in both hematological malignancies and solid tumors, as these combinations would also benefit from reduced inhibition of NK cell function. The results also indicate that Formula (2) can be combined synergistically with anti-CD19 NK cell therapies, such as NK cells expressing CD 19-targeted chimeric antigen receptors, while Formula (10) cannot be similarly combined without loss of function for NK cell therapies.

### Example 8 - Effects of BTK Inhibition on Antibody-Dependent NK Cell Mediated Cytotoxicity Using Obinutuzumab

It has been shown above that ibrutinib undesirably antagonizes rituximab ADCC effects mediated by NK cells, and that Formula (2) does not antagonize rituximab ADCC effects and instead allows for a synergistic combination. As noted previously, this may be due to ibrutinib's secondary irreversible binding to ITK, which is required for FcR-stimulated NK cell function including calcium mobilization, granule release, and overall ADCC. H. E. Kohrt, et al., Blood 2014,123, 1957-60. The potential for ibrutinib antagonization of obinutuzumab (GA-101) ADCC as mediated by NK cells was also explored and compared to the effects of Formula (2).

The NK cell degranulation/ADCC assay was performed using a whole blood assay with CLL targets added to normal donor whole blood, in the presence or absence of different doses of Formula (2) and ibrutinib, followed by opsonization with the anti-CD20 antibody obinutuzumab. Ibrutinib was used as a control, and two blinded samples of BTK inhibitors, Formula (2) and a second sample of ibrutinib, were provided to the investigators. Degranulation in whole blood was performed as follows. CLL targets (MEC-1 cells) were expanded in RPMI 1640 medium (Life Technologies, Inc.) with 10% fetal bovine serum (FBS). Exponentially growing cells were used. On the day of the experiment, 8 mL of blood was drawn from a normal volunteer into a test tube containing desirudin to obtain a final concentration of 50 µg/mL. A white blood cell (WBC) count of whole blood was performed. MEC-1 cells were re-suspended at the concentration of WBC in whole blood (*e.g.,* if 6 × 10⁶ WBC/mL was measured, MEC-1 cells were re-suspended at 6 × 10⁶ cells/mL, to allow for a final WBC:MEC-1 cell ratio of 1:1). The ibrutinib control and two blinded BTK inhibitors were diluted in X-VIVO 15 serum-free hematopoietic cell medium (Lonza Group, Ltd.) to concentrations of 200 µM, 20 µM and 2 µM. 170 µL aliquots of unmanipulated whole blood were incubated with 10 µL BTK inhibitors or X-VIVO 15 medium for one hour into a plate. Cetuximab and obinutuzumab (GA-101) were diluted in X-VIVO 15 medium to a concentration of 20 µg/mL. Equal volumes of MEC-1 cells and antibodies were incubated for 5 minutes. After incubation, 20 µL of MEC-1 cells and antibodies was added to whole blood and the BTK inhibitors/X-VIVO 15 medium (for a final volume of 200 µL). The samples were placed in a 5% CO₂ incubator for 4 hours at 37 °C. The experimental conditions thus achieved a WBC:MEC-1 cell ratio of 1:1, with final concentrations of the BTK inhibitors in the assay of 10 µM, 1 µM and 0.1 µM and final concentrations of the antibodies of 1 µg/mL.

After 4 hours, the samples were mixed gently and 50 µL aliquots were removed from each well and placed in fluorescence-activated cell sorting (FACS) test tubes. A 20 µL aliquot of anti-CD56-APC antibody and anti-CD107a-PE antibody was added. The samples were incubated for 20 minutes at room temperature in the dark. An aliquot of 2 mL of FACS lysing solution (BD Biosceinces) was added. The samples were again incubated for 5 minutes, and then centrifuged at 2000 rpm for 5 minutes. Supernatant was discarded and the cell pellet was resuspended in 500 µL of PBS. The samples were analyzed on the flow cytometer for CD107a⁺ NK cells (CD56⁺).

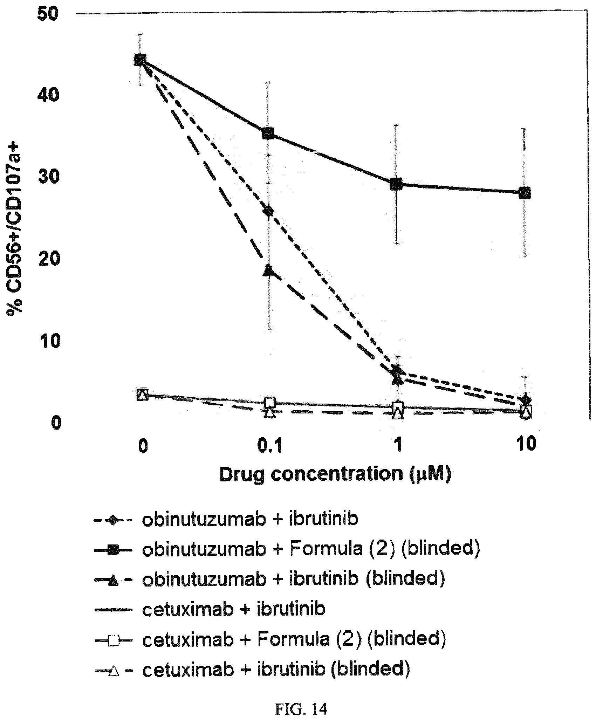

The NK cell degranulation results are summarized in FIG. 14 for n = 3 experiments, which shows the effects on whole blood after pretreatment for 1 hour with the BTK inhibitors at the concentrations shown and subsequent stimulation with MEC-1 opsonised with obinutuzumab or cetuximab at 1 µg/mL for 4 hours. A strong reduction in the percentage of CD56⁺/CD107a⁺ NK cells is observed using ibrutinib (both as a control and blinded BTK inhibitor), which indicates that ibrutinib undesirably antagonizes NK cells. In contrast, Formula (2) shows little antagonism towards NK cells, and had a minimal effect on obinutuzumab-stimulated NK cell degranulation while ibrutinib reduced obinutuzumab-stimulated NK degranulation by greater than 40%. These results support the synergistic combination of obinutuzumab and Formula (2) in treatment of human B cell malignancies.

### Example 9 - Effects of BTK Inhibition on Generalized NK Cell Mediated Cytotoxicity

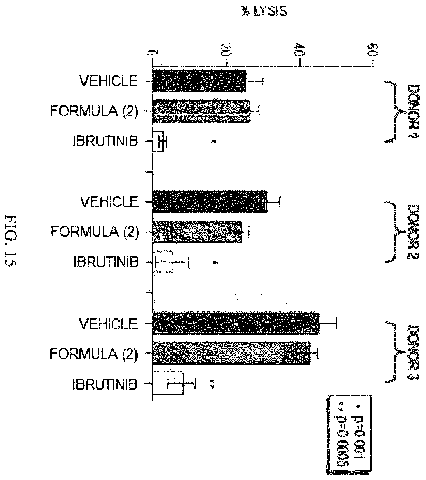

An assay was performed to assess the effects of BTK inhibition using Formula (2) on generalized NK killing (non-ADCC killing). The targets (K562 cells) do not express major histocompatibility complex (MHC) class I, so they do not inactivate NK cells. Target cells were grown to mid-log phase, and 5 × 10⁵ cells were labeled in 100 µL of assay medium (IMDM with 10% FCS and penicillin/streptomycin) with 100 µCi of ⁵¹Cr for 1 hour at 37 °C. Cells were washed twice and resuspended in assay medium. A total of 5000 target cells/well was used in the assay. Effector cells were resuspended in assay medium, distributed on a V-bottom 96-well plate, and mixed with labeled target cells at 40:1 E:T ratios. Maximum release was determined by incubating target cells in 1% Triton X-100. For spontaneous release, targets were incubated without effectors in assay medium alone. After a 1 minute centrifugation at 1000 rpm, plates were incubated for 4 and 16 hours at 37 °C. Supernatant was harvested and ⁵¹Cr release was measured in a gamma counter. Percentage of specific release was calculated as (experimental release-spontaneous release)/(maximum release-spontaneous release) × 100. The results are shown in FIG. 15.

### Example 10 - Effects of BTK Inhibition on T Cells

An assay was performed to assess the effects of BTK inhibition using Formula (2) on T cells. Enriched CD4⁺ T cells are plated on 24-well culture dishes that have been precoated 2 hr with 250 µL anti-TCRβ (0.5 µg/mL) plus anti-CD28 (5 µg/mL) at 37 °C in PBS. The cells are then supplemented with media containing BTK inhibitors along with the skewing cytokines as indicated in the following. The Th17 and Treg cultures are grown for 4 days before analysis. The cells are maintained for an additional 3 days with skewing cytokines (Th17; 20 ng/mL IL-6, 0.5 ng/mL TGF-β, 5 µg/mL IL-4, 5 µg/mL IFN-γ and Treg; 0.5 ng/mL TGF-β, 5 µg/mL IL-4, 5 µg/mL IFN-y) and are supplemented with IL2 as a growth factor.

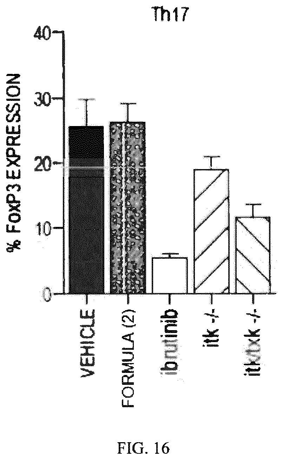

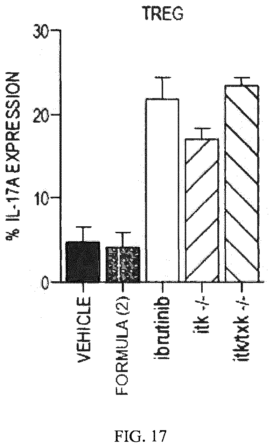

The results are shown in FIG. 16 and FIG. 17, and further illustrate the surprising properties of Formula (2) in comparison to Formula (10) (ibrutinib). Because of the lack of activity of Formula (2) on ITK and Txk, no adverse effect on Th17 and Treg development was observed. Since ibrutinib inhibits both ITK and Txk, a profound inhibition of Th17 cells and an increase in Treg development is observed, which is comparable to the murine ITK/Txk double knock-out cells which were used as a control.

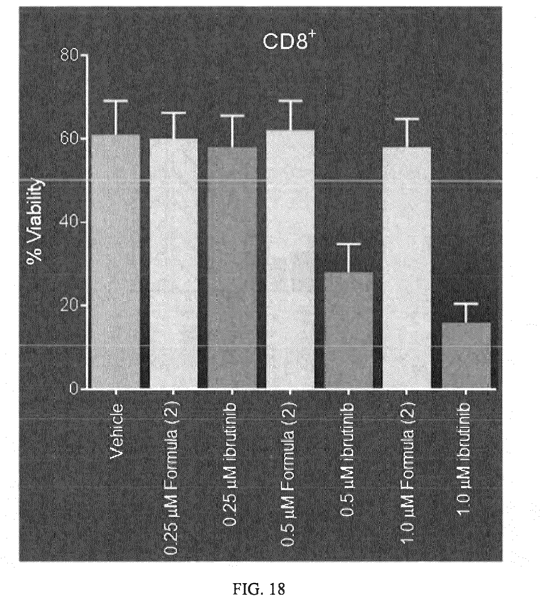

The effects of ibrutinib in comparison to Formula (2) on CD8⁺ T cell viability were also assessed. Total T cells were plated on anti-TCR and anti-CD28 coated wells in the presence of both BTK inhibitors. Neutral culture conditions were used that will not polarize T cells to a helper lineage. The cells are grown for 4 days and are then stained with anti-CD4, anti-CD8 and LIVE/DEAD reagent to determine if the drugs have selective effects on either the CD4⁺ or CD8⁺ cells. Statistical significance was calculated using the Mann Whitney T-test. The results, shown in FIG. 18, indicate that higher concentrations of Formula (10) (ibrutinib) have a strong, negative effect on CD8⁺ T cell viability that is not observed with Formula (2) at any concentration.

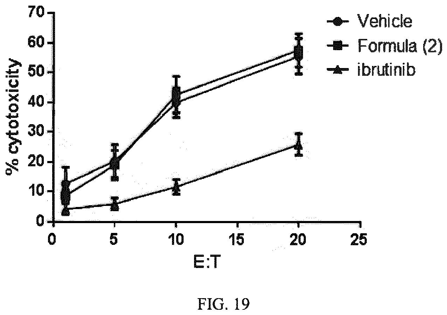

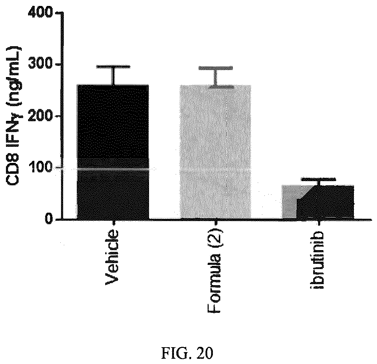

Without being bound by any theory, CD8⁺ T cells have two primary effector functions: (1) produce large amounts of IFN-γ (which activates macrophages), and (2) cytolytic activity. A cytotoxic T cell (CTL) assay was performed to compare the BTK inhibitors of Formula (2) and Formula (10) (ibrutinib). Effectors were prepared by generating CTL by culturing MHC mismatched splenocytes for 4 days with (500 nM) and without Formula (2) or Formula (10) (ibrutinib). The targets were B lymphoblasts from lipopolysaccharide (LPS) treated cultures. The assay was performed by incubating different ratios of effectors: targets for 4 hours. In FIG. 19, the results show that Formula (10) (ibrutinib) affects CD8⁺ T cell function as measured by % cytotoxicity. Formula (2), in contrast, has no effect on CD8⁺ T cell function as measured by % cytotoxicity relative to vehicle. The effect on CD8⁺ T cell function can also be observed by measurement of IFN-γ levels, as shown in FIG. 20, where Formula (10) (ibrutinib) again results in a significant loss of function relative to Formula (2) and vehicle.

As described herein, the tumor microenvironment provides stromal support for tumor growth and conceals the tumor from immune surveillance. Tumor-associated stroma comprises a mix of fibroblasts, Tregs, MDSCs, and TAMs that promote tumor growth and restrain immune-mediated tumor cell killing. The targeting of immune infiltrates may impair stromal support and enhance immune-mediated killing of cancer cells. Specifically, myeloid cells significantly infiltrate the tumor microenvironment and suppress anti-tumor immunity, promote tumor growth and tumor metastasis. Two subsets of myeloid cells with significant immunosuppressive activity are MDSCs and TAMs. During tumor progression, MDSCs and TAMs facilitate tumor growth by down regulating the host immune response through suppression of CD8⁺ T cell activation and function. BTK is expressed among cells of hematopoietic origin including B cells, myeloid cells, mast cells and platelets, but not T cells, where it regulates multiple cellular processes. BTK-dependent activation of myeloid cells and lymphocytes sustain the complex microenvironment needed for lymphoid and solid tumor maintenance. BTK inhibition shifts the microenvironment from suppressive to stimulatory by inhibiting the infiltration and function and of innate immune subsets (MDSCs and TAMs), thus allowing the activation of cytotoxic CD8⁺ T cells. The unique selectivity of the BTK inhibitor of Formula (2), as compared to, *e.g.,* the BTK inhibitor of Formula (10) (ibrutinib), prevents loss of function for CD8⁺ T cells as shown in this example. As a result, T cell therapies including CAR-T (CD19-targeted chimeric antigen receptor expressing CD8⁺ T cells) can be used in combination with Formula (2) and other selective BTK inhibitors described herein in a synergistic manner. Formula (10) cannot be coadministered without loss of function for CD8⁺ CD19-targeted chimeric antigen receptor expressing T cells.

Recent clinical trials have shown that CAR-T cells are a promising therapy for hematological and solid tumor malignancies. CARs include fusion proteins combining the antigen-recognition fragment of a monoclonal antibody with T cell activation domains from the T-cell receptor complex, such as the ζ chain, and costimulatory endodomains, from CD28, 4-1BB, or OX40. In clinical trials, up to 90% complete response rates have been seen after CD19-CAR-T administration, even in chemotherapy-refractory acute lymphocytic leukemia. Results in other B-cell cancers, such as chronic lymphocytic leukemia (CLL) and diffuse large B-cell lymphoma (DLBCL), however, have been less striking. One explanation for the different response rates among tumor types is that CAR-T functionality may be inhibited by an immunosuppressive tumor microenvironment. As mentioned above, TAMs, macrophages, and MDSCs inhibit T cell responses and promote recruitment of regulatory T cells. Thus, a BTK inhibitor that promotes T cell function (such as Formula (2)) may have diverse and synergistic benefits in combination with adoptive T cell therapy and CAR-T cells in clinical practice through reducing the inhibitory tumor microenvironment by inhibiting the infiltration and function and of innate immune subsets in the tumor microenvironment.

### Example 11 - Clinical Study of a BTK Inhibitor in Leukemia/Lymphoma and Effects on Bone Marrow and Lymphoid Microenvironments

Clinical studies have shown that targeting the BCR signaling pathway by inhibiting BTK produces significant clinical benefit in patients with non-Hodgkin's lymphoma (NHL). The second generation BTK inhibitor, Formula (2), achieves significant oral bioavailability and potency, and has favorable preclinical characteristics, as described above. The purpose of this study is to evaluate the safety and efficacy of the second generation BTK inhibitor of Formula (2) in treating subjects with chronic lymphocytic leukemia (CLL) and small lymphocytic lymphoma (SLL).

The design and conduct of this study is supported by an understanding of the history and current therapies for subjects with lymphoid cancers; knowledge of the activity and safety of a first-generation BTK inhibitor, ibrutinib, in subjects with hematologic cancers; and the available nonclinical information regarding Formula (2). The collective data support the following conclusions. BTK expression plays an important role in the biology of lymphoid neoplasms, which represent serious and life-threatening disorders with continuing unmet medical need. Clinical evaluation of Formula (2) as a potential treatment for these disorders has sound scientific rationale based on observations that the compound selectively abrogates BTK activity and shows activity in nonclinical models of lymphoid cancers. These data are supported by clinical documentation that ibrutinib, a first-generation BTK inhibitor, is clinically active in these diseases. Ibrutinib clinical data and Formula (2) nonclinical safety pharmacology and toxicology studies support the safety of testing Formula (2) in subjects with B cell malignancies.

The primary objectives of the clinical study are as follows: (1) establish the safety and the MTD of orally administered Formula (2) in subjects with CLL/SLL; (2) determine pharmacokinetics (PK) of orally administered Formula (2) and identification of its major metabolite(s); and (3) measure pharmacodynamic (PD) parameters including drug occupancy of BTK, the target enzyme, and effect on biologic markers of B cell function.

The secondary objective of the clinical study is to evaluate tumor responses in patients treated with Formula (2).

This study is a multicenter, open-label, nonrandomized, sequential group, dose escalation study. The following dose cohorts will be evaluated:
Cohort 1: 100 mg/day for 28 days (= 1 cycle)
Cohort 2: 175 mg/day for 28 days (= 1 cycle)
Cohort 3: 250 mg/day for 28 days (= 1 cycle)
Cohort 4: 350 mg/day for 28 days (= 1 cycle)
Cohort 5: 450 mg/day for 28 days (= 1 cycle)
Cohort 6: To be determined amount in mg/day for 28 days (= 1 cycle)

Each cohort will be enrolled sequentially with 6 subjects per cohort. If ≤ 1 dose-limiting toxicity (DLT) is observed in the cohort during Cycle 1, escalation to the next cohort will proceed. Subjects may be enrolled in the next cohort if 4 of the 6 subjects enrolled in the cohort completed Cycle 1 without experiencing a DLT, while the remaining 2 subjects are completing evaluation. If ≥ 2 DLTs are observed during Cycle 1, dosing at that dose and higher will be suspended and the MTD will be established as the previous cohort. The MTD is defined as the largest daily dose for which fewer than 33% of the subjects experience a DLT during Cycle 1. Dose escalation will end when either the MTD is achieved or at 3 dose levels above full BTK occupancy, whichever occurs first. Full BTK occupancy is defined as Formula (2) active-site occupancy of > 80% (average of all subjects in cohort) at 24 hours postdose. Should escalation to Cohort 6 be necessary, the dose will be determined based on the aggregate data from Cohorts 1 to 5, which includes safety, efficacy, and PK/PD results. The dose for Cohort 6 will not exceed 900 mg/day.

Treatment with Formula (2) may be continued for > 28 days until disease progression or an unacceptable drug-related toxicity occurs. Subjects with disease progression will be removed from the study. All subjects who discontinue study drug will have a safety follow-up visit 30 (±7) days after the last dose of study drug unless they have started another cancer therapy within that timeframe. Radiologic tumor assessment will be done at screening and at the end of Cycle 2, Cycle 4, and Cycle 12 and at investigator discretion. Confirmation of complete response (CR) will require bone marrow analysis and radiologic tumor assessment. For subjects who remain on study for > 11 months, a mandatory bone marrow aspirate and biopsy is required in Cycle 12 concurrent with the radiologic tumor assessment.

All subjects will have standard hematology, chemistry, and urinalysis safety panels done at screening. This study also includes pancreatic function assessment (serum amylase and serum lipase) due to the pancreatic findings in the 28-day GLP rat toxicity study. Once dosing commences, all subjects will be evaluated for safety once weekly for the first 4 weeks, every other week for Cycle 2, and monthly thereafter. Blood samples will be collected during the first week of treatment for PK/PD assessments. ECGs will be done at screening, and on Day 1-2, 8, 15, 22, 28 of Cycle 1, Day 15 and 28 of Cycle 2, and monthly thereafter through Cycle 6. ECGs are done in triplicate for screening only. Thereafter, single ECG tests are done unless a repeat ECG testing is required.

Dose-limiting toxicity is defined as any of the following events (if not related to disease progression): (1) any Grade ≥ 3 non-hematologic toxicity (except alopecia) persisting despite receipt of a single course of standard outpatient symptomatic therapy (e.g., Grade 3 diarrhea that responds to a single, therapeutic dose of Imodium^{®} would not be considered a DLT); (2) grade ≥ 3 prolongation of the corrected QT interval (QTc), as determined by a central ECG laboratory overread; (3) grade 4 neutropenia (absolute neutrophil count [ANC] < 500/µL) lasting > 7 days after discontinuation of therapy without growth factors or lasting > 5 days after discontinuation of therapy while on growth factors (i.e., Grade 4 neutropenia not lasting as long as specified will not be considered a DLT), (4) grade 4 thrombocytopenia (platelet count < 20,000/µL) lasting > 7 days after discontinuation of therapy or requiring transfusion (*i.e*., Grade 4 thrombocytopenia not lasting as long as specified will not be considered a DLT), and (5) dosing delay due to toxicity for > 7 consecutive days.

The efficacy parameters for the study include overall response rate, duration of response, and progression-free survival (PFS). The safety parameters for the study include DLTs and MTD, frequency, severity, and attribution of adverse events (AEs) based on the Common Terminology Criteria for Adverse Events (CTCAE v4.03) for non-hematologic AEs. Hallek, et al., Blood 2008, 111, 5446-5456.

The schedule of assessments is as follows, with all days stated in the following meaning the given day or +/- 2 days from the given day. A physical examination, including vital signs and weight, are performed at screening, during cycle 1 at 1, 8, 15, 22, and 28 days, during cycle 2 at 15 and 28 days, during cycles 3 to 24 at 28 days, and at follow up (after the last dose). The screening physical examination includes, at a minimum, the general appearance of the subject, height (screening only) and weight, and examination of the skin, eyes, ears, nose, throat, lungs, heart, abdomen, extremities, musculoskeletal system, lymphatic system, and nervous system. Symptom-directed physical exams are done thereafter. Vital signs (blood pressure, pulse, respiratory rate, and temperature) are assessed after the subject has rested in the sitting position. Eastern Cooperative Oncology Group (ECOG) status is assessed at screening, during cycle 1 at 1, 8, 15, 22, and 28 days, during cycle 2 at 15 and 28 days, during cycles 3 to 24 at 28 days, and at follow up, using the published ECOG performance status indications described in Oken, et al., Am. J. Clin. Oncol. 1982, 5, 649-655. ECG testing is performed at screening, during cycle 1 at 1, 2, 8, 15, 22, and 28 days, during cycle 2 at 15 and 28 days, during cycles 3 to 24 at 28 days, and at follow up. The 12-lead ECG test will be done in triplicate (≥ 1 minute apart) at screening. The calculated QTc average of the 3 ECGs must be <480 ms for eligibility. On cycle 1, day 1 and cycle 1, day 8, single ECGs are done predose and at 1, 2, 4, and 6 hours postdose. The single ECG on Cycle 1 Day 2 is done predose. On cycle 1, day 15, day 22, and day 28, a single ECG is done 2 hours post-dose. Starting with cycle 2, a single ECG is done per visit. Subjects should be in supine position and resting for at least 10 minutes before study-related ECGs. Two consecutive machine-read QTc > 500 ms or > 60 ms above baseline require central ECG review. Hematology, including complete blood count with differential and platelet and reticulocyte counts, is assesed at screening, during cycle 1 at 1, 8, 15, 22, and 28 days, during cycle 2 at 15 and 28 days, during cycles 3 to 24 at 28 days, and at follow up. Serum chemistry is assesed at screening, during cycle 1 at 1, 8, 15, 22, and 28 days, during cycle 2 at 15 and 28 days, during cycles 3 to 24 at 28 days, and at follow up. Serum chemistry includes albumin, alkaline phosphatase, ALT, AST, bicarbonate, blood urea nitrogen (BUN), calcium, chloride, creatinine, glucose, lactate dehydrogenase (LDH), magnesium, phosphate, potassium, sodium, total bilirubin, total protein, and uric acid. Cell counts and serum immunoglobulin are performed at screening, at cycle 2, day 28, and at every 6 months thereafter until last dose and include T/B/NK/monocyte cell counts (CD3, CD4, CD8, CD14, CD19, CD19, CD16/56, and others as needed) and serum immunoglobulin (IgG, IgM, IgA, and total immunoglobulin). Bone marrow aspirates are performed at cycle 12. Pharmacodynamics samples are drawn during cycle 1 at 1, 2, and 8 days, and at follow up. On days 1 and 8, pharmacodynamic samples are drawn pre-dose and 4 hours (±10 minutes) post-dose, and on day 2, pharmacodynamic samples are drawn predose. Pharmacokinetics samples are drawn during cycle 1 at 1, 2, 8, 15, 22, and 28 days. Pharmacokinetic samples for Cycle 1 Day 1 are drawn pre-dose and at 0.5, 1, 2, 4, 6 and 24 hours (before dose on Day 2) post-dose. Samples for Cycle 1 Day 8 are drawn pre-dose and at 0.5, 1, 2, 4, and 6 hours post-dose. On Cycle 1 Day 15, 22, and 28, a PK sample is drawn predose and the second PK sample must be drawn before (up to 10 minutes before) the ECG acquisition, which is 2 hours postdose. Pretreatment radiologic tumor assessments are performed within 30 days before the first dose. A computed tomography (CT) scan (with contrast unless contraindicated) is required of the chest, abdomen, and pelvis. In addition, a positron emission tomography (PET) or PET/CT must done for subjects with SLL. Radiologic tumor assessments are mandatory at the end of Cycle 2 (-7 days), Cycle 4 (- 7days), and Cycle 12 (-7 days). Otherwise, radiologic tumor assessments are done at investigator discretion. A CT (with contrast unless contraindicated) scan of the chest, abdomen, and pelvis is required for subjects with CLL. In addition, a PET/CT is required in subjects with SLL. Bone marrow and radiologic assessments are both required for confirmation of a complete response (CR). Clinical assessments of tumor response should be done at the end of Cycle 6 and every 3 months thereafter. Molecular markers are measured at screening, and include interphase cytogenetics, stimulated karyotype, IgHV mutational status, Zap-70 methylation, and beta-2 microglobulin levels. Urinalysis is performed at screening, and includes pH, ketones, specific gravity, bilirubin, protein, blood, and glucose. Other assessments, including informed consent, eligibility, medical history, and pregnancy test are done at the time of screening.

The investigator rates the subject's response to treatment based on recent guidelines for CLL, as given in Hallek, et al., Blood 2008, 111, 5446-56, and for SLL, as given in Cheson, et al., J. Clin. Oncol. 2007, 25, 579-586. The response assessment criteria for CLL are summarized in Table 12.

**TABLE 12. Response Assessment Criteria for CLL. Abbreviations: ANC = absolute neutrophil count; CR = complete remission; CRi = CR with incomplete blood count recovery; PR = partial remission.**

| **Response** | **Peripheral Blood** | **Bone Marrow (if performed)** | **Nodes, Liver, and Spleen^{a}** |
|---|---|---|---|
| CR | Lymphocytes < 4 × 10⁹/L ANC >1.5 × 10⁹/L^{b} Platelets > 100 × 10⁹/L^{b} Hemoglobin > 11.0 g/dL (untransfused)^{b} | Normocellular <30% lymphocytes No B-lymphoid nodules | Normal (e.g., no lymph nodes >1.5 cm) |
| CRi | Lymphocytes < 4 × 10⁹/L Persistent anemia, thrombocytopenia, or neutropenia related to drug toxicity | Hypocellular <30% lymphocytes | Normal (*e.g*., no lymph nodes >1.5 cm) |
| PR | Lymphocytes ≥ 50% decrease from baseline ANC > 1.5 × 10⁹/L | Not assessed | ≥50% reduction in lymphadenopathy^{c} and/or in spleen or liver enlargement |
| | or | | |
| | Platelets > 100 × 10⁹/L or 50% improvement over baseline^{b} | | |
| | or | | |
| | Hemoglobin > 11.0 g/dL | | |
| | or | | |
| | 50% improvement over baseline (untransfused)^{b} | | |

| | | | |
|---|---|---|---|
| a. Computed tomography (CT) scan of abdomen, pelvis, and chest is required for this evaluation b. Without need for exogenous growth factors c. In the sum products of ≤ 6 lymph nodes or in the largest diameter of the enlarged lymph node(s) detected before therapy and no increase in any lymph node or new enlarged lymph nodes | | | |

The response assessment criteria for SLL are summarized in Table 13.

**TABLE 13. Response Assessment Criteria for SLL. Abbreviations: CR = complete remission, CT = computed tomography, FDG = [¹⁸F]fluorodeoxyglucose, PET = positron-emission tomography, PR = partial remission, SD = stable disease, SPD = sum of the product of the diameters.**

| **Response** | **Definition** | **Nodal Masses** | **Spleen, Liver** | **Bone Marrow** |
|---|---|---|---|---|
| CR | Disappearance of all evidence of disease | (a) FDG-avid or PET positive prior to therapy; mass of any size permitted if PET negative | Not palpable, nodules disappeared | If infiltrate present at screening, infiltrate cleared on repeat biopsy; if indeterminate by morphology, immunohistochemistry should be negative |
| | | (b) Variably FDG-avid or PET negative; regression to normal size on CT | | |
| PR | Regression of measurable disease and no new sites | ≥ 50% decrease in SPD of up to 6 largest dominant masses; no increase in size of other nodes | ≥ 50% decrease in SPD of nodules (for single nodule in greatest transverse diameter); no increase in size of liver or spleen | Irrelevant if positive prior to therapy; cell type should be specified |
| | | (a) FDG-avid or PET positive prior to therapy; ≥ 1 PET positive at previously involved site | | |
| | | (b) Variably FDG-avid or PET negative; regression on CT | | |
| SD | Failure to attain CR/PR or progressive disease | (a) FDG-avid or PET positive prior to therapy; PET positive at prior sites of disease, and no new sites on CT or PET | | |
| | | (b) Variably FDG avid or PET negative; no change in size of previous lesions on CT | | |

The PK parameters of the study are as follows. The plasma PK of Formula (2) and a metabolite is characterized using noncompartmental analysis. The following PK parameters are calculated, whenever possible, from plasma concentrations of Formula (2):
AUC₍₀₋ₜ₎: Area under the plasma concentration-time curve calculated using linear trapezoidal summation from time 0 to time t, where t is the time of the last measurable concentration (Ct),
AUC₍₀₋₂₄₎: Area under the plasma concentration-time curve from 0 to 24 hours, calculated using linear trapezoidal summation,
AUC_{(0-∞)}: Area under the plasma concentration-time curve from 0 to infinity, calculated using the formula: AUC_{(0-∞)} = AUC₍₀₋ₜ₎ + Ct / λz, where λz is the apparent terminal elimination rate constant,
Cₘₐₓ: Maximum observed plasma concentration,
Tₘₐₓ: Time of the maximum plasma concentration (obtained without interpolation),
t_{½}: Terminal elimination half-life (whenever possible),
λ_{z}: Terminal elimination rate constant (whenever possible),
Cl/F: Oral clearance.

The PD parameters of the study are as follows. The occupancy of BTK by Formula (2) are measured in peripheral blood mononuclear cells (PBMCs) with the aid of a biotin-tagged Formula (2) analogue probe. The effect of Formula (2) on biologic markers of B cell function will also be evaluated.

The statistical analysis used in the study is as follows. No formal statistical tests of hypotheses are performed. Descriptive statistics (including means, standard deviations, and medians for continuous variables and proportions for discrete variables) are used to summarize data as appropriate.

The following definitions are used for the safety and efficacy analysis sets: Safety analysis set: All enrolled subjects who receive ≥ 1 dose of study drug; Per-protocol (PP) analysis set: All enrolled subjects who receive ≥ 1 dose of study drug and with ≥ 1 tumor response assessment after treatment. The safety analysis set will be used for evaluating the safety parameters in this study. The PP analysis sets will be analyzed for efficacy parameters in this study.

No imputation of values for missing data is performed except for missing or partial start and end dates for adverse events and concomitant medication will be imputed according to prespecified, conservative imputation rules. Subjects lost to follow-up (or drop out) will be included in statistical analyses to the point of their last evaluation.

The safety endpoint analysis was performed as follows. Safety summaries will include summaries in the form of tables and listings. The frequency (number and percentage) of treatment emergent adverse events will be reported in each treatment group by Medical Dictionary for Regulatory Activities (MedDRA) System Organ Class and Preferred Term. Summaries will also be presented by the severity of the adverse event and by relationship to study drug. Laboratory shift tables containing counts and percentages will be prepared by treatment assignment, laboratory parameter, and time. Summary tables will be prepared for each laboratory parameter. Figures of changes in laboratory parameters over time will be generated. Vital signs, ECGs, and physical exams will be tabulated and summarized.

Additional analyses include summaries of subject demographics, baseline characteristics, compliance, and concurrent treatments. Concomitant medications will be coded according to the World Health Organization (WHO) Drug Dictionary and tabulated.

The analysis of efficacy parameters was performed as follows. The point estimate of the overall response rate will be calculated for the PP analysis set. The corresponding 95% confidence interval also will be derived. The duration of overall response is measured from the time measurement criteria are met for CR or PR (whichever is first recorded) until the first date that recurrent or progressive disease is objectively documented (taking as reference for progressive disease the smallest measurements recorded since the treatment started). Kaplan-Meier methodology will be used to estimate event-free curves and corresponding quantiles (including the median). Progression-free survival is measured from the time of first study drug administration until the first date that recurrent or progressive disease is objectively documented (taking as reference for progressive disease the smallest measurements recorded since the treatment started). Kaplan-Meier methodology will be used to estimate the event-free curves and corresponding quantiles (including the median).

The study scheme is a sequential cohort escalation. Each cohort consists of six subjects. The sample size of the study is 24 to 36 subjects, depending on dose escalation into subsequent cohorts. Cohort 1 (N = 6) consists of Formula (2), 100 mg QD for 28 days. Cohort 2 (N = 6) consists of Formula (2), 175 mg QD for 28 days. Cohort 3 (N = 6) consists of Formula (2), 250 mg QD for 28 days. Cohort 4 (N = 6) consists of Formula (2), 350 mg QD for 28 days. Cohort 5 (N = 6) consists of Formula (2), 450 mg QD for 28 days. Cohort 6 (N = 6) consists of Formula (2), at a dose to be determined QD for 28 days. The dose level for Cohort 6 will be determined based on the safety and efficacy of Cohorts 1 to 5, and will not exceed 900 mg/day. Escalation will end with either the MTD cohort or three levels above full BTK occupancy, whichever is observed first. An additional arm of the study will explore 100 mg BID dosing. Treatment with oral Formula (2) may be continued for greater than 28 days until disease progression or an unacceptable drug-related toxicity occurs.

The inclusion criteria for the study are as follows: (1) men and women ≥ 18 years of age with a confirmed diagnosis of CLL/SLL, which has relapsed after, or been refractory to, ≥ 2 previous treatments for CLL/SLL; however, subjects with 17p deletion are eligible if they have relapsed after, or been refractory to, 1 prior treatment for CLL/SLL; (2) body weight ≥ 60 kg, (3) ECOG performance status of ≤ 2; (4) agreement to use contraception during the study and for 30 days after the last dose of study drug if sexually active and able to bear children; (5) willing and able to participate in all required evaluations and procedures in this study protocol including swallowing capsules without difficulty; or (6) ability to understand the purpose and risks of the study and provide signed and dated informed consent and authorization to use protected health information (in accordance with national and local subject privacy regulations).

The dosage form and strength of Formula (2) used in the clinical study is a hard gelatin capsules prepared using standard pharmaceutical grade excipients (microcrystalline cellulose) and containing 25 mg of Formula (2) each. The color of the capsules is Swedish orange. The route of administration is oral (*per os*, or PO). The dose regimen is once daily or twice daily, as defined by the cohort, on an empty stomach (defined as no food 2 hours before and 30 minutes after dosing).

The baseline characteristics for the patients enrolled in the clinical study are given in Table 14.

**TABLE 14. Relapsed/refractory CLL baseline characteristics.**

| **Characteristic** | | **CLL (N=44)** |
|---|---|---|
| Patient Demographics | | |
| | Age (years), median (range) | 62 (45-84) |
| | Sex, men (%) | 33 (75) |
| | Prior therapies, median (range), n | 3 (1-10) |
| | ≥3 prior therapies, n (%) | 26 (59) |
| Clinical Details | | |
| | ECOG performance status ≥ 1 (%) | 28 (63) |
| | Rai stage III/IV | 16 (36) |
| | Bulky disease ≥ 5 cm, n (%) | 15 (34) |
| | Cytopenia at baseline | 33 (75) |
| Cytogenic Status | | |
| | Chromosome 11q22.3 deletion (Del 11q), n (%) | 18 (41) |
| | Chromosome 17p13.1 (Del 17p), n (%) | 19 (34) |
| | IgV_{H} status (unmutated), n (%) | 28 (64) |

The results of the clinical study in relapsed/refractory CLL patients are summarized in Table 15.

**TABLE 15. Activity of Formula (2) in relapsed/refractory CLL. (PR = partial response; PR+L = partial response with lymphocytosis; SD = stable disease; PD = progressive disease.)**

| **n (%)** | **All Cohorts (N=31)** | **100 mg QD (N=8)** | **175 mg QD (N=8)** | **250 mg QD (N=7)** | **100 mg BID (N=3)** | **400 mg QD (N=5)** |
|---|---|---|---|---|---|---|
| **PR** | 22 (71) | 7 (88) | 5 (63) | 5 (71) | 3 (100) | 2 (40) |
| **PR+L** | 7 (23) | 0 (0) | 3 (37) | 2 (29) | 0 (0) | 2 (40) |
| **SD** | 2 (6) | 1 (12) | 0 (0) | 0 (0) | 0 (0) | 1 (20) |
| **PD** | 0 (0) | 0 (0) | 0 (0) | 0 (0) | 0 (0) | 0 (0) |

| Median (range) Cycles | | | | | | |
|---|---|---|---|---|---|---|
| | **7.3 (3.0-10.8)** | **10.0 (9.0-10.8)** | **8.6 (3.0-8.8)** | **7.0 (7.0-7.3)** | **5.2 (4.7-5.5)** | **5.0 (4.8-5.5)** |

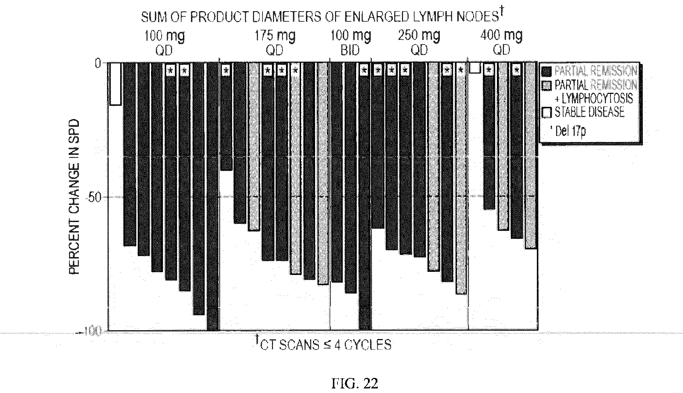

FIG. 21 shows the median % change in ALC and SPD from baseline in the clinical study of Formula (2), plotted in comparison to the results reported for ibrutinib in Figure 1A of Byrd, et al., N. Engl. J. Med. 2013, 369, 32-42. The results show that Formula (2) leads to a more rapid patient response in CLL than corresponding treatment with ibrutinib. This effect is illustrated, for example, by the median % change in SPD, which achieved the same status in the present study at 7 months of treatment with Formula (2) as compared to 18 months for ibrutinib. The % change in SPD observed in the different cohorts (*i.e.* by dose and dosing regimen) is shown in FIG. 22, and in all cases shows significant responses.

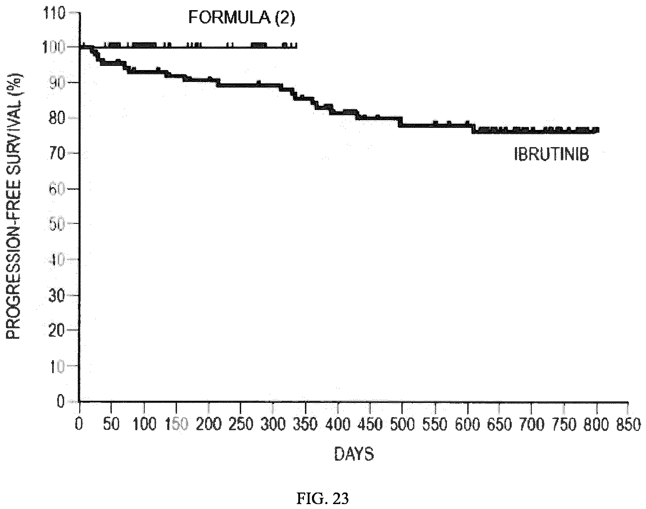

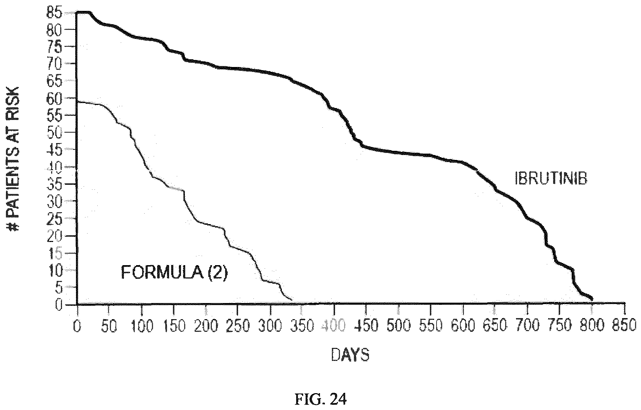

A Kaplan-Meier curve showing PFS from the clinical CLL study of Formula (2) is shown in FIG. 23. A comparison of survival curves was performed using the Log-Rank (Mantle-Cox) test, with a p-value of 0.0206 indicating that the survival curves are different. The number of patients at risk is shown in FIG. 24. Both FIG. 23 and FIG. 24 show the results for Formula (2) in comparison to the results reported for Formula (10) (ibrutinib) in Byrd, et al., N. Engl. J. Med. 2013, 369, 32-42. An improvement in survival and a reduction in risk are observed in CLL patients treated with Formula (2) in comparison to patients treated with ibrutinib.

Based on the data and comparisons shown above, the CLL study showed that the efficacy of Formula (2) was surprisingly superior to that of Formula (10) (ibrutinib).

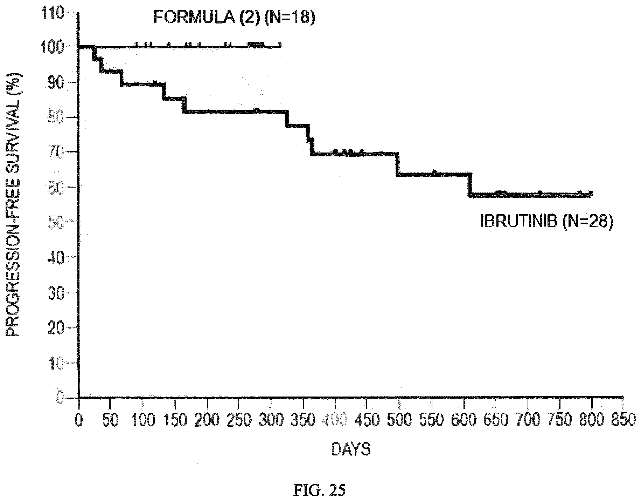

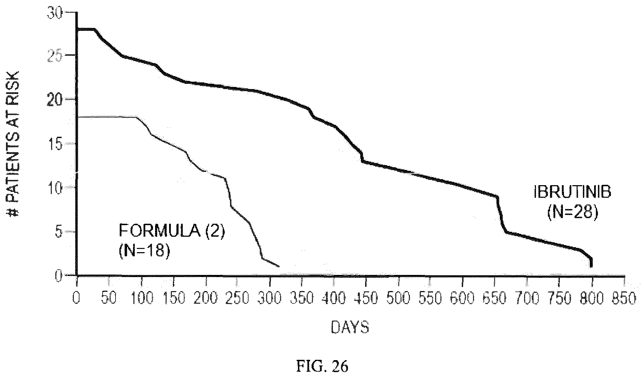

In the literature study of ibrutinib, increased disease progression was associated with patients with high-risk cytogenetic lesions (17p13.1 deletion or 11q22.3 deletion), as shown in Figure 3A in Byrd, et al., N. Engl. J. Med. 2013, 369, 32-42, which shows ibrutinib PFS including PFS broken down by genetic abnormality. The 17p and 11q deletions are validated high-risk characteristics of CLL, and the 17p deletion is the highest risk. In FIG. 25, the PFS is shown for Formula (2) in patients with the 17p deletion in comparison to the results obtained for ibrutinib in Byrd, et al., N. Engl. J. Med. 2013, 369, 32-42. A p-value of 0.0696 was obtained. In FIG. 26, the number of patients at risk with the 17p deletion is compared. To date, no 17p patients have progressed on Formula (2).

The adverse events observed in the clinical study in relapsed/refractory CLL are given in Table 16. No DLTs were observed. The MTD was not reached. No treatment-related serious adverse events (SAEs) were observed. No prophylactic antivirals or antibiotics were needed.

**TABLE 16. Treatment-related adverse events reported in the clinical study of Formula (2) in relapsed/refractory CLL. (Reported in ≥ 5% of patients.)**

| **Adverse Events (Treatment-Related), n (%)** | **Grade** | **All (N=44)** |
|---|---|---|
| Headache | 1/2 | 7 (16) |
| Increased tendency to bruise | 1 | 6 (14) |
| Diarrhea | 1 | 4 (9) |
| Petechiae | 1 | 3 (7) |

The clinical study of Formula (2) thus showed other unexpectedly superior results compared to ibrutinib therapy. A lack of lymphocytosis was observed in the study. Furthermore, only grade 1 AEs were observed, and these AEs were attributable to the high BTK selectivity of Formula (2).

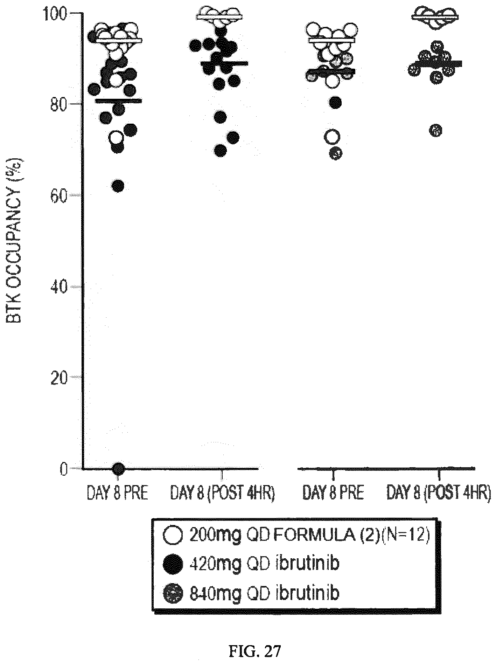

BTK target occupancy was measured for relapsed/refractory CLL patients with the results shown in FIG. 27. For 200 mg QD dosing of the BTK inhibitor of Formula (2), about 94% - 99% BTK occupancy was observed, with superior 24 hour coverage and less inter-patient variability also observed. For 420 mg and 840 mg QD of the BTK inhibitor ibrutinib, 80% - 90% BTK occupancy was observed, with more inter-patient variability and capped occupancy. These results indicate that the BTK inhibitor of Formula (2) achieves superior BTK occupancy in CLL patients than ibrutinib.

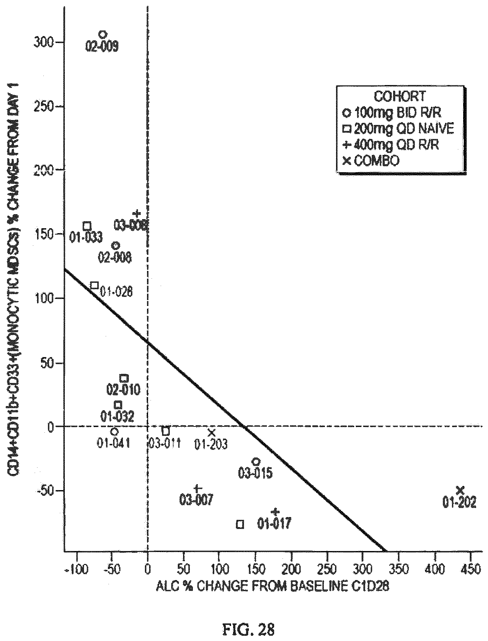

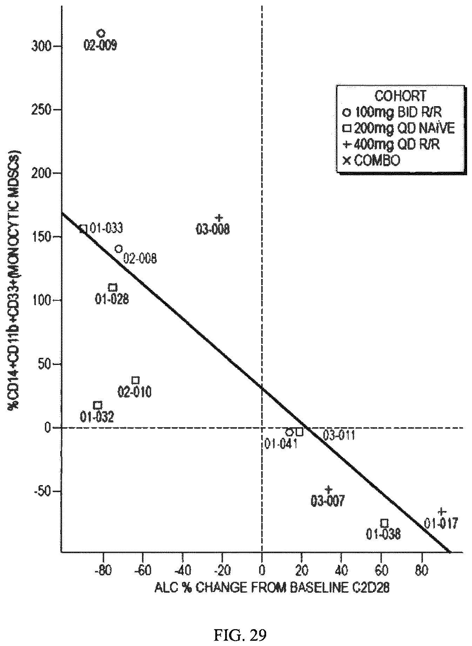

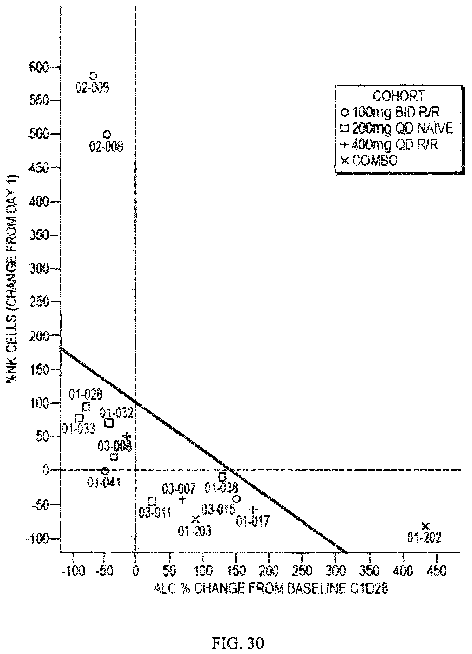

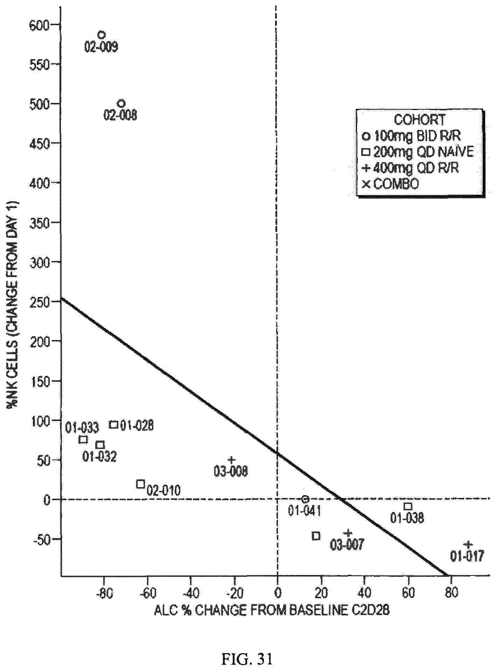

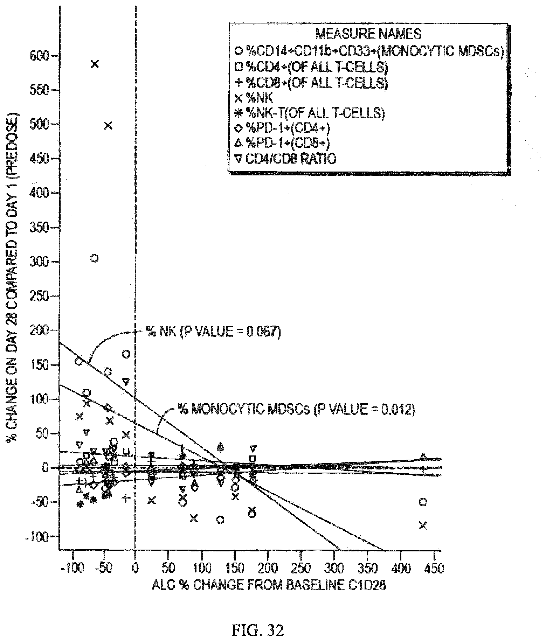

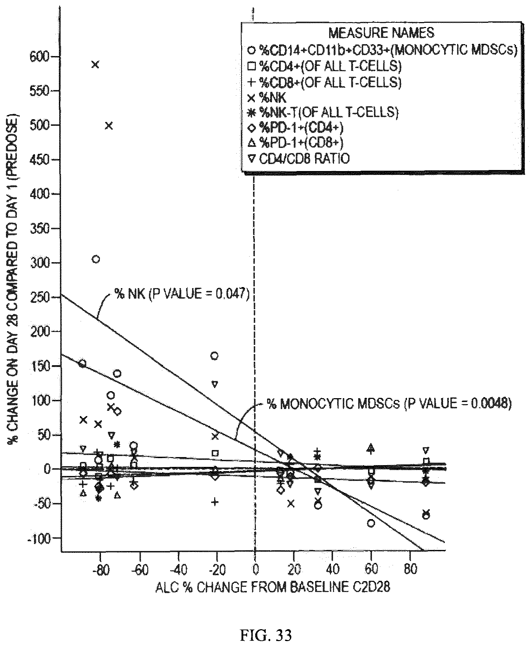

The effects of Formula (2) on cell subset percentages were also evaluated using flow cytometry analysis of peripheral blood, with the results shown in FIG. 28, FIG. 29, FIG. 30, FIG. 31, FIG. 32, and FIG. 33. PBMC samples from CLL patient samples drawn prior to (predose) and after 28 days of dosing with Formula (2) were compared for potential changes in cell subsets. PBMCs were stained with monoclonal antibodies conjugated to fluorescent tags (flourochromes) to identify cell subsets via flow cytometry. Non-viable cells were excluded from the analysis using the dye 7-aminoactinomycin D (7-AAD). To produce the metric of percent change, the following steps were taken. First, each cell subset was defined by hierarchical flow cytometry gating. Then, the change in frequency (between day 1 and day 28) was calculated for each cell subset. MDSC subsets were measured as a % of all myeloid cells. T cell subsets were measured as a % of all CD3⁺ cells, and NK cells were measured as a % of all live CD45⁺ cells. In FIG. 28 and FIG. 29, the results show the % change in MDSC (monocytic) level over 28 days versus % ALC change at cycle 1 day 28 (C1D28) and at cycle 2 day 28 (C2D28). A cycle is 28 days. A trend is observed wherein patients with decreasing ALC % had increasing MDSC (monocytic) %. This may include patients who had quickly resolving lymphocytosis and those with no initial lymphocytosis. This provides evidence that treatment with Formula (2) mobilizes MDSCs and thus affects the CLL tumor microenvironment in marrow and lymph nodes, which is an unexpected indication of superior efficacy. In FIG. 30 and FIG. 31, the results show the % change in NK cell level over 28 days versus % ALC change, measured at C1D28 or C2D28, and similar trends are observed wherein patients with decreasing ALC % had increasing NK cell %. This may include patients who had quickly resolving lymphocytosis and those having no initial lymphocytosis. The effects in FIG. 28 to FIG. 31 are observed in multiple cohorts, at doses including 100 mg BID, 200 mg QD, and 400 mg QD. In FIG. 32 and FIG. 33, the effects on NK cells and MDSC cells are compared to a number of other markers versus % change in ALC at C1D28 and C2D28. These other markers include CD4+ T cells, CD8+ T cells, CD4+/CD8+ T cell ratio, NK-T cells, PD-1+ CD4+ T cells, and PD-1+ CD8+ T cells. The effects on NK cells and MDSC cells are observed to be much more pronounced than on any of these other markers.

These results suggest that after Formula (2) administration, the CLL microenvironment undergoes a change wherein NK cells and monocytic MDSC subsets increase in frequency in the peripheral blood in patients with falling ALC counts, an important clinical parameter in CLL. The NK cell increase may reflect an overall increase in cytolytic activity against B-CLL resulting in the ALC % to drop. The increase in MDSC % in the blood may be due to a movement of these cells out of the lymph nodes, spleen, and bone marrow, which are all possible sites of CLL proliferation. Fewer MDSCs at the CLL proliferation centers would likely result in a reduced immunosuppressive microenvironment leading to an increase in cell-mediated immunity against the tumor, decreased tumor proliferation, and eventually lower ALC% in the circulation.

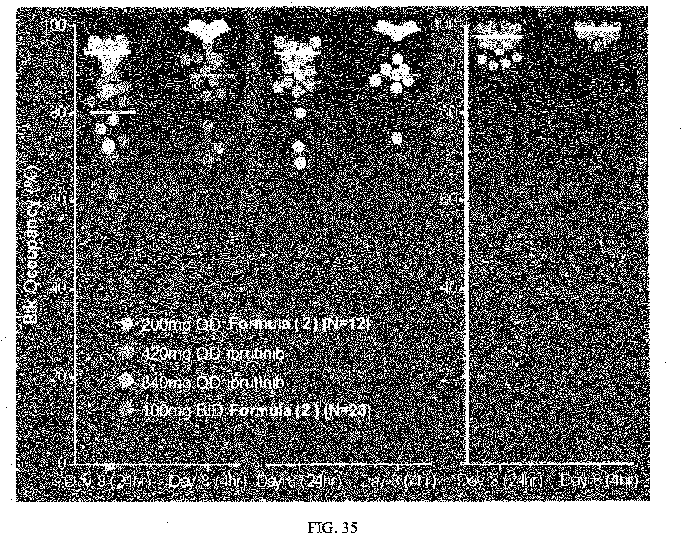

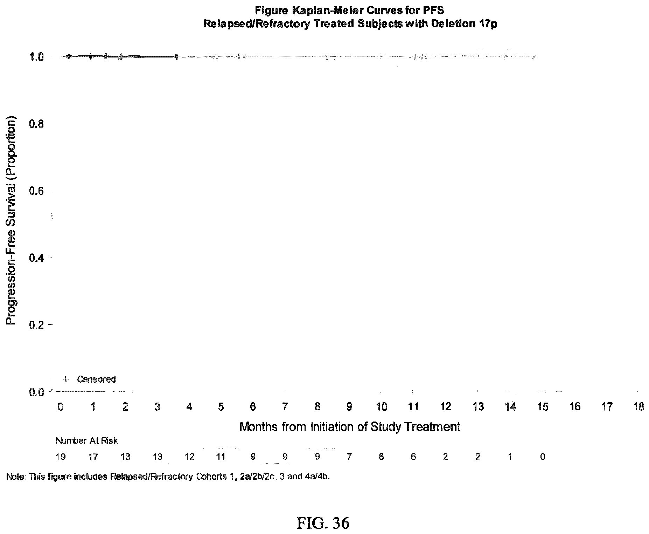

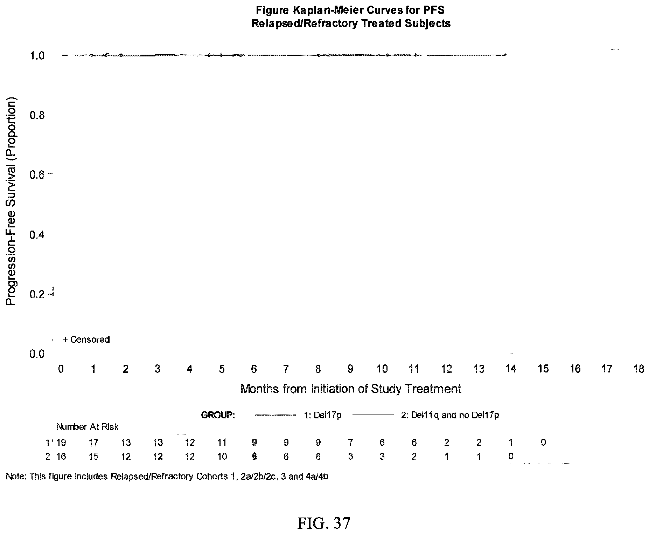

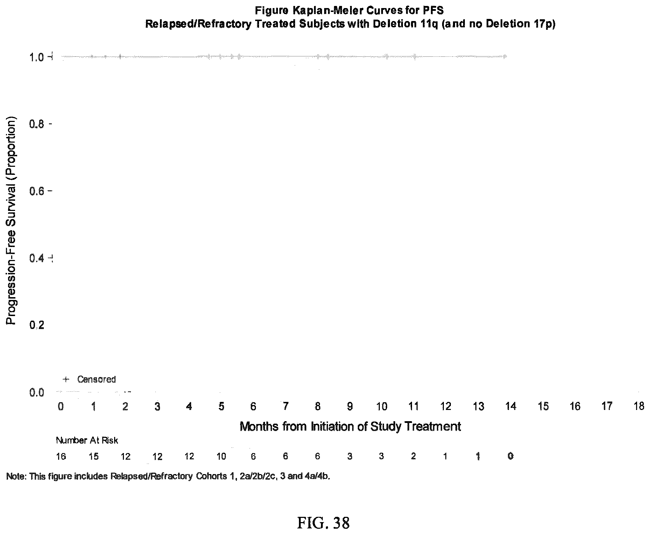

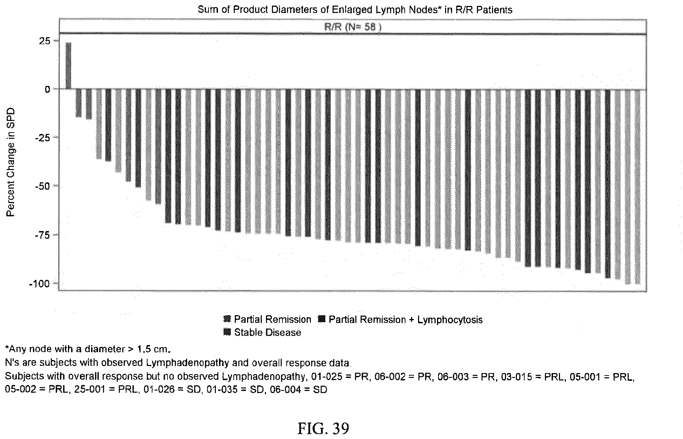

Updated clinical results from the CLL study are shown in FIG. 34 to FIG. 39. FIG. 34 shows an update of the data presented in FIG. 21. FIG. 35 shows an update of the data presented in FIG. 27, and includes BID dosing results. Formula (2) 200 mg QD dosing resulted in 94% - 99% BTK occupancy, 24 hour coverage, and less inter-patient variability. Ibrutinib 420 mg and 840 mg QD dosing resulted in 80% - 90% BTK occupancy, more inter-patient variability, and capped occupancy. Formula (2) 100 mg BID dosing resulted in 97% - 99% BTK occupancy, complete BTK coverage, and less inter-patient variability. The PFS for patients with 17p deletions and 11q deletions are illustrated in FIG. 36, FIG. 37, and FIG. 38. Updated SPD results are illustrated in FIG. 39, and again show significant results across all cohorts and dosing regimens.

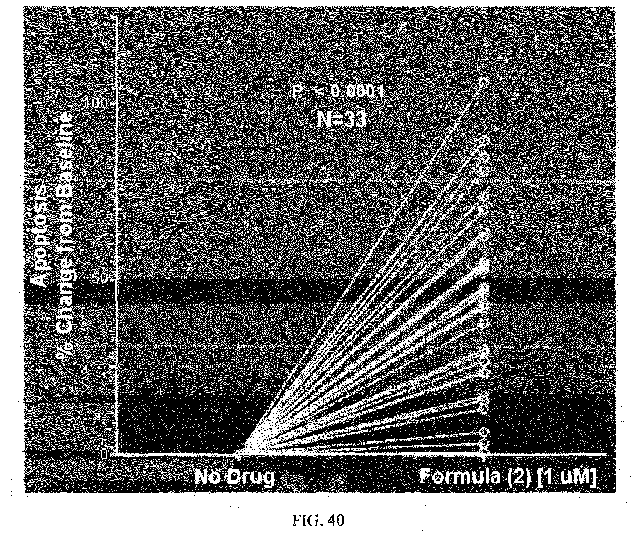

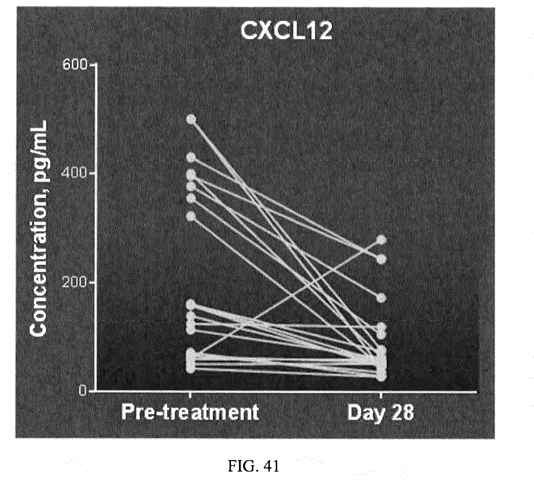

Treatment of CLL patients with Formula (2) also resulted in increased apoptosis, as illustrated in FIG. 40. Apoptotic B-CLL was defined by flow cytometry as having cleaved PARP⁺, Caspase 3⁺, CD19⁺, and CD5⁺ phenotypes. 82% of samples tested had a baseline change greater than 25%. Treatment of CLL patients also showed that Formula (2) decreased plasma chemokines associated with MDSC homing and retention. A significant decrease in CXCL12 levels has been observed in patients treated with Formula (2), as shown in FIG. 41.

### Example 12 - Effects of BTK Inhibitors on Thrombosis

Clinical studies have shown that targeting the BCR signaling pathway by inhibiting BTK produces significant clinical benefit (Byrd, et al., N. Engl. J. Med. 2013, 369(1), 32-42, Wang, et al., N. Engl. J. Med. 2013, 369(6), 507-16). However, in these studies, bleeding has been reported in up to 50% of ibrutinib-treated patients. Most bleeding events were of grade 1-2 (spontaneous bruising or petechiae) but, in 5% of patients, they were of grade 3 or higher after trauma. These results are reflected in the prescribing information for ibrutinib, where bleeding events of any grade, including bruising and petechiae, were reported in about half of patients treated with ibrutinib (INMRUVICA package insert and prescribing information, revised July 2014, U.S. Food and Drug Administration).

Constitutive or aberrant activation of the BCR signaling cascade has been implicated in the propagation and maintenance of a variety of B cell malignancies. Small molecule inhibitors of BTK, a protein early in this cascade and specifically expressed in B cells, have emerged as a new class of targeted agents. There are several BTK inhibitors, including Formula (17) (CC-292), and Formula (10) (ibrutinib), in clinical development. Importantly, early stage clinical trials have found ibrutinib to be particularly active in chronic lymphocytic leukemia (CLL) and mantle cell lymphoma (MCL), suggesting that this class of inhibitors may play a significant role in various types of cancers (Aalipour and Advani, Br. J. Haematol. 2013, 163, 436-43). However, their effects are not limited to leukemia or lymphomas as platelets also rely on the Tec kinases family members BTK and Tec for signal transduction in response to various thrombogenic stimuli (Oda, et al., Blood 2000, 95(5), 1663-70; Atkinson, et al., Blood 2003, 102(10), 3592-99). In fact, both Tec and BTK play an important role in the regulation of phospholipase Cγ2 (PLCy2) downstream of the collagen receptor glycoprotein VI (GPVI) in human platelets. In addition, BTK is activated and undergoes tyrosine phosphorylation upon challenge of the platelet thrombin receptor, which requires the engagement of αIIbβ3 integrin and PI3K activity (Laffargue, et al., FEBSLett. 1999, 443(1), 66-70). It has also been implicated in GPIbα-dependent thrombus stability at sites of vascular injury (Liu, et al., Blood 2006, 108(8), 2596-603). Thus, BTK and Tec are involved in several processes important in supporting the formation of a stable hemostatic plug, which is critical for preventing significant blood loss in response to vascular injury. Hence, the effects of the BTK inhibitors of Formula (2) and Formula (10) (ibrutinib) were evaluated on human platelet-mediated thrombosis by utilizing the *in vivo* human thrombus formation in the VWF HA1 mice model described in Chen, et al., Nat. Biotechnol. 2008, 26(1), 114-19.

Administration of anesthesia, insertion of venous and arterial catheters, fluorescent labeling and administration of human platelets (5 × 10⁸/ml), and surgical preparation of the cremaster muscle in mice have been previously described (Chen et al., Nat Biotechnol. 2008, 26(1), 114-19). Injury to the vessel wall of arterioles (~40-65 mm diameter) was performed using a pulsed nitrogen dye laser (440 nm, Photonic Instruments) applied through a 20× water-immersion Olympus objective (LUMPlanFl, 0.5 numerical aperture (NA)) of a Zeiss Axiotech vario microscope. Human platelet and wall interactions were visualized by fluorescence microscopy using a system equipped with a Yokogawa CSU-22 spinning disk confocal scanner, iXON EM camera, and 488 nm and 561 nm laser lines to detect BCECF-labeled and rhodamine-labeled platelets, respectively (Revolution XD, Andor Technology). The extent of thrombus formation was assessed for 2 minutes after injury and the area (µm²) of coverage determined (Image IQ, Andor Technology). For the Formula (2), Formula (17) (CC-292), and Formula (10) (ibrutinib) inhibition studies, the BTK inhibitors were added to purified human platelets for 30 minutes before administration.

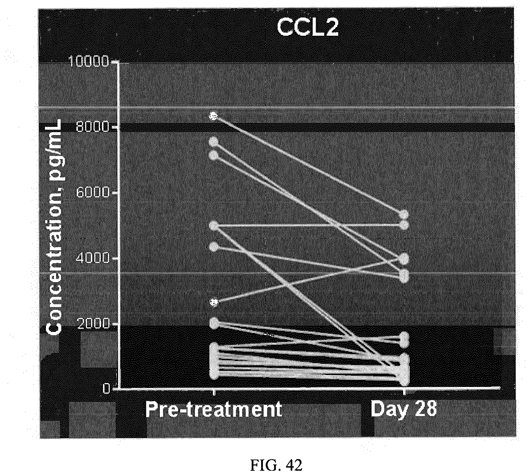

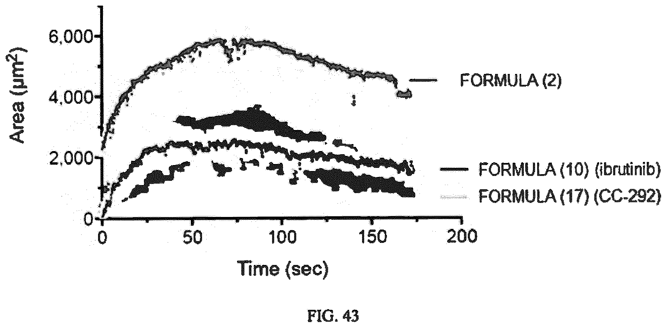

The *in vivo* throbus effects of the BTK inhibitors, Formula (2), Formula (17) (CC-292), and Formula (10) (ibrutinib), were evaluated on human platelet-mediated thrombosis by utilizing the *in vivo* human thrombus formation in the VWF HA1 mice model, which has been previously described (Chen, et al., NatBiotechnol. 2008, 26(1), 114-19). Purified human platelets were preincubated with various concentrations of the BTK inhibitors (0.1 µM, 0.5 µM, or 1 µM) or DMSO and then administered to VWF HA1 mice, followed by laser-induced thrombus formation. The BTK inhibitor-treated human platelets were fluorescently labeled and infused continuously through a catheter inserted into the femoral artery. Their behavior in response to laser-induced vascular injury was monitored in real time using two-channel confocal intravital microscopy (Furie and Furie, J. Clin. Invest. 2005, 115(12), 2255-62). Upon induction of arteriole injury untreated platelets rapidly formed thrombi with an average thrombus size of 6,450 ± 292 mm² (mean ± s.e.m.), as shown in FIG. 42 and FIG. 43. Similarly, Formula (2) (1 µM) treated platelets formed a slightly smaller but not significantly different thrombi with an average thrombus size of 5733 ± 393 mm² (mean ± s.e.m.). In contrast, a dramatic reduction in thrombus size occurred in platelets pretreated with 1 µM of Formula (10) (ibrutinib), 2600 ± 246 mm² (mean ± s.e.m.), resulting in a reduction in maximal thrombus size by about 61% compared with control (P > 0.001) (FIG. 42). Similar results were obtained with platelets pretreated with 500 nM of Formula (2) or ibrutinib: thrombus size of 5946 ± 283 mm², and 2710 ± 325 mm² respectively. These initial results may provide some mechanic background and explanation on the reported 44% bleeding related adverse event rates in the Phase III RESONATE study comparing ibrutinib with ofatumumab. The results obtained for Formula (17) (CC-292) were similar to that for Formula (10) (ibrutinib), as shown in FIG. 42 and 43. These results demonstrate the surprising advantage of the BTK inhibitor of Formula (2), which does not interfere with thrombus formation, while the BTK inhibitors of Formula (17) (CC-292) and Formula (10) (ibrutinib) interfere with thrombus formation.

The objective of this study was to evaluate *in vivo* thrombus formation in the presence of BTK inhibitors. *In vivo* testing of novel antiplatelet agents requires informative biomarkers. By utilizing a genetic modified mouse von Willebrand factor (VWFR1326H) model that supports human but not mouse platelet-mediated thrombosis, we evaluated the effects of Formula (2), Formula (17) (CC-292), and Formula (10) (ibrutinib) on thrombus formation. These results show that Formula (2) had no significant effect on human platelet-mediated thrombus formation while Formula (10) (ibrutinib) was able to limit this process, resulting in a reduction in maximal thrombus size by 61% compared with control. Formula (17) (CC-292) showed an effect similar to Formula (10) (ibrutinib). These results, which show reduced thrombus formation for ibrutinib at physiologically relevant concentrations, may provide some mechanistic background for the Grade ≥ 3 bleeding events (*e.g*., subdural hematoma, gastrointestinal bleeding, hematuria and postprocedural hemorrhage) that have been reported in ≤ 6% of patients treated with Formula (10) (ibrutinib).

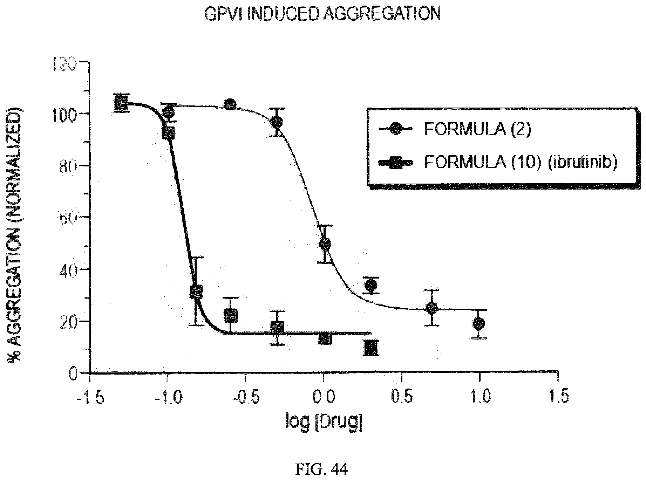

GPVI platelet aggregation was measured for Formula (2) and Formula (10) (ibrutinib). Blood was obtained from untreated humans, and platelets were purified from plasma-rich protein by centrifugation. Cells were resuspended to a final concentration of 350,000/µL in buffer containing 145 mmol/L NaCl, 10 mmol/L HEPES, 0.5 mmol/L Na₂HPO₄, 5 mmol/L KCl, 2 mmol/L MgCl₂, 1 mmol/L CaCl₂, and 0.1% glucose, at pH 7.4. Stock solutions of Convulxin (CVX) GPVI were prepared on the day of experimentation and added to platelet suspensions 5 minutes (37 °C, 1200 rpm) before the induction of aggregation. Aggregation was assessed with a Chronolog Lumi-Aggregometer (model 540 VS; Chronolog, Havertown, PA) and permitted to proceed for 6 minutes after the addition of agonist. The results are reported as maximum percent change in light transmittance from baseline with platelet buffer used as a reference. The results are shown in FIG. 44.

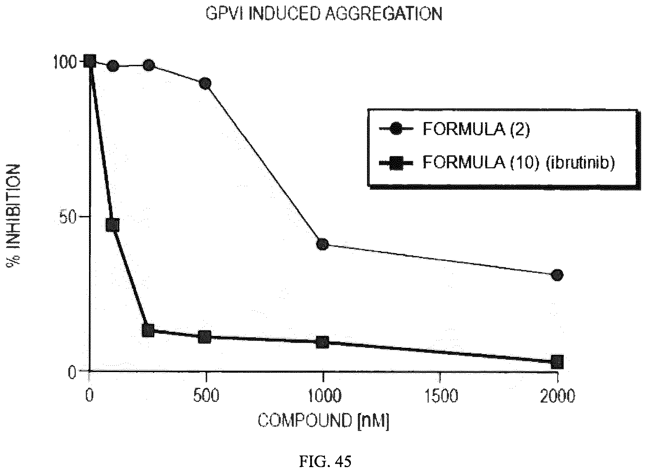

In FIG. 45, the results of CVX-induced (250 ng/mL) human platelet aggregation results before and 15 minutes after administration of the BTK inhibitors to 6 healthy individuals are shown.

The results depicted in FIG. 44 and FIG. 45 indicate that the BTK inhibitor of Formula (10) (ibrutinib) significantly inhibits GPVI platelet aggregation, while the BTK inhibitor of Formula (2) does not, further illustrating the surprising benefits of the latter compound.

### Example 13 - Synergistic Combinations of BTK Inhibitors and CD19 Inhibitors

Combination experiments may be performed to assess the synergistic behavior of drug combinations of BTK inhibitors and CD19 inhibitors. Syngeneic animal models for B cell lymphomas, including murine models, may be used to assess this behavior using intravenous, intrasplenic, intraperitoneal, subcutaneous, intramuscular, or intracerebral induction.

A mouse xenograft model may also be used to assess the synergistic behavior of drug combinations of BTK inhibitors and CD19 inhibitors. In this study, 6 to 10-week-old NOD-SCID-yc-/- (NSG) mice are obtained from the Jackson Laboratory (Bar Harbor, ME) and the CD19+ human ALL line Nalm-6 is obtained from American Type Culture Collection (ATCC; Manassas, VA). Animals are injected via the tail vein with 10⁶ viable Nalm-6 cells in 0.2 mL of sterile phosphate buffered saline (PBS). Starting at 3 to 5 weeks after injection, mice are screened for engraftment every 3 weeks by retro-orbital sampling of peripheral blood. Engraftment is detected by flow cytometric analysis using antibodies recognizing CD19 and CD45. In addition, mice are screened using anti-CD3 and anti-CD33 to ensure engraftment was of the lymphoblast population and not of a T-cell or myeloid lineage. Xenografted mice were randomized to treatment with (i) the BTK inhibitor of Formula (2), (ii) a murine anti-CD19 monoclonal antibody (BioXcell, Clone 1D3), (iii) a combination of the BTK inhibitor of Formula (2) and the same murine anti-CD19 monoclonal antibody, or (iv) vehicle, after establishment of disease, defined as more than 5% blasts detected in peripheral blood. Response to treatment was measured weekly by retro-orbital bleeds to determine white blood counts and percent blasts by flow cytometry.

### Example 14 - In Vivo Study of CD19 CAR-T Cells and BTK Synergistic Combinations

To evaluate the effect of inhibiting BTK in tumors treated with CD19 CAR-T cells, eight-week-old SCID-Beige mice (Charles River) mice are injected by tail vein on day 1 with 1 × 10⁶ green fluorescent protein-firefly luciferase (GFP-FFLuc) transduced NALM-6 tumor cells. Seven days after tumor engraftment, mice are injected intravenously with 1 × 10⁷ CAR-T cells (day 7 and day 14). Tumor bioluminescence is monitored using a Xenogen *in vivo* Imaging System (IVIS; Caliper Life Sciences, Hopkinton, MA) for acquisition of imaging data sets. To evaluate a BTK inhibitor effect, GFP-FFLuc transduced NALM-6 cells are injected *via* the tail vein, and mice are treated with (A) oral Formula (2) at a dose of 15 mg/kg BID, (B) intravenous CAR-T cells as described above, or (C) a combination of Formula (2) at a dose of 15 mg/kg BID and intravenous CAR-T cells as described above. The CAR-T cells may use a variety of signaling domains, including CD28, 4-1BB, and CD3ζ domains, different transmembrane domains, and different CD19 scFv domains to evaluate the optimal combination.

## Claims

1. A pharmaceutical combination for use in the treatment of cancer, comprising therapeutically effective amounts of (1) a CD19 inhibitor selected from an anti-CD19 antibody or an anti-CD19 chimeric antigen receptor, or an antigen-binding fragment or conjugate thereof, and (2) a Bruton's tyrosine kinase (BTK) inhibitor, wherein the BTK inhibitor is the compound of Formula (II): 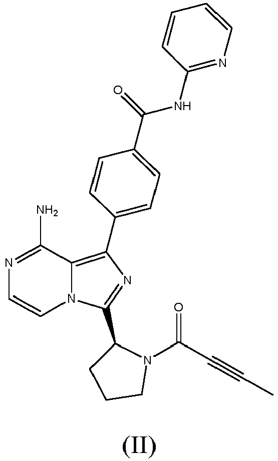 or a pharmaceutically acceptable salt thereof.

2. The combination for use according to Claim 1, wherein the CD19 inhibitor is selected from the group consisting of blinatumomab, coltuximab ravtansine, denintuzumab mafodotin, and antigen-binding fragments, conjugates, variants, and biosimilars thereof.

3. The combination for use according to Claim 1, wherein the CD19 inhibitor is a T cell comprising an anti-CD19 chimeric antigen receptor.

4. The combination for use according to Claim 1, wherein the CD19 inhibitor is an NK cell comprising an anti-CD19 chimeric antigen receptor.

5. The combination for use according to Claim 3 or 4, wherein the anti-CD 19 chimeric antigen receptor comprises intracellular signaling domain, a transmembrane domain, and an anti-CD 19 extracellular domain, wherein the anti-CD 19 extracellular domain is selected from the group consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, and conservative amino acid substitutions thereof.

6. The combination for use according to Claim 5, wherein the intracellular signaling domain comprises a CD3ζ signaling domain and a costimulatory domain selected from the group consisting of a 4-1BB signaling domain, a CD28 signaling domain, and combinations thereof.

7. The combination for use according to Claim 5, wherein the intracellular signaling domain is selected from the group consisting of SEQ ID NO:40, SEQ ID NO:41, SEQ ID NO:42, conservative amino acid substitutions thereof, and combinations thereof.

8. The combination for use according to Claim 3 or 4, wherein the anti-CD 19 chimeric antigen receptor is selected from the group consisting of SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, and conservative amino acid substitutions thereof.

9. The combination for use according to any one of the preceding Claims, further comprising a therapeutically effective amount of an anti-CD20 antibody.

10. The combination for use according to Claim 9, wherein the anti-CD20 antibody is selected from the group consisting of rituximab, obinutuzumab, ofatumumab, veltuzumab, tositumomab, ibritumomab, and fragments, conjugates, variants, radioisotope-labeled complexes, biosimilars thereof, and combinations thereof.

11. The combination for use according to any one of the preceding Claims, further comprising a therapeutically effective amount of a chemotherapeutic regimen selected from the group consisting of (1) fludarabine, cyclophosphamide, and rituximab (FCR); (2) rituximab, cyclophosphamide, doxorubicin, vincristine, and prednisone (R-CHOP); and (3) bendamustine and rituximab (BR).

12. The combination for use according to any one of the preceding Claims, wherein the cancer is a B cell hematological malignancy.

13. The combination for use according to Claim 12, wherein the B cell hematological malignancy is selected from the group consisting of chronic lymphocytic leukemia (CLL), small lymphocytic leukemia (SLL), non-Hodgkin's lymphoma (NHL), diffuse large B cell lymphoma (DLBCL), follicular lymphoma (FL), mantle cell lymphoma (MCL), Hodgkin's lymphoma, B cell acute lymphoblastic leukemia (B-ALL), Burkitt's lymphoma, Waldenström's macroglobulinemia (WM), Burkitt's lymphoma, multiple myeloma, and myelofibrosis.

14. The combination for use according to any one of the preceding Claims, wherein the cancer is a solid tumor cancer.

15. The combination for use according to Claim 14, wherein the solid tumor cancer is selected from the group consisting of bladder cancer, non-small cell lung cancer, cervical cancer, anal cancer, pancreatic cancer, squamous cell carcinoma including head and neck cancer, renal cell carcinoma, melanoma, ovarian cancer, small cell lung cancer, glioblastoma, gastrointestinal stromal tumor, breast cancer, lung cancer, colorectal cancer, thyroid cancer, bone sarcoma, stomach cancer, oral cavity cancer, oropharyngeal cancer, gastric cancer, kidney cancer, liver cancer, prostate cancer, esophageal cancer, testicular cancer, gynecological cancer, colon cancer, and brain cancer.

## Patentansprüche

1. Pharmazeutische Kombination zur Verwendung bei der Behandlung von Krebs, umfassend therapeutisch wirksame Mengen (1) eines CD19-Inhibitors, der aus einem Anti-CD19-Antikörper oder einem chimären Anti-CD19-Antigenrezeptor oder einem antigenbindenden Fragment oder Konjugat davon ausgewählt ist, und (2) eines BTK(Bruton's Tyrosine Kinase)-Inhibitors, wobei es sich bei dem BTK-Inhibitor um die Verbindung der Formel (II): 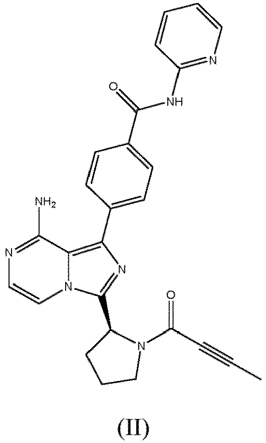 oder ein pharmazeutisch unbedenkliches Salz davon handelt.

2. Kombination zur Verwendung nach Anspruch 1, wobei der CD-19-Inhibitor aus der Gruppe bestehend aus Blinatumomab, Coltuximab Ravtansin, Denintuzumab Mafodotin sowie antigenbindenden Fragmenten, Konjugaten, Varianten und Bioanalogen davon ausgewählt ist.

3. Kombination zur Verwendung nach Anspruch 1, wobei es sich bei dem CD-19-Inhibitor um eine einen chimären Anti-CD19-Antigenrezeptor umfassende T-Zelle handelt.

4. Kombination zur Verwendung nach Anspruch 1, wobei es sich bei dem CD-19-Inhibitor um eine einen chimären Anti-CD19-Antigenrezeptor umfassende NK-Zelle handelt.

5. Kombination zur Verwendung nach Anspruch 3 oder 4, wobei der chimäre Anti-CD19-Antigenrezeptor eine intrazelluläre Signalgebungsdomäne, eine Transmembrandomäne und eine extrazelluläre Anti-CD19-Domäne umfasst, wobei die extrazelluläre Anti-CD19-Domäne aus der Gruppe bestehend aus SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39 und konservativen Aminosäuresubstitutionen davon ausgewählt ist.

6. Kombination zur Verwendung nach Anspruch 5, wobei die intrazelluläre Signalgebungsdomäne eine CD3ζ-Signalgebungsdomäne und eine aus der Gruppe bestehend aus einer 4-1BB-Signalgebungsdomäne, einer CD28-Signalgebungsdomäne und Kombinationen davon ausgewählte Costimulatordomäne umfasst.

7. Kombination zur Verwendung nach Anspruch 5, wobei die intrazelluläre Signalgebungsdomäne aus der Gruppe bestehend aus SEQ ID NO:40, SEQ ID NO:41, SEQ ID NO:42, konservativen Aminosäuresubstitutionen davon und Kombinationen davon ausgewählt ist.

8. Kombination zur Verwendung nach Anspruch 3 oder 4, wobei der chimäre Anti-CD19-Antigenrezeptor aus der Gruppe bestehend aus SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59 und konservativen Aminosäuresubstitutionen davon ausgewählt ist.

9. Kombination zur Verwendung nach einem der vorhergehenden Ansprüche, ferner umfassend eine therapeutisch wirksame Menge eines Anti-CD20-Antikörpers.

10. Kombination zur Verwendung nach Anspruch 9, wobei der Anti-CD20-Antikörper aus der Gruppe bestehend aus Rituximab, Obinutuzumab, Ofatumumab, Veltuzumab, Tositumomab, Ibritumomab sowie Fragmenten, Konjugaten, Varianten, radioisotopenmarkierten Komplexen, Bioanalogen davon und Kombinationen davon ausgewählt ist.

11. Kombination zur Verwendung nach einem der vorhergehenden Ansprüche, ferner umfassend eine therapeutisch wirksame Menge eines Chemotherapieplans, der aus der Gruppe bestehend aus (1) Fludarabin, Cyclophosphamid und Rituximab (FCR); (2) Rituximab, Cyclophosphamid, Doxorubicin, Vincristin und Prednison (R-CHOP); und (3) Bendamustin und Rituximab (BR) ausgewählt ist.

12. Kombination zur Verwendung nach einem der vorhergehenden Ansprüche, wobei es sich bei dem Krebs um ein hämatologisches B-Zell-Malignom handelt.

13. Kombination zur Verwendung nach Anspruch 12, wobei das hämatologische B-Zell-Malignom aus der Gruppe bestehend aus chronischer lymphatischer Leukämie (CLL), kleinzelligem lymphozytischem Lymphom (SLL), Non-Hodgkin-Lymphom (NHL), diffusem großzelligem B-Zell-Lymphom (DLBCL), follikulärem Lymphom (FL), Mantelzell-Lymphom (MCL), Hodgkin-Lymphom, akuter lymphoblastischer B-Zell-Leukämie (B-ALL), Burkitt-Lymphom, Morbus Waldenström (WM), Burkitt-Lymphom, multiplem Myelom und Myelofibrose ausgewählt ist.

14. Kombination zur Verwendung nach einem der vorhergehenden Ansprüche, wobei es sich bei dem Krebs um eine Krebserkrankung mit solidem Tumor handelt.

15. Kombination zur Verwendung nach Anspruch 14, wobei die Krebserkrankung mit solidem Tumor aus der Gruppe bestehend aus Blasenkrebs, nichtkleinzelligem Lungenkrebs, Gebärmutterhalskrebs, Analkrebs, Bauchspeicheldrüsenkrebs, Plattenepithelkarzinom, einschließlich Krebs an Kopf und Hals, Nierenzellkarzinom, Melanom, Ovarialkarzinom, kleinzelligem Lungenkrebs, Glioblastom, Stromatumor des Verdauungstrakts, Brustkrebs, Lungenkrebs, Kolorektalkarzinom, Schilddrüsenkrebs, Knochensarkom, Magenkrebs, Mundhöhlenkrebs, Krebs des Mund- und Rachenraums, Magenkarzinom, Nierenkrebs, Leberkrebs, Prostatakrebs, Speiseröhrenkrebs, Hodenkrebs, gynäkologischen Krebserkrankungen, Darmkrebs und Hirntumoren ausgewählt ist.

## Revendications

1. Combinaison pharmaceutique pour une utilisation dans le traitement d'un cancer, comprenant des quantités thérapeutiquement efficaces de (1) un inhibiteur de CD19 choisi parmi un anticorps anti-CD19 ou un récepteur antigénique chimérique anti-CD19, ou un fragment ou conjugué de liaison à un antigène correspondant, et (2) un inhibiteur de tyrosine kinase de Bruton (BTK), l'inhibiteur de BTK étant le composé de formule (II) : 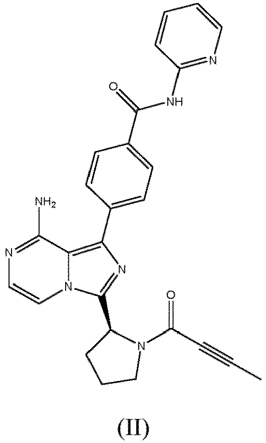 ou un sel pharmaceutiquement acceptable correspondant.

2. Combinaison pour une utilisation selon la revendication 1, l'inhibiteur de CD 19 étant choisi dans le groupe constitué par blinatumomab, coltuximab ravtansine, dénintutuzumab mafodotin et des fragments de liaison à un antigène, des conjugués, des variants et des biosimilaires correspondants.

3. Combinaison pour une utilisation selon la revendication 1, l'inhibiteur de CD 19 étant une cellule T comprenant un récepteur antigénique chimérique anti-CD19.

4. Combinaison pour une utilisation selon la revendication 1, l'inhibiteur de CD 19 étant une cellule NK comprenant un récepteur antigénique chimérique anti-CD19.

5. Combinaison pour utilisation selon la revendication 3 ou 4, le récepteur antigénique chimérique anti-CD19 comprenant un domaine de signalisation intracellulaire, un domaine transmembranaire, et un domaine extracellulaire anti-CD19, le domaine extracellulaire anti-CD19 étant choisi dans le groupe constitué par SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39 et des substitutions conservatrices d'acides aminés.

6. Combinaison pour une utilisation selon la revendication 5, le domaine de signalisation intracellulaire comprenant un domaine de signalisation CD3ζ et un domaine de co-stimulation choisi dans le groupe constitué par un domaine de signalisation 4-1BB, un domaine de signalisation CD28 et des combinaisons correspondantes.

7. Combinaison pour une utilisation selon la revendication 5, le domaine de signalisation intracellulaire étant choisi dans le groupe constitué par SEQ ID NO: 40, SEQ ID NO: 41, SEQ ID NO: 42, des substitutions conservatrices d'acides aminés et des combinaisons correspondantes.

8. Combinaison pour une utilisation selon la revendication 3 ou 4, le récepteur antigénique chimérique anti-CD19 étant choisi dans le groupe constitué par SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, et des substitutions conservatrices d'acides aminés.

9. Combinaison pour une utilisation selon l'une quelconque des revendications précédentes, comprenant en outre une quantité thérapeutiquement efficace d'anticorps anti-CD20.

10. Combinaison pour une utilisation selon la revendication 9, l'anticorps anti-CD20 étant choisi dans le groupe constitué par rituximab, obinutuzumab, ofatumumab, veltuzumab, tositumab, ibritumomab et des fragments, des conjugués, des variants, des complexes marqués par un radioisotope, des biosimilaires correspondants et des combinaisons correspondantes.

11. Combinaison pour une utilisation selon l'une quelconque des revendications précédentes, comprenant en outre une quantité thérapeutiquement efficace d'un régime chimiothérapeutique choisi dans le groupe constitué par (1) fludarabine, cyclophosphamide et rituximab (FCR) ; (2) rituximab, cyclophosphamide, doxorubicine, vincristine et prednisone (R-CHOP) ; et (3) bendamustine et rituximab (BR).

12. Combinaison pour une utilisation selon l'une quelconque des revendications précédentes, le cancer étant une malignité hématologique à cellules B.

13. Combinaison selon la revendication 12, la tumeur hématologique à cellules B étant choisie dans le groupe constitué par une leucémie lymphoïde chronique (LLC), une leucémie lymphocytaire à petites cellules (LLPC), un lymphome non hodgkinien (LNH), un lymphome diffus à grandes cellules B (LDGCB), un lymphome folliculaire (LF), un lymphome à cellules du manteau (LCM), un lymphome de Hodgkin, une leucémie aiguë lymphoblastique à cellules B (LAL B), un lymphome de Burkitt, une macroglobulinémie de Waldenstrom (WM), un lymphome de Burkitt, un myélome multiple et une myélofibrose.

14. Combinaison pour une utilisation selon l'une quelconque des revendications précédentes, le cancer étant un cancer à tumeur solide.

15. Combinaison selon la revendication 14, le cancer de la tumeur solide étant choisi dans le groupe constitué par le cancer de la vessie, le cancer du poumon non à petites cellules, le cancer du col de l'utérus, le cancer anal, le cancer pancréatique, un carcinome à cellules squameuses y compris un cancer de la tête et du cou, un carcinome de cellules rénales, un mélanome, le cancer de l'ovaire, le cancer du poumon à petites cellules, un glioblastome, une tumeur stromale gastro-intestinale, le cancer du sein, le cancer du poumon, le cancer colorectal, le cancer de la thyroïde, un sarcome des os, le cancer de l'estomac, le cancer de la cavité buccale, le cancer de l'oropharynx, le cancer gastrique, le cancer du rein, le cancer du foie, le cancer de la prostate, le cancer de l'œsophage, le cancer testiculaire, le cancer gynécologique, le cancer du côlon et le cancer du cerveau.
